# EUROPEAN PATENT APPLICATION

(11) **EP 3 978 606 A1**
(43) Date of publication of application: **06.04.2022**
(21) Application number: 20199855.6
(22) Date of filing: 02.10.2020
(51) Int. Cl.: C12N 9/88, C12N 1/20

(54) **MESOPHILIC, METHYLOTROPHIC BACTERIA FOR THE PH-INDEPENDENT PRODUCTION OF BIOCHEMICALS**

(71) Applicant: Acies Bio d.o.o., 1000 Ljubljana (SI)
(72) Inventor: Kruis, Aleksander Johannes, 4260 Bled (SI); Virant, David, 1233 Dob (SI); Svagelj, Mirjan, 9000 Murska Sobota (SI); Kosec, Gregor, 1000 Ljubljana (SI); Fujs, Stefan, 1000 Ljubljana (SI)
(74) Representative: Zacco GmbH

(57) **Abstract**

The present invention generally relates to the biotechnology engineering, and specifically to genetically modified mesophilic, methylotrophic bacteria, which allow for the production of biochemicals in a pH-independent manner. More specifically, the present invention provides a mesophilic, methylotrophic bacterium which expresses a polypeptide which has glutamate decarboxylase (GAD) activity and is catalytically active at a pH above 5.5. The present invention further provides a mesophilic, methylotrophic bacterium which expresses a) a polypeptide having α-ketoglutarate decarboxylase (KDC) activity and b) a polypeptide having GABA transaminase (GABA-TA) activity. The present invention also provides methods for the pH-independent production of biochemicals, such as γ-amino butyric acid (GABA) or related compounds, using a bacterium of the invention.

SEQUENCE LISTING

<110>  Acies Bio d.o.o.

<120>  Mesophilic, methylotrophic bacteria for the pH-independent
       production of biochemicals

<130>  P49905EP00

<160>  92

<170>  PatentIn version 3.5

<210>  1
<211>  466
<212>  PRT
<213>  Escherichia coli

<400>  1

Met Asp Lys Lys Gln Val Thr Asp Leu Arg Ser Glu Leu Leu Asp Ser
1               5                   10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
        180                 185                 190

Arg Met Ile Glu Ala Cys Asp Glu Asn Thr Ile Gly Val Val Pro Thr
    195                 200                 205

Phe Gly Val Thr Tyr Thr Gly Asn Tyr Glu Phe Pro Gln Pro Leu His
210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

<210>  2
<211>  457
<212>  PRT
<213>  Sulfobacillus thermosulfidooxidans

<400>  2

Met Lys Lys Ala Ser Glu Glu Val Ala Ile Asn Pro Leu Phe Ala Arg
1               5                   10                  15

Arg Gly Glu Glu Ser Val Pro Arg Tyr Arg Leu Ser Glu Glu Gly Leu
            20                  25                  30

Leu Pro Glu Thr Ala Tyr Gln Ile Val His Asp Glu Leu Thr Leu Asp
        35                  40                  45

Gly Ser Ala Arg Leu Asn Leu Ala Thr Phe Val Gly Thr Trp Met Glu
    50                  55                  60

Pro Glu Ala Gln Lys Leu Tyr Val Glu Ala Ala Asn Lys Asn Ile Ile
65                  70                  75                  80

Asp Lys Asp Glu Tyr Pro Glu Thr Ala Ala Ile Glu Glu Arg Cys Leu
                85                  90                  95

Lys Ile Leu Ala Asn Leu Trp Asn Ala Pro Asp Pro Ala Asn Ser Ile
            100                 105                 110

Gly Val Ser Thr Thr Gly Ser Ser Glu Ala Cys Met Leu Gly Gly Leu
        115                 120                 125

Ala Leu Lys Arg Arg Trp Gln Leu Ser Arg Arg Gln Gln Gly Lys Ser
    130                 135                 140

Thr Asp His Pro Asn Ile Val Phe Ser Ser Ala Val Gln Val Val Trp
145                 150                 155                 160

Glu Lys Phe Ala Asn Tyr Phe Glu Val Glu Pro Arg Tyr Val Asn Ile
                165                 170                 175

Thr Pro Glu His Pro Tyr Leu Thr Pro Glu Gly Val Ile Lys Ala Val
        180                 185                 190

Asp Glu Asn Thr Ile Gly Val Val Pro Ile Leu Gly Val Thr Tyr Thr
    195                 200                 205

Gly Ser Tyr Glu Pro Val Glu Ala Ile Ala Gln Ala Leu Asp Asp Leu
    210                 215                 220

Glu Lys Arg Thr Gly Leu Ser Ile Pro Ile Val Asp Ala Ala Ser Gly
225                 230                 235                 240

Gly Phe Val Ala Pro Phe Leu Gln Pro Asp Leu Val Trp Asp Phe Arg
                245                 250                 255

Leu Pro Arg Val His Ser Ile Asn Thr Ser Gly His Lys Tyr Gly Leu
            260                 265                 270

Val Tyr Pro Gly Leu Gly Trp Val Leu Trp Arg Asp Lys Thr Leu Leu
        275                 280                 285

Pro Glu Glu Leu Ile Phe Asn Val Ser Tyr Leu Gly Gly Ser Met Pro
    290                 295                 300

Thr Phe Gly Leu Asn Phe Ser Arg Pro Gly Ala Gln Val Leu Leu Gln
305                 310                 315                 320

Tyr Tyr Asn Phe Leu Arg Leu Gly Arg Gln Gly Tyr Tyr Thr Val Gln
                325                 330                 335

Lys Thr Cys Gln Asn Val Ala His Phe Leu Ala Gln Glu Ile Gly Ala
            340                 345                 350

Met Glu Pro Phe Glu Leu Val Thr Asp Gly Ser Asp Leu Pro Val Phe
        355                 360                 365

Ala Trp Arg Lys Arg Pro Glu Glu Asn Pro Leu Trp Ser Leu Glu Asp
    370                 375                 380

Leu Ser Phe Val Leu Arg Glu Arg Gly Trp Gln Val Pro Ala Tyr Pro
385                 390                 395                 400

Leu Pro Pro Thr Met Gln Asp Val Thr Ile Met Arg Ile Val Val Arg
                405                 410                 415

Asn Ser Phe Ser Met Asp Met Ala Gln Leu Phe Leu Asp Asp Leu Asn

420                 425                 430

Arg Ala Val Ala Tyr Leu Gln Gly Leu Asp His Pro Phe Pro Gln Ala
        435                 440                 445

Leu Arg Gln His Lys Pro Phe Ala His
    450                 455

<210>  3
<211>  467
<212>  PRT
<213>  Bacillus megaterium

<400>  3

Met Pro Gln Trp His Pro His Arg Glu Gln Lys Asn Leu Pro Asp Glu
1               5                   10                  15

Phe Pro Val Asn Pro Leu Phe Ser Arg Gln Gly Glu Val Thr Ile Pro
            20                  25                  30

Arg Leu Arg Ile Ser Asp Gln Gly Met Leu Pro Glu Thr Ala Tyr Gln
        35                  40                  45

Ile Ile His Asp Glu Ile Ala Leu Asp Gly Asn Ala Arg Leu Asn Leu
    50                  55                  60

Ala Thr Phe Val Thr Thr Trp Met Glu Pro Asp Ala Lys Arg Leu Tyr
65                  70                  75                  80

Gly Glu Ser Phe Asp Lys Asn Met Ile Asp Lys Asp Glu Tyr Pro Gln
                85                  90                  95

Thr Ala Ala Ile Glu Glu Arg Cys Val Arg Ile Leu Ala Asp Leu Trp
            100                 105                 110

Asn Ser Pro Asn Pro Asp Thr Thr Met Gly Val Ser Thr Thr Gly Ser
        115                 120                 125

Ser Glu Ala Cys Met Leu Gly Gly Leu Ala Leu Lys Arg Arg Trp Gln
    130                 135                 140

Lys Leu Arg Lys Ser Lys Gly Leu Ser Thr Asp Arg Pro Asn Ile Val
145                 150                 155                 160

Phe Ser Ser Ser Val Gln Val Val Trp Glu Lys Phe Ala Asn Tyr Trp
                165                 170                 175

Asp Val Glu Pro Arg Tyr Val Asn Ile Asn Pro Asp His Pro Tyr Leu

            180                 185                 190

Asp Ala Glu Gly Val Ile Asn Ala Val Asp Glu Asn Thr Ile Gly Val
        195                 200                 205

Val Pro Ile Leu Gly Val Thr Tyr Thr Gly Val Tyr Glu Pro Ile Ala
    210                 215                 220

Ala Ile Ala Lys Ala Leu Asp Glu Leu Gln Glu Lys Thr Gly Leu Asp
225                 230                 235                 240

Ile Pro Ile His Val Asp Ala Ala Ser Gly Gly Phe Ile Ala Pro Phe
                245                 250                 255

Leu Gln Pro Asp Leu Ile Trp Asp Phe Arg Leu Pro Arg Val Lys Ser
            260                 265                 270

Ile Asn Val Ser Gly His Lys Tyr Gly Leu Val Tyr Pro Gly Leu Gly
        275                 280                 285

Trp Val Ile Trp Arg Glu Lys Glu Asp Leu Pro Glu Asp Leu Ile Phe
    290                 295                 300

Arg Val Ser Tyr Leu Gly Gly Asn Met Pro Thr Phe Ala Leu Asn Phe
305                 310                 315                 320

Ser Arg Pro Gly Ala Gln Val Leu Leu Gln Tyr Tyr Asn Phe Leu Arg
                325                 330                 335

Leu Gly Lys Asp Gly Tyr Tyr Ala Val Gln Lys Thr Ser Gln Glu Asn
            340                 345                 350

Ala Leu Phe Leu Ser Lys Glu Ile Gly Glu Met Glu Ala Phe Glu Ile
        355                 360                 365

Leu Ala Asp Gly Ser Asp Ile Pro Val Leu Ala Trp Lys Leu Lys Glu
    370                 375                 380

Gly Tyr Thr Pro Asn Trp Thr Leu Tyr Asp Leu Ser Arg Gln Leu Arg
385                 390                 395                 400

Thr Tyr Gly Trp Gln Val Pro Ala Tyr Pro Leu Pro Ala Asp Met Glu
                405                 410                 415

Glu Ile Thr Ile Met Arg Ile Val Val Arg Asn Gly Phe Ser Arg Asp
            420                 425                 430

Leu Ala His Leu Phe Met Val Asn Phe Lys Gln Ala Val Glu Phe Leu
        435                 440                 445

Asn Ser Leu Asp Arg Pro Val Leu Lys Asp Thr Lys Tyr Asp Asn Gly
    450                 455                 460

Phe His His
465

<210>  4
<211>  467
<212>  PRT
<213>  Listeria innocua

<400>  4

Met Leu Tyr Ser Glu Asn Asp Lys Arg Lys His Glu Ser Tyr Arg Ile
1               5                   10                  15

Pro Leu Phe Gly Ser Glu Glu Glu Ser Thr Ser Ile Pro Lys Tyr Val
            20                  25                  30

Leu Lys Lys Glu Pro Met Glu Pro Arg Ile Ala Tyr Gln Leu Val Lys
        35                  40                  45

Asp Gln Leu Met Asp Glu Gly Asn Ala Arg Gln Asn Leu Ala Thr Phe
    50                  55                  60

Cys Gln Thr Tyr Met Glu Lys Glu Ala Glu Ile Leu Met Ala Glu Thr
65                  70                  75                  80

Leu Glu Lys Asn Ala Ile Asp Lys Ser Glu Tyr Pro Gln Thr Ala Glu
                85                  90                  95

Leu Glu Asn Arg Cys Val Asn Ile Leu Ala Asp Leu Trp Asn Ala Pro
            100                 105                 110

Lys Glu Met Ser Tyr Leu Gly Thr Ser Thr Val Gly Ser Ser Glu Ala
        115                 120                 125

Cys Met Leu Gly Gly Leu Ala Met Lys Phe Arg Trp Arg Asn Asn Ala
    130                 135                 140

Glu Lys Arg Gly Leu Asp Ile Gln Ala Lys Arg Pro Asn Leu Ile Ile
145                 150                 155                 160

Ser Ser Gly Tyr Gln Val Cys Trp Glu Lys Phe Cys Val Tyr Trp Asp
                165                 170                 175

Val Asp Met Arg Val Val Pro Met Asp Lys Glu His Leu Ser Leu Asp
            180                 185                 190

Val Glu Lys Val Phe Glu Leu Val Asp Glu Tyr Thr Ile Gly Ile Val
        195                 200                 205

Gly Ile Leu Gly Ile Thr Tyr Thr Gly Lys Phe Asp Asp Ile Ala Leu
    210                 215                 220

Leu Asp Glu Lys Val Glu Ala Tyr Asn Glu Ala Asn Glu His Gln Leu
225                 230                 235                 240

Val Ile His Ile Asp Gly Ala Ser Gly Ala Met Phe Thr Pro Phe Val
                245                 250                 255

Asn Pro Glu Leu Pro Trp Asp Phe Arg Leu Lys Asn Val Val Ser Ile
            260                 265                 270

Asn Thr Ser Gly His Lys Tyr Gly Leu Val Tyr Pro Gly Val Gly Trp
        275                 280                 285

Ile Leu Trp Lys Asp Lys Glu Tyr Leu Pro Lys Glu Leu Ile Phe Glu
    290                 295                 300

Val Ser Tyr Leu Gly Gly Ser Met Pro Thr Met Ala Ile Asn Phe Ser
305                 310                 315                 320

Arg Ser Ala Ser Gln Ile Ile Gly Gln Tyr Tyr Asn Phe Leu Arg Tyr
                325                 330                 335

Gly Phe Glu Gly Tyr Arg Glu Ile His Glu Lys Thr Lys Lys Thr Ala
            340                 345                 350

Leu Tyr Leu Ser Lys Thr Val Glu Lys Ser Gly Tyr Phe Glu Ile Ile
        355                 360                 365

Asn Asp Gly Ser Asn Leu Pro Ile Val Cys Tyr Lys Leu Lys Asp Asp
    370                 375                 380

Leu Asp Val Glu Trp Thr Leu Tyr Asp Leu Ala Asp Gln Leu Leu Met
385                 390                 395                 400

Lys Gly Trp Gln Val Pro Ala Tyr Pro Leu Pro Ala Asp Leu Ser Asp
                405                 410                 415

Thr Ile Ile Gln Arg Phe Val Cys Arg Ala Asp Leu Gly Tyr Asn Val
            420                 425                 430

Ala Glu Glu Phe Ala Ala Asp Phe Ala Asp Ala Leu His Asn Leu Glu
        435                 440                 445

His Ala Arg Val Leu Tyr His Asp Lys Glu Arg Asn Asp Ser Tyr Gly
    450                 455                 460

Phe Thr His
465

<210>  5
<211>  462
<212>  PRT
<213>  Listeria monocytogenes

<400>  5

Met Phe Lys Thr Asn Val Glu Gln Asn Asn Val Pro Val Phe Gly Ser
1               5                   10                  15

Phe Glu Ser Gly Gln Asp Leu Pro Glu Lys Arg Met Asn Lys Glu Ser
            20                  25                  30

Val Asp Pro Arg Ile Ala Tyr Gln Leu Val Lys Asp Gln Leu Ile Asp
        35                  40                  45

Glu Gly Ser Ala Arg Gln Asn Leu Ala Thr Phe Cys Gln Thr Tyr Met
    50                  55                  60

Glu Pro Glu Ala Glu Gln Ile Met Ala Glu Thr Met Glu Lys Asn Ala
65                  70                  75                  80

Ile Asp Lys Ser Glu Tyr Pro Gln Thr Ala Lys Leu Glu Ser Ser Cys
                85                  90                  95

Val Asn Met Leu Ala Asp Leu Trp Asn Val Asp Glu Ser Glu His Tyr
            100                 105                 110

Met Gly Thr Ser Thr Val Gly Ser Ser Glu Ala Cys Met Leu Gly Gly
        115                 120                 125

Met Ala Met Lys Phe Arg Trp Arg Ser Ala Ala Leu Lys Asn Gly Leu
    130                 135                 140

Asp Ile His Ala Lys Lys Pro Ser Leu Val Ile Ser Ser Gly Tyr Gln
145                 150                 155                 160

Val Cys Trp Glu Lys Phe Cys Val Tyr Trp Asp Ile Glu Leu Arg Glu
                165                 170                 175

Val Pro Met Ser Glu Glu His Leu Ser Ile Asn Thr Asp Ile Ile Met
            180                 185                 190

Asp Tyr Val Asp Glu Tyr Thr Ile Gly Ile Val Gly Ile Leu Gly Ile
        195                 200                 205

Thr Tyr Thr Gly Lys Phe Asp Asp Ile Met Thr Leu Asn Asp Leu Val
    210                 215                 220

Glu Asp Tyr Asn Asn Thr His Asp Asn Glu Val Val Ile His Val Asp
225                 230                 235                 240

Gly Ala Ser Gly Ala Met Phe Thr Pro Phe Val Glu Pro Gly Leu Glu
                245                 250                 255

Trp Asp Phe Arg Leu Pro Asn Val Val Ser Ile Asn Thr Ser Gly His
            260                 265                 270

Lys Tyr Gly Leu Val Tyr Pro Gly Val Gly Trp Ile Leu Trp Arg Asp
        275                 280                 285

Lys Glu Tyr Leu Pro Glu Glu Leu Val Phe Asp Val Ser Tyr Leu Gly
    290                 295                 300

Gly His Met Pro Thr Met Ala Ile Asn Phe Ser Arg Ser Ala Ser Gln
305                 310                 315                 320

Ile Ile Gly Gln Tyr Tyr Asn Phe Leu Arg Phe Gly Tyr Glu Gly Tyr
                325                 330                 335

Arg Gln Ile His Met Arg Thr Arg Asp Gly Ala Leu Gln Leu Ser Gln
            340                 345                 350

Ala Val Ala Glu Thr Gly Leu Phe Glu Ile Tyr Asn Asp Gly Ala Asn
        355                 360                 365

Leu Pro Ile Val Cys Tyr Lys Leu Lys Asp Asp Ala Asn Val Ala Trp
    370                 375                 380

Thr Leu Tyr Asp Leu Ala Asp Arg Leu Gln Met Lys Gly Trp Gln Val
385                 390                 395                 400

Pro Ala Tyr Pro Leu Pro Lys Glu Met Gly Asn Thr Ile Ile Gln Arg
                405                 410                 415

Tyr Val Cys Arg Gly Asp Leu Gly Gln Asn Met Val Thr Ala Phe Lys
            420                 425                 430

Asn Asp Leu Ser Glu Ser Ile Glu Glu Leu Asn Asn Ala His Ile Leu
        435                 440                 445

Tyr His Asp Val Asn Thr Ser Lys Thr His Gly Phe Thr His
    450                 455                 460

<210>  6
<211>  466
<212>  PRT
<213>  Shigella flexneri

<400>  6

Met Asp Gln Lys Leu Leu Thr Asp Phe Arg Ser Glu Leu Leu Asp Ser
1               5                   10                  15

Arg Phe Gly Ala Lys Ala Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Gln Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu

435                 440                 445

Ser Asp His Pro Lys Leu Gln Gly Ile Ala Gln Gln Asn Ser Phe Lys
    450                 455                 460

His Thr
465

<210>  7
<211>  466
<212>  PRT
<213>  Lactococcus lactis subsp. lactis

<400>  7

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Glu Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Ile Tyr Trp Asp Ile
                165                 170                 175

Glu Met Arg Glu Val Pro Met Asp Lys Glu His Met Ser Ile Asn Leu

            180                 185                 190

Asp Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Phe Leu Ala Lys Glu Ile Glu Lys Thr Gly Met Phe Glu Ile Met Asn
        355                 360                 365

Asp Gly Ser Gln Leu Pro Ile Val Cys Tyr Lys Leu Lys Glu Asp Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Glu Ala Leu Asn Lys
        435                 440                 445

Ala His Ile Leu Tyr His Glu Glu Pro Glu Asn Lys Thr Tyr Gly Phe
    450                 455                 460

Thr His
465

<210>  8
<211>  466
<212>  PRT
<213>  Lactococcus lactis subsp. cremoris

<400>  8

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Glu Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Val Tyr Trp Asp Ile
                165                 170                 175

Glu Met Arg Glu Val Pro Met Asp Arg Glu His Met Ser Ile Asn Leu
            180                 185                 190

Glu Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Tyr Leu Ala Glu Glu Ile Glu Lys Thr Gly Met Phe Glu Ile Met Asn
        355                 360                 365

Asp Gly Ala Gln Leu Pro Ile Val Cys Tyr Lys Leu Lys Glu Asn Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Asp Ala Leu Asn Lys
        435                 440                 445

Ala His Ile Leu Phe His Gln Glu Pro Glu Asn Lys Thr Tyr Gly Phe
    450                 455                 460

Thr His
465

<210>  9
<211>  464
<212>  PRT
<213>  Aliivibrio fischeri

<400>  9

Met Pro Leu His Ser Lys Asn Ala Val Arg Asp Asp Leu Leu Asp Asp
1               5                   10                  15

Ile Tyr Ser Ser Ala Asp Leu Ser Leu Ser Met Pro Lys Tyr Lys Met
            20                  25                  30

Pro Glu Gln Glu His Asp Pro Arg His Ala Tyr Gln Val Ile His Asp
        35                  40                  45

Glu Leu Met Met Asp Gly Asn Ser Arg Gln Asn Leu Ala Thr Phe Cys
    50                  55                  60

Gln Thr Trp Val Glu Asp Glu Val His Lys Leu Met Asp Glu Cys Ile
65                  70                  75                  80

Asp Lys Asn Met Ile Asp Lys Asp Glu Tyr Pro Gln Thr Ala Glu Leu
                85                  90                  95

Glu Ser Arg Cys Val His Met Leu Ala Asp Leu Trp Asn Ser Pro Asp
            100                 105                 110

Ala Glu Asn Thr Leu Gly Cys Ser Thr Thr Gly Ser Ser Glu Ala Ala
        115                 120                 125

Met Leu Gly Gly Met Ala Leu Lys Trp Ala Trp Arg Glu Lys Met Lys
    130                 135                 140

Lys Leu Gly Lys Pro Thr Asp Lys Pro Asn Met Ile Cys Gly Pro Val
145                 150                 155                 160

Gln Val Cys Trp His Lys Phe Ala Arg Tyr Trp Asp Ile Glu Leu Arg
                165                 170                 175

Glu Ile Pro Met Glu Gly Asp Arg Leu Ile Met Thr Pro Glu Glu Val
            180                 185                 190

Ile Lys Arg Cys Asp Glu Asn Thr Ile Gly Val Val Pro Thr Leu Gly
        195                 200                 205

Val Thr Phe Thr Cys Gln Tyr Glu Pro Val Lys Ala Val His Glu Ala
    210                 215                 220

Leu Asp Lys Leu Gln Glu Glu Thr Gly Leu Asp Ile Pro Met His Ile
225                 230                 235                 240

Asp Ala Ala Ser Gly Gly Phe Leu Ala Pro Phe Cys Asp Pro Asp Leu
                245                 250                 255

Glu Trp Asp Phe Arg Leu Pro Arg Val Lys Ser Ile Asn Ala Ser Gly
            260                 265                 270

His Lys Phe Gly Leu Ser Pro Leu Gly Val Gly Trp Val Ile Trp Arg
        275                 280                 285

Asp Ala Ser Ala Leu His Glu Asp Leu Ile Phe Asn Val Asn Tyr Leu
    290                 295                 300

Gly Gly Asn Met Pro Thr Phe Ala Leu Asn Phe Ser Arg Pro Gly Gly
305                 310                 315                 320

Gln Ile Val Ala Gln Tyr Tyr Asn Phe Leu Arg Leu Gly Lys Glu Gly
                325                 330                 335

Tyr Arg Lys Ile His Gln Ala Cys Tyr Asp Thr Ala Val Tyr Leu Ser
            340                 345                 350

Ser Glu Ile Glu Lys Leu Gly Met Phe Glu Ile Ile Tyr Asp Gly Lys
        355                 360                 365

Gly Gly Ile Pro Ala Met Ser Trp Ser Leu Lys Glu Gly Val Asp Pro
    370                 375                 380

Gly Phe Asn Leu Phe Asp Leu Ser Asp Arg Ile Arg Ser Arg Gly Trp
385                 390                 395                 400

Gln Ile Ala Ala Tyr Ala Met Pro Pro Lys Arg Glu Asp Leu Val Ile
                405                 410                 415

Met Arg Ile Leu Val Arg His Gly Phe Ser Arg Asp Gln Ala Asp Leu
            420                 425                 430

Leu Val Ala Asp Leu Lys His Cys Val Glu Phe Phe Ala Lys His Pro
        435                 440                 445

Ile Ser His Gly Ser Asp Glu Leu Glu Ser Ser Gly Phe Asn His Gly
    450                 455                 460

<210>  10
<211>  468
<212>  PRT
<213>  Lactobacillus brevis

<400>  10

Met Ala Met Leu Tyr Gly Lys His Thr His Glu Thr Asp Glu Thr Leu
1               5                   10                  15

Thr Pro Ile Phe Gly Ala Thr Ala Glu Arg His Asp Leu Pro Lys Tyr
            20                  25                  30

Lys Leu Ala Lys His Ala Leu Glu Pro Arg Glu Ala Asp Arg Leu Val
        35                  40                  45

Arg Asp Gln Leu Leu Asp Glu Gly Asn Ser Arg Leu Asn Leu Ala Thr
    50                  55                  60

Phe Cys Gln Thr Tyr Met Glu Pro Glu Ala Val Glu Leu Met Lys Asp
65                  70                  75                  80

Thr Leu Glu Lys Asn Ala Ile Asp Lys Ser Glu Tyr Pro Arg Thr Ala
                85                  90                  95

Glu Ile Glu Asn Arg Cys Val Asn Ile Ile Ala Asn Leu Trp His Ala
            100                 105                 110

Pro Glu Ala Glu Ser Phe Thr Gly Thr Ser Thr Ile Gly Ser Ser Glu
        115                 120                 125

Ala Cys Met Leu Ala Gly Leu Ala Met Lys Phe Ala Trp Arg Lys Arg
    130                 135                 140

Ala Lys Ala Asn Gly Leu Asp Leu Thr Ala His Gln Pro Asn Ile Val
145                 150                 155                 160

Ile Ser Ala Gly Tyr Gln Val Cys Trp Glu Lys Phe Cys Val Tyr Trp
                165                 170                 175

Asp Ile Asp Met His Val Val Pro Met Asp Asp Asp His Met Ser Leu
            180                 185                 190

Asn Val Asp His Val Leu Asp Tyr Val Asp Asp Tyr Thr Ile Gly Ile
        195                 200                 205

Val Gly Ile Met Gly Ile Thr Tyr Thr Gly Gln Tyr Asp Asp Leu Ala
    210                 215                 220

Arg Leu Asp Ala Val Val Glu Arg Tyr Asn Arg Thr Thr Lys Phe Pro
225                 230                 235                 240

Val Tyr Ile His Val Asp Ala Ala Ser Gly Gly Phe Tyr Thr Pro Phe
                245                 250                 255

Ile Glu Pro Glu Leu Lys Trp Asp Phe Arg Leu Asn Asn Val Ile Ser
            260                 265                 270

Ile Asn Ala Ser Gly His Lys Tyr Gly Leu Val Tyr Pro Gly Val Gly
        275                 280                 285

Trp Val Ile Trp Arg Asp Gln Gln Tyr Leu Pro Lys Glu Leu Val Phe
    290                 295                 300

Lys Val Ser Tyr Leu Gly Gly Glu Leu Pro Thr Met Ala Ile Asn Phe
305                 310                 315                 320

Ser His Ser Ala Ser Gln Leu Ile Gly Gln Tyr Tyr Asn Phe Ile Arg
                325                 330                 335

Phe Gly Phe Asp Gly Tyr Arg Glu Ile Gln Glu Lys Thr His Asp Val
            340                 345                 350

Ala Arg Tyr Leu Ala Lys Ser Leu Thr Lys Leu Gly Gly Phe Ser Leu
        355                 360                 365

Ile Asn Asp Gly His Glu Leu Pro Leu Ile Cys Tyr Glu Leu Thr Ala
    370                 375                 380

Asp Ser Asp Arg Glu Trp Thr Leu Tyr Asp Leu Ser Asp Arg Leu Leu
385                 390                 395                 400

Met Lys Gly Trp Gln Val Pro Thr Tyr Pro Leu Pro Lys Asn Met Thr
                405                 410                 415

Asp Arg Val Ile Gln Arg Ile Val Val Arg Ala Asp Phe Gly Met Ser
            420                 425                 430

Met Ala His Asp Phe Ile Asp Asp Leu Thr Gln Ala Ile His Asp Leu

435                 440                 445

Asp Gln Ala His Ile Val Phe His Ser Asp Pro Gln Pro Lys Lys Tyr
    450                 455                 460

Gly Phe Thr His
465

<210>  11
<211>  466
<212>  PRT
<213>  Artificial Sequence

<220>
<223>  E. coli GAD mutant E89Q

<400>  11

Met Asp Lys Lys Gln Val Thr Asp Leu Arg Ser Glu Leu Leu Asp Ser
1               5                   10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Gln Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
        180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp

420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

<210>  12
<211>  466
<212>  PRT
<213>  Artificial Sequence

<220>
<223>  E. coli GAD mutant H465A

<400>  12

Met Asp Lys Lys Gln Val Thr Asp Leu Arg Ser Glu Leu Leu Asp Ser
1               5                   10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr

405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

Ala Thr
465

<210>  13
<211>  466
<212>  PRT
<213>  Artificial Sequence

<220>
<223>  E. coli GAD mutant E89Q+H465A

<400>  13

Met Asp Lys Lys Gln Val Thr Asp Leu Arg Ser Glu Leu Leu Asp Ser
1               5                   10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Gln Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg

385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

Ala Thr
465

<210>  14
<211>  457
<212>  PRT
<213>  Artificial Sequence

<220>
<223>  S. thermosulfidooxidans GAD mutant E84Q

<400>  14

Met Lys Lys Ala Ser Glu Glu Val Ala Ile Asn Pro Leu Phe Ala Arg
1               5                   10                  15

Arg Gly Glu Glu Ser Val Pro Arg Tyr Arg Leu Ser Glu Glu Gly Leu
            20                  25                  30

Leu Pro Glu Thr Ala Tyr Gln Ile Val His Asp Glu Leu Thr Leu Asp
        35                  40                  45

Gly Ser Ala Arg Leu Asn Leu Ala Thr Phe Val Gly Thr Trp Met Glu
    50                  55                  60

Pro Glu Ala Gln Lys Leu Tyr Val Glu Ala Ala Asn Lys Asn Ile Ile
65                  70                  75                  80

Asp Lys Asp Gln Tyr Pro Glu Thr Ala Ala Ile Glu Glu Arg Cys Leu
                85                  90                  95

Lys Ile Leu Ala Asn Leu Trp Asn Ala Pro Asp Pro Ala Asn Ser Ile
            100                 105                 110

Gly Val Ser Thr Thr Gly Ser Ser Glu Ala Cys Met Leu Gly Gly Leu
        115                 120                 125

Ala Leu Lys Arg Arg Trp Gln Leu Ser Arg Arg Gln Gln Gly Lys Ser
    130                 135                 140

Thr Asp His Pro Asn Ile Val Phe Ser Ser Ala Val Gln Val Val Trp
145                 150                 155                 160

Glu Lys Phe Ala Asn Tyr Phe Glu Val Glu Pro Arg Tyr Val Asn Ile
                165                 170                 175

Thr Pro Glu His Pro Tyr Leu Thr Pro Glu Gly Val Ile Lys Ala Val
            180                 185                 190

Asp Glu Asn Thr Ile Gly Val Val Pro Ile Leu Gly Val Thr Tyr Thr
        195                 200                 205

Gly Ser Tyr Glu Pro Val Glu Ala Ile Ala Gln Ala Leu Asp Asp Leu
    210                 215                 220

Glu Lys Arg Thr Gly Leu Ser Ile Pro Ile Val Asp Ala Ala Ser Gly
225                 230                 235                 240

Gly Phe Val Ala Pro Phe Leu Gln Pro Asp Leu Val Trp Asp Phe Arg
                245                 250                 255

Leu Pro Arg Val His Ser Ile Asn Thr Ser Gly His Lys Tyr Gly Leu
            260                 265                 270

Val Tyr Pro Gly Leu Gly Trp Val Leu Trp Arg Asp Lys Thr Leu Leu
        275                 280                 285

Pro Glu Glu Leu Ile Phe Asn Val Ser Tyr Leu Gly Gly Ser Met Pro
    290                 295                 300

Thr Phe Gly Leu Asn Phe Ser Arg Pro Gly Ala Gln Val Leu Leu Gln
305                 310                 315                 320

Tyr Tyr Asn Phe Leu Arg Leu Gly Arg Gln Gly Tyr Tyr Thr Val Gln
                325                 330                 335

Lys Thr Cys Gln Asn Val Ala His Phe Leu Ala Gln Glu Ile Gly Ala
            340                 345                 350

Met Glu Pro Phe Glu Leu Val Thr Asp Gly Ser Asp Leu Pro Val Phe
        355                 360                 365

Ala Trp Arg Lys Arg Pro Glu Glu Asn Pro Leu Trp Ser Leu Glu Asp

370                 375                 380

Leu Ser Phe Val Leu Arg Glu Arg Gly Trp Gln Val Pro Ala Tyr Pro
385                 390                 395                 400

Leu Pro Pro Thr Met Gln Asp Val Thr Ile Met Arg Ile Val Val Arg
                405                 410                 415

Asn Ser Phe Ser Met Asp Met Ala Gln Leu Phe Leu Asp Asp Leu Asn
            420                 425                 430

Arg Ala Val Ala Tyr Leu Gln Gly Leu Asp His Pro Phe Pro Gln Ala
        435                 440                 445

Leu Arg Gln His Lys Pro Phe Ala His
    450                 455

<210>  15
<211>  457
<212>  PRT
<213>  Artificial Sequence

<220>
<223>  S. thermosulfidooxidans GAD mutant H457A

<400>  15

Met Lys Lys Ala Ser Glu Glu Val Ala Ile Asn Pro Leu Phe Ala Arg
1               5                   10                  15

Arg Gly Glu Glu Ser Val Pro Arg Tyr Arg Leu Ser Glu Glu Gly Leu
            20                  25                  30

Leu Pro Glu Thr Ala Tyr Gln Ile Val His Asp Glu Leu Thr Leu Asp
        35                  40                  45

Gly Ser Ala Arg Leu Asn Leu Ala Thr Phe Val Gly Thr Trp Met Glu
    50                  55                  60

Pro Glu Ala Gln Lys Leu Tyr Val Glu Ala Ala Asn Lys Asn Ile Ile
65                  70                  75                  80

Asp Lys Asp Glu Tyr Pro Glu Thr Ala Ala Ile Glu Glu Arg Cys Leu
                85                  90                  95

Lys Ile Leu Ala Asn Leu Trp Asn Ala Pro Asp Pro Ala Asn Ser Ile
            100                 105                 110

Gly Val Ser Thr Thr Gly Ser Ser Glu Ala Cys Met Leu Gly Gly Leu
        115                 120                 125

Ala Leu Lys Arg Arg Trp Gln Leu Ser Arg Arg Gln Gln Gly Lys Ser
    130                 135                 140

Thr Asp His Pro Asn Ile Val Phe Ser Ser Ala Val Gln Val Val Trp
145                 150                 155                 160

Glu Lys Phe Ala Asn Tyr Phe Glu Val Glu Pro Arg Tyr Val Asn Ile
                165                 170                 175

Thr Pro Glu His Pro Tyr Leu Thr Pro Glu Gly Val Ile Lys Ala Val
            180                 185                 190

Asp Glu Asn Thr Ile Gly Val Val Pro Ile Leu Gly Val Thr Tyr Thr
        195                 200                 205

Gly Ser Tyr Glu Pro Val Glu Ala Ile Ala Gln Ala Leu Asp Asp Leu
    210                 215                 220

Glu Lys Arg Thr Gly Leu Ser Ile Pro Ile Val Asp Ala Ala Ser Gly
225                 230                 235                 240

Gly Phe Val Ala Pro Phe Leu Gln Pro Asp Leu Val Trp Asp Phe Arg
                245                 250                 255

Leu Pro Arg Val His Ser Ile Asn Thr Ser Gly His Lys Tyr Gly Leu
            260                 265                 270

Val Tyr Pro Gly Leu Gly Trp Val Leu Trp Arg Asp Lys Thr Leu Leu
        275                 280                 285

Pro Glu Glu Leu Ile Phe Asn Val Ser Tyr Leu Gly Gly Ser Met Pro
    290                 295                 300

Thr Phe Gly Leu Asn Phe Ser Arg Pro Gly Ala Gln Val Leu Leu Gln
305                 310                 315                 320

Tyr Tyr Asn Phe Leu Arg Leu Gly Arg Gln Gly Tyr Tyr Thr Val Gln
                325                 330                 335

Lys Thr Cys Gln Asn Val Ala His Phe Leu Ala Gln Glu Ile Gly Ala
            340                 345                 350

Met Glu Pro Phe Glu Leu Val Thr Asp Gly Ser Asp Leu Pro Val Phe
        355                 360                 365

Ala Trp Arg Lys Arg Pro Glu Glu Asn Pro Leu Trp Ser Leu Glu Asp

370                 375                 380

Leu Ser Phe Val Leu Arg Glu Arg Gly Trp Gln Val Pro Ala Tyr Pro
385                 390                 395                 400

Leu Pro Pro Thr Met Gln Asp Val Thr Ile Met Arg Ile Val Val Arg
                405                 410                 415

Asn Ser Phe Ser Met Asp Met Ala Gln Leu Phe Leu Asp Asp Leu Asn
            420                 425                 430

Arg Ala Val Ala Tyr Leu Gln Gly Leu Asp His Pro Phe Pro Gln Ala
        435                 440                 445

Leu Arg Gln His Lys Pro Phe Ala Ala
    450                 455

<210>  16
<211>  457
<212>  PRT
<213>  Artificial Sequence

<220>
<223>  S. thermosulfidooxidans GAD mutant E84Q+H457A

<400>  16

Met Lys Lys Ala Ser Glu Glu Val Ala Ile Asn Pro Leu Phe Ala Arg
1               5                   10                  15

Arg Gly Glu Glu Ser Val Pro Arg Tyr Arg Leu Ser Glu Glu Gly Leu
            20                  25                  30

Leu Pro Glu Thr Ala Tyr Gln Ile Val His Asp Glu Leu Thr Leu Asp
        35                  40                  45

Gly Ser Ala Arg Leu Asn Leu Ala Thr Phe Val Gly Thr Trp Met Glu
    50                  55                  60

Pro Glu Ala Gln Lys Leu Tyr Val Glu Ala Ala Asn Lys Asn Ile Ile
65                  70                  75                  80

Asp Lys Asp Gln Tyr Pro Glu Thr Ala Ala Ile Glu Glu Arg Cys Leu
                85                  90                  95

Lys Ile Leu Ala Asn Leu Trp Asn Ala Pro Asp Pro Ala Asn Ser Ile
            100                 105                 110

Gly Val Ser Thr Thr Gly Ser Ser Glu Ala Cys Met Leu Gly Gly Leu
        115                 120                 125

Ala Leu Lys Arg Arg Trp Gln Leu Ser Arg Arg Gln Gln Gly Lys Ser
    130                 135                 140

Thr Asp His Pro Asn Ile Val Phe Ser Ser Ala Val Gln Val Val Trp
145                 150                 155                 160

Glu Lys Phe Ala Asn Tyr Phe Glu Val Glu Pro Arg Tyr Val Asn Ile
                165                 170                 175

Thr Pro Glu His Pro Tyr Leu Thr Pro Glu Gly Val Ile Lys Ala Val
            180                 185                 190

Asp Glu Asn Thr Ile Gly Val Val Pro Ile Leu Gly Val Thr Tyr Thr
        195                 200                 205

Gly Ser Tyr Glu Pro Val Glu Ala Ile Ala Gln Ala Leu Asp Asp Leu
    210                 215                 220

Glu Lys Arg Thr Gly Leu Ser Ile Pro Ile Val Asp Ala Ala Ser Gly
225                 230                 235                 240

Gly Phe Val Ala Pro Phe Leu Gln Pro Asp Leu Val Trp Asp Phe Arg
                245                 250                 255

Leu Pro Arg Val His Ser Ile Asn Thr Ser Gly His Lys Tyr Gly Leu
            260                 265                 270

Val Tyr Pro Gly Leu Gly Trp Val Leu Trp Arg Asp Lys Thr Leu Leu
        275                 280                 285

Pro Glu Glu Leu Ile Phe Asn Val Ser Tyr Leu Gly Gly Ser Met Pro
    290                 295                 300

Thr Phe Gly Leu Asn Phe Ser Arg Pro Gly Ala Gln Val Leu Leu Gln
305                 310                 315                 320

Tyr Tyr Asn Phe Leu Arg Leu Gly Arg Gln Gly Tyr Tyr Thr Val Gln
                325                 330                 335

Lys Thr Cys Gln Asn Val Ala His Phe Leu Ala Gln Glu Ile Gly Ala
            340                 345                 350

Met Glu Pro Phe Glu Leu Val Thr Asp Gly Ser Asp Leu Pro Val Phe
        355                 360                 365

Ala Trp Arg Lys Arg Pro Glu Glu Asn Pro Leu Trp Ser Leu Glu Asp

370                 375                 380

Leu Ser Phe Val Leu Arg Glu Arg Gly Trp Gln Val Pro Ala Tyr Pro
385                 390                 395                 400

Leu Pro Pro Thr Met Gln Asp Val Thr Ile Met Arg Ile Val Val Arg
                405                 410                 415

Asn Ser Phe Ser Met Asp Met Ala Gln Leu Phe Leu Asp Asp Leu Asn
            420                 425                 430

Arg Ala Val Ala Tyr Leu Gln Gly Leu Asp His Pro Phe Pro Gln Ala
        435                 440                 445

Leu Arg Gln His Lys Pro Phe Ala Ala
    450                 455

<210>  17
<211>  467
<212>  PRT
<213>  Artificial Sequence

<220>
<223>  B. megaterium GAD mutant E93Q

<400>  17

Met Pro Gln Trp His Pro His Arg Glu Gln Lys Asn Leu Pro Asp Glu
1               5                   10                  15

Phe Pro Val Asn Pro Leu Phe Ser Arg Gln Gly Glu Val Thr Ile Pro
            20                  25                  30

Arg Leu Arg Ile Ser Asp Gln Gly Met Leu Pro Glu Thr Ala Tyr Gln
        35                  40                  45

Ile Ile His Asp Glu Ile Ala Leu Asp Gly Asn Ala Arg Leu Asn Leu
    50                  55                  60

Ala Thr Phe Val Thr Thr Trp Met Glu Pro Asp Ala Lys Arg Leu Tyr
65                  70                  75                  80

Gly Glu Ser Phe Asp Lys Asn Met Ile Asp Lys Asp Gln Tyr Pro Gln
                85                  90                  95

Thr Ala Ala Ile Glu Glu Arg Cys Val Arg Ile Leu Ala Asp Leu Trp
            100                 105                 110

Asn Ser Pro Asn Pro Asp Thr Thr Met Gly Val Ser Thr Thr Gly Ser
        115                 120                 125

Ser Glu Ala Cys Met Leu Gly Gly Leu Ala Leu Lys Arg Arg Trp Gln
    130                 135                 140

Lys Leu Arg Lys Ser Lys Gly Leu Ser Thr Asp Arg Pro Asn Ile Val
145                 150                 155                 160

Phe Ser Ser Ser Val Gln Val Val Trp Glu Lys Phe Ala Asn Tyr Trp
                165                 170                 175

Asp Val Glu Pro Arg Tyr Val Asn Ile Asn Pro Asp His Pro Tyr Leu
            180                 185                 190

Asp Ala Glu Gly Val Ile Asn Ala Val Asp Glu Asn Thr Ile Gly Val
        195                 200                 205

Val Pro Ile Leu Gly Val Thr Tyr Thr Gly Val Tyr Glu Pro Ile Ala
    210                 215                 220

Ala Ile Ala Lys Ala Leu Asp Glu Leu Gln Glu Lys Thr Gly Leu Asp
225                 230                 235                 240

Ile Pro Ile His Val Asp Ala Ala Ser Gly Gly Phe Ile Ala Pro Phe
                245                 250                 255

Leu Gln Pro Asp Leu Ile Trp Asp Phe Arg Leu Pro Arg Val Lys Ser
            260                 265                 270

Ile Asn Val Ser Gly His Lys Tyr Gly Leu Val Tyr Pro Gly Leu Gly
        275                 280                 285

Trp Val Ile Trp Arg Glu Lys Glu Asp Leu Pro Glu Asp Leu Ile Phe
    290                 295                 300

Arg Val Ser Tyr Leu Gly Gly Asn Met Pro Thr Phe Ala Leu Asn Phe
305                 310                 315                 320

Ser Arg Pro Gly Ala Gln Val Leu Leu Gln Tyr Tyr Asn Phe Leu Arg
                325                 330                 335

Leu Gly Lys Asp Gly Tyr Tyr Ala Val Gln Lys Thr Ser Gln Glu Asn
            340                 345                 350

Ala Leu Phe Leu Ser Lys Glu Ile Gly Glu Met Glu Ala Phe Glu Ile
        355                 360                 365

Leu Ala Asp Gly Ser Asp Ile Pro Val Leu Ala Trp Lys Leu Lys Glu

370                 375                 380

Gly Tyr Thr Pro Asn Trp Thr Leu Tyr Asp Leu Ser Arg Gln Leu Arg
385                 390                 395                 400

Thr Tyr Gly Trp Gln Val Pro Ala Tyr Pro Leu Pro Ala Asp Met Glu
                405                 410                 415

Glu Ile Thr Ile Met Arg Ile Val Val Arg Asn Gly Phe Ser Arg Asp
            420                 425                 430

Leu Ala His Leu Phe Met Val Asn Phe Lys Gln Ala Val Glu Phe Leu
        435                 440                 445

Asn Ser Leu Asp Arg Pro Val Leu Lys Asp Thr Lys Tyr Asp Asn Gly
    450                 455                 460

Phe His His
465

<210>  18
<211>  467
<212>  PRT
<213>  Artificial Sequence

<220>
<223>  B. megaterium GAD mutant H467A

<400>  18

Met Pro Gln Trp His Pro His Arg Glu Gln Lys Asn Leu Pro Asp Glu
1               5                   10                  15

Phe Pro Val Asn Pro Leu Phe Ser Arg Gln Gly Glu Val Thr Ile Pro
            20                  25                  30

Arg Leu Arg Ile Ser Asp Gln Gly Met Leu Pro Glu Thr Ala Tyr Gln
        35                  40                  45

Ile Ile His Asp Glu Ile Ala Leu Asp Gly Asn Ala Arg Leu Asn Leu
    50                  55                  60

Ala Thr Phe Val Thr Thr Trp Met Glu Pro Asp Ala Lys Arg Leu Tyr
65                  70                  75                  80

Gly Glu Ser Phe Asp Lys Asn Met Ile Asp Lys Asp Glu Tyr Pro Gln
                85                  90                  95

Thr Ala Ala Ile Glu Glu Arg Cys Val Arg Ile Leu Ala Asp Leu Trp
            100                 105                 110

Asn Ser Pro Asn Pro Asp Thr Thr Met Gly Val Ser Thr Thr Gly Ser
        115                 120                 125

Ser Glu Ala Cys Met Leu Gly Gly Leu Ala Leu Lys Arg Arg Trp Gln
    130                 135                 140

Lys Leu Arg Lys Ser Lys Gly Leu Ser Thr Asp Arg Pro Asn Ile Val
145                 150                 155                 160

Phe Ser Ser Ser Val Gln Val Val Trp Glu Lys Phe Ala Asn Tyr Trp
                165                 170                 175

Asp Val Glu Pro Arg Tyr Val Asn Ile Asn Pro Asp His Pro Tyr Leu
            180                 185                 190

Asp Ala Glu Gly Val Ile Asn Ala Val Asp Glu Asn Thr Ile Gly Val
        195                 200                 205

Val Pro Ile Leu Gly Val Thr Tyr Thr Gly Val Tyr Glu Pro Ile Ala
    210                 215                 220

Ala Ile Ala Lys Ala Leu Asp Glu Leu Gln Glu Lys Thr Gly Leu Asp
225                 230                 235                 240

Ile Pro Ile His Val Asp Ala Ala Ser Gly Gly Phe Ile Ala Pro Phe
                245                 250                 255

Leu Gln Pro Asp Leu Ile Trp Asp Phe Arg Leu Pro Arg Val Lys Ser
            260                 265                 270

Ile Asn Val Ser Gly His Lys Tyr Gly Leu Val Tyr Pro Gly Leu Gly
        275                 280                 285

Trp Val Ile Trp Arg Glu Lys Glu Asp Leu Pro Glu Asp Leu Ile Phe
    290                 295                 300

Arg Val Ser Tyr Leu Gly Gly Asn Met Pro Thr Phe Ala Leu Asn Phe
305                 310                 315                 320

Ser Arg Pro Gly Ala Gln Val Leu Leu Gln Tyr Tyr Asn Phe Leu Arg
                325                 330                 335

Leu Gly Lys Asp Gly Tyr Tyr Ala Val Gln Lys Thr Ser Gln Glu Asn
            340                 345                 350

Ala Leu Phe Leu Ser Lys Glu Ile Gly Glu Met Glu Ala Phe Glu Ile

        355                 360                 365

Leu Ala Asp Gly Ser Asp Ile Pro Val Leu Ala Trp Lys Leu Lys Glu
    370                 375                 380

Gly Tyr Thr Pro Asn Trp Thr Leu Tyr Asp Leu Ser Arg Gln Leu Arg
385                 390                 395                 400

Thr Tyr Gly Trp Gln Val Pro Ala Tyr Pro Leu Pro Ala Asp Met Glu
                405                 410                 415

Glu Ile Thr Ile Met Arg Ile Val Val Arg Asn Gly Phe Ser Arg Asp
            420                 425                 430

Leu Ala His Leu Phe Met Val Asn Phe Lys Gln Ala Val Glu Phe Leu
        435                 440                 445

Asn Ser Leu Asp Arg Pro Val Leu Lys Asp Thr Lys Tyr Asp Asn Gly
    450                 455                 460

Phe His Ala
465

<210> 19
<211> 467
<212> PRT
<213> Artificial Sequence

<220>
<223> B. megaterium GAD mutant E93Q+H467A

<400> 19

Met Pro Gln Trp His Pro His Arg Glu Gln Lys Asn Leu Pro Asp Glu
1               5                   10                  15

Phe Pro Val Asn Pro Leu Phe Ser Arg Gln Gly Glu Val Thr Ile Pro
            20                  25                  30

Arg Leu Arg Ile Ser Asp Gln Gly Met Leu Pro Glu Thr Ala Tyr Gln
        35                  40                  45

Ile Ile His Asp Glu Ile Ala Leu Asp Gly Asn Ala Arg Leu Asn Leu
    50                  55                  60

Ala Thr Phe Val Thr Thr Trp Met Glu Pro Asp Ala Lys Arg Leu Tyr
65                  70                  75                  80

Gly Glu Ser Phe Asp Lys Asn Met Ile Asp Lys Asp Gln Tyr Pro Gln
                85                  90                  95

Thr Ala Ala Ile Glu Glu Arg Cys Val Arg Ile Leu Ala Asp Leu Trp
            100                 105                 110

Asn Ser Pro Asn Pro Asp Thr Thr Met Gly Val Ser Thr Thr Gly Ser
        115                 120                 125

Ser Glu Ala Cys Met Leu Gly Gly Leu Ala Leu Lys Arg Arg Trp Gln
    130                 135                 140

Lys Leu Arg Lys Ser Lys Gly Leu Ser Thr Asp Arg Pro Asn Ile Val
145                 150                 155                 160

Phe Ser Ser Ser Val Gln Val Val Trp Glu Lys Phe Ala Asn Tyr Trp
                165                 170                 175

Asp Val Glu Pro Arg Tyr Val Asn Ile Asn Pro Asp His Pro Tyr Leu
            180                 185                 190

Asp Ala Glu Gly Val Ile Asn Ala Val Asp Glu Asn Thr Ile Gly Val
        195                 200                 205

Val Pro Ile Leu Gly Val Thr Tyr Thr Gly Val Tyr Glu Pro Ile Ala
    210                 215                 220

Ala Ile Ala Lys Ala Leu Asp Glu Leu Gln Glu Lys Thr Gly Leu Asp
225                 230                 235                 240

Ile Pro Ile His Val Asp Ala Ala Ser Gly Gly Phe Ile Ala Pro Phe
                245                 250                 255

Leu Gln Pro Asp Leu Ile Trp Asp Phe Arg Leu Pro Arg Val Lys Ser
            260                 265                 270

Ile Asn Val Ser Gly His Lys Tyr Gly Leu Val Tyr Pro Gly Leu Gly
        275                 280                 285

Trp Val Ile Trp Arg Glu Lys Glu Asp Leu Pro Glu Asp Leu Ile Phe
    290                 295                 300

Arg Val Ser Tyr Leu Gly Gly Asn Met Pro Thr Phe Ala Leu Asn Phe
305                 310                 315                 320

Ser Arg Pro Gly Ala Gln Val Leu Leu Gln Tyr Tyr Asn Phe Leu Arg
                325                 330                 335

Leu Gly Lys Asp Gly Tyr Tyr Ala Val Gln Lys Thr Ser Gln Glu Asn

340                 345                 350

Ala Leu Phe Leu Ser Lys Glu Ile Gly Glu Met Glu Ala Phe Glu Ile
        355                 360                 365

Leu Ala Asp Gly Ser Asp Ile Pro Val Leu Ala Trp Lys Leu Lys Glu
    370                 375                 380

Gly Tyr Thr Pro Asn Trp Thr Leu Tyr Asp Leu Ser Arg Gln Leu Arg
385                 390                 395                 400

Thr Tyr Gly Trp Gln Val Pro Ala Tyr Pro Leu Pro Ala Asp Met Glu
                405                 410                 415

Glu Ile Thr Ile Met Arg Ile Val Val Arg Asn Gly Phe Ser Arg Asp
            420                 425                 430

Leu Ala His Leu Phe Met Val Asn Phe Lys Gln Ala Val Glu Phe Leu
        435                 440                 445

Asn Ser Leu Asp Arg Pro Val Leu Lys Asp Thr Lys Tyr Asp Asn Gly
    450                 455                 460

Phe His Ala
465

<210>  20
<211>  467
<212>  PRT
<213>  Artificial Sequence

<220>
<223>  L. innocua GAD mutant E90Q

<400>  20

Met Leu Tyr Ser Glu Asn Asp Lys Arg Lys His Glu Ser Tyr Arg Ile
1               5                   10                  15

Pro Leu Phe Gly Ser Glu Glu Glu Ser Thr Ser Ile Pro Lys Tyr Val
            20                  25                  30

Leu Lys Lys Glu Pro Met Glu Pro Arg Ile Ala Tyr Gln Leu Val Lys
        35                  40                  45

Asp Gln Leu Met Asp Glu Gly Asn Ala Arg Gln Asn Leu Ala Thr Phe
    50                  55                  60

Cys Gln Thr Tyr Met Glu Lys Glu Ala Glu Ile Leu Met Ala Glu Thr
65                  70                  75                  80

Leu Glu Lys Asn Ala Ile Asp Lys Ser Gln Tyr Pro Gln Thr Ala Glu
                85                  90                  95

Leu Glu Asn Arg Cys Val Asn Ile Leu Ala Asp Leu Trp Asn Ala Pro
            100                 105                 110

Lys Glu Met Ser Tyr Leu Gly Thr Ser Thr Val Gly Ser Ser Glu Ala
        115                 120                 125

Cys Met Leu Gly Gly Leu Ala Met Lys Phe Arg Trp Arg Asn Asn Ala
    130                 135                 140

Glu Lys Arg Gly Leu Asp Ile Gln Ala Lys Arg Pro Asn Leu Ile Ile
145                 150                 155                 160

Ser Ser Gly Tyr Gln Val Cys Trp Glu Lys Phe Cys Val Tyr Trp Asp
                165                 170                 175

Val Asp Met Arg Val Val Pro Met Asp Lys Glu His Leu Ser Leu Asp
            180                 185                 190

Val Glu Lys Val Phe Glu Leu Val Asp Glu Tyr Thr Ile Gly Ile Val
        195                 200                 205

Gly Ile Leu Gly Ile Thr Tyr Thr Gly Lys Phe Asp Asp Ile Ala Leu
    210                 215                 220

Leu Asp Glu Lys Val Glu Ala Tyr Asn Glu Ala Asn Glu His Gln Leu
225                 230                 235                 240

Val Ile His Ile Asp Gly Ala Ser Gly Ala Met Phe Thr Pro Phe Val
                245                 250                 255

Asn Pro Glu Leu Pro Trp Asp Phe Arg Leu Lys Asn Val Val Ser Ile
            260                 265                 270

Asn Thr Ser Gly His Lys Tyr Gly Leu Val Tyr Pro Gly Val Gly Trp
        275                 280                 285

Ile Leu Trp Lys Asp Lys Glu Tyr Leu Pro Lys Glu Leu Ile Phe Glu
    290                 295                 300

Val Ser Tyr Leu Gly Gly Ser Met Pro Thr Met Ala Ile Asn Phe Ser
305                 310                 315                 320

Arg Ser Ala Ser Gln Ile Ile Gly Gln Tyr Tyr Asn Phe Leu Arg Tyr

325                 330                 335

Gly Phe Glu Gly Tyr Arg Glu Ile His Glu Lys Thr Lys Lys Thr Ala
            340                 345                 350

Leu Tyr Leu Ser Lys Thr Val Glu Lys Ser Gly Tyr Phe Glu Ile Ile
        355                 360                 365

Asn Asp Gly Ser Asn Leu Pro Ile Val Cys Tyr Lys Leu Lys Asp Asp
    370                 375                 380

Leu Asp Val Glu Trp Thr Leu Tyr Asp Leu Ala Asp Gln Leu Leu Met
385                 390                 395                 400

Lys Gly Trp Gln Val Pro Ala Tyr Pro Leu Pro Ala Asp Leu Ser Asp
                405                 410                 415

Thr Ile Ile Gln Arg Phe Val Cys Arg Ala Asp Leu Gly Tyr Asn Val
            420                 425                 430

Ala Glu Glu Phe Ala Ala Asp Phe Ala Asp Ala Leu His Asn Leu Glu
        435                 440                 445

His Ala Arg Val Leu Tyr His Asp Lys Glu Arg Asn Asp Ser Tyr Gly
    450                 455                 460

Phe Thr His
465

<210>  21
<211>  467
<212>  PRT
<213>  Artificial Sequence

<220>
<223>  L. innocua GAD mutant H467A

<400>  21

Met Leu Tyr Ser Glu Asn Asp Lys Arg Lys His Glu Ser Tyr Arg Ile
1               5                   10                  15

Pro Leu Phe Gly Ser Glu Glu Glu Ser Thr Ser Ile Pro Lys Tyr Val
            20                  25                  30

Leu Lys Lys Glu Pro Met Glu Pro Arg Ile Ala Tyr Gln Leu Val Lys
        35                  40                  45

Asp Gln Leu Met Asp Glu Gly Asn Ala Arg Gln Asn Leu Ala Thr Phe
    50                  55                  60

Cys Gln Thr Tyr Met Glu Lys Glu Ala Glu Ile Leu Met Ala Glu Thr
65                  70                  75                  80

Leu Glu Lys Asn Ala Ile Asp Lys Ser Glu Tyr Pro Gln Thr Ala Glu
                85                  90                  95

Leu Glu Asn Arg Cys Val Asn Ile Leu Ala Asp Leu Trp Asn Ala Pro
            100                 105                 110

Lys Glu Met Ser Tyr Leu Gly Thr Ser Thr Val Gly Ser Ser Glu Ala
        115                 120                 125

Cys Met Leu Gly Gly Leu Ala Met Lys Phe Arg Trp Arg Asn Asn Ala
    130                 135                 140

Glu Lys Arg Gly Leu Asp Ile Gln Ala Lys Arg Pro Asn Leu Ile Ile
145                 150                 155                 160

Ser Ser Gly Tyr Gln Val Cys Trp Glu Lys Phe Cys Val Tyr Trp Asp
                165                 170                 175

Val Asp Met Arg Val Val Pro Met Asp Lys Glu His Leu Ser Leu Asp
            180                 185                 190

Val Glu Lys Val Phe Glu Leu Val Asp Glu Tyr Thr Ile Gly Ile Val
        195                 200                 205

Gly Ile Leu Gly Ile Thr Tyr Thr Gly Lys Phe Asp Asp Ile Ala Leu
    210                 215                 220

Leu Asp Glu Lys Val Glu Ala Tyr Asn Glu Ala Asn Glu His Gln Leu
225                 230                 235                 240

Val Ile His Ile Asp Gly Ala Ser Gly Ala Met Phe Thr Pro Phe Val
                245                 250                 255

Asn Pro Glu Leu Pro Trp Asp Phe Arg Leu Lys Asn Val Val Ser Ile
            260                 265                 270

Asn Thr Ser Gly His Lys Tyr Gly Leu Val Tyr Pro Gly Val Gly Trp
        275                 280                 285

Ile Leu Trp Lys Asp Lys Glu Tyr Leu Pro Lys Glu Leu Ile Phe Glu
    290                 295                 300

Val Ser Tyr Leu Gly Gly Ser Met Pro Thr Met Ala Ile Asn Phe Ser

305                 310                 315                 320

Arg Ser Ala Ser Gln Ile Ile Gly Gln Tyr Tyr Asn Phe Leu Arg Tyr
                325                 330                 335

Gly Phe Glu Gly Tyr Arg Glu Ile His Glu Lys Thr Lys Lys Thr Ala
            340                 345                 350

Leu Tyr Leu Ser Lys Thr Val Glu Lys Ser Gly Tyr Phe Glu Ile Ile
        355                 360                 365

Asn Asp Gly Ser Asn Leu Pro Ile Val Cys Tyr Lys Leu Lys Asp Asp
    370                 375                 380

Leu Asp Val Glu Trp Thr Leu Tyr Asp Leu Ala Asp Gln Leu Leu Met
385                 390                 395                 400

Lys Gly Trp Gln Val Pro Ala Tyr Pro Leu Pro Ala Asp Leu Ser Asp
                405                 410                 415

Thr Ile Ile Gln Arg Phe Val Cys Arg Ala Asp Leu Gly Tyr Asn Val
            420                 425                 430

Ala Glu Glu Phe Ala Ala Asp Phe Ala Asp Ala Leu His Asn Leu Glu
        435                 440                 445

His Ala Arg Val Leu Tyr His Asp Lys Glu Arg Asn Asp Ser Tyr Gly
    450                 455                 460

Phe Thr Ala
465

<210>  22
<211>  467
<212>  PRT
<213>  Artificial Sequence

<220>
<223>  L. innocua GAD mutant E90Q+H467A

<400>  22

Met Leu Tyr Ser Glu Asn Asp Lys Arg Lys His Glu Ser Tyr Arg Ile
1               5                   10                  15

Pro Leu Phe Gly Ser Glu Glu Glu Ser Thr Ser Ile Pro Lys Tyr Val
            20                  25                  30

Leu Lys Lys Glu Pro Met Glu Pro Arg Ile Ala Tyr Gln Leu Val Lys
        35                  40                  45

Asp Gln Leu Met Asp Glu Gly Asn Ala Arg Gln Asn Leu Ala Thr Phe
    50                  55                  60

Cys Gln Thr Tyr Met Glu Lys Glu Ala Glu Ile Leu Met Ala Glu Thr
65                  70                  75                  80

Leu Glu Lys Asn Ala Ile Asp Lys Ser Gln Tyr Pro Gln Thr Ala Glu
                85                  90                  95

Leu Glu Asn Arg Cys Val Asn Ile Leu Ala Asp Leu Trp Asn Ala Pro
            100                 105                 110

Lys Glu Met Ser Tyr Leu Gly Thr Ser Thr Val Gly Ser Ser Glu Ala
        115                 120                 125

Cys Met Leu Gly Gly Leu Ala Met Lys Phe Arg Trp Arg Asn Asn Ala
    130                 135                 140

Glu Lys Arg Gly Leu Asp Ile Gln Ala Lys Arg Pro Asn Leu Ile Ile
145                 150                 155                 160

Ser Ser Gly Tyr Gln Val Cys Trp Glu Lys Phe Cys Val Tyr Trp Asp
                165                 170                 175

Val Asp Met Arg Val Val Pro Met Asp Lys Glu His Leu Ser Leu Asp
            180                 185                 190

Val Glu Lys Val Phe Glu Leu Val Asp Glu Tyr Thr Ile Gly Ile Val
        195                 200                 205

Gly Ile Leu Gly Ile Thr Tyr Thr Gly Lys Phe Asp Asp Ile Ala Leu
    210                 215                 220

Leu Asp Glu Lys Val Glu Ala Tyr Asn Glu Ala Asn Glu His Gln Leu
225                 230                 235                 240

Val Ile His Ile Asp Gly Ala Ser Gly Ala Met Phe Thr Pro Phe Val
                245                 250                 255

Asn Pro Glu Leu Pro Trp Asp Phe Arg Leu Lys Asn Val Val Ser Ile
            260                 265                 270

Asn Thr Ser Gly His Lys Tyr Gly Leu Val Tyr Pro Gly Val Gly Trp
        275                 280                 285

Ile Leu Trp Lys Asp Lys Glu Tyr Leu Pro Lys Glu Leu Ile Phe Glu

290                 295                 300

Val Ser Tyr Leu Gly Gly Ser Met Pro Thr Met Ala Ile Asn Phe Ser
305                 310                 315                 320

Arg Ser Ala Ser Gln Ile Ile Gly Gln Tyr Tyr Asn Phe Leu Arg Tyr
                325                 330                 335

Gly Phe Glu Gly Tyr Arg Glu Ile His Glu Lys Thr Lys Lys Thr Ala
            340                 345                 350

Leu Tyr Leu Ser Lys Thr Val Glu Lys Ser Gly Tyr Phe Glu Ile Ile
        355                 360                 365

Asn Asp Gly Ser Asn Leu Pro Ile Val Cys Tyr Lys Leu Lys Asp Asp
    370                 375                 380

Leu Asp Val Glu Trp Thr Leu Tyr Asp Leu Ala Asp Gln Leu Leu Met
385                 390                 395                 400

Lys Gly Trp Gln Val Pro Ala Tyr Pro Leu Pro Ala Asp Leu Ser Asp
                405                 410                 415

Thr Ile Ile Gln Arg Phe Val Cys Arg Ala Asp Leu Gly Tyr Asn Val
            420                 425                 430

Ala Glu Glu Phe Ala Ala Asp Phe Ala Asp Ala Leu His Asn Leu Glu
        435                 440                 445

His Ala Arg Val Leu Tyr His Asp Lys Glu Arg Asn Asp Ser Tyr Gly
    450                 455                 460

Phe Thr Ala
465

<210>  23
<211>  462
<212>  PRT
<213>  Artificial Sequence

<220>
<223>  L. monocytogenes GAD mutant E85Q

<400>  23

Met Phe Lys Thr Asn Val Glu Gln Asn Asn Val Pro Val Phe Gly Ser
1               5                   10                  15

Phe Glu Ser Gly Gln Asp Leu Pro Glu Lys Arg Met Asn Lys Glu Ser
            20                  25                  30

Val Asp Pro Arg Ile Ala Tyr Gln Leu Val Lys Asp Gln Leu Ile Asp
        35                  40                  45

Glu Gly Ser Ala Arg Gln Asn Leu Ala Thr Phe Cys Gln Thr Tyr Met
    50                  55                  60

Glu Pro Glu Ala Glu Gln Ile Met Ala Glu Thr Met Glu Lys Asn Ala
65                  70                  75                  80

Ile Asp Lys Ser Gln Tyr Pro Gln Thr Ala Lys Leu Glu Ser Ser Cys
                85                  90                  95

Val Asn Met Leu Ala Asp Leu Trp Asn Val Asp Glu Ser Glu His Tyr
            100                 105                 110

Met Gly Thr Ser Thr Val Gly Ser Ser Glu Ala Cys Met Leu Gly Gly
        115                 120                 125

Met Ala Met Lys Phe Arg Trp Arg Ser Ala Ala Leu Lys Asn Gly Leu
    130                 135                 140

Asp Ile His Ala Lys Lys Pro Ser Leu Val Ile Ser Ser Gly Tyr Gln
145                 150                 155                 160

Val Cys Trp Glu Lys Phe Cys Val Tyr Trp Asp Ile Glu Leu Arg Glu
                165                 170                 175

Val Pro Met Ser Glu Glu His Leu Ser Ile Asn Thr Asp Ile Ile Met
            180                 185                 190

Asp Tyr Val Asp Glu Tyr Thr Ile Gly Ile Val Gly Ile Leu Gly Ile
        195                 200                 205

Thr Tyr Thr Gly Lys Phe Asp Asp Ile Met Thr Leu Asn Asp Leu Val
    210                 215                 220

Glu Asp Tyr Asn Asn Thr His Asp Asn Glu Val Val Ile His Val Asp
225                 230                 235                 240

Gly Ala Ser Gly Ala Met Phe Thr Pro Phe Val Glu Pro Gly Leu Glu
                245                 250                 255

Trp Asp Phe Arg Leu Pro Asn Val Val Ser Ile Asn Thr Ser Gly His
            260                 265                 270

Lys Tyr Gly Leu Val Tyr Pro Gly Val Gly Trp Ile Leu Trp Arg Asp

275                 280                 285

Lys Glu Tyr Leu Pro Glu Glu Leu Val Phe Asp Val Ser Tyr Leu Gly
    290                 295                 300

Gly His Met Pro Thr Met Ala Ile Asn Phe Ser Arg Ser Ala Ser Gln
305                 310                 315                 320

Ile Ile Gly Gln Tyr Tyr Asn Phe Leu Arg Phe Gly Tyr Glu Gly Tyr
                325                 330                 335

Arg Gln Ile His Met Arg Thr Arg Asp Gly Ala Leu Gln Leu Ser Gln
            340                 345                 350

Ala Val Ala Glu Thr Gly Leu Phe Glu Ile Tyr Asn Asp Gly Ala Asn
        355                 360                 365

Leu Pro Ile Val Cys Tyr Lys Leu Lys Asp Asp Ala Asn Val Ala Trp
    370                 375                 380

Thr Leu Tyr Asp Leu Ala Asp Arg Leu Gln Met Lys Gly Trp Gln Val
385                 390                 395                 400

Pro Ala Tyr Pro Leu Pro Lys Glu Met Gly Asn Thr Ile Ile Gln Arg
                405                 410                 415

Tyr Val Cys Arg Gly Asp Leu Gly Gln Asn Met Val Thr Ala Phe Lys
            420                 425                 430

Asn Asp Leu Ser Glu Ser Ile Glu Glu Leu Asn Asn Ala His Ile Leu
        435                 440                 445

Tyr His Asp Val Asn Thr Ser Lys Thr His Gly Phe Thr His
    450                 455                 460

<210>  24
<211>  462
<212>  PRT
<213>  Artificial Sequence

<220>
<223>  L. monocytogenes GAD mutant H462A

<400>  24

Met Phe Lys Thr Asn Val Glu Gln Asn Asn Val Pro Val Phe Gly Ser
1               5                   10                  15

Phe Glu Ser Gly Gln Asp Leu Pro Glu Lys Arg Met Asn Lys Glu Ser
            20                  25                  30

Val Asp Pro Arg Ile Ala Tyr Gln Leu Val Lys Asp Gln Leu Ile Asp
        35                  40                  45

Glu Gly Ser Ala Arg Gln Asn Leu Ala Thr Phe Cys Gln Thr Tyr Met
    50                  55                  60

Glu Pro Glu Ala Glu Gln Ile Met Ala Glu Thr Met Glu Lys Asn Ala
65                  70                  75                  80

Ile Asp Lys Ser Glu Tyr Pro Gln Thr Ala Lys Leu Glu Ser Ser Cys
                85                  90                  95

Val Asn Met Leu Ala Asp Leu Trp Asn Val Asp Glu Ser Glu His Tyr
            100                 105                 110

Met Gly Thr Ser Thr Val Gly Ser Ser Glu Ala Cys Met Leu Gly Gly
        115                 120                 125

Met Ala Met Lys Phe Arg Trp Arg Ser Ala Ala Leu Lys Asn Gly Leu
    130                 135                 140

Asp Ile His Ala Lys Lys Pro Ser Leu Val Ile Ser Ser Gly Tyr Gln
145                 150                 155                 160

Val Cys Trp Glu Lys Phe Cys Val Tyr Trp Asp Ile Glu Leu Arg Glu
                165                 170                 175

Val Pro Met Ser Glu Glu His Leu Ser Ile Asn Thr Asp Ile Ile Met
            180                 185                 190

Asp Tyr Val Asp Glu Tyr Thr Ile Gly Ile Val Gly Ile Leu Gly Ile
        195                 200                 205

Thr Tyr Thr Gly Lys Phe Asp Asp Ile Met Thr Leu Asn Asp Leu Val
    210                 215                 220

Glu Asp Tyr Asn Asn Thr His Asp Asn Glu Val Val Ile His Val Asp
225                 230                 235                 240

Gly Ala Ser Gly Ala Met Phe Thr Pro Phe Val Glu Pro Gly Leu Glu
                245                 250                 255

Trp Asp Phe Arg Leu Pro Asn Val Val Ser Ile Asn Thr Ser Gly His
            260                 265                 270

Lys Tyr Gly Leu Val Tyr Pro Gly Val Gly Trp Ile Leu Trp Arg Asp

275                 280                 285

Lys Glu Tyr Leu Pro Glu Glu Leu Val Phe Asp Val Ser Tyr Leu Gly
    290                 295                 300

Gly His Met Pro Thr Met Ala Ile Asn Phe Ser Arg Ser Ala Ser Gln
305                 310                 315                 320

Ile Ile Gly Gln Tyr Tyr Asn Phe Leu Arg Phe Gly Tyr Glu Gly Tyr
                325                 330                 335

Arg Gln Ile His Met Arg Thr Arg Asp Gly Ala Leu Gln Leu Ser Gln
            340                 345                 350

Ala Val Ala Glu Thr Gly Leu Phe Glu Ile Tyr Asn Asp Gly Ala Asn
        355                 360                 365

Leu Pro Ile Val Cys Tyr Lys Leu Lys Asp Asp Ala Asn Val Ala Trp
    370                 375                 380

Thr Leu Tyr Asp Leu Ala Asp Arg Leu Gln Met Lys Gly Trp Gln Val
385                 390                 395                 400

Pro Ala Tyr Pro Leu Pro Lys Glu Met Gly Asn Thr Ile Ile Gln Arg
                405                 410                 415

Tyr Val Cys Arg Gly Asp Leu Gly Gln Asn Met Val Thr Ala Phe Lys
            420                 425                 430

Asn Asp Leu Ser Glu Ser Ile Glu Glu Leu Asn Asn Ala His Ile Leu
        435                 440                 445

Tyr His Asp Val Asn Thr Ser Lys Thr His Gly Phe Thr Ala
    450                 455                 460

<210>  25
<211>  462
<212>  PRT
<213>  Artificial Sequence

<220>
<223>  L. monocytogenes GAD mutant E85Q+H462A

<400>  25

Met Phe Lys Thr Asn Val Glu Gln Asn Asn Val Pro Val Phe Gly Ser
1               5                   10                  15

Phe Glu Ser Gly Gln Asp Leu Pro Glu Lys Arg Met Asn Lys Glu Ser
            20                  25                  30

Val Asp Pro Arg Ile Ala Tyr Gln Leu Val Lys Asp Gln Leu Ile Asp
        35                  40                  45

Glu Gly Ser Ala Arg Gln Asn Leu Ala Thr Phe Cys Gln Thr Tyr Met
    50                  55                  60

Glu Pro Glu Ala Glu Gln Ile Met Ala Glu Thr Met Glu Lys Asn Ala
65                  70                  75                  80

Ile Asp Lys Ser Gln Tyr Pro Gln Thr Ala Lys Leu Glu Ser Ser Cys
                85                  90                  95

Val Asn Met Leu Ala Asp Leu Trp Asn Val Asp Glu Ser Glu His Tyr
            100                 105                 110

Met Gly Thr Ser Thr Val Gly Ser Ser Glu Ala Cys Met Leu Gly Gly
        115                 120                 125

Met Ala Met Lys Phe Arg Trp Arg Ser Ala Ala Leu Lys Asn Gly Leu
    130                 135                 140

Asp Ile His Ala Lys Lys Pro Ser Leu Val Ile Ser Ser Gly Tyr Gln
145                 150                 155                 160

Val Cys Trp Glu Lys Phe Cys Val Tyr Trp Asp Ile Glu Leu Arg Glu
                165                 170                 175

Val Pro Met Ser Glu Glu His Leu Ser Ile Asn Thr Asp Ile Ile Met
            180                 185                 190

Asp Tyr Val Asp Glu Tyr Thr Ile Gly Ile Val Gly Ile Leu Gly Ile
        195                 200                 205

Thr Tyr Thr Gly Lys Phe Asp Asp Ile Met Thr Leu Asn Asp Leu Val
    210                 215                 220

Glu Asp Tyr Asn Asn Thr His Asp Asn Glu Val Val Ile His Val Asp
225                 230                 235                 240

Gly Ala Ser Gly Ala Met Phe Thr Pro Phe Val Glu Pro Gly Leu Glu
                245                 250                 255

Trp Asp Phe Arg Leu Pro Asn Val Val Ser Ile Asn Thr Ser Gly His
            260                 265                 270

Lys Tyr Gly Leu Val Tyr Pro Gly Val Gly Trp Ile Leu Trp Arg Asp

275                 280                 285

Lys Glu Tyr Leu Pro Glu Glu Leu Val Phe Asp Val Ser Tyr Leu Gly
    290                 295                 300

Gly His Met Pro Thr Met Ala Ile Asn Phe Ser Arg Ser Ala Ser Gln
305                 310                 315                 320

Ile Ile Gly Gln Tyr Tyr Asn Phe Leu Arg Phe Gly Tyr Glu Gly Tyr
                325                 330                 335

Arg Gln Ile His Met Arg Thr Arg Asp Gly Ala Leu Gln Leu Ser Gln
            340                 345                 350

Ala Val Ala Glu Thr Gly Leu Phe Glu Ile Tyr Asn Asp Gly Ala Asn
        355                 360                 365

Leu Pro Ile Val Cys Tyr Lys Leu Lys Asp Asp Ala Asn Val Ala Trp
    370                 375                 380

Thr Leu Tyr Asp Leu Ala Asp Arg Leu Gln Met Lys Gly Trp Gln Val
385                 390                 395                 400

Pro Ala Tyr Pro Leu Pro Lys Glu Met Gly Asn Thr Ile Ile Gln Arg
                405                 410                 415

Tyr Val Cys Arg Gly Asp Leu Gly Gln Asn Met Val Thr Ala Phe Lys
            420                 425                 430

Asn Asp Leu Ser Glu Ser Ile Glu Glu Leu Asn Asn Ala His Ile Leu
        435                 440                 445

Tyr His Asp Val Asn Thr Ser Lys Thr His Gly Phe Thr Ala
    450                 455                 460

<210>  26
<211>  466
<212>  PRT
<213>  Artificial Sequence

<220>
<223>  S. flexneri GAD mutant E89Q

<400>  26

Met Asp Gln Lys Leu Leu Thr Asp Phe Arg Ser Glu Leu Leu Asp Ser
1               5                   10                  15

Arg Phe Gly Ala Lys Ala Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Gln Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile

        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Gln Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

His Thr
465

<210> 27
<211> 466
<212> PRT
<213> Artificial Sequence

<220>
<223> S. flexneri GAD mutant H465A

<400> 27

Met Asp Gln Lys Leu Leu Thr Asp Phe Arg Ser Glu Leu Leu Asp Ser
1               5                   10                  15

Arg Phe Gly Ala Lys Ala Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Glu Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala

260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Gln Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

Ala Thr
465

<210>  28
<211>  466
<212>  PRT
<213>  Artificial Sequence

<220>
<223>  S. flexneri GAD mutant E89Q+H465A

<400>  28

Met Asp Gln Lys Leu Leu Thr Asp Phe Arg Ser Glu Leu Leu Asp Ser
1               5                   10                  15

Arg Phe Gly Ala Lys Ala Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Gln Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro

                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Gln Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu
        435                 440                 445

Ser Asp His Pro Lys Leu Gln Gly Ile Ala Gln Gln Asn Ser Phe Lys
    450                 455                 460

Ala Thr
465

<210> 29
<211> 466
<212> PRT
<213> Artificial Sequence

<220>
<223>  L. lactis subsp. lactis GAD mutant E89Q

<400>  29

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Gln Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Ile Tyr Trp Asp Ile
                165                 170                 175

Glu Met Arg Glu Val Pro Met Asp Lys Glu His Met Ser Ile Asn Leu
            180                 185                 190

Asp Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr

225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Phe Leu Ala Lys Glu Ile Glu Lys Thr Gly Met Phe Glu Ile Met Asn
        355                 360                 365

Asp Gly Ser Gln Leu Pro Ile Val Cys Tyr Lys Leu Lys Glu Asp Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Glu Ala Leu Asn Lys
        435                 440                 445

Ala His Ile Leu Tyr His Glu Glu Pro Glu Asn Lys Thr Tyr Gly Phe
    450                 455                 460

Thr His
465

<210> 30
<211> 466
<212> PRT
<213> Artificial Sequence

<220>
<223> L. lactis subsp. lactis GAD mutant H466A

<400> 30

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Glu Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Ile Tyr Trp Asp Ile
                165                 170                 175

Glu Met Arg Glu Val Pro Met Asp Lys Glu His Met Ser Ile Asn Leu
            180                 185                 190

Asp Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu

210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Phe Leu Ala Lys Glu Ile Glu Lys Thr Gly Met Phe Glu Ile Met Asn
        355                 360                 365

Asp Gly Ser Gln Leu Pro Ile Val Cys Tyr Lys Leu Lys Glu Asp Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Glu Ala Leu Asn Lys
        435                 440                 445

Ala His Ile Leu Tyr His Glu Glu Pro Glu Asn Lys Thr Tyr Gly Phe
    450                 455                 460

Thr Ala
465

<210> 31
<211> 466
<212> PRT
<213> Artificial Sequence

<220>
<223> L. lactis subsp. lactis GAD mutant E89Q+H466A

<400> 31

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Gln Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Ile Tyr Trp Asp Ile
                165                 170                 175

Glu Met Arg Glu Val Pro Met Asp Lys Glu His Met Ser Ile Asn Leu
            180                 185                 190

Asp Lys Val Met Asp Tyr Val Asp Glu Tyr Thr Ile Gly Val Val Gly

        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Phe Leu Ala Lys Glu Ile Glu Lys Thr Gly Met Phe Glu Ile Met Asn
        355                 360                 365

Asp Gly Ser Gln Leu Pro Ile Val Cys Tyr Lys Leu Lys Glu Asp Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Glu Ala Leu Asn Lys
        435                 440                 445

Ala His Ile Leu Tyr His Glu Glu Pro Glu Asn Lys Thr Tyr Gly Phe
    450                 455                 460

Thr Ala
465

<210>  32
<211>  466
<212>  PRT
<213>  Artificial Sequence

<220>
<223>  L. lactis subsp. cremoris GAD mutant E89Q

<400>  32

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Gln Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Val Tyr Trp Asp Ile
                165                 170                 175

Glu Met Arg Glu Val Pro Met Asp Arg Glu His Met Ser Ile Asn Leu

            180                 185                 190

Glu Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Tyr Leu Ala Glu Glu Ile Glu Lys Thr Gly Met Phe Glu Ile Met Asn
        355                 360                 365

Asp Gly Ala Gln Leu Pro Ile Val Cys Tyr Lys Leu Lys Glu Asn Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Asp Ala Leu Asn Lys
        435                 440                 445

Ala His Ile Leu Phe His Gln Glu Pro Glu Asn Lys Thr Tyr Gly Phe
    450                 455                 460

Thr His
465

<210>  33
<211>  466
<212>  PRT
<213>  Artificial Sequence

<220>
<223>  L. lactis subsp. cremoris GAD mutant H466A

<400>  33

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Glu Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser
145                 150                 155                 160

Ser Gly Tyr Gln Val Cys Trp Glu Lys Phe Cys Val Tyr Trp Asp Ile

                165                 170                 175

Glu Met Arg Glu Val Pro Met Asp Arg Glu His Met Ser Ile Asn Leu
            180                 185                 190

Glu Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Tyr Leu Ala Glu Glu Ile Glu Lys Thr Gly Met Phe Glu Ile Met Asn
        355                 360                 365

Asp Gly Ala Gln Leu Pro Ile Val Cys Tyr Lys Leu Lys Glu Asn Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Asp Ala Leu Asn Lys
        435                 440                 445

Ala His Ile Leu Phe His Gln Glu Pro Glu Asn Lys Thr Tyr Gly Phe
    450                 455                 460

Thr Ala
465

<210>  34
<211>  466
<212>  PRT
<213>  Artificial Sequence

<220>
<223>  L. lactis subsp. cremoris GAD mutant E89Q+H466A

<400>  34

Met Leu Tyr Gly Lys Glu Asn Arg Asp Glu Ala Glu Phe Leu Glu Pro
1               5                   10                  15

Ile Phe Gly Ser Glu Ser Glu Gln Val Asp Leu Pro Lys Tyr Lys Leu
            20                  25                  30

Ala Gln Gln Ser Ile Glu Pro Arg Val Ala Tyr Gln Leu Val Gln Asp
        35                  40                  45

Glu Met Leu Asp Glu Gly Asn Ala Arg Leu Asn Leu Ala Thr Phe Cys
    50                  55                  60

Gln Thr Tyr Met Glu Pro Glu Ala Val Lys Leu Met Ser Gln Thr Leu
65                  70                  75                  80

Glu Lys Asn Ala Ile Asp Lys Ser Gln Tyr Pro Arg Thr Thr Glu Ile
                85                  90                  95

Glu Asn Arg Cys Val Asn Met Ile Ala Asp Leu Trp Asn Ala Ser Glu
            100                 105                 110

Lys Glu Lys Phe Met Gly Thr Ser Thr Ile Gly Ser Ser Glu Ala Cys
        115                 120                 125

Met Leu Gly Gly Met Ala Met Lys Phe Ser Trp Arg Lys Arg Ala Glu
    130                 135                 140

Lys Leu Gly Leu Asp Ile Asn Ala Lys Lys Pro Asn Leu Val Ile Ser

145                 150                 155                 160

Ser Gly Tyr Gln Val Cys Trp Glu Lys Phe Cys Val Tyr Trp Asp Ile
                165                 170                 175

Glu Met Arg Glu Val Pro Met Asp Arg Glu His Met Ser Ile Asn Leu
            180                 185                 190

Glu Lys Val Met Asp Tyr Val Asp Glu Tyr Thr Ile Gly Val Val Gly
        195                 200                 205

Ile Met Gly Ile Thr Tyr Thr Gly Arg Tyr Asp Asp Ile Lys Ala Leu
    210                 215                 220

Asp Asn Leu Ile Glu Glu Tyr Asn Lys Gln Thr Asp Tyr Lys Val Tyr
225                 230                 235                 240

Ile His Val Asp Ala Ala Ser Gly Gly Leu Tyr Ala Pro Phe Val Glu
                245                 250                 255

Pro Glu Leu Glu Trp Asp Phe Arg Leu Lys Asn Val Ile Ser Ile Asn
            260                 265                 270

Thr Ser Gly His Lys Tyr Gly Leu Val Tyr Pro Gly Val Gly Trp Val
        275                 280                 285

Leu Trp Arg Asp Lys Lys Tyr Leu Pro Glu Glu Leu Ile Phe Lys Val
    290                 295                 300

Ser Tyr Leu Gly Gly Glu Leu Pro Thr Met Ala Ile Asn Phe Ser His
305                 310                 315                 320

Ser Ala Ser Gln Leu Ile Gly Gln Tyr Tyr Asn Phe Val Arg Tyr Gly
                325                 330                 335

Phe Asp Gly Tyr Lys Ala Ile His Glu Arg Thr His Lys Val Ala Met
            340                 345                 350

Tyr Leu Ala Glu Glu Ile Glu Lys Thr Gly Met Phe Glu Ile Met Asn
        355                 360                 365

Asp Gly Ala Gln Leu Pro Ile Val Cys Tyr Lys Leu Lys Glu Asn Ser
    370                 375                 380

Asn Arg Gly Trp Asn Leu Tyr Asp Leu Ala Asp Arg Leu Leu Met Lys
385                 390                 395                 400

Gly Trp Gln Val Pro Ala Tyr Pro Leu Pro Lys Asn Leu Glu Asn Glu
                405                 410                 415

Ile Ile Gln Arg Leu Val Ile Arg Ala Asp Phe Gly Met Asn Met Ala
            420                 425                 430

Phe Asn Tyr Val Gln Asp Met Gln Glu Ala Ile Asp Ala Leu Asn Lys
        435                 440                 445

Ala His Ile Leu Phe His Gln Glu Pro Glu Asn Lys Thr Tyr Gly Phe
    450                 455                 460

Thr Ala
465

<210>  35
<211>  464
<212>  PRT
<213>  Artificial Sequence

<220>
<223>  A. fischeri GAD mutant E89Q

<400>  35

Met Pro Leu His Ser Lys Asn Ala Val Arg Asp Asp Leu Leu Asp Asp
1               5                   10                  15

Ile Tyr Ser Ser Ala Asp Leu Ser Leu Ser Met Pro Lys Tyr Lys Met
            20                  25                  30

Pro Glu Gln Glu His Asp Pro Arg His Ala Tyr Gln Val Ile His Asp
        35                  40                  45

Glu Leu Met Met Asp Gly Asn Ser Arg Gln Asn Leu Ala Thr Phe Cys
    50                  55                  60

Gln Thr Trp Val Glu Asp Glu Val His Lys Leu Met Asp Glu Cys Ile
65                  70                  75                  80

Asp Lys Asn Met Ile Asp Lys Asp Gln Tyr Pro Gln Thr Ala Glu Leu
                85                  90                  95

Glu Ser Arg Cys Val His Met Leu Ala Asp Leu Trp Asn Ser Pro Asp
            100                 105                 110

Ala Glu Asn Thr Leu Gly Cys Ser Thr Thr Gly Ser Ser Glu Ala Ala
        115                 120                 125

Met Leu Gly Gly Met Ala Leu Lys Trp Ala Trp Arg Glu Lys Met Lys

130                 135                 140

Lys Leu Gly Lys Pro Thr Asp Lys Pro Asn Met Ile Cys Gly Pro Val
145                 150                 155                 160

Gln Val Cys Trp His Lys Phe Ala Arg Tyr Trp Asp Ile Glu Leu Arg
                165                 170                 175

Glu Ile Pro Met Glu Gly Asp Arg Leu Ile Met Thr Pro Glu Glu Val
            180                 185                 190

Ile Lys Arg Cys Asp Glu Asn Thr Ile Gly Val Val Pro Thr Leu Gly
        195                 200                 205

Val Thr Phe Thr Cys Gln Tyr Glu Pro Val Lys Ala Val His Glu Ala
    210                 215                 220

Leu Asp Lys Leu Gln Glu Glu Thr Gly Leu Asp Ile Pro Met His Ile
225                 230                 235                 240

Asp Ala Ala Ser Gly Gly Phe Leu Ala Pro Phe Cys Asp Pro Asp Leu
                245                 250                 255

Glu Trp Asp Phe Arg Leu Pro Arg Val Lys Ser Ile Asn Ala Ser Gly
            260                 265                 270

His Lys Phe Gly Leu Ser Pro Leu Gly Val Gly Trp Val Ile Trp Arg
        275                 280                 285

Asp Ala Ser Ala Leu His Glu Asp Leu Ile Phe Asn Val Asn Tyr Leu
    290                 295                 300

Gly Gly Asn Met Pro Thr Phe Ala Leu Asn Phe Ser Arg Pro Gly Gly
305                 310                 315                 320

Gln Ile Val Ala Gln Tyr Tyr Asn Phe Leu Arg Leu Gly Lys Glu Gly
                325                 330                 335

Tyr Arg Lys Ile His Gln Ala Cys Tyr Asp Thr Ala Val Tyr Leu Ser
            340                 345                 350

Ser Glu Ile Glu Lys Leu Gly Met Phe Glu Ile Ile Tyr Asp Gly Lys
        355                 360                 365

Gly Gly Ile Pro Ala Met Ser Trp Ser Leu Lys Glu Gly Val Asp Pro
    370                 375                 380

Gly Phe Asn Leu Phe Asp Leu Ser Asp Arg Ile Arg Ser Arg Gly Trp
385                 390                 395                 400

Gln Ile Ala Ala Tyr Ala Met Pro Pro Lys Arg Glu Asp Leu Val Ile
                405                 410                 415

Met Arg Ile Leu Val Arg His Gly Phe Ser Arg Asp Gln Ala Asp Leu
            420                 425                 430

Leu Val Ala Asp Leu Lys His Cys Val Glu Phe Phe Ala Lys His Pro
        435                 440                 445

Ile Ser His Gly Ser Asp Glu Leu Glu Ser Ser Gly Phe Asn His Gly
    450                 455                 460

<210>  36
<211>  464
<212>  PRT
<213>  Artificial Sequence

<220>
<223>  A. fischeri GAD mutant H463A

<400>  36

Met Pro Leu His Ser Lys Asn Ala Val Arg Asp Asp Leu Leu Asp Asp
1               5                   10                  15

Ile Tyr Ser Ser Ala Asp Leu Ser Leu Ser Met Pro Lys Tyr Lys Met
            20                  25                  30

Pro Glu Gln Glu His Asp Pro Arg His Ala Tyr Gln Val Ile His Asp
        35                  40                  45

Glu Leu Met Met Asp Gly Asn Ser Arg Gln Asn Leu Ala Thr Phe Cys
    50                  55                  60

Gln Thr Trp Val Glu Asp Glu Val His Lys Leu Met Asp Glu Cys Ile
65                  70                  75                  80

Asp Lys Asn Met Ile Asp Lys Asp Glu Tyr Pro Gln Thr Ala Glu Leu
                85                  90                  95

Glu Ser Arg Cys Val His Met Leu Ala Asp Leu Trp Asn Ser Pro Asp
            100                 105                 110

Ala Glu Asn Thr Leu Gly Cys Ser Thr Thr Gly Ser Ser Glu Ala Ala
        115                 120                 125

Met Leu Gly Gly Met Ala Leu Lys Trp Ala Trp Arg Glu Lys Met Lys

130                 135                 140

Lys Leu Gly Lys Pro Thr Asp Lys Pro Asn Met Ile Cys Gly Pro Val
145                 150                 155                 160

Gln Val Cys Trp His Lys Phe Ala Arg Tyr Trp Asp Ile Glu Leu Arg
                165                 170                 175

Glu Ile Pro Met Glu Gly Asp Arg Leu Ile Met Thr Pro Glu Glu Val
            180                 185                 190

Ile Lys Arg Cys Asp Glu Asn Thr Ile Gly Val Val Pro Thr Leu Gly
        195                 200                 205

Val Thr Phe Thr Cys Gln Tyr Glu Pro Val Lys Ala Val His Glu Ala
    210                 215                 220

Leu Asp Lys Leu Gln Glu Glu Thr Gly Leu Asp Ile Pro Met His Ile
225                 230                 235                 240

Asp Ala Ala Ser Gly Gly Phe Leu Ala Pro Phe Cys Asp Pro Asp Leu
                245                 250                 255

Glu Trp Asp Phe Arg Leu Pro Arg Val Lys Ser Ile Asn Ala Ser Gly
            260                 265                 270

His Lys Phe Gly Leu Ser Pro Leu Gly Val Gly Trp Val Ile Trp Arg
        275                 280                 285

Asp Ala Ser Ala Leu His Glu Asp Leu Ile Phe Asn Val Asn Tyr Leu
    290                 295                 300

Gly Gly Asn Met Pro Thr Phe Ala Leu Asn Phe Ser Arg Pro Gly Gly
305                 310                 315                 320

Gln Ile Val Ala Gln Tyr Tyr Asn Phe Leu Arg Leu Gly Lys Glu Gly
                325                 330                 335

Tyr Arg Lys Ile His Gln Ala Cys Tyr Asp Thr Ala Val Tyr Leu Ser
            340                 345                 350

Ser Glu Ile Glu Lys Leu Gly Met Phe Glu Ile Ile Tyr Asp Gly Lys
        355                 360                 365

Gly Gly Ile Pro Ala Met Ser Trp Ser Leu Lys Glu Gly Val Asp Pro
    370                 375                 380

Gly Phe Asn Leu Phe Asp Leu Ser Asp Arg Ile Arg Ser Arg Gly Trp
385                 390                 395                 400

Gln Ile Ala Ala Tyr Ala Met Pro Pro Lys Arg Glu Asp Leu Val Ile
                405                 410                 415

Met Arg Ile Leu Val Arg His Gly Phe Ser Arg Asp Gln Ala Asp Leu
            420                 425                 430

Leu Val Ala Asp Leu Lys His Cys Val Glu Phe Phe Ala Lys His Pro
        435                 440                 445

Ile Ser His Gly Ser Asp Glu Leu Glu Ser Ser Gly Phe Asn Ala Gly
    450                 455                 460

<210>  37
<211>  464
<212>  PRT
<213>  Artificial Sequence

<220>
<223>  A. fischeri GAD mutant E89Q+H463A

<400>  37

Met Pro Leu His Ser Lys Asn Ala Val Arg Asp Asp Leu Leu Asp Asp
1               5                   10                  15

Ile Tyr Ser Ser Ala Asp Leu Ser Leu Ser Met Pro Lys Tyr Lys Met
            20                  25                  30

Pro Glu Gln Glu His Asp Pro Arg His Ala Tyr Gln Val Ile His Asp
        35                  40                  45

Glu Leu Met Met Asp Gly Asn Ser Arg Gln Asn Leu Ala Thr Phe Cys
    50                  55                  60

Gln Thr Trp Val Glu Asp Glu Val His Lys Leu Met Asp Glu Cys Ile
65                  70                  75                  80

Asp Lys Asn Met Ile Asp Lys Asp Gln Tyr Pro Gln Thr Ala Glu Leu
                85                  90                  95

Glu Ser Arg Cys Val His Met Leu Ala Asp Leu Trp Asn Ser Pro Asp
            100                 105                 110

Ala Glu Asn Thr Leu Gly Cys Ser Thr Thr Gly Ser Ser Glu Ala Ala
        115                 120                 125

Met Leu Gly Gly Met Ala Leu Lys Trp Ala Trp Arg Glu Lys Met Lys

130                 135                 140

Lys Leu Gly Lys Pro Thr Asp Lys Pro Asn Met Ile Cys Gly Pro Val
145                 150                 155                 160

Gln Val Cys Trp His Lys Phe Ala Arg Tyr Trp Asp Ile Glu Leu Arg
                165                 170                 175

Glu Ile Pro Met Glu Gly Asp Arg Leu Ile Met Thr Pro Glu Glu Val
            180                 185                 190

Ile Lys Arg Cys Asp Glu Asn Thr Ile Gly Val Val Pro Thr Leu Gly
        195                 200                 205

Val Thr Phe Thr Cys Gln Tyr Glu Pro Val Lys Ala Val His Glu Ala
    210                 215                 220

Leu Asp Lys Leu Gln Glu Glu Thr Gly Leu Asp Ile Pro Met His Ile
225                 230                 235                 240

Asp Ala Ala Ser Gly Gly Phe Leu Ala Pro Phe Cys Asp Pro Asp Leu
                245                 250                 255

Glu Trp Asp Phe Arg Leu Pro Arg Val Lys Ser Ile Asn Ala Ser Gly
            260                 265                 270

His Lys Phe Gly Leu Ser Pro Leu Gly Val Gly Trp Val Ile Trp Arg
        275                 280                 285

Asp Ala Ser Ala Leu His Glu Asp Leu Ile Phe Asn Val Asn Tyr Leu
    290                 295                 300

Gly Gly Asn Met Pro Thr Phe Ala Leu Asn Phe Ser Arg Pro Gly Gly
305                 310                 315                 320

Gln Ile Val Ala Gln Tyr Tyr Asn Phe Leu Arg Leu Gly Lys Glu Gly
                325                 330                 335

Tyr Arg Lys Ile His Gln Ala Cys Tyr Asp Thr Ala Val Tyr Leu Ser
            340                 345                 350

Ser Glu Ile Glu Lys Leu Gly Met Phe Glu Ile Ile Tyr Asp Gly Lys
        355                 360                 365

Gly Gly Ile Pro Ala Met Ser Trp Ser Leu Lys Glu Gly Val Asp Pro
    370                 375                 380

Gly Phe Asn Leu Phe Asp Leu Ser Asp Arg Ile Arg Ser Arg Gly Trp
385                 390                 395                 400

Gln Ile Ala Ala Tyr Ala Met Pro Pro Lys Arg Glu Asp Leu Val Ile
                405                 410                 415

Met Arg Ile Leu Val Arg His Gly Phe Ser Arg Asp Gln Ala Asp Leu
            420                 425                 430

Leu Val Ala Asp Leu Lys His Cys Val Glu Phe Phe Ala Lys His Pro
        435                 440                 445

Ile Ser His Gly Ser Asp Glu Leu Glu Ser Ser Gly Phe Asn Ala Gly
    450                 455                 460

<210>  38
<211>  468
<212>  PRT
<213>  Artificial Sequence

<220>
<223>  L. brevis GAD 4AA mutant

<400>  38

Met Ala Met Leu Tyr Gly Lys His Thr His Glu Thr Asp Glu Thr Leu
1               5                   10                  15

Ile Pro Ile Phe Gly Ala Thr Ala Glu Arg His Asp Leu Pro Lys Tyr
            20                  25                  30

Lys Leu Ala Lys His Ala Leu Glu Pro Arg Glu Ala Asp Arg Leu Val
        35                  40                  45

Arg Asp Gln Leu Leu Asp Glu Gly Asn Ser Arg Leu Asn Leu Ala Thr
    50                  55                  60

Phe Cys Gln Thr Tyr Met Glu Pro Glu Ala Val Glu Leu Met Lys Asp
65                  70                  75                  80

Thr Leu Glu Lys Asn Ala Ile Asp Lys Ser Glu Tyr Pro Arg Thr Ala
                85                  90                  95

Glu Ile Glu Asn Arg Cys Val Asn Ile Ile Ala Asn Leu Trp His Ala
            100                 105                 110

Pro Glu Ala Glu Ser Phe Thr Gly Thr Ser Thr Ile Gly Ser Ser Glu
        115                 120                 125

Ala Cys Met Leu Ala Gly Leu Ala Met Lys Phe Ala Trp Arg Lys Arg

130                 135                 140

Ala Lys Ala Asn Gly Leu Asp Leu Thr Ala His Gln Pro Asn Ile Val
145                 150                 155                 160

Ile Ser Ala Gly Tyr Gln Val Cys Trp Glu Lys Phe Cys Val Tyr Trp
                165                 170                 175

Asp Ile Asp Met His Val Val Pro Met Asp Asp Asp His Met Ser Leu
            180                 185                 190

Asn Val Asp His Val Leu Asp Tyr Val Asp Asp Tyr Thr Ile Gly Ile
        195                 200                 205

Val Gly Ile Met Gly Ile Thr Tyr Thr Gly Gln Tyr Asp Asp Leu Ala
    210                 215                 220

Arg Leu Asp Ala Val Val Glu Arg Tyr Asn Arg Thr Thr Lys Phe Pro
225                 230                 235                 240

Val Tyr Ile His Val Asp Ala Ala Ser Gly Gly Phe Tyr Thr Pro Phe
                245                 250                 255

Ile Glu Pro Glu Leu Lys Trp Asp Phe Arg Leu Asn Asn Val Ile Ser
            260                 265                 270

Ile Asn Ala Ser Gly His Lys Tyr Gly Leu Val Tyr Pro Gly Val Gly
        275                 280                 285

Trp Val Ile Trp Arg Gly Gln Gln Tyr Leu Pro Lys Glu Leu Val Phe
    290                 295                 300

Lys Val Ser Tyr Leu Gly Gly Ser Leu Pro Thr Met Ala Ile Asn Phe
305                 310                 315                 320

Ser His Ser Ala Ser Gln Leu Ile Gly Gln Tyr Tyr Asn Phe Ile Arg
                325                 330                 335

Phe Gly Phe Asp Gly Tyr Arg Glu Ile His Glu Lys Thr His Asp Val
            340                 345                 350

Ala Arg Tyr Leu Ala Lys Ser Leu Thr Lys Leu Gly Gly Phe Ser Leu
        355                 360                 365

Ile Asn Asp Gly His Glu Leu Pro Leu Ile Cys Tyr Glu Leu Thr Ala
    370                 375                 380

Asp Ser Asp Arg Glu Trp Thr Leu Tyr Asp Leu Ser Asp Arg Leu Leu
385                 390                 395                 400

Met Lys Gly Trp Gln Val Pro Thr Tyr Pro Leu Pro Lys Asn Met Thr
                405                 410                 415

Asp Arg Val Ile Gln Arg Ile Val Val Arg Ala Asp Phe Gly Met Ser
            420                 425                 430

Met Ala His Asp Phe Ile Asp Asp Leu Thr Gln Ala Ile His Asp Leu
        435                 440                 445

Asp Gln Ala His Ile Val Phe His Ser Asp Pro Gln Pro Lys Lys Tyr
    450                 455                 460

Gly Phe Thr His
465

<210>  39
<211>  614
<212>  PRT
<213>  Euglena gracilis

<400>  39

Met Gly Ile Ala Leu Asp Gly Pro Asp Arg Asp Arg Met Phe Asn Ala
1               5                   10                  15

Tyr Leu His Ser Ser Arg Pro Ser Phe Gly Pro Ala Thr Asp Val Phe
            20                  25                  30

Val Gln Cys Leu Glu Ala Glu Lys Ile Asp Trp Ile Phe Gly Ile Pro
        35                  40                  45

Gly Glu Glu Asn Leu Asp Leu Leu His Ser Leu Ala Lys Ala His Arg
    50                  55                  60

Arg Glu Gly Gly Glu Thr Met Lys Leu Val Val Thr Arg His Glu Gln
65                  70                  75                  80

Ala Ala Gly Phe Met Ala Ala Thr Val Gly Arg Leu Thr Gly His Pro
                85                  90                  95

Ala Ala Cys Leu Ser Thr Leu Gly Pro Gly Ala Thr Asn Phe Thr Thr
            100                 105                 110

Ala Leu Ala Tyr Ser Lys Leu Gly Gly Phe Pro Leu Ile Cys Ile Thr
        115                 120                 125

Gly Gln Lys Pro Ile Arg His Ser Lys Gln Gly Ala Phe Gln Ile Leu
    130                 135                 140

Asp Ile Cys Gly His Phe Arg Asp Val Val Lys Ser Thr Lys Ser Ile
145                 150                 155                 160

Glu Asp Pro Asp Leu Ile Pro Ser Thr Ile Arg Asn Ala Val Leu Gln
                165                 170                 175

Ala Arg Glu Glu Lys Pro Gly Pro Val His Ile Glu Leu Ala Glu Asp
            180                 185                 190

Ile Ala Ala Leu Glu Val Ser Lys Glu Gly Gly Trp Val Phe Pro Ala
        195                 200                 205

Pro Thr Arg Thr Pro Ile Ala Arg Gly Arg Arg Pro Leu Val Glu Pro
    210                 215                 220

Lys Cys Ile Glu His Ala Val Thr Leu Ile His Ala Ala Lys Arg Pro
225                 230                 235                 240

Leu Ile Cys Ile Ala Ala Gly Ala Asn Arg Lys Asn Thr His His Ala
                245                 250                 255

Met Glu Val Leu Met Arg Lys Thr Gly Leu Met Val Val Cys Thr Gln
            260                 265                 270

Met Gly Lys Gly Val Val Tyr Glu Asp Asn Leu Gly Tyr Ile Gly Cys
        275                 280                 285

Thr Ala Leu Ser Asp Lys Asp Leu Val His Val Ala Ile Asn Thr Ala
    290                 295                 300

Asp Leu Ile Val Asn Val Gly His Asp Ile Ser Glu Lys Pro Pro Phe
305                 310                 315                 320

Ile Met Asn His Asn Lys Pro Pro Leu Val Ile His Ala Asn Phe Thr
                325                 330                 335

Arg Pro Leu Val Asp Pro Val Tyr Phe Pro His Leu Val Leu Val Gly
            340                 345                 350

Asp Ile Asn Asp Cys Leu Trp Gln Leu Ala Thr Arg Ile Arg Pro Gln
        355                 360                 365

Pro His Trp Asp Phe Asn Pro Phe Gln Met Val Arg Arg Glu Ile Glu
    370                 375                 380

Arg Thr Val Trp Asn Ser Pro Tyr Ala Gln Ser Asp Ala Phe Pro Leu
385                 390                 395                 400

Thr Val Gln Arg Leu Val Ser Asp Val Arg Lys Ala Met Pro Val Asp
                405                 410                 415

Gly Ile Val Ser Leu Asp Asn Gly Met Tyr Lys Ile Trp Phe Ala Arg
            420                 425                 430

Gln Tyr Lys Thr Met Met Gly Asn Thr Leu Leu Leu Asp Asn Ala Leu
        435                 440                 445

Ala Thr Met Gly Ala Gly Leu Pro Ser Cys Ile Ala Ala Lys Leu Val
    450                 455                 460

Tyr Pro Glu Arg Val Cys Ile Ala Val Cys Gly Asp Gly Gly Phe Met
465                 470                 475                 480

Met Asn Ser Gln Glu Ile Glu Thr Ala Leu Arg Leu Asn Leu His Ile
                485                 490                 495

Val Val Ile Val Leu Asn Asn Asn Ser Tyr Gly Met Ile Ala Trp Lys
            500                 505                 510

Ala Thr Ala Met Gly Met Asp Asp Phe Gly Leu Asn Tyr Gly Asn Pro
        515                 520                 525

Asp Phe Ala Gln Tyr Ala Arg Ala Tyr Gly Ala Ile Gly His Asn Val
    530                 535                 540

Lys Ser Thr Ala Glu Phe Leu Pro Thr Leu Glu Lys Ala Ile Lys Glu
545                 550                 555                 560

His Gly Val His Ile Ile Asp Leu Pro Ile Ser Tyr Glu Thr Ser Asp
                565                 570                 575

Lys Ala Leu Phe Glu Asp Leu Pro Lys Glu Val Glu Glu Leu Lys Lys
            580                 585                 590

Ala Val Ala Lys Ala Ile Ser Glu Glu Lys Lys Phe Asp Trp Asp Ala
        595                 600                 605

Val Thr Ala Ala Gln Ser
    610

<210>  40
<211>  545

<212>  PRT
<213>  Synechococcus sp.

<400>  40

Met Asn Thr Ala Glu Leu Leu Ile Arg Cys Leu Glu Asn Glu Gly Val
1               5                   10                  15

Glu Tyr Ile Phe Gly Leu Pro Gly Glu Glu Asn Leu His Ile Leu Glu
            20                  25                  30

Ala Leu Lys Glu Ser Pro Ile Arg Phe Ile Thr Val Arg His Glu Gln
        35                  40                  45

Gly Ala Ala Phe Met Ala Asp Val Tyr Gly Arg Leu Thr Gly Lys Ala
    50                  55                  60

Gly Val Cys Leu Ser Thr Leu Gly Pro Gly Ala Thr Asn Leu Met Thr
65                  70                  75                  80

Gly Val Ala Asp Ala Asn Leu Asp Gly Ala Pro Leu Ile Ala Ile Thr
                85                  90                  95

Gly Gln Val Gly Thr Asp Arg Met His Ile Glu Ser His Gln Tyr Leu
            100                 105                 110

Asp Leu Val Ala Met Phe Ala Pro Val Thr Lys Trp Asn Lys Gln Ile
        115                 120                 125

Val Arg Pro Asn Thr Thr Pro Glu Val Val Arg Arg Ala Phe Lys Ile
    130                 135                 140

Ala Gln Gln Glu Lys Pro Gly Ala Val His Ile Asp Leu Pro Glu Asn
145                 150                 155                 160

Ile Ala Ala Met Pro Val Glu Gly Gln Pro Leu Gln Arg Asp Gly Arg
                165                 170                 175

Glu Lys Ile Tyr Ala Ser Ser Arg Ser Leu Asn Arg Ala Ala Glu Ala
            180                 185                 190

Ile Ala His Ala Lys Ser Pro Leu Ile Leu Val Gly Asn Gly Ile Ile
        195                 200                 205

Arg Ala Asp Ala Ala Glu Ala Leu Thr Asp Phe Ala Thr Gln Leu Asn
    210                 215                 220

Ile Pro Val Val Asn Thr Phe Met Gly Lys Gly Ala Ile Pro Tyr Thr
225                 230                 235                 240

His Pro Leu Ser Leu Trp Thr Val Gly Leu Gln Gln Arg Asp Phe Val
                245                 250                 255

Thr Cys Ala Phe Glu Gln Ser Asp Leu Val Ile Ala Val Gly Tyr Asp
            260                 265                 270

Leu Ile Glu Tyr Ser Pro Lys Arg Trp Asn Pro Glu Gly Thr Thr Pro
        275                 280                 285

Ile Ile His Ile Gly Glu Val Ala Ala Glu Ile Asp Ser Ser Tyr Ile
    290                 295                 300

Pro Leu Thr Glu Val Val Gly Asp Ile Gly Asp Ala Leu Asn Glu Ile
305                 310                 315                 320

Arg Lys Arg Thr Asp Arg Glu Gly Lys Thr Ala Pro Lys Phe Leu Asn
                325                 330                 335

Val Arg Ala Glu Ile Arg Glu Asp Tyr Glu Arg His Gly Thr Asp Ala
            340                 345                 350

Ser Phe Pro Val Lys Pro Gln Lys Ile Ile Tyr Asp Leu Arg Gln Val
        355                 360                 365

Met Ala Pro Glu Asp Ile Val Ile Ser Asp Val Gly Ala His Lys Met
    370                 375                 380

Trp Met Ala Arg His Tyr His Cys Asp Arg Pro Asn Thr Cys Leu Ile
385                 390                 395                 400

Ser Asn Gly Phe Ala Ala Met Gly Ile Ala Ile Pro Gly Ala Val Ala
                405                 410                 415

Ala Lys Leu Val Tyr Pro Glu Lys Asn Val Val Ala Val Thr Gly Asp
            420                 425                 430

Gly Gly Phe Met Met Asn Cys Gln Glu Leu Glu Thr Ala Leu Arg Ile
        435                 440                 445

Gly Ala Asn Phe Val Thr Leu Ile Phe Asn Asp Gly Gly Tyr Gly Leu
    450                 455                 460

Ile Gly Trp Lys Gln Ile Asn Gln Phe Gly Ala Pro Ala Phe Val Glu
465                 470                 475                 480

Phe Gly Asn Pro Asp Phe Val Gln Phe Ala Glu Ser Met Gly Leu Lys

485                 490                 495

Gly Tyr Arg Ile Thr Ala Ala Ala Asp Leu Val Pro Thr Leu Lys Glu
            500                 505                 510

Ala Leu Ala Gln Asp Val Pro Ala Val Ile Asp Cys Pro Val Asp Tyr
        515                 520                 525

Ser Glu Asn Val Lys Phe Ser Gln Lys Ser Gly Asp Leu Ile Cys Arg
    530                 535                 540

Met
545

<210>  41
<211>  1231
<212>  PRT
<213>  M. tuberculosis

<400>  41

Met Ala Asn Ile Ser Ser Pro Phe Gly Gln Asn Glu Trp Leu Val Glu
1               5                   10                  15

Glu Met Tyr Arg Lys Phe Arg Asp Asp Pro Ser Ser Val Asp Pro Ser
            20                  25                  30

Trp His Glu Phe Leu Val Asp Tyr Ser Pro Glu Pro Thr Ser Gln Pro
        35                  40                  45

Ala Ala Glu Pro Thr Arg Val Thr Ser Pro Leu Val Ala Glu Arg Ala
    50                  55                  60

Ala Ala Ala Ala Pro Gln Ala Pro Pro Lys Pro Ala Asp Thr Ala Ala
65                  70                  75                  80

Ala Gly Asn Gly Val Val Ala Ala Leu Ala Ala Lys Thr Ala Val Pro
                85                  90                  95

Pro Pro Ala Glu Gly Asp Glu Val Ala Val Leu Arg Gly Ala Ala Ala
            100                 105                 110

Ala Val Val Lys Asn Met Ser Ala Ser Leu Glu Val Pro Thr Ala Thr
        115                 120                 125

Ser Val Arg Ala Val Pro Ala Lys Leu Leu Ile Asp Asn Arg Ile Val
    130                 135                 140

Ile Asn Asn Gln Leu Lys Arg Thr Arg Gly Gly Lys Ile Ser Phe Thr

145                 150                 155                 160

His Leu Leu Gly Tyr Ala Leu Val Gln Ala Val Lys Lys Phe Pro Asn
                165                 170                 175

Met Asn Arg His Tyr Thr Glu Val Asp Gly Lys Pro Thr Ala Val Thr
            180                 185                 190

Pro Ala His Thr Asn Leu Gly Leu Ala Ile Asp Leu Gln Gly Lys Asp
        195                 200                 205

Gly Lys Arg Ser Leu Val Val Ala Gly Ile Lys Arg Cys Glu Thr Met
    210                 215                 220

Arg Phe Ala Gln Phe Val Thr Ala Tyr Glu Asp Ile Val Arg Arg Ala
225                 230                 235                 240

Arg Asp Gly Lys Leu Thr Thr Glu Asp Phe Ala Gly Val Thr Ile Ser
                245                 250                 255

Leu Thr Asn Pro Gly Thr Ile Gly Thr Val His Ser Val Pro Arg Leu
            260                 265                 270

Met Pro Gly Gln Gly Ala Ile Ile Gly Val Gly Ala Met Glu Tyr Pro
        275                 280                 285

Ala Glu Phe Gln Gly Ala Ser Glu Glu Arg Ile Ala Glu Leu Gly Ile
    290                 295                 300

Gly Lys Leu Ile Thr Leu Thr Ser Thr Tyr Asp His Arg Ile Ile Gln
305                 310                 315                 320

Gly Ala Glu Ser Gly Asp Phe Leu Arg Thr Ile His Glu Leu Leu Leu
                325                 330                 335

Ser Asp Gly Phe Trp Asp Glu Val Phe Arg Glu Leu Ser Ile Pro Tyr
            340                 345                 350

Leu Pro Val Arg Trp Ser Thr Asp Asn Pro Asp Ser Ile Val Asp Lys
        355                 360                 365

Asn Ala Arg Val Met Asn Leu Ile Ala Ala Tyr Arg Asn Arg Gly His
    370                 375                 380

Leu Met Ala Asp Thr Asp Pro Leu Arg Leu Asp Lys Ala Arg Phe Arg
385                 390                 395                 400

Ser His Pro Asp Leu Glu Val Leu Thr His Gly Leu Thr Leu Trp Asp
                405                 410                 415

Leu Asp Arg Val Phe Lys Val Asp Gly Phe Ala Gly Ala Gln Tyr Lys
            420                 425                 430

Lys Leu Arg Asp Val Leu Gly Leu Leu Arg Asp Ala Tyr Cys Arg His
        435                 440                 445

Ile Gly Val Glu Tyr Ala His Ile Leu Asp Pro Glu Gln Lys Glu Trp
    450                 455                 460

Leu Glu Gln Arg Val Glu Thr Lys His Val Lys Pro Thr Val Ala Gln
465                 470                 475                 480

Gln Lys Tyr Ile Leu Ser Lys Leu Asn Ala Ala Glu Ala Phe Glu Thr
                485                 490                 495

Phe Leu Gln Thr Lys Tyr Val Gly Gln Lys Arg Phe Ser Leu Glu Gly
            500                 505                 510

Ala Glu Ser Val Ile Pro Met Met Asp Ala Ala Ile Asp Gln Cys Ala
        515                 520                 525

Glu His Gly Leu Asp Glu Val Val Ile Gly Met Pro His Arg Gly Arg
    530                 535                 540

Leu Asn Val Leu Ala Asn Ile Val Gly Lys Pro Tyr Ser Gln Ile Phe
545                 550                 555                 560

Thr Glu Phe Glu Gly Asn Leu Asn Pro Ser Gln Ala His Gly Ser Gly
                565                 570                 575

Asp Val Lys Tyr His Leu Gly Ala Thr Gly Leu Tyr Leu Gln Met Phe
            580                 585                 590

Gly Asp Asn Asp Ile Gln Val Ser Leu Thr Ala Asn Pro Ser His Leu
        595                 600                 605

Glu Ala Val Asp Pro Val Leu Glu Gly Leu Val Arg Ala Lys Gln Asp
    610                 615                 620

Leu Leu Asp His Gly Ser Ile Asp Ser Asp Gly Gln Arg Ala Phe Ser
625                 630                 635                 640

Val Val Pro Leu Met Leu His Gly Asp Ala Ala Phe Ala Gly Gln Gly
                645                 650                 655

Val Val Ala Glu Thr Leu Asn Leu Ala Asn Leu Pro Gly Tyr Arg Val
            660                 665                 670

Gly Gly Thr Ile His Ile Ile Val Asn Asn Gln Ile Gly Phe Thr Thr
        675                 680                 685

Ala Pro Glu Tyr Ser Arg Ser Ser Glu Tyr Cys Thr Asp Val Ala Lys
    690                 695                 700

Met Ile Gly Ala Pro Ile Phe His Val Asn Gly Asp Asp Pro Glu Ala
705                 710                 715                 720

Cys Val Trp Val Ala Arg Leu Ala Val Asp Phe Arg Gln Arg Phe Lys
                725                 730                 735

Lys Asp Val Val Ile Asp Met Leu Cys Tyr Arg Arg Arg Gly His Asn
            740                 745                 750

Glu Gly Asp Asp Pro Ser Met Thr Asn Pro Tyr Val Tyr Asp Val Val
        755                 760                 765

Asp Thr Lys Arg Gly Ala Arg Lys Ser Tyr Thr Glu Ala Leu Ile Gly
    770                 775                 780

Arg Gly Asp Ile Ser Met Lys Glu Ala Glu Asp Ala Leu Arg Asp Tyr
785                 790                 795                 800

Gln Gly Gln Leu Glu Arg Val Phe Asn Glu Val Arg Glu Leu Glu Lys
                805                 810                 815

His Gly Val Gln Pro Ser Glu Ser Val Glu Ser Asp Gln Met Ile Pro
            820                 825                 830

Ala Gly Leu Ala Thr Ala Val Asp Lys Ser Leu Leu Ala Arg Ile Gly
        835                 840                 845

Asp Ala Phe Leu Ala Leu Pro Asn Gly Phe Thr Ala His Pro Arg Val
    850                 855                 860

Gln Pro Val Leu Glu Lys Arg Arg Glu Met Ala Tyr Glu Gly Lys Ile
865                 870                 875                 880

Asp Trp Ala Phe Gly Glu Leu Leu Ala Leu Gly Ser Leu Val Ala Glu
                885                 890                 895

Gly Lys Leu Val Arg Leu Ser Gly Gln Asp Ser Arg Arg Gly Thr Phe
            900                 905                 910

Ser Gln Arg His Ser Val Leu Ile Asp Arg His Thr Gly Glu Glu Phe
        915                 920                 925

Thr Pro Leu Gln Leu Leu Ala Thr Asn Ser Asp Gly Ser Pro Thr Gly
    930                 935                 940

Gly Lys Phe Leu Val Tyr Asp Ser Pro Leu Ser Glu Tyr Ala Ala Val
945                 950                 955                 960

Gly Phe Glu Tyr Gly Tyr Thr Val Gly Asn Pro Asp Ala Val Val Leu
                965                 970                 975

Trp Glu Ala Gln Phe Gly Asp Phe Val Asn Gly Ala Gln Ser Ile Ile
            980                 985                 990

Asp Glu Phe Ile Ser Ser Gly Glu Ala Lys Trp Gly Gln Leu Ser Asn
        995                 1000                1005

Val Val Leu Leu Leu Pro His Gly His Glu Gly Gln Gly Pro Asp
    1010                1015                1020

His Thr Ser Ala Arg Ile Glu Arg Phe Leu Gln Leu Trp Ala Glu
    1025                1030                1035

Gly Ser Met Thr Ile Ala Met Pro Ser Thr Pro Ser Asn Tyr Phe
    1040                1045                1050

His Leu Leu Arg Arg His Ala Leu Asp Gly Ile Gln Arg Pro Leu
    1055                1060                1065

Ile Val Phe Thr Pro Lys Ser Met Leu Arg His Lys Ala Ala Val
    1070                1075                1080

Ser Glu Ile Lys Asp Phe Thr Glu Ile Lys Phe Arg Ser Val Leu
    1085                1090                1095

Glu Glu Pro Thr Tyr Glu Asp Gly Ile Gly Asp Arg Asn Lys Val
    1100                1105                1110

Ser Arg Ile Leu Leu Thr Ser Gly Lys Leu Tyr Tyr Glu Leu Ala
    1115                1120                1125

Ala Arg Lys Ala Lys Asp Asn Arg Asn Asp Leu Ala Ile Val Arg
    1130                1135                1140

Leu Glu Gln Leu Ala Pro Leu Pro Arg Arg Arg Leu Arg Glu Thr

1145                1150                1155

Leu Asp Arg Tyr Glu Asn Val Lys Glu Phe Phe Trp Val Gln Glu
    1160                1165                1170

Glu Pro Ala Asn Gln Gly Ala Trp Pro Arg Phe Gly Leu Glu Leu
    1175                1180                1185

Pro Glu Leu Leu Pro Asp Lys Leu Ala Gly Ile Lys Arg Ile Ser
    1190                1195                1200

Arg Arg Ala Met Ser Ala Pro Ser Ser Gly Ser Ser Lys Val His
    1205                1210                1215

Ala Val Glu Gln Gln Glu Ile Leu Asp Glu Ala Phe Gly
    1220                1225                1230

<210>  42
<211>  580
<212>  PRT
<213>  Bacillus subtilis

<400>  42

Met Thr Val Asn Pro Ile Thr His Tyr Ile Gly Ser Phe Ile Asp Glu
1               5                   10                  15

Phe Ala Leu Ser Gly Ile Thr Asp Ala Val Val Cys Pro Gly Ser Arg
            20                  25                  30

Ser Thr Pro Leu Ala Val Leu Cys Ala Ala His Pro Asp Ile Ser Val
        35                  40                  45

His Val Gln Ile Asp Glu Arg Ser Ala Gly Phe Phe Ala Leu Gly Leu
    50                  55                  60

Ala Lys Ala Lys Gln Arg Pro Val Leu Leu Ile Cys Thr Ser Gly Thr
65                  70                  75                  80

Ala Ala Ala Asn Phe Tyr Pro Ala Val Val Glu Ala His Tyr Ser Arg
                85                  90                  95

Val Pro Ile Ile Val Leu Thr Ala Asp Arg Pro His Glu Leu Arg Glu
            100                 105                 110

Val Gly Ala Pro Gln Ala Ile Asn Gln His Phe Leu Phe Gly Asn Phe
        115                 120                 125

Val Lys Phe Phe Thr Asp Ser Ala Leu Pro Glu Glu Ser Pro Gln Met

130                 135                 140

Leu Arg Tyr Ile Arg Thr Leu Ala Ser Arg Ala Ala Gly Glu Ala Gln
145                 150                 155                 160

Lys Arg Pro Met Gly Pro Val His Val Asn Val Pro Leu Arg Glu Pro
                165                 170                 175

Leu Met Pro Asp Leu Ser Asp Glu Pro Phe Gly Arg Met Arg Thr Gly
            180                 185                 190

Arg His Val Ser Val Lys Thr Gly Thr Gln Ser Val Asp Arg Glu Ser
        195                 200                 205

Leu Ser Asp Val Ala Glu Met Leu Ala Glu Ala Glu Lys Gly Met Ile
    210                 215                 220

Val Cys Gly Glu Leu His Ser Asp Ala Asp Lys Glu Asn Ile Ile Ala
225                 230                 235                 240

Leu Ser Lys Ala Leu Gln Tyr Pro Ile Leu Ala Asp Pro Leu Ser Asn
                245                 250                 255

Leu Arg Asn Gly Val His Asp Lys Ser Thr Val Ile Asp Ala Tyr Asp
            260                 265                 270

Ser Phe Leu Lys Asp Asp Glu Leu Lys Arg Lys Leu Arg Pro Asp Val
        275                 280                 285

Val Ile Arg Phe Gly Pro Met Pro Val Ser Lys Pro Val Phe Leu Trp
    290                 295                 300

Leu Lys Asp Asp Pro Thr Ile Gln Gln Ile Val Ile Asp Glu Asp Gly
305                 310                 315                 320

Gly Trp Arg Asp Pro Thr Gln Ala Ser Ala His Met Ile His Cys Asn
                325                 330                 335

Ala Ser Val Phe Ala Glu Glu Ile Met Ala Gly Leu Thr Ala Ala Thr
            340                 345                 350

Arg Ser Ser Glu Trp Leu Glu Lys Trp Gln Phe Val Asn Gly Arg Phe
        355                 360                 365

Arg Glu His Leu Gln Thr Ile Ser Ser Glu Asp Val Ser Phe Glu Gly
    370                 375                 380

Asn Leu Tyr Arg Ile Leu Gln His Leu Val Pro Glu Asn Ser Ser Leu
385                 390                 395                 400

Phe Val Gly Asn Ser Met Pro Ile Arg Asp Val Asp Thr Phe Phe Glu
                405                 410                 415

Lys Gln Asp Arg Pro Phe Arg Ile Tyr Ser Asn Arg Gly Ala Asn Gly
            420                 425                 430

Ile Asp Gly Val Val Ser Ser Ala Met Gly Val Cys Glu Gly Thr Lys
        435                 440                 445

Ala Pro Val Thr Leu Val Ile Gly Asp Leu Ser Phe Tyr His Asp Leu
    450                 455                 460

Asn Gly Leu Leu Ala Ala Lys Lys Leu Gly Ile Pro Leu Thr Val Ile
465                 470                 475                 480

Leu Val Asn Asn Asp Gly Gly Gly Ile Phe Ser Phe Leu Pro Gln Ala
                485                 490                 495

Ser Glu Lys Thr His Phe Glu Asp Leu Phe Gly Thr Pro Thr Gly Leu
            500                 505                 510

Asp Phe Lys His Ala Ala Ala Leu Tyr Gly Gly Thr Tyr Ser Cys Pro
        515                 520                 525

Ala Ser Trp Asp Glu Phe Lys Thr Ala Tyr Ala Pro Gln Ala Asp Lys
    530                 535                 540

Pro Gly Leu His Leu Ile Glu Ile Lys Thr Asp Arg Gln Ser Arg Val
545                 550                 555                 560

Gln Leu His Arg Asp Met Leu Asn Glu Ala Val Arg Glu Val Lys Lys
                565                 570                 575

Gln Trp Glu Leu
            580

<210>  43
<211>  426
<212>  PRT
<213>  Escherichia coli

<400>  43

Met Asn Ser Asn Lys Glu Leu Met Gln Arg Arg Ser Gln Ala Ile Pro
1               5                   10                  15

Arg Gly Val Gly Gln Ile His Pro Ile Phe Ala Asp Arg Ala Glu Asn
            20                  25                  30

Cys Arg Val Trp Asp Val Glu Gly Arg Glu Tyr Leu Asp Phe Ala Gly
        35                  40                  45

Gly Ile Ala Val Leu Asn Thr Gly His Leu His Pro Lys Val Val Ala
    50                  55                  60

Ala Val Glu Ala Gln Leu Lys Lys Leu Ser His Thr Cys Phe Gln Val
65                  70                  75                  80

Leu Ala Tyr Glu Pro Tyr Leu Glu Leu Cys Glu Ile Met Asn Gln Lys
                85                  90                  95

Val Pro Gly Asp Phe Ala Lys Lys Thr Leu Leu Val Thr Thr Gly Ser
            100                 105                 110

Glu Ala Val Glu Asn Ala Val Lys Ile Ala Arg Ala Ala Thr Lys Arg
        115                 120                 125

Ser Gly Thr Ile Ala Phe Ser Gly Ala Tyr His Gly Arg Thr His Tyr
    130                 135                 140

Thr Leu Ala Leu Thr Gly Lys Val Asn Pro Tyr Ser Ala Gly Met Gly
145                 150                 155                 160

Leu Met Pro Gly His Val Tyr Arg Ala Leu Tyr Pro Cys Pro Leu His
                165                 170                 175

Gly Ile Ser Glu Asp Asp Ala Ile Ala Ser Ile His Arg Ile Phe Lys
            180                 185                 190

Asn Asp Ala Ala Pro Glu Asp Ile Ala Ala Ile Val Ile Glu Pro Val
        195                 200                 205

Gln Gly Glu Gly Gly Phe Tyr Ala Ser Ser Pro Ala Phe Met Gln Arg
    210                 215                 220

Leu Arg Ala Leu Cys Asp Glu His Gly Ile Met Leu Ile Ala Asp Glu
225                 230                 235                 240

Val Gln Ser Gly Ala Gly Arg Thr Gly Thr Leu Phe Ala Met Glu Gln
                245                 250                 255

Met Gly Val Ala Pro Asp Leu Thr Thr Phe Ala Lys Ser Ile Ala Gly
            260                 265                 270

Gly Phe Pro Leu Ala Gly Val Thr Gly Arg Ala Glu Val Met Asp Ala
        275                 280                 285

Val Ala Pro Gly Gly Leu Gly Gly Thr Tyr Ala Gly Asn Pro Ile Ala
    290                 295                 300

Cys Val Ala Ala Leu Glu Val Leu Lys Val Phe Glu Gln Glu Asn Leu
305                 310                 315                 320

Leu Gln Lys Ala Asn Asp Leu Gly Gln Lys Leu Lys Asp Gly Leu Leu
                325                 330                 335

Ala Ile Ala Glu Lys His Pro Glu Ile Gly Asp Val Arg Gly Leu Gly
            340                 345                 350

Ala Met Ile Ala Ile Glu Leu Phe Glu Asp Gly Asp His Asn Lys Pro
        355                 360                 365

Asp Ala Lys Leu Thr Ala Glu Ile Val Ala Arg Ala Arg Asp Lys Gly
    370                 375                 380

Leu Ile Leu Leu Ser Cys Gly Pro Tyr Tyr Asn Val Leu Arg Ile Leu
385                 390                 395                 400

Val Pro Leu Thr Ile Glu Asp Ala Gln Ile Arg Gln Gly Leu Glu Ile
                405                 410                 415

Ile Ser Gln Cys Phe Asp Glu Ala Lys Gln
            420                 425

<210>  44
<211>  504
<212>  PRT
<213>  Arabidopsis thaliana

<400>  44

Met Val Val Ile Asn Ser Leu Arg Arg Leu Ala Arg Thr Thr Gln Val
1               5                   10                  15

His Leu His Ser Lys Tyr Ala Thr Cys Met Ser Gly Asn Ser Thr Ser
            20                  25                  30

Arg Arg Ile Phe Thr Thr Glu Ala Ala Pro Glu Lys Lys Asn Thr Val
        35                  40                  45

Gly Ser Lys Gly His Asp Met Leu Ala Pro Phe Thr Ala Gly Trp Gln
    50                  55                  60

Ser Ala Asp Leu Asp Pro Leu Val Ile Ala Lys Ser Glu Gly Ser Tyr
65                  70                  75                  80

Val Tyr Asp Asp Thr Gly Lys Lys Tyr Leu Asp Ser Leu Ala Gly Leu
                85                  90                  95

Trp Cys Thr Ala Leu Gly Gly Asn Glu Pro Arg Leu Val Ser Ala Ala
            100                 105                 110

Val Glu Gln Leu Asn Thr Leu Pro Phe Tyr His Ser Phe Trp Asn Arg
        115                 120                 125

Thr Thr Lys Pro Ser Leu Asp Leu Ala Lys Val Leu Leu Glu Met Phe
    130                 135                 140

Thr Ala Asn Lys Met Ala Lys Ala Phe Phe Thr Ser Gly Gly Ser Asp
145                 150                 155                 160

Ala Asn Asp Thr Gln Val Lys Leu Val Trp Tyr Tyr Asn Asn Ala Leu
                165                 170                 175

Gly Arg Pro Glu Lys Lys Lys Phe Ile Ala Arg Lys Lys Ser Tyr His
            180                 185                 190

Gly Ser Thr Leu Ile Ser Ala Ser Leu Ser Gly Leu Pro Pro Leu His
        195                 200                 205

Gln Asn Phe Asp Leu Pro Ala Pro Phe Val Leu His Thr Asp Cys Pro
    210                 215                 220

His Tyr Trp Arg Phe His Leu Pro Gly Glu Thr Glu Glu Glu Phe Ser
225                 230                 235                 240

Thr Arg Leu Ala Lys Asn Leu Glu Asp Leu Ile Ile Lys Glu Gly Pro
                245                 250                 255

Glu Thr Ile Gly Ala Phe Ile Ala Glu Pro Val Met Gly Ala Gly Gly
            260                 265                 270

Val Ile Pro Pro Pro Ala Thr Tyr Phe Glu Lys Val Gln Ala Val Val
        275                 280                 285

Lys Lys Tyr Asp Ile Leu Phe Ile Ala Asp Glu Val Ile Cys Ala Phe
    290                 295                 300

Gly Arg Leu Gly Thr Met Phe Gly Cys Asp Lys Tyr Asn Ile Lys Pro
305                 310                 315                 320

Asp Leu Val Thr Leu Ala Lys Ala Leu Ser Ser Ala Tyr Met Pro Ile
                325                 330                 335

Gly Ala Ile Leu Met Ser Gln Glu Val Ala Asp Val Ile Asn Ser His
            340                 345                 350

Ser Ser Lys Leu Gly Val Phe Ser His Gly Phe Thr Tyr Ser Gly His
        355                 360                 365

Pro Val Ser Cys Ala Val Ala Ile Glu Ala Leu Lys Ile Tyr Lys Glu
    370                 375                 380

Arg Asn Ile Pro Glu Tyr Val Ala Lys Val Ala Pro Arg Phe Gln Asp
385                 390                 395                 400

Gly Val Lys Ala Phe Ala Ser Gly Ser Pro Ile Ile Gly Glu Thr Arg
                405                 410                 415

Gly Thr Gly Leu Ile Leu Gly Thr Glu Phe Val Asp Asn Lys Ser Pro
            420                 425                 430

Asn Glu Pro Phe Pro Pro Glu Trp Gly Val Gly Ala Phe Phe Gly Ala
        435                 440                 445

Glu Cys Gln Lys His Gly Met Leu Val Arg Val Ala Gly Asp Gly Ile
    450                 455                 460

Leu Met Ser Pro Pro Leu Ile Ile Ser Pro Glu Glu Ile Asp Glu Leu
465                 470                 475                 480

Ile Ser Ile Tyr Gly Lys Ala Leu Lys Ala Thr Glu Glu Lys Val Lys
                485                 490                 495

Glu Leu Lys Ala Gln His Lys Lys
            500

<210>  45
<211>  515
<212>  PRT
<213>  Solanum lycopersicum

<400>  45

Met Ala Lys Ile Ser Arg Leu Phe Gly Ser Thr Val Lys Ala Ala Ile
1               5                   10                  15

Thr Ala Gln Ala Gly Phe His Gly Lys Arg Ile Pro Ala Val Ser Ser
            20                  25                  30

Leu Gln Glu His Ile Val Lys Ser Thr Pro Ala Arg Tyr Asn Ser Thr
        35                  40                  45

Gln Ala Cys Leu Glu Asn Asp Ile Ser Gly Thr Asp Asn Lys Gly Phe
    50                  55                  60

Lys Gly His Asp Met Leu Ala Pro Phe Thr Ala Gly Trp Gln Ser Thr
65                  70                  75                  80

Asp Val Asp Pro Leu Ile Ile Glu Lys Ser Glu Gly Ser His Val Tyr
                85                  90                  95

Asp Met Gln Gly Arg Lys Tyr Ile Asp Thr Leu Ala Gly Leu Trp Cys
            100                 105                 110

Thr Ala Leu Gly Gly Asn Glu Pro Arg Leu Val Asp Ala Ala Thr Lys
        115                 120                 125

Gln Leu Asn Thr Leu Pro Phe Tyr His Ser Phe Trp Asn Arg Thr Thr
    130                 135                 140

Lys Pro Ser Leu Asp Leu Ala Lys Glu Leu Leu Asp Met Phe Thr Ala
145                 150                 155                 160

Lys Lys Met Ala Lys Ala Phe Phe Thr Asn Ser Gly Ser Glu Ala Asn
                165                 170                 175

Asp Thr Gln Val Lys Leu Val Trp Tyr Tyr Asn Asn Ala Leu Gly Arg
            180                 185                 190

Pro Asn Lys Lys Lys Phe Ile Ala Arg Ala Lys Ala Tyr His Gly Ser
        195                 200                 205

Thr Leu Ile Ser Ala Ser Leu Thr Gly Leu Pro Ala Leu His Gln Asn
    210                 215                 220

Phe Asp Leu Pro Ala Pro Phe Val Leu His Thr Asp Cys Pro His Tyr
225                 230                 235                 240

Trp Arg Tyr His Leu Pro Gly Glu Thr Glu Glu Glu Phe Ser Thr Arg
                245                 250                 255

Leu Ala Lys Asn Leu Glu Asp Leu Ile Leu Lys Glu Gly Pro Glu Thr
            260                 265                 270

Ile Ala Ala Phe Ile Ala Glu Pro Val Met Gly Ala Gly Gly Val Ile

275                 280                 285

Pro Pro Pro Ala Thr Tyr Phe Asp Lys Ile Gln Ala Val Val Lys Lys
    290                 295                 300

Tyr Asp Ile Leu Phe Ile Ala Asp Glu Val Ile Cys Ala Phe Gly Arg
305                 310                 315                 320

Leu Gly Thr Met Phe Gly Ser Asp Met Tyr Asn Ile Lys Pro Asp Leu
                325                 330                 335

Val Thr Leu Ala Lys Ala Leu Ser Ser Ala Tyr Met Pro Ile Gly Ala
            340                 345                 350

Val Leu Val Ser Pro Glu Val Ser Asp Val Ile His Ser Gln Ser Asn
        355                 360                 365

Lys Leu Gly Ser Phe Ser His Gly Phe Thr Tyr Ser Gly His Pro Val
    370                 375                 380

Ala Cys Ala Val Ala Leu Glu Ala Ile Lys Ile Tyr Lys Glu Arg Asn
385                 390                 395                 400

Met Val Glu Arg Val Asn Arg Ile Ser Pro Lys Phe Gln Glu Gly Leu
                405                 410                 415

Lys Ala Phe Ser Asp Ser Pro Ile Ile Gly Glu Ile Arg Gly Leu Gly
            420                 425                 430

Leu Ile Leu Ala Thr Glu Phe Ala Asn Asn Lys Ser Pro Asn Asp His
        435                 440                 445

Phe Pro Pro Glu Trp Gly Val Gly Ala Tyr Phe Gly Ala Gln Cys Gln
    450                 455                 460

Lys Asn Gly Met Leu Val Arg Val Ala Gly Asp Thr Ile Met Met Ser
465                 470                 475                 480

Pro Pro Phe Val Val Thr Pro Glu Glu Leu Asp Glu Leu Ile Arg Ile
                485                 490                 495

Tyr Gly Lys Ala Leu Arg Glu Thr Glu Lys Arg Val Glu Glu Leu Lys
            500                 505                 510

Ser Gln Lys
        515

<210>  46
<211>  520
<212>  PRT
<213>  Solanum lycopersicum

<400>  46

Met Ala Lys Ile Thr Ser Leu Ile Gly Ser Gly Ile Val Ala Ala Thr
1               5                   10                  15

Asn Gln Val Gly Pro His Val Lys His Ile Pro Ala Val Gly Asn Leu
            20                  25                  30

Gln Lys Gln Ile Val Ser Asp Gln Ile Gln Val Arg Trp Ser Ser Thr
        35                  40                  45

Glu Thr Ser Leu Lys Asn Asp Ile Ser Ala Thr Asp Val Arg Gly Tyr
    50                  55                  60

Lys Gly His Asp Met Leu Ala Pro Phe Thr Ala Gly Trp His Ser Thr
65                  70                  75                  80

Asp Leu Glu Pro Leu Val Ile Gln Lys Ser Glu Gly Ser Tyr Val Tyr
                85                  90                  95

Asp Val Asn Gly Lys Lys Tyr Leu Asp Ala Leu Ala Gly Leu Trp Cys
            100                 105                 110

Thr Ser Leu Gly Gly Asn Glu Pro Arg Leu Val Ala Ala Ala Thr Lys
        115                 120                 125

Gln Leu Asn Glu Leu Ala Phe Tyr His Ser Phe Trp Asn Arg Ser Thr
    130                 135                 140

Lys Pro Ser Leu Asp Leu Ala Lys Glu Leu Leu Asp Leu Phe Thr Ala
145                 150                 155                 160

Asn Lys Met Ala Lys Ala Phe Phe Thr Asn Ser Gly Ser Glu Ala Asn
                165                 170                 175

Asp Thr Gln Val Lys Leu Val Trp Tyr Tyr Asn Asn Ala Leu Gly Arg
            180                 185                 190

Pro Asp Lys Lys Lys Phe Ile Ala Arg Thr Lys Ser Tyr His Gly Ser
        195                 200                 205

Thr Leu Ile Ser Ala Ser Leu Ser Gly Leu Pro Ala Leu His Gln Gln
    210                 215                 220

Phe Asp Leu Pro Ala Pro Phe Val Leu His Thr Asp Cys Pro His Phe
225                 230                 235                 240

Trp Arg Phe His Gln Pro Gly Glu Thr Glu Glu Glu Phe Ser Thr Arg
                245                 250                 255

Leu Ala Asn Asn Leu Glu Asn Leu Ile Leu Lys Glu Gly Pro Glu Thr
            260                 265                 270

Ile Ala Ala Phe Ile Ala Glu Pro Val Met Gly Ala Gly Gly Val Ile
        275                 280                 285

Pro Pro Pro Ala Thr Tyr Phe Glu Lys Val Gln Ala Ile Leu Lys Lys
    290                 295                 300

Tyr Asp Ile Leu Phe Ile Ala Asp Glu Val Ile Cys Gly Phe Gly Arg
305                 310                 315                 320

Leu Gly Thr Met Phe Gly Cys Glu Lys Tyr Asn Ile Lys Pro Asp Leu
                325                 330                 335

Val Ser Val Ala Lys Ala Leu Ser Ser Gly Tyr Met Pro Ile Gly Ala
            340                 345                 350

Val Leu Val Ser Pro Glu Val Ser Asp Val Ile Tyr Ser Gln Ser Asn
        355                 360                 365

Lys Leu Gly Thr Phe Ser His Gly Phe Thr Tyr Ser Gly His Pro Val
    370                 375                 380

Ser Cys Ala Val Ala Leu Glu Thr Leu Lys Ile Tyr Lys Glu Arg Asn
385                 390                 395                 400

Ile Ile Glu Gln Val Asn Arg Ile Ser Pro Lys Phe Gln Glu Gly Leu
                405                 410                 415

Lys Ala Phe Ser Asp Ser Pro Ile Ile Gly Glu Ile Arg Gly Thr Gly
            420                 425                 430

Leu Leu His Gly Thr Glu Phe Thr Asp Asn Lys Ser Pro Asn Asp Pro
        435                 440                 445

Phe Pro Pro Glu Trp Gly Ile Gly Ala Tyr Phe Gly Ala Arg Cys Glu
    450                 455                 460

Lys His Gly Val Leu Val Arg Val Ala Gly Asp Asn Ile Met Met Ser
465                 470                 475                 480

Pro Pro Tyr Ile Leu Ser Leu Glu Glu Ile Asp Glu Leu Ile Ile Lys
                485                 490                 495

Tyr Gly Lys Ala Leu Lys Asp Thr Glu Asn Arg Val Glu Glu Leu Lys
            500                 505                 510

Ser Gln Lys Lys Ile Lys Ser Ser
        515                 520

<210>  47
<211>  458
<212>  PRT
<213>  Solanum lycopersicum

<400>  47

Met Ala Lys Thr Asn Gly Phe Met Gly His Asp Met Leu Ala Pro Phe
1               5                   10                  15

Thr Ala Ala Trp Met Ile Asp Met Gly Pro Leu Val Ile Asp Lys Ala
            20                  25                  30

Glu Gly Ser Tyr Val Tyr Gly Val Asn Gly Lys Lys Tyr Leu Asp Ser
        35                  40                  45

Leu Ser Gly Leu Trp Cys Thr Val Leu Gly Gly Ser Glu Pro Arg Leu
    50                  55                  60

Ile Glu Ala Ala Ser Lys Gln Leu Asn Lys Ser Ala Phe Tyr His Ser
65                  70                  75                  80

Phe Trp Asn Arg Thr Thr Lys Pro Ser Leu Asp Leu Ala Lys Glu Leu
                85                  90                  95

Ile Asn Met Phe Thr Ala Asn Lys Met Gly Lys Val Phe Phe Thr Ser
            100                 105                 110

Ser Gly Ser Glu Ala Asn Asp Thr Gln Val Lys Leu Val Trp Tyr Tyr
        115                 120                 125

Asn Asn Ala Ile Gly Arg Pro Asn Lys Lys Lys Ile Ile Ser Arg Lys
    130                 135                 140

Asn Ala Tyr His Gly Ser Thr Tyr Met Thr Ala Gly Leu Ser Gly Leu
145                 150                 155                 160

Pro Ser Leu His Leu Lys Phe Asp Leu Pro Pro Pro Tyr Ile Leu His
                165                 170                 175

Thr Asp Cys Pro His Tyr Trp Asn Tyr His Leu Pro Gly Glu Thr Glu
            180                 185                 190

Glu Glu Tyr Ser Thr Arg Leu Ala Asn Asn Leu Glu Asn Leu Ile Leu
        195                 200                 205

Lys Glu Gly Pro Glu Thr Val Ala Ala Phe Ile Ala Glu Pro Val Met
    210                 215                 220

Gly Gly Ala Gly Val Ile Ile Pro Pro Ala Thr Tyr Phe Glu Lys Ile
225                 230                 235                 240

Gln Ala Val Leu Lys Lys Tyr Asp Ile Leu Phe Ile Ala Asp Glu Val
                245                 250                 255

Ile Cys Gly Phe Gly Arg Leu Gly Thr Met Phe Gly Cys Asp Lys Tyr
            260                 265                 270

Asn Ile Lys Pro Asp Leu Val Ser Ile Ala Lys Ala Leu Ser Gly Gly
        275                 280                 285

Tyr Ile Pro Ile Gly Ala Val Leu Val Ser Glu Glu Ile Ser Lys Val
    290                 295                 300

Ile Met Ser Gln Ser Asn Gln Leu Gly Val Phe Cys His Gly Phe Thr
305                 310                 315                 320

Tyr Ser Gly His Pro Val Ala Cys Ala Val Ala Leu Glu Ala Leu Lys
                325                 330                 335

Ile Tyr Lys Glu Lys Asn Ile Thr Glu Val Val Asn Lys Leu Ser Pro
            340                 345                 350

Lys Leu Gln Glu Gly Leu Lys Ala Phe Ile Asp Ser Pro Ile Ile Gly
        355                 360                 365

Glu Ile Arg Gly Thr Gly Leu Val Leu Ser Thr Glu Phe Val Asp Asn
    370                 375                 380

Lys Ser Pro Asn Asp Pro Phe Pro Pro Glu Trp Gly Val Gly Thr Tyr
385                 390                 395                 400

Phe Gly Ser Gln Cys Gln Lys His Gly Met Leu Val Ser Phe Ser Gly
                405                 410                 415

Asp His Val Asn Met Ala Pro Pro Phe Thr Leu Ser Leu Glu Glu Leu
            420                 425                 430

Asp Glu Met Ile Ser Ile Tyr Gly Lys Ala Leu Lys Asp Thr Glu Lys
        435                 440                 445

Arg Val Glu Glu Leu Lys Ser Gln Lys Lys
    450                 455

<210>  48
<211>  448
<212>  PRT
<213>  Pseudomonas aeruginosa

<400>  48

Met Asn Gln Pro Leu Asn Val Ala Pro Pro Val Ser Ser Glu Leu Asn
1               5                   10                  15

Leu Arg Ala His Trp Met Pro Phe Ser Ala Asn Arg Asn Phe Gln Lys
            20                  25                  30

Asp Pro Arg Ile Ile Val Ala Ala Glu Gly Ser Trp Leu Thr Asp Asp
        35                  40                  45

Lys Gly Arg Lys Val Tyr Asp Ser Leu Ser Gly Leu Trp Thr Cys Gly
    50                  55                  60

Ala Gly His Ser Arg Lys Glu Ile Gln Glu Ala Val Ala Arg Gln Leu
65                  70                  75                  80

Gly Thr Leu Asp Tyr Ser Pro Gly Phe Gln Tyr Gly His Pro Leu Ser
                85                  90                  95

Phe Gln Leu Ala Glu Lys Ile Ala Gly Leu Leu Pro Gly Glu Leu Asn
            100                 105                 110

His Val Phe Phe Thr Gly Ser Gly Ser Glu Cys Ala Asp Thr Ser Ile
        115                 120                 125

Lys Met Ala Arg Ala Tyr Trp Arg Leu Lys Gly Gln Pro Gln Lys Thr
    130                 135                 140

Lys Leu Ile Gly Arg Ala Arg Gly Tyr His Gly Val Asn Val Ala Gly
145                 150                 155                 160

Thr Ser Leu Gly Gly Ile Gly Gly Asn Arg Lys Met Phe Gly Gln Leu
                165                 170                 175

Met Asp Val Asp His Leu Pro His Thr Leu Gln Pro Gly Met Ala Phe
            180                 185                 190

Thr Arg Gly Met Ala Gln Thr Gly Gly Val Glu Leu Ala Asn Glu Leu
        195                 200                 205

Leu Lys Leu Ile Glu Leu His Asp Ala Ser Asn Ile Ala Ala Val Ile
    210                 215                 220

Val Glu Pro Met Ser Gly Ser Ala Gly Val Leu Val Pro Pro Val Gly
225                 230                 235                 240

Tyr Leu Gln Arg Leu Arg Glu Ile Cys Asp Gln His Asn Ile Leu Leu
                245                 250                 255

Ile Phe Asp Glu Val Ile Thr Ala Phe Gly Arg Leu Gly Thr Tyr Ser
            260                 265                 270

Gly Ala Glu Tyr Phe Gly Val Thr Pro Asp Leu Met Asn Val Ala Lys
        275                 280                 285

Gln Val Thr Asn Gly Ala Val Pro Met Gly Ala Val Ile Ala Ser Ser
    290                 295                 300

Glu Ile Tyr Asp Thr Phe Met Asn Gln Ala Leu Pro Glu His Ala Val
305                 310                 315                 320

Glu Phe Ser His Gly Tyr Thr Tyr Ser Ala His Pro Val Ala Cys Ala
                325                 330                 335

Ala Gly Leu Ala Ala Leu Asp Ile Leu Ala Arg Asp Asn Leu Val Gln
            340                 345                 350

Gln Ser Ala Glu Leu Ala Pro His Phe Glu Lys Gly Leu His Gly Leu
        355                 360                 365

Gln Gly Ala Lys Asn Val Ile Asp Ile Arg Asn Cys Gly Leu Ala Gly
    370                 375                 380

Ala Ile Gln Ile Ala Pro Arg Asp Gly Asp Pro Thr Val Arg Pro Phe
385                 390                 395                 400

Glu Ala Gly Met Lys Leu Trp Gln Gln Gly Phe Tyr Val Arg Phe Gly
                405                 410                 415

Gly Asp Thr Leu Gln Phe Gly Pro Thr Phe Asn Ala Arg Pro Glu Glu
            420                 425                 430

Leu Asp Arg Leu Phe Asp Ala Val Gly Glu Ala Leu Asn Gly Ile Ala

435                 440                 445

<210>  49
<211>  483
<212>  PRT
<213>  Oryza sativa subsp. japonica

<400>  49

Met Thr Thr Pro His Gly Leu Leu Gln Tyr Ser Ser Gly Ala Phe Ser
1               5                   10                  15

Asp Gln Val Pro Ala Asp Asp Ser Ala Glu Glu His Gly Val Lys Asp
            20                  25                  30

His Ala Met Leu Ala Pro Phe Thr Ala Ala Trp Gln Thr Ala Ile Ser
        35                  40                  45

Pro Pro Leu Val Ile Glu Arg Ser Glu Gly Cys Tyr Val Tyr Asp Val
    50                  55                  60

Asn Gly Thr Lys Tyr Leu Asp Ala Leu Ala Gly Leu Leu Ser Thr Ala
65                  70                  75                  80

Leu Gly Gly Ser Glu Pro Arg Leu Val Lys Ala Ala Thr Glu Gln Leu
                85                  90                  95

Asn Lys Leu Pro Phe Tyr His Ser Phe Trp Asn His Thr Thr Arg Pro
            100                 105                 110

Ser Leu Asp Leu Ala Lys Glu Leu Ile Ser Met Phe Thr Ala Arg Glu
        115                 120                 125

Met Gly Lys Val Phe Phe Thr Asn Ser Gly Ser Glu Ala Asn Asp Ser
    130                 135                 140

Gln Val Lys Ile Val Trp Tyr Tyr Asn Asn Ala Leu Gly Arg Pro Lys
145                 150                 155                 160

Lys Lys Asn Ile Ile Ser Arg Thr Gln Ser Tyr His Gly Thr Thr Phe
                165                 170                 175

Ile Ser Ala Ser Leu Ser Gly Leu Pro Thr Leu His Gln Asp Phe Asp
            180                 185                 190

Leu Pro Gly Arg Phe Val Leu His Thr Asp Cys Pro His Tyr Trp Arg
        195                 200                 205

Phe His Leu Pro Gly Glu Thr Glu Glu Glu Phe Ala Thr Arg Leu Ala

210                 215                 220

Asp Asn Leu Glu Asn Leu Ile Leu Lys Gln Gly Pro Glu Thr Ile Ala
225                 230                 235                 240

Ala Phe Ile Ala Glu Pro Val Ile Gly Ala Gly Gly Val Ile Leu Pro
                245                 250                 255

Pro Lys Thr Tyr Phe Glu Lys Ile Gln Ala Val Val Lys Lys Tyr Asp
            260                 265                 270

Ile Leu Phe Ile Val Asp Glu Val Ile Thr Gly Phe Gly Arg Leu Gly
        275                 280                 285

Thr Met Phe Gly Ser Asp Leu Tyr Asn Ile Lys Pro Asp Leu Val Ser
    290                 295                 300

Leu Ala Lys Ala Leu Ser Ser Ala Tyr Ala Pro Ile Gly Ala Ile Leu
305                 310                 315                 320

Val Ser Pro Glu Ile Ser Asp Val Ile His Ser His Ser Asn Lys Leu
                325                 330                 335

Gly Thr Phe Ala His Gly Phe Thr Tyr Ser Gly His Pro Val Ser Cys
            340                 345                 350

Ala Val Ala Leu Glu Ala Leu Lys Ile Tyr Arg Glu Arg Asp Ile Pro
        355                 360                 365

Gly His Val Thr His Val Ala Gln Arg Phe Gln Glu Gly Ile Lys Ala
    370                 375                 380

Phe Ala Ala Gly Ser Pro Ile Val Gly Glu Thr Arg Gly Val Gly Leu
385                 390                 395                 400

Leu Ile Ala Thr Glu Phe Thr Asp Asn Lys Ser Pro Tyr Glu Leu Phe
                405                 410                 415

Pro Phe Glu Trp Gly Val Gly Glu Ile Phe Gly Gln Glu Cys Lys Lys
            420                 425                 430

Arg Gly Met Met Val Lys Val Leu Gly Asn Leu Ile Ala Met Ser Pro
        435                 440                 445

Pro Leu Ile Ile Thr Arg Glu Glu Ile Asp Lys Leu Val Ser Ile Tyr
    450                 455                 460

Gly Glu Ala Leu Lys Ala Thr Glu Glu Arg Val Ala Glu Leu Lys Ser
465                 470                 475                 480

Lys Lys Asn

<210>  50
<211>  516
<212>  PRT
<213>  Oryza sativa subsp. japonica

<400>  50

Met Val Ile Ala Arg Gly Leu Leu Arg Ser Asn Ala Ser Ser Ser Ser
1               5                   10                  15

Ser Gln Ala Ile Asn Leu Leu Lys Tyr Val Thr Ser Thr Gly Ser Leu
            20                  25                  30

Gln Gly His Thr Gln Asn Leu Cys Asp Ala Ser Thr Arg His Phe Ser
        35                  40                  45

Ser Val Pro Ser Pro Gln Tyr Asn Ser Thr Glu Glu Asn Gly Phe Lys
    50                  55                  60

Gly His Gly Met Leu Ala Pro Phe Thr Ala Gly Trp Gln Ser Thr Asp
65                  70                  75                  80

Val His Pro Leu Val Ile Glu Arg Ser Glu Gly Ser Tyr Val Tyr Asp
                85                  90                  95

Ile Asp Gly Lys Lys Tyr Leu Asp Ser Leu Ala Gly Leu Trp Cys Thr
            100                 105                 110

Ala Leu Gly Gly Ser Glu Pro Arg Leu Val Lys Ala Ala Thr Glu Gln
        115                 120                 125

Leu His Lys Leu Pro Phe Tyr His Ser Phe Trp Asn Arg Thr Thr Lys
    130                 135                 140

Pro Ser Leu Asp Leu Ala Lys Glu Leu Leu Ser Met Phe Thr Ala Arg
145                 150                 155                 160

Glu Met Gly Lys Val Phe Phe Thr Asn Ser Gly Ser Glu Ala Asn Asp
                165                 170                 175

Ser Gln Val Lys Leu Val Trp Tyr Tyr Asn Asn Ala Leu Gly Arg Pro
            180                 185                 190

Asp Lys Lys Lys Phe Ile Ala Arg Ser Lys Ser Tyr His Gly Ser Thr
        195                 200                 205

Leu Ile Ser Ala Ser Leu Ser Gly Leu Pro Ala Leu His Gln Lys Phe
    210                 215                 220

Asp Leu Pro Ala Pro Phe Val Leu His Thr Asp Cys Pro His Tyr Trp
225                 230                 235                 240

Arg Phe His Leu Pro Gly Glu Thr Glu Glu Glu Phe Ala Thr Arg Leu
                245                 250                 255

Ala Asn Asn Leu Glu Glu Leu Ile Leu Lys Glu Gly Pro Glu Thr Ile
            260                 265                 270

Ala Ala Phe Ile Ala Glu Pro Val Met Gly Ala Gly Gly Val Ile Pro
        275                 280                 285

Pro Pro Lys Thr Tyr Phe Glu Lys Val Gln Ala Ile Val Lys Lys Tyr
    290                 295                 300

Asp Ile Leu Phe Ile Ala Asp Glu Val Ile Thr Ala Phe Gly Arg Leu
305                 310                 315                 320

Gly Thr Met Phe Gly Ser Asp Met Tyr Asn Ile Lys Pro Asp Leu Val
                325                 330                 335

Ser Met Ala Lys Ala Leu Ser Ser Ala Tyr Val Pro Ile Gly Ala Ile
            340                 345                 350

Met Val Ser Pro Glu Ile Ser Asp Val Ile His Ser Gln Ser Asn Lys
        355                 360                 365

Leu Gly Ser Phe Ala His Gly Phe Thr Tyr Ser Gly His Pro Val Ala
    370                 375                 380

Cys Ala Val Ala Ile Glu Ala Leu Lys Ile Tyr Gln Glu Arg Asn Ile
385                 390                 395                 400

Pro Asp His Val Lys Gln Ile Ser Pro Arg Phe Gln Glu Gly Val Lys
                405                 410                 415

Ala Phe Ala Gly Ser Pro Ile Val Gly Glu Ile Arg Gly Val Gly Leu
            420                 425                 430

Ile Leu Gly Thr Glu Phe Ala Asp Asn Lys Ser Pro Asn Asp Pro Phe
        435                 440                 445

Pro Ala Glu Trp Gly Val Gly Ala Ile Phe Gly Ala Glu Cys Gln Lys
    450                 455                 460

Arg Gly Met Leu Val Arg Val Ala Gly Asp Asn Ile Met Met Ser Pro
465                 470                 475                 480

Pro Leu Ile Met Thr Pro Asp Glu Val Glu Glu Leu Val Ser Ile Tyr
                485                 490                 495

Gly Asp Ala Leu Lys Ala Thr Glu Glu Arg Val Ala Glu Leu Lys Ser
            500                 505                 510

Lys Lys Asn Asn
        515

<210>  51
<211>  497
<212>  PRT
<213>  Oryza sativa subsp. japonica

<400>  51

Met Asn Leu Ile Lys His Ala Ala Phe Ala Ala Ser Phe Gln Gly Glu
1               5                   10                  15

Thr Asp Cys Thr Ser His Ala Ser Ala Arg Lys Phe Ser Thr Ser Gly
            20                  25                  30

Ser Ser Pro Leu Leu Asp Ser Thr Glu Gly Asn Gly Phe Lys Gly His
        35                  40                  45

Ser Met Leu Ala Pro Phe Thr Ala Gly Trp His Ser Thr Asp Leu Glu
    50                  55                  60

Pro Leu Ile Ile Glu Arg Ser Glu Gly Ser Tyr Val Tyr Asp Ser Lys
65                  70                  75                  80

Gly Asn Lys Tyr Leu Asp Thr Leu Ala Gly Leu Trp Cys Thr Ala Leu
                85                  90                  95

Gly Gly Ser Glu Pro Arg Leu Val Lys Ala Ala Thr Asp Gln Leu Asn
            100                 105                 110

Lys Leu Pro Phe Tyr His Ser Phe Trp Asn Ser Thr Ala Lys Pro Pro
        115                 120                 125

Leu Asp Leu Ala Glu Glu Leu Ile Ser Met Phe Thr Ala Lys Glu Met
    130                 135                 140

Gly Lys Val Phe Phe Thr Asn Ser Gly Ser Glu Ala Asn Asp Ser Gln
145                 150                 155                 160

Val Lys Leu Val Trp Tyr Tyr Asn Asn Ala Leu Gly Arg Pro Asn Lys
                165                 170                 175

Lys Lys Ile Ile Ala Gln Ser Gln Ala Tyr His Gly Ser Thr Leu Ile
            180                 185                 190

Ser Ala Ser Leu Ser Gly Leu Pro Ala Met His Leu Lys Phe Asp Leu
        195                 200                 205

Pro Ala Pro Phe Val Leu His Thr Asp Cys Pro His Tyr Trp Arg Phe
    210                 215                 220

Gly Leu Pro Gly Glu Ala Glu Glu Glu Phe Ala Thr Arg Leu Ala Asp
225                 230                 235                 240

Asn Leu Glu Asn Leu Ile Leu Lys Glu Gly Pro Glu Thr Val Ala Ala
                245                 250                 255

Phe Ile Ala Glu Pro Val Ile Gly Ala Gly Gly Val Ile Pro Pro Pro
            260                 265                 270

Lys Thr Tyr Phe Glu Lys Ile Gln Ala Val Leu Gln Lys Tyr Asp Val
        275                 280                 285

Leu Phe Ile Ala Asp Glu Val Ile Thr Gly Phe Gly Arg Leu Gly Thr
    290                 295                 300

Met Phe Gly Ser Asp Leu Tyr Asn Ile Lys Pro Asp Leu Val Ser Leu
305                 310                 315                 320

Ala Lys Ala Leu Ser Ser Ala Tyr Val Pro Ile Gly Ala Thr Leu Val
                325                 330                 335

Ser Pro Glu Ile Ser Asp Val Val His Ser Gln Ser Asn Lys Ile Gly
            340                 345                 350

Phe Phe Ala His Gly Phe Thr Tyr Ser Gly His Pro Val Ser Cys Ala
        355                 360                 365

Val Ala Leu Glu Ala Leu Lys Ile Tyr Arg Glu Arg Asn Ile Pro Ala
    370                 375                 380

His Val Lys Gln Ile Ser Pro Arg Phe Gln Glu Gly Ile Lys Ala Phe
385                 390                 395                 400

Ala Gly Ser Ser Ile Ile Gly Glu Thr Arg Gly Val Gly Leu Leu Leu
                405                 410                 415

Ala Thr Glu Phe Ala Asn Asn Lys Ser Pro Asn Asp Pro Phe Pro Val
            420                 425                 430

Glu Trp Gly Val Ala Gln Ile Phe Gly Ala Glu Cys Lys Lys Arg Gly
        435                 440                 445

Met Leu Val Lys Val Val Gly Asp Glu Ile Ala Met Ser Pro Pro Leu
    450                 455                 460

Ile Met Ser Gln Arg Glu Val Asp Gly Leu Val Ser Ile Tyr Gly Glu
465                 470                 475                 480

Ala Leu Lys Ala Thr Glu Glu Arg Val Ala Glu Leu Arg Ser Lys Lys
                485                 490                 495

Lys

<210>  52
<211>  510
<212>  PRT
<213>  Oryza sativa subsp. japonica

<400>  52

Met Ile Cys Arg Ser Leu Leu Leu Leu Arg Ser Asn Ala Ala Ser Lys
1               5                   10                  15

Ala Ser Asn Ile Val Lys His Val Ala Ala Thr Gly Cys Leu Pro Lys
            20                  25                  30

Tyr Ser Ser Glu Ala Pro Ala Arg Tyr Phe Ser Ser Glu Pro Ser Leu
        35                  40                  45

Gln Val Asp Ser Thr Glu Glu Asn Gly Phe Lys Gly His Gly Met Leu
    50                  55                  60

Ala Pro Phe Thr Ala Gly Trp Gln Ser Thr Asp Leu His Pro Leu Val
65                  70                  75                  80

Ile Asp Arg Ser Glu Gly Ser Tyr Val Tyr Asp Ile Asn Gly Lys Lys
                85                  90                  95

Tyr Ile Asp Ala Leu Ala Gly Leu Trp Ser Thr Ala Leu Gly Gly Asn
            100                 105                 110

Glu Pro Arg Leu Ile Lys Ala Ala Thr Asp Gln Leu Asn Lys Leu Pro
        115                 120                 125

Phe Tyr His Ser Phe Trp Asn Arg Thr Thr Lys Pro Ser Leu Asp Leu
    130                 135                 140

Ala Asn Glu Ile Leu Ser Met Phe Thr Ala Arg Glu Met Gly Lys Ile
145                 150                 155                 160

Phe Phe Thr Asn Ser Gly Ser Glu Ala Asn Asp Ser Gln Val Lys Leu
                165                 170                 175

Val Trp Tyr Tyr Asn Asn Ala Leu Gly Arg Pro Asn Lys Lys Lys Phe
            180                 185                 190

Ile Ala Arg Ser Lys Ser Tyr His Gly Ser Thr Leu Val Ser Ala Ser
        195                 200                 205

Leu Ser Gly Leu Pro Ala Leu His Gln Lys Phe Asp Leu Pro Ala Pro
    210                 215                 220

Phe Val Leu His Thr Asp Cys Pro His Tyr Trp Arg Phe His Leu Pro
225                 230                 235                 240

Asp Glu Thr Glu Glu Glu Phe Ala Thr Arg Leu Ala Thr Asn Leu Glu
                245                 250                 255

Asn Leu Ile Leu Lys Glu Gly Pro Glu Thr Ile Ala Ala Phe Ile Ala
            260                 265                 270

Glu Pro Val Met Gly Ala Gly Gly Val Ile Pro Pro Pro Lys Thr Tyr
        275                 280                 285

Phe Glu Lys Ile Gln Ala Val Leu Lys Lys Tyr Asp Ile Leu Leu Ile
    290                 295                 300

Ala Asp Glu Val Ile Thr Ala Phe Gly Arg Leu Gly Thr Met Phe Gly
305                 310                 315                 320

Cys Asp Met Tyr Asp Ile Lys Pro Asp Leu Val Ser Ile Ala Lys Ala
                325                 330                 335

Leu Ser Ser Ala Tyr Met Pro Ile Gly Ala Ile Leu Val Ser Pro Glu
            340                 345                 350

Ile Thr Asp Val Ile Tyr Ser Gln Ser Asn Lys Leu Gly Ser Phe Ala

355                 360                 365

His Gly Phe Thr Tyr Ser Gly His Pro Val Ser Cys Ala Val Ala Ile
    370                 375                 380

Glu Ala Leu Lys Ile Tyr Lys Glu Arg Asn Ile Ile Glu His Val Gln
385                 390                 395                 400

Lys Ile Ala Pro Arg Phe Gln Glu Gly Ile Lys Ala Phe Ser Gly Ser
                405                 410                 415

Pro Ile Val Gly Glu Ile Arg Gly Leu Gly Leu Ile Leu Gly Thr Glu
            420                 425                 430

Phe Val Asp Asn Lys Ser Pro Asn Asp Pro Phe Pro Ala Glu Trp Gly
        435                 440                 445

Val Gly Ser Leu Phe Gly Ala Glu Cys Glu Lys Arg Gly Met Leu Ile
    450                 455                 460

Arg Val Ala Gly Asp Asn Ile Met Leu Ser Pro Pro Leu Ile Met Thr
465                 470                 475                 480

Pro Asp Glu Val Glu Glu Ile Ile Cys Lys Tyr Gly Asp Ala Leu Lys
                485                 490                 495

Ala Thr Glu Glu Arg Ile Ala Glu Leu Lys Ala Lys Arg Gly
            500                 505                 510

<210>  53
<211>  497
<212>  PRT
<213>  Oryza sativa subsp. indica

<400>  53

Met Asn Leu Ile Lys His Ala Ala Phe Ala Ala Ser Phe Gln Gly Glu
1               5                   10                  15

Thr Asp Cys Thr Ser His Ala Ser Ala Arg Lys Phe Ser Thr Ser Gly
            20                  25                  30

Ser Ser Pro Leu Leu Asp Ser Thr Glu Gly Asn Gly Phe Lys Gly His
        35                  40                  45

Ser Met Leu Ala Pro Phe Thr Ala Gly Trp His Ser Thr Asp Leu Glu
    50                  55                  60

Pro Leu Ile Ile Glu Arg Ser Glu Gly Ser Tyr Val Tyr Asp Ser Lys

65                  70                  75                  80

Gly Asn Lys Tyr Leu Asp Thr Leu Ala Gly Leu Trp Cys Thr Ala Leu
                85                  90                  95

Gly Gly Ser Glu Pro Arg Leu Val Lys Ala Ala Thr Asp Gln Leu Asn
            100                 105                 110

Lys Leu Pro Phe Tyr His Ser Phe Trp Asn Ser Thr Ala Lys Pro Pro
        115                 120                 125

Leu Asp Leu Ala Glu Glu Leu Ile Ser Met Phe Thr Ala Lys Glu Met
    130                 135                 140

Gly Lys Val Phe Phe Thr Asn Ser Gly Ser Glu Ala Asn Asp Ser Gln
145                 150                 155                 160

Val Lys Leu Val Trp Tyr Tyr Asn Asn Ala Leu Gly Arg Pro Asn Lys
                165                 170                 175

Lys Lys Ile Ile Ala Gln Ser Gln Ala Tyr His Gly Ser Thr Leu Ile
            180                 185                 190

Ser Ala Ser Leu Ser Gly Leu Pro Ala Met His Leu Lys Phe Asp Leu
        195                 200                 205

Pro Ala Pro Phe Val Leu His Thr Asp Cys Pro His Tyr Trp Arg Phe
    210                 215                 220

Gly Leu Pro Gly Glu Ala Glu Glu Glu Phe Ala Thr Arg Leu Ala Asp
225                 230                 235                 240

Asn Leu Glu Asn Leu Ile Leu Lys Glu Gly Pro Glu Thr Val Ala Ala
                245                 250                 255

Phe Ile Ala Glu Pro Val Ile Gly Ala Gly Gly Val Ile Pro Pro Pro
            260                 265                 270

Lys Thr Tyr Phe Glu Lys Ile Gln Ala Val Leu Gln Lys Tyr Asp Val
        275                 280                 285

Leu Phe Ile Ala Asp Glu Val Ile Thr Gly Phe Gly Arg Leu Gly Thr
    290                 295                 300

Met Phe Gly Ser Asp Leu Tyr Asn Ile Lys Pro Asp Leu Val Ser Leu
305                 310                 315                 320

Ala Lys Ala Leu Ser Ser Ala Tyr Val Pro Ile Gly Ala Thr Leu Val
                325                 330                 335

Ser Pro Glu Ile Ser Asp Val Val His Ser Gln Ser Asn Lys Ile Gly
            340                 345                 350

Phe Phe Ala His Gly Phe Thr Tyr Ser Gly His Pro Val Ser Cys Ala
        355                 360                 365

Val Ala Leu Glu Ala Leu Lys Ile Tyr Arg Glu Arg Asn Ile Pro Ala
    370                 375                 380

His Val Lys Gln Ile Ser Pro Arg Phe Gln Glu Gly Ile Lys Ala Phe
385                 390                 395                 400

Ala Gly Ser Ser Ile Ile Gly Glu Thr Arg Gly Val Gly Leu Leu Leu
                405                 410                 415

Ala Thr Glu Phe Ala Asn Asn Lys Ser Pro Asn Asp Pro Phe Pro Val
            420                 425                 430

Glu Trp Gly Val Ala Gln Ile Phe Gly Ala Glu Cys Lys Lys Arg Gly
        435                 440                 445

Met Leu Val Lys Val Val Gly Asp Glu Ile Ala Met Ser Pro Pro Leu
    450                 455                 460

Ile Met Ser Gln Arg Glu Val Asp Gly Leu Val Ser Ile Tyr Gly Glu
465                 470                 475                 480

Ala Leu Lys Ala Thr Glu Glu Arg Val Ala Glu Leu Arg Ser Lys Lys
                485                 490                 495

Lys

<210>  54
<211>  516
<212>  PRT
<213>  Oryza sativa subsp. indica

<400>  54

Met Val Ile Ala Arg Gly Leu Leu Arg Ser Asn Ala Ser Ser Ser Ser
1               5                   10                  15

Ser Gln Ala Ile Asn Leu Leu Lys Tyr Val Thr Ser Thr Gly Ser Leu
            20                  25                  30

Gln Gly His Thr Gln Asn Leu Cys Asp Ala Ser Thr Arg His Phe Ser
        35                  40                  45

Ser Val Pro Ser Pro Gln Ser Asn Ser Thr Glu Glu Asn Gly Phe Lys
    50                  55                  60

Gly His Gly Met Leu Ala Pro Phe Thr Ala Gly Trp Gln Ser Thr Asp
65                  70                  75                  80

Val His Pro Leu Val Ile Glu Arg Ser Glu Gly Ser Tyr Val Tyr Asp
                85                  90                  95

Ile Asp Gly Lys Lys Tyr Leu Asp Ser Leu Ala Gly Leu Trp Cys Thr
            100                 105                 110

Ala Leu Gly Gly Ser Glu Pro Arg Leu Ala Lys Ala Ala Thr Glu Gln
        115                 120                 125

Leu His Lys Leu Pro Phe Tyr His Ser Phe Trp Asn Arg Thr Thr Lys
    130                 135                 140

Pro Ser Leu Asp Leu Ala Lys Glu Leu Leu Ser Met Phe Thr Ala Arg
145                 150                 155                 160

Glu Met Gly Lys Val Phe Phe Thr Asn Ser Gly Ser Glu Ala Asn Asp
                165                 170                 175

Ser Gln Val Lys Leu Val Trp Tyr Tyr Asn Asn Ala Leu Gly Arg Pro
            180                 185                 190

Asp Lys Lys Lys Phe Ile Ala Arg Ser Lys Ser Tyr His Gly Ser Thr
        195                 200                 205

Leu Ile Ser Ala Ser Leu Ser Gly Leu Pro Ala Leu His Gln Lys Phe
    210                 215                 220

Asp Leu Pro Ala Pro Phe Val Leu His Thr Asp Cys Pro His Tyr Trp
225                 230                 235                 240

Arg Phe His Leu Pro Gly Glu Thr Glu Glu Glu Phe Ala Thr Arg Leu
                245                 250                 255

Ala Asn Asn Leu Glu Glu Leu Ile Leu Lys Glu Gly Pro Glu Thr Ile
            260                 265                 270

Ala Ala Phe Ile Ala Glu Pro Val Met Gly Ala Gly Gly Val Ile Pro
        275                 280                 285

Pro Pro Lys Thr Tyr Phe Glu Lys Val Gln Ala Ile Val Lys Lys Tyr
    290                 295                 300

Asp Ile Leu Phe Ile Ala Asp Glu Val Ile Thr Ala Phe Gly Arg Leu
305                 310                 315                 320

Gly Thr Met Phe Gly Ser Asp Met Tyr Asn Ile Lys Pro Asp Leu Val
                325                 330                 335

Ser Met Ala Lys Ala Leu Ser Ser Ala Tyr Val Pro Ile Gly Ala Ile
            340                 345                 350

Met Val Ser Pro Glu Ile Ser Asp Val Ile His Ser Gln Ser Asn Lys
        355                 360                 365

Leu Gly Ser Phe Ala His Gly Phe Thr Tyr Ser Gly His Pro Val Ala
    370                 375                 380

Cys Ala Val Ala Ile Glu Ala Leu Lys Ile Tyr Gln Glu Arg Asn Ile
385                 390                 395                 400

Pro Asp His Val Lys Gln Ile Ser Pro Arg Phe Gln Glu Gly Val Lys
                405                 410                 415

Ala Phe Ala Gly Ser Pro Ile Val Gly Glu Ile Arg Gly Val Gly Leu
            420                 425                 430

Ile Leu Gly Thr Glu Phe Ala Asp Asn Lys Ser Pro Asn Asp Pro Phe
        435                 440                 445

Pro Ala Glu Trp Gly Val Gly Ala Ile Phe Gly Ala Glu Cys Gln Lys
    450                 455                 460

Arg Gly Met Leu Val Arg Val Ala Gly Asp Asn Ile Met Met Ser Pro
465                 470                 475                 480

Pro Leu Ile Met Thr Pro Asp Glu Val Glu Glu Leu Val Ser Ile Tyr
                485                 490                 495

Gly Asp Ala Leu Lys Ala Thr Glu Glu Arg Val Ala Glu Leu Lys Ser
            500                 505                 510

Lys Lys Asn Asn
        515

<210>  55
<211>  510

<212>  PRT
<213>  Oryza sativa subsp. indica

<400>  55

Met Ile Cys Arg Ser Leu Leu Leu Leu Arg Ser Asn Ala Ala Ser Lys
1               5                   10                  15

Ala Ser Ser Ile Val Lys His Val Ala Ala Thr Gly Cys Leu Pro Glu
            20                  25                  30

Tyr Ser Ser Glu Ala Pro Ala Arg Tyr Phe Ser Ser Glu Ser Ser Leu
        35                  40                  45

Gln Val Asp Ser Thr Glu Glu Asn Gly Phe Lys Gly His Gly Met Leu
    50                  55                  60

Ala Pro Phe Thr Ala Gly Trp Gln Ser Thr Asp Leu His Pro Leu Val
65                  70                  75                  80

Ile Asp Arg Ser Glu Gly Ser Tyr Val Tyr Asp Ile Asn Gly Lys Lys
                85                  90                  95

Tyr Ile Asp Ala Leu Ala Gly Leu Trp Ser Thr Ala Leu Gly Gly Asn
            100                 105                 110

Glu Pro Arg Leu Ile Lys Ala Ala Thr Asp Gln Leu Asn Lys Leu Pro
        115                 120                 125

Phe Tyr His Ser Phe Trp Asn Arg Thr Thr Lys Pro Ser Leu Asp Leu
    130                 135                 140

Ala Asn Glu Ile Leu Ser Met Phe Thr Ala Arg Glu Met Gly Lys Ile
145                 150                 155                 160

Phe Phe Thr Asn Ser Gly Ser Glu Ala Asn Asp Ser Gln Val Lys Leu
                165                 170                 175

Val Trp Tyr Tyr Asn Asn Ala Leu Gly Arg Pro Asn Lys Lys Lys Phe
            180                 185                 190

Ile Ala Arg Ser Lys Ser Tyr His Gly Ser Thr Leu Val Ser Ala Ser
        195                 200                 205

Leu Ser Gly Leu Pro Ala Leu His Gln Lys Phe Asp Leu Pro Ala Pro
    210                 215                 220

Phe Val Leu His Thr Asp Cys Pro His Tyr Trp Arg Phe His Leu Pro
225                 230                 235                 240

Asp Glu Thr Glu Glu Glu Phe Ala Thr Arg Leu Ala Thr Asn Leu Glu
                245                 250                 255

Asn Leu Ile Leu Lys Glu Gly Pro Glu Thr Ile Ala Ala Phe Ile Ala
            260                 265                 270

Glu Pro Val Met Gly Ala Gly Gly Val Ile Pro Pro Pro Lys Thr Tyr
        275                 280                 285

Phe Glu Lys Ile Gln Ala Val Leu Lys Lys Tyr Asp Ile Leu Leu Ile
    290                 295                 300

Ala Asp Glu Val Ile Thr Ala Phe Gly Arg Leu Gly Thr Met Phe Gly
305                 310                 315                 320

Cys Asp Met Tyr Asp Ile Lys Pro Asp Leu Val Ser Ile Ala Lys Ala
                325                 330                 335

Leu Ser Ser Ala Tyr Met Pro Ile Gly Ala Ile Leu Val Ser Pro Glu
            340                 345                 350

Ile Thr Asp Val Ile Tyr Ser Gln Ser Asn Lys Leu Gly Ser Phe Ala
        355                 360                 365

His Gly Phe Thr Tyr Ser Gly His Pro Val Ser Cys Ala Val Ala Ile
    370                 375                 380

Glu Ala Leu Lys Ile Tyr Lys Glu Arg Asn Ile Ile Glu His Val Gln
385                 390                 395                 400

Lys Ile Ala Pro Arg Phe Gln Glu Gly Ile Lys Ala Phe Ser Gly Ser
                405                 410                 415

Pro Ile Val Gly Glu Ile Arg Gly Leu Gly Leu Ile Leu Gly Thr Glu
            420                 425                 430

Phe Val Asp Asn Lys Ser Pro Asn Asp Pro Phe Pro Ala Glu Trp Gly
        435                 440                 445

Val Gly Ser Leu Phe Gly Ala Glu Cys Glu Lys Arg Gly Met Leu Ile
    450                 455                 460

Arg Val Ala Gly Asp Asn Ile Met Leu Ser Pro Pro Leu Ile Met Thr
465                 470                 475                 480

Pro Asp Glu Val Glu Glu Ile Ile Ser Lys Tyr Gly Asp Ala Leu Lys

485                 490                 495

Ala Thr Glu Glu Arg Ile Ala Glu Leu Lys Ala Lys Arg Gly
            500                 505                 510

<210>  56
<211>  426
<212>  PRT
<213>  Escherichia coli

<400>  56

Met Asn Ser Asn Lys Glu Leu Met Gln Arg Arg Ser Gln Ala Ile Pro
1               5                   10                  15

Arg Gly Val Gly Gln Ile His Pro Ile Phe Ala Asp Arg Ala Glu Asn
            20                  25                  30

Cys Arg Val Trp Asp Val Glu Gly Arg Glu Tyr Leu Asp Phe Ala Gly
        35                  40                  45

Gly Ile Ala Val Leu Asn Thr Gly His Leu His Pro Lys Val Val Ala
    50                  55                  60

Ala Val Glu Ala Gln Leu Lys Lys Leu Ser His Thr Cys Phe Gln Val
65                  70                  75                  80

Leu Ala Tyr Glu Pro Tyr Leu Glu Leu Cys Glu Ile Met Asn Gln Lys
                85                  90                  95

Val Pro Gly Asp Phe Ala Lys Lys Thr Leu Leu Val Thr Thr Gly Ser
            100                 105                 110

Glu Ala Val Glu Asn Ala Val Lys Ile Ala Arg Ala Ala Thr Lys Arg
        115                 120                 125

Ser Gly Thr Ile Ala Phe Ser Gly Ala Tyr His Gly Arg Thr His Tyr
    130                 135                 140

Thr Leu Ala Leu Thr Gly Lys Val Asn Pro Tyr Ser Ala Gly Met Gly
145                 150                 155                 160

Leu Met Pro Gly His Val Tyr Arg Ala Leu Tyr Pro Cys Pro Leu His
                165                 170                 175

Gly Ile Ser Glu Asp Asp Ala Ile Ala Ser Ile His Arg Ile Phe Lys
            180                 185                 190

Asn Asp Ala Ala Pro Glu Asp Ile Ala Ala Ile Val Ile Glu Pro Val

195                 200                 205

Gln Gly Glu Gly Gly Phe Tyr Ala Ser Ser Pro Ala Phe Met Gln Arg
    210                 215                 220

Leu Arg Ala Leu Cys Asp Glu His Gly Ile Met Leu Ile Ala Asp Glu
225                 230                 235                 240

Val Gln Ser Gly Ala Gly Arg Thr Gly Thr Leu Phe Ala Met Glu Gln
                245                 250                 255

Met Gly Val Ala Pro Asp Leu Thr Thr Phe Ala Lys Ser Ile Ala Gly
            260                 265                 270

Gly Phe Pro Leu Ala Gly Val Thr Gly Arg Ala Glu Val Met Asp Ala
        275                 280                 285

Val Ala Pro Gly Gly Leu Gly Gly Thr Tyr Ala Gly Asn Pro Ile Ala
    290                 295                 300

Cys Val Ala Ala Leu Glu Val Leu Lys Val Phe Glu Gln Glu Asn Leu
305                 310                 315                 320

Leu Gln Lys Ala Asn Asp Leu Gly Gln Lys Leu Lys Asp Gly Leu Leu
                325                 330                 335

Ala Ile Ala Glu Lys His Pro Glu Ile Gly Asp Val Arg Gly Leu Gly
            340                 345                 350

Ala Met Ile Ala Ile Glu Leu Phe Glu Asp Gly Asp His Asn Lys Pro
        355                 360                 365

Asp Ala Lys Leu Thr Ala Glu Ile Val Ala Arg Ala Arg Asp Lys Gly
    370                 375                 380

Leu Ile Leu Leu Ser Cys Gly Pro Tyr Tyr Asn Val Leu Arg Ile Leu
385                 390                 395                 400

Val Pro Leu Thr Ile Glu Asp Ala Gln Ile Arg Gln Gly Leu Glu Ile
                405                 410                 415

Ile Ser Gln Cys Phe Asp Glu Ala Lys Gln
            420                 425

<210>  57
<211>  500
<212>  PRT
<213>  Homo sapiens

<400>  57

<210>  58
<211>  500
<212>  PRT
<213>  Rattus norvegicus

<400>  58

Met Ala Phe Leu Leu Thr Thr Arg Arg Leu Val Cys Ser Ser Gln Lys
1               5                   10                  15

Asn Leu His Leu Phe Thr Pro Gly Ser Arg Tyr Ile Ser Gln Ala Ala
            20                  25                  30

Ala Lys Val Asp Phe Glu Phe Asp Tyr Asp Gly Pro Leu Met Lys Thr
        35                  40                  45

Glu Val Pro Gly Pro Arg Ser Gln Glu Leu Met Lys Gln Leu Asn Thr
    50                  55                  60

Ile Gln Asn Ala Glu Ala Val His Phe Phe Cys Asn Tyr Glu Glu Ser
65                  70                  75                  80

Arg Gly Asn Tyr Leu Val Asp Val Asp Gly Asn Arg Met Leu Asp Leu
                85                  90                  95

Tyr Ser Gln Ile Ser Ser Val Pro Ile Gly Tyr Asn His Pro Ala Leu
            100                 105                 110

Ala Lys Leu Val Gln Gln Pro Gln Asn Ala Ser Thr Phe Ile Asn Arg
        115                 120                 125

Pro Ala Leu Gly Ile Leu Pro Pro Glu Asn Phe Val Asp Lys Leu Arg
    130                 135                 140

Glu Ser Leu Met Ser Val Ala Pro Lys Gly Met Cys Gln Leu Ile Thr
145                 150                 155                 160

Met Ala Cys Gly Ser Cys Ser Asn Glu Asn Ala Phe Lys Thr Ile Phe
                165                 170                 175

Met Trp Tyr Arg Ser Lys Glu Arg Gly Gln Arg Gly Phe Ser Lys Glu
            180                 185                 190

Glu Leu Glu Thr Cys Met Val Asn Gln Ser Pro Gly Cys Pro Asp Tyr
        195                 200                 205

Ser Ile Leu Ser Phe Met Gly Ala Phe His Gly Arg Thr Met Gly Cys
    210                 215                 220

Leu Ala Thr Thr His Ser Lys Ala Ile His Lys Ile Asp Ile Pro Ser
225                 230                 235                 240

Phe Asp Trp Pro Ile Ala Pro Phe Pro Arg Leu Lys Tyr Pro Leu Glu
                245                 250                 255

Glu Phe Val Thr Asp Asn Gln Gln Glu Glu Ala Arg Cys Leu Glu Glu
            260                 265                 270

Val Glu Asp Leu Ile Val Lys Tyr Arg Lys Lys Lys Arg Thr Val Ala
        275                 280                 285

Gly Ile Ile Val Glu Pro Ile Gln Ser Glu Gly Gly Asp Asn His Ala
    290                 295                 300

Ser Asp Asp Phe Phe Arg Lys Leu Arg Asp Ile Ala Arg Lys His Gly
305                 310                 315                 320

Cys Ala Phe Leu Val Asp Glu Val Gln Thr Gly Gly Gly Cys Thr Gly
                325                 330                 335

Lys Phe Trp Ala His Glu His Trp Gly Leu Asp Asp Pro Ala Asp Val
            340                 345                 350

Met Ser Phe Ser Lys Lys Met Met Thr Gly Gly Phe Phe His Lys Glu
        355                 360                 365

Glu Phe Arg Pro Ser Ala Pro Tyr Arg Ile Phe Asn Thr Trp Leu Gly
    370                 375                 380

Asp Pro Ser Lys Asn Leu Leu Leu Ala Glu Val Ile Asn Ile Ile Lys
385                 390                 395                 400

Arg Glu Asp Leu Leu Asn Asn Val Ala His Ala Gly Lys Thr Leu Leu
                405                 410                 415

Thr Gly Leu Leu Asp Leu Gln Ala Gln Tyr Pro Gln Phe Val Ser Arg
            420                 425                 430

Val Arg Gly Arg Gly Thr Phe Cys Ser Phe Asp Thr Pro Asp Glu Ala
        435                 440                 445

Ile Arg Asn Lys Leu Ile Leu Ile Ala Arg Asn Lys Gly Val Val Leu
    450                 455                 460

Gly Gly Cys Gly Asp Lys Ser Ile Arg Phe Arg Pro Thr Leu Val Phe
465                 470                 475                 480

Arg Asp His His Ala His Leu Phe Leu Asn Ile Phe Ser Gly Ile Leu
                485                 490                 495

Ala Asp Phe Lys
            500

<210>  59
<211>  500
<212>  PRT
<213>  Sus scrofa

<400>  59

Met Ala Ser Val Leu Leu Thr Arg Arg Leu Ala Cys Ser Phe Arg His
1               5                   10                  15

Asn His Arg Leu Leu Val Pro Gly Trp Arg His Ile Ser Gln Ala Ala
            20                  25                  30

Ala Lys Val Asp Val Glu Phe Asp Tyr Asp Gly Pro Leu Met Lys Thr
        35                  40                  45

Glu Val Pro Gly Pro Arg Ser Arg Glu Leu Met Lys Gln Leu Asn Ile
    50                  55                  60

Ile Gln Asn Ala Glu Ala Val His Phe Phe Cys Asn Tyr Glu Glu Ser
65                  70                  75                  80

Arg Gly Asn Tyr Leu Val Asp Val Asp Gly Asn Arg Met Leu Asp Leu
                85                  90                  95

Tyr Ser Gln Ile Ser Ser Ile Pro Ile Gly Tyr Ser His Pro Ala Leu
            100                 105                 110

Val Lys Leu Val Gln Gln Pro Gln Asn Val Ser Thr Phe Ile Asn Arg
        115                 120                 125

Pro Ala Leu Gly Ile Leu Pro Pro Glu Asn Phe Val Glu Lys Leu Arg
    130                 135                 140

Glu Ser Leu Leu Ser Val Ala Pro Lys Gly Met Ser Gln Leu Ile Thr
145                 150                 155                 160

Met Ala Cys Gly Ser Cys Ser Asn Glu Asn Ala Phe Lys Thr Ile Phe
                165                 170                 175

Met Trp Tyr Arg Ser Lys Glu Arg Gly Gln Ser Ala Phe Ser Lys Glu
            180                 185                 190

Glu Leu Glu Thr Cys Met Ile Asn Gln Ala Pro Gly Cys Pro Asp Tyr
        195                 200                 205

Ser Ile Leu Ser Phe Met Gly Ala Phe His Gly Arg Thr Met Gly Cys
    210                 215                 220

Leu Ala Thr Thr His Ser Lys Ala Ile His Lys Ile Asp Ile Pro Ser
225                 230                 235                 240

Phe Asp Trp Pro Ile Ala Pro Phe Pro Arg Leu Lys Tyr Pro Leu Glu
                245                 250                 255

Glu Phe Val Lys Glu Asn Gln Gln Glu Glu Ala Arg Cys Leu Glu Glu
            260                 265                 270

Val Glu Asp Leu Ile Val Lys Tyr Arg Lys Lys Lys Lys Thr Val Ala
        275                 280                 285

Gly Ile Ile Val Glu Pro Ile Gln Ser Glu Gly Gly Asp Asn His Ala
    290                 295                 300

Ser Asp Asp Phe Phe Arg Lys Leu Arg Asp Ile Ser Arg Lys His Gly
305                 310                 315                 320

Cys Ala Phe Leu Val Asp Glu Val Gln Thr Gly Gly Gly Ser Thr Gly
                325                 330                 335

Lys Phe Trp Ala His Glu His Trp Gly Leu Asp Asp Pro Ala Asp Val
            340                 345                 350

Met Thr Phe Ser Lys Lys Met Met Thr Gly Gly Phe Phe His Lys Glu
        355                 360                 365

Glu Phe Arg Pro Asn Ala Pro Tyr Arg Ile Phe Asn Thr Trp Leu Gly
    370                 375                 380

Asp Pro Ser Lys Asn Leu Leu Leu Ala Glu Val Ile Asn Ile Ile Lys
385                 390                 395                 400

Arg Glu Asp Leu Leu Ser Asn Ala Ala His Ala Gly Lys Val Leu Leu
                405                 410                 415

Thr Gly Leu Leu Asp Leu Gln Ala Arg Tyr Pro Gln Phe Ile Ser Arg

420                 425                 430

Val Arg Gly Arg Gly Thr Phe Cys Ser Phe Asp Thr Pro Asp Glu Ser
        435                 440                 445

Ile Arg Asn Lys Leu Ile Ser Ile Ala Arg Asn Lys Gly Val Met Leu
    450                 455                 460

Gly Gly Cys Gly Asp Lys Ser Ile Arg Phe Arg Pro Thr Leu Val Phe
465                 470                 475                 480

Arg Asp His His Ala His Leu Phe Leu Asn Ile Phe Ser Asp Ile Leu
                485                 490                 495

Ala Asp Phe Lys
            500

<210>  60
<211>  421
<212>  PRT
<213>  Escherichia coli

<400>  60

Met Ser Asn Asn Glu Phe His Gln Arg Arg Leu Ser Ala Thr Pro Arg
1               5                   10                  15

Gly Val Gly Val Met Cys Asn Phe Phe Ala Gln Ser Ala Glu Asn Ala
            20                  25                  30

Thr Leu Lys Asp Val Glu Gly Asn Glu Tyr Ile Asp Phe Ala Ala Gly
        35                  40                  45

Ile Ala Val Leu Asn Thr Gly His Arg His Pro Asp Leu Val Ala Ala
    50                  55                  60

Val Glu Gln Gln Leu Gln Gln Phe Thr His Thr Ala Tyr Gln Ile Val
65                  70                  75                  80

Pro Tyr Glu Ser Tyr Val Thr Leu Ala Glu Lys Ile Asn Ala Leu Ala
                85                  90                  95

Pro Val Ser Gly Gln Ala Lys Thr Ala Phe Phe Thr Thr Gly Ala Glu
            100                 105                 110

Ala Val Glu Asn Ala Val Lys Ile Ala Arg Ala His Thr Gly Arg Pro
        115                 120                 125

Gly Val Ile Ala Phe Ser Gly Gly Phe His Gly Arg Thr Tyr Met Thr

130                 135                 140

Met Ala Leu Thr Gly Lys Val Ala Pro Tyr Lys Ile Gly Phe Gly Pro
145                 150                 155                 160

Phe Pro Gly Ser Val Tyr His Val Pro Tyr Pro Ser Asp Leu His Gly
                165                 170                 175

Ile Ser Thr Gln Asp Ser Leu Asp Ala Ile Glu Arg Leu Phe Lys Ser
            180                 185                 190

Asp Ile Glu Ala Lys Gln Val Ala Ala Ile Ile Phe Glu Pro Val Gln
        195                 200                 205

Gly Glu Gly Gly Phe Asn Val Ala Pro Lys Glu Leu Val Ala Ala Ile
    210                 215                 220

Arg Arg Leu Cys Asp Glu His Gly Ile Val Met Ile Ala Asp Glu Val
225                 230                 235                 240

Gln Ser Gly Phe Ala Arg Thr Gly Lys Leu Phe Ala Met Asp His Tyr
                245                 250                 255

Ala Asp Lys Pro Asp Leu Met Thr Met Ala Lys Ser Leu Ala Gly Gly
            260                 265                 270

Met Pro Leu Ser Gly Val Val Gly Asn Ala Asn Ile Met Asp Ala Pro
        275                 280                 285

Ala Pro Gly Gly Leu Gly Gly Thr Tyr Ala Gly Asn Pro Leu Ala Val
    290                 295                 300

Ala Ala Ala His Ala Val Leu Asn Ile Ile Asp Lys Glu Ser Leu Cys
305                 310                 315                 320

Glu Arg Ala Asn Gln Leu Gly Gln Arg Leu Lys Asn Thr Leu Ile Asp
                325                 330                 335

Ala Lys Glu Ser Val Pro Ala Ile Ala Ala Val Arg Gly Leu Gly Ser
            340                 345                 350

Met Ile Ala Val Glu Phe Asn Asp Pro Gln Thr Gly Glu Pro Ser Ala
        355                 360                 365

Ala Ile Ala Gln Lys Ile Gln Gln Arg Ala Leu Ala Gln Gly Leu Leu
    370                 375                 380

Leu Leu Thr Cys Gly Ala Tyr Gly Asn Val Ile Arg Phe Leu Tyr Pro
385                 390                 395                 400

Leu Thr Ile Pro Asp Ala Gln Phe Asp Ala Ala Met Lys Ile Leu Gln
                405                 410                 415

Asp Ala Leu Ser Asp
            420

<210>  61
<211>  500
<212>  PRT
<213>  Mus musculus

<400>  61

Met Ala Phe Leu Leu Ile Thr Arg Arg Leu Ala Cys Ser Ser Gln Lys
1               5                   10                  15

Asn Leu His Leu Phe Ile Pro Gly Ser Arg Tyr Ile Ser Gln Ala Ala
            20                  25                  30

Ala Lys Val Asp Ile Glu Phe Asp Tyr Asp Gly Pro Leu Met Lys Thr
        35                  40                  45

Glu Val Pro Gly Pro Arg Ser Lys Glu Leu Met Lys Gln Leu Asn Thr
    50                  55                  60

Ile Gln Asn Ala Glu Ala Val His Phe Phe Cys Asn Tyr Glu Glu Ser
65                  70                  75                  80

Arg Gly Asn Tyr Leu Val Asp Val Asp Gly Asn Arg Met Leu Asp Leu
                85                  90                  95

Tyr Ser Gln Ile Ser Ser Val Pro Ile Gly Tyr Asn His Pro Ala Leu
            100                 105                 110

Ala Lys Leu Val Gln Gln Pro Gln Asn Ala Ser Thr Phe Ile Asn Arg
        115                 120                 125

Pro Ala Leu Gly Ile Leu Pro Pro Glu Asn Phe Val Asp Lys Leu Gln
    130                 135                 140

Glu Ser Leu Met Ser Val Ala Pro Arg Gly Met Ser Gln Leu Ile Thr
145                 150                 155                 160

Met Ala Cys Gly Ser Cys Ser Asn Glu Asn Ala Phe Lys Thr Ile Phe
                165                 170                 175

Met Trp Tyr Arg Ser Lys Glu Arg Gly Gln Arg Gly Phe Ser Lys Glu
            180                 185                 190

Glu Leu Glu Thr Cys Met Val Asn Gln Ser Pro Gly Cys Pro Asp Tyr
        195                 200                 205

Ser Ile Leu Ser Phe Met Gly Ala Phe His Gly Arg Thr Met Gly Cys
    210                 215                 220

Leu Ala Thr Thr His Ser Lys Ala Ile His Lys Ile Asp Ile Pro Ser
225                 230                 235                 240

Phe Asp Trp Pro Ile Ala Pro Phe Pro Arg Leu Lys Tyr Pro Leu Glu
                245                 250                 255

Glu Phe Thr Thr Asp Asn Gln Gln Glu Glu Ala Arg Cys Leu Glu Glu
            260                 265                 270

Val Glu Asp Leu Ile Val Lys Tyr Arg Lys Lys Lys Arg Thr Val Ala
        275                 280                 285

Gly Ile Ile Val Glu Pro Ile Gln Ser Glu Gly Gly Asp Asn His Ala
    290                 295                 300

Ser Asp Asp Phe Phe Arg Lys Leu Arg Asp Ile Ala Arg Lys His Gly
305                 310                 315                 320

Cys Ala Phe Leu Val Asp Glu Val Gln Thr Gly Gly Gly Cys Thr Gly
                325                 330                 335

Lys Phe Trp Ala His Glu His Trp Gly Leu Asp Asp Pro Ala Asp Val
            340                 345                 350

Met Thr Phe Ser Lys Lys Met Met Thr Gly Gly Phe Phe His Lys Glu
        355                 360                 365

Glu Phe Arg Pro Ser Ala Pro Tyr Arg Ile Phe Asn Thr Trp Leu Gly
    370                 375                 380

Asp Pro Ser Lys Asn Leu Leu Leu Ala Glu Val Ile Asn Ile Ile Lys
385                 390                 395                 400

Arg Glu Asp Leu Leu Asn Asn Val Ala Arg Val Gly Lys Thr Leu Leu
                405                 410                 415

Thr Gly Leu Leu Asp Leu Gln Ala Gln Tyr Pro Gln Phe Ile Ser Arg
            420                 425                 430

Val Arg Gly Arg Gly Thr Phe Cys Ser Phe Asp Thr Pro Asp Glu Ala
        435                 440                 445

Ile Arg Asn Lys Leu Ile Leu Ile Ala Arg Asn Lys Gly Val Val Leu
    450                 455                 460

Gly Gly Cys Gly Asp Lys Ser Ile Arg Phe Arg Pro Thr Leu Val Phe
465                 470                 475                 480

Arg Asp His His Ala His Leu Phe Leu Ser Ile Phe Ser Gly Ile Leu
                485                 490                 495

Ala Asp Phe Lys
            500

<210>  62
<211>  500
<212>  PRT
<213>  Bos taurus

<400>  62

Met Ala Ser Met Leu Val Ala Gln Arg Leu Ala Cys Ser Phe Gln His
1               5                   10                  15

Ser Tyr Arg Leu Leu Val Pro Gly Ser Arg His Ile Ser Gln Ala Ala
            20                  25                  30

Ala Lys Val Asp Val Glu Phe Asp Tyr Asp Gly Arg Leu Met Lys Thr
        35                  40                  45

Glu Val Pro Gly Leu Arg Cys Gln Glu Leu Met Lys Gln Leu Asn Ile
    50                  55                  60

Ile Gln Asn Ala Glu Ala Val His Phe Phe Cys Asn Tyr Glu Glu Ser
65                  70                  75                  80

Arg Gly Asn Tyr Leu Val Asp Val Asp Gly Asn Arg Met Leu Asp Leu
                85                  90                  95

Tyr Ser Gln Ile Ser Ser Val Pro Ile Gly Tyr Ser His Pro Gly Leu
            100                 105                 110

Leu Lys Leu Ile Gln Gln Pro Gln Asn Ala Ser Met Phe Val Asn Arg
        115                 120                 125

Pro Ala Leu Gly Ile Leu Leu Pro Glu Asn Phe Val Glu Lys Leu Arg
    130                 135                 140

Gln Ser Leu Leu Ser Val Ala Pro Lys Gly Met Ser Gln Leu Ile Thr
145                 150                 155                 160

Met Ala Cys Gly Ser Cys Ser Asn Glu Asn Gly Leu Lys Thr Ile Phe
                165                 170                 175

Met Trp Tyr Arg Ser Lys Glu Arg Gly Gln Arg Gly Phe Pro Gln Glu
            180                 185                 190

Glu Leu Glu Thr Cys Met Ile Asn Gln Ala Pro Trp Cys Pro Asp Tyr
        195                 200                 205

Ser Ile Leu Ser Phe Met Gly Ala Phe His Gly Arg Thr Met Gly Cys
    210                 215                 220

Leu Ala Thr Thr His Ser Lys Ala Ile His Lys Ile Asp Ile Pro Ser
225                 230                 235                 240

Phe Asp Trp Pro Ile Ala Pro Phe Pro Arg Leu Lys Tyr Pro Leu Glu
                245                 250                 255

Glu Phe Val Lys Glu Asn Gln Gln Glu Glu Ala Arg Cys Leu Glu Glu
            260                 265                 270

Val Glu Asp Leu Ile Val Lys Tyr Arg Lys Lys Lys Lys Thr Val Ala
        275                 280                 285

Gly Ile Ile Val Glu Pro Ile Gln Ser Glu Gly Gly Asp Asn His Ala
    290                 295                 300

Ser Asp Asp Phe Phe Arg Lys Leu Arg Asp Ile Pro Arg Lys Gln Cys
305                 310                 315                 320

Cys Ala Phe Leu Val Asp Val Val Gln Thr Gly Gly Gly Cys Thr Gly
                325                 330                 335

Lys Phe Trp Ala His Glu His Trp Ala Arg Asp Asp Pro Glu Asp Val
            340                 345                 350

Met Thr Ser Ser Lys Lys Met Met Thr Gly Gly Phe Phe His Lys Glu
        355                 360                 365

Glu Phe Arg Pro Asn Ala Pro Tyr Arg Ile Phe Asn Thr Trp Leu Gly
    370                 375                 380

Asp Pro Ser Lys Asn Leu Leu Leu Ala Glu Val Ile Asn Ile Ile Lys
385                 390                 395                 400

Arg Glu Asp Leu Leu Asn Asn Ala Ala His Ala Gly Lys Ala Leu Leu
                405                 410                 415

Thr Gly Leu Leu Asp Leu Gln Ala Arg Tyr Pro Gln Phe Ile Ser Arg
            420                 425                 430

Val Arg Gly Arg Gly Thr Phe Cys Ser Phe Asp Thr Pro Asp Asp Ser
        435                 440                 445

Ile Arg Asn Lys Leu Ile Leu Ile Ala Arg Asn Lys Gly Val Val Leu
    450                 455                 460

Gly Gly Cys Gly Asp Lys Ser Ile Arg Phe Arg Pro Thr Leu Val Phe
465                 470                 475                 480

Arg Asp His His Ala His Leu Phe Leu Asn Ile Phe Ser Asp Ile Leu
                485                 490                 495

Ala Asp Phe Lys
            500

<210>  63
<211>  471
<212>  PRT
<213>  Saccharomyces cerevisiae

<400>  63

Met Ser Ile Cys Glu Gln Tyr Tyr Pro Glu Glu Pro Thr Lys Pro Thr
1               5                   10                  15

Val Lys Thr Glu Ser Ile Pro Gly Pro Glu Ser Gln Lys Gln Leu Lys
            20                  25                  30

Glu Leu Gly Glu Val Phe Asp Thr Arg Pro Ala Tyr Phe Leu Ala Asp
        35                  40                  45

Tyr Glu Lys Ser Leu Gly Asn Tyr Ile Thr Asp Val Asp Gly Asn Thr
    50                  55                  60

Tyr Leu Asp Leu Tyr Ala Gln Ile Ser Ser Ile Ala Leu Gly Tyr Asn
65                  70                  75                  80

Asn Pro Ala Leu Ile Lys Ala Ala Gln Ser Pro Glu Met Ile Arg Ala
                85                  90                  95

Leu Val Asp Arg Pro Ala Leu Gly Asn Phe Pro Ser Lys Asp Leu Asp
            100                 105                 110

Lys Ile Leu Lys Gln Ile Leu Lys Ser Ala Pro Lys Gly Gln Asp His
        115                 120                 125

Val Trp Ser Gly Leu Ser Gly Ala Asp Ala Asn Glu Leu Ala Phe Lys
    130                 135                 140

Ala Ala Phe Ile Tyr Tyr Arg Ala Lys Gln Arg Gly Tyr Asp Ala Asp
145                 150                 155                 160

Phe Ser Glu Lys Glu Asn Leu Ser Val Met Asp Asn Asp Ala Pro Gly
                165                 170                 175

Ala Pro His Leu Ala Val Leu Ser Phe Lys Arg Ala Phe His Gly Arg
            180                 185                 190

Leu Phe Ala Ser Gly Ser Thr Thr Cys Ser Lys Pro Ile His Lys Leu
        195                 200                 205

Asp Phe Pro Ala Phe His Trp Pro His Ala Glu Tyr Pro Ser Tyr Gln
    210                 215                 220

Tyr Pro Leu Asp Glu Asn Ser Asp Ala Asn Arg Lys Glu Asp Asp His
225                 230                 235                 240

Cys Leu Ala Ile Val Glu Glu Leu Ile Lys Thr Trp Ser Ile Pro Val
                245                 250                 255

Ala Ala Leu Ile Ile Glu Pro Ile Gln Ser Glu Gly Gly Asp Asn His
            260                 265                 270

Ala Ser Lys Tyr Phe Leu Gln Lys Leu Arg Asp Ile Thr Leu Lys Tyr
        275                 280                 285

Asn Val Val Tyr Ile Ile Asp Glu Val Gln Thr Gly Val Gly Ala Thr
    290                 295                 300

Gly Lys Leu Trp Cys His Glu Tyr Ala Asp Ile Gln Pro Pro Val Asp
305                 310                 315                 320

Leu Val Thr Phe Ser Lys Lys Phe Gln Ser Ala Gly Tyr Phe Phe His
                325                 330                 335

Asp Pro Lys Phe Ile Pro Asn Lys Pro Tyr Arg Gln Phe Asn Thr Trp
            340                 345                 350

Cys Gly Glu Pro Ala Arg Met Ile Ile Ala Gly Ala Ile Gly Gln Glu

355                 360                 365

Ile Ser Asp Lys Lys Leu Thr Glu Gln Cys Ser Arg Val Gly Asp Tyr
    370                 375                 380

Leu Phe Lys Lys Leu Glu Gly Leu Gln Lys Lys Tyr Pro Glu Asn Phe
385                 390                 395                 400

Gln Asn Leu Arg Gly Lys Gly Arg Gly Thr Phe Ile Ala Trp Asp Leu
                405                 410                 415

Pro Thr Gly Glu Lys Arg Asp Leu Leu Leu Lys Lys Leu Lys Leu Asn
            420                 425                 430

Gly Cys Asn Val Gly Gly Cys Ala Val His Ala Val Arg Leu Arg Pro
        435                 440                 445

Ser Leu Thr Phe Glu Glu Lys His Ala Asp Ile Phe Ile Glu Ala Leu
    450                 455                 460

Ala Lys Ser Val Asn Glu Leu
465                 470

<210>  64
<211>  474
<212>  PRT
<213>  Schizosaccharomyces pombe

<400>  64

Met Ser Ser Thr Ala Thr Val Thr Glu Ser Thr His Phe Phe Pro Asn
1               5                   10                  15

Glu Pro Gln Gly Pro Ser Ile Lys Thr Glu Thr Ile Pro Gly Pro Lys
            20                  25                  30

Gly Lys Ala Ala Ala Glu Glu Met Ser Lys Tyr His Asp Ile Ser Ala
        35                  40                  45

Val Lys Phe Pro Val Asp Tyr Glu Lys Ser Ile Gly Asn Tyr Leu Val
    50                  55                  60

Asp Leu Asp Gly Asn Val Leu Leu Asp Val Tyr Ser Gln Ile Ala Thr
65                  70                  75                  80

Ile Pro Ile Gly Tyr Asn Asn Pro Thr Leu Leu Lys Ala Ala Lys Ser
                85                  90                  95

Asp Glu Val Ala Thr Ile Leu Met Asn Arg Pro Ala Leu Gly Asn Tyr

            100                 105                 110

Pro Pro Lys Glu Trp Ala Arg Val Ala Tyr Glu Gly Ala Ile Lys Tyr
        115                 120                 125

Ala Pro Lys Gly Gln Lys Tyr Val Tyr Phe Gln Met Ser Gly Ser Asp
    130                 135                 140

Ala Asn Glu Ile Ala Tyr Lys Leu Ala Met Leu His His Phe Asn Asn
145                 150                 155                 160

Lys Pro Arg Pro Thr Gly Asp Tyr Thr Ala Glu Glu Asn Glu Ser Cys
                165                 170                 175

Leu Asn Asn Ala Ala Pro Gly Ser Pro Glu Val Ala Val Leu Ser Phe
            180                 185                 190

Arg His Ser Phe His Gly Arg Leu Phe Gly Ser Leu Ser Thr Thr Arg
        195                 200                 205

Ser Lys Pro Val His Lys Leu Gly Met Pro Ala Phe Pro Trp Pro Gln
    210                 215                 220

Ala Asp Phe Pro Ala Leu Lys Tyr Pro Leu Glu Glu His Val Glu Glu
225                 230                 235                 240

Asn Ala Lys Glu Glu Gln Arg Cys Ile Asp Gln Val Glu Gln Ile Leu
                245                 250                 255

Thr Asn His His Cys Pro Val Val Ala Cys Ile Ile Glu Pro Ile Gln
            260                 265                 270

Ser Glu Gly Gly Asp Asn His Ala Ser Pro Asp Phe Phe His Lys Leu
        275                 280                 285

Gln Ala Thr Leu Lys Lys His Asp Val Lys Phe Ile Val Asp Glu Val
    290                 295                 300

Gln Thr Gly Val Gly Ser Thr Gly Thr Leu Trp Ala His Glu Gln Trp
305                 310                 315                 320

Asn Leu Pro Tyr Pro Pro Asp Met Val Thr Phe Ser Lys Lys Phe Gln
                325                 330                 335

Ala Ala Gly Ile Phe Tyr His Asp Leu Ala Leu Arg Pro His Ala Tyr
            340                 345                 350

Gln His Phe Asn Thr Trp Met Gly Asp Pro Phe Arg Ala Val Gln Ser
        355                 360                 365

Arg Tyr Ile Leu Gln Glu Ile Gln Asp Lys Asp Leu Leu Asn Asn Val
    370                 375                 380

Lys Ser Val Gly Asp Phe Leu Tyr Ala Gly Leu Glu Glu Leu Ala Arg
385                 390                 395                 400

Lys His Pro Gly Lys Ile Asn Asn Leu Arg Gly Lys Gly Lys Gly Thr
                405                 410                 415

Phe Ile Ala Trp Asp Cys Glu Ser Pro Ala Ala Arg Asp Lys Phe Cys
            420                 425                 430

Ala Asp Met Arg Ile Asn Gly Val Asn Ile Gly Gly Cys Gly Val Ala
        435                 440                 445

Ala Ile Arg Leu Arg Pro Met Leu Val Phe Gln Lys His His Ala Gln
    450                 455                 460

Ile Leu Leu Lys Lys Ile Asp Glu Leu Ile
465                 470

<210>  65
<211>  483
<212>  PRT
<213>  Caenorhabditis elegans

<400>  65

Met Leu Pro Arg Leu Ala Lys Ser Ser Leu Ile Gln Gln Val Arg Gly
1               5                   10                  15

Val Ser Ala Ile Ala Asn Ala Glu Pro Ser Gly Pro Ser Ile Ser Thr
            20                  25                  30

Ser Ile Pro Gly Pro Lys Ser Lys Ala Leu Lys Gln Glu Met Asp Lys
        35                  40                  45

Val His Gln Thr Thr Ser Val Arg Phe His Val Asp Tyr Glu Lys Ser
    50                  55                  60

Phe Gly Asn Tyr Val Val Asp Ala Asp Gly Asn Ala Leu Leu Asp Val
65                  70                  75                  80

Tyr Thr Gln Ile Ser Ser Leu Pro Leu Gly Tyr Asn His Pro Asp Leu
                85                  90                  95

Val Lys Val Ala Ser Gln Pro His Leu Ile Thr Ser Leu Val Ser Arg
            100                 105                 110

Pro Ala Leu Gly Ser Phe Pro Arg Thr Asp Phe Ala Asp Gly Ile Ser
        115                 120                 125

His Ala Leu Thr Ser Ile Ala Pro Lys Gly Leu Lys Ala Val Gln Thr
    130                 135                 140

Met Leu Cys Gly Thr Ser Ala Asn Glu Asn Ala Ile Lys Thr Ala Phe
145                 150                 155                 160

Ile Trp Tyr Gln Ala Gln Arg Arg Gly Gly Leu Gly Pro Asp Ala Leu
                165                 170                 175

His Leu Glu Ser Cys Met Asn Gln Gln Lys Pro Gly Thr Pro Asn Leu
            180                 185                 190

Ser Val Met Gly Phe Glu Gly Ala Phe His Gly Arg Ser Leu Cys Met
        195                 200                 205

Leu Ser Val Thr Arg Ser Lys Pro Ile His Lys Val Asp Ile Pro Ala
    210                 215                 220

Phe Asp Trp Pro Ile Ala Lys Phe Pro Arg Tyr Lys Tyr Pro Leu Asp
225                 230                 235                 240

Gln Asn Val Ala Tyr Asn Lys Lys Gln Asp Gln Glu Cys Leu Ala Asp
                245                 250                 255

Val Glu Ala Lys Ile Ser Glu Trp Lys Arg Arg Asp Asn Asp Val Ala
            260                 265                 270

Ala Ile Ile Val Glu Pro Ile Gln Ala Glu Gly Gly Asp His Tyr Gly
        275                 280                 285

Ser Pro Ala Phe Phe Gln Gly Leu Arg Asp Ile Thr Ser Lys His Gly
    290                 295                 300

Ile Val Phe Ile Val Asp Glu Val Gln Thr Gly Gly Gly Ala Thr Gly
305                 310                 315                 320

Asp Ile Trp Ala His Asp His Trp Asn Leu Ser Ser Pro Pro Asp Met
                325                 330                 335

Val Thr Phe Ser Lys Lys Leu Leu Thr Gly Gly Tyr Phe Tyr Gly Glu
            340                 345                 350

His Leu Arg Val Lys Glu Ala Tyr Arg Ile Tyr Asn Thr Trp Met Gly
        355                 360                 365

Asp Pro Thr Lys Leu Leu Leu Leu Glu Lys Ala Val Glu Val Ile Lys
    370                 375                 380

Arg Asp Gly Leu Ile Glu Gln Ser Arg Glu Val Gly Ala Glu Phe Gln
385                 390                 395                 400

Lys Arg Leu Gly Glu Leu Gln Ala Ser Ser Gly Gly Lys Leu Asp Gln
                405                 410                 415

Ala Arg Gly Arg Gly Thr Phe Ala Ala Val Asp Phe Pro Ser Gly Ser
            420                 425                 430

Leu Arg Asp Lys Phe Val Asp Leu Ala Ile Ser Asn Gly Leu His Cys
        435                 440                 445

Gly Gly Cys Gly Asp Arg Ser Leu Arg Phe Arg Pro Ser Leu Val Tyr
    450                 455                 460

Thr Lys Lys His Leu Asp Leu Thr Phe Asp Leu Leu Asp Lys Thr Leu
465                 470                 475                 480

Lys Gly Leu

<210>  66
<211>  498
<212>  PRT
<213>  Emericella nidulans

<400>  66

Met Ala Ser Ala Phe Arg Ser Ser Leu Lys Leu Arg Ala Ser Ala Arg
1               5                   10                  15

Leu Pro Ala Val Arg Thr Ile Thr Thr Thr Pro Arg Leu Arg Ala Ala
            20                  25                  30

Glu Lys Pro Tyr Phe Pro Asn Glu Pro Thr Ala Pro Lys Leu Ala Thr
        35                  40                  45

Ala Ile Pro Gly Pro Lys Asn Lys Ala Ala Ser Glu Gln Leu Asn Glu
    50                  55                  60

Val Phe Asp Val Arg Ser Leu Asn Met Leu Ala Asp Tyr Thr Lys Ser
65                  70                  75                  80

Val Gly Asn Tyr Ile Ala Asp Leu Asp Gly Asn Met Leu Leu Asp Val
                85                  90                  95

Tyr Ala Gln Ile Ala Ser Ile Pro Val Gly Tyr Asn Asn Pro His Leu
            100                 105                 110

Leu Lys Val Ala Ala Ser Pro Glu Met Ala Thr Ser Leu Ile Asn Arg
        115                 120                 125

Pro Ala Leu Gly Asn Phe Pro Ser Ala Asp Trp Ala His Ile Leu Lys
    130                 135                 140

Thr Gly Ile Leu Lys Val Ala Pro Lys Gly Leu Asp Gln Val Phe Thr
145                 150                 155                 160

Ala Met Ala Gly Ser Asp Ala Asn Glu Thr Ala Tyr Lys Ala Ala Phe
                165                 170                 175

Met Tyr Tyr Arg Gln Gln Gln Arg Gly Gly Pro Glu Lys Glu Phe Thr
            180                 185                 190

Glu Glu Glu Ile Gln Ser Ser Met Leu Asn Gln Thr Pro Gly Ser Pro
        195                 200                 205

Gln Leu Ser Ile Met Ser Phe Lys Ala Gly Phe His Gly Arg Leu Phe
    210                 215                 220

Gly Ser Leu Ser Thr Thr Arg Ser Lys Pro Ile His Lys Leu Asp Ile
225                 230                 235                 240

Pro Ala Phe Asp Trp Pro Gln Ala Pro Phe Pro Ser Leu Lys Tyr Pro
                245                 250                 255

Leu Glu Glu His Ala Lys Glu Asn Ala Glu Glu Glu Gln Arg Cys Leu
            260                 265                 270

Gln Glu Ala Glu Arg Leu Ile Lys Glu Trp His Asn Pro Val Ala Ala
        275                 280                 285

Ile Ile Val Glu Pro Ile Gln Ser Glu Gly Gly Asp Asn His Ala Ser
    290                 295                 300

Pro Ala Phe Phe Arg Gly Leu Arg Glu Ile Thr Lys Arg Asn Asn Val
305                 310                 315                 320

Leu Phe Ile Val Asp Glu Val Gln Thr Gly Val Gly Ala Thr Gly Lys
                325                 330                 335

Phe Trp Ala His Asp His Trp Asn Leu Glu Thr Pro Pro Asp Met Val
            340                 345                 350

Thr Phe Ser Lys Lys Ala Gln Thr Ala Gly Tyr Tyr Phe Gly Asn Pro
        355                 360                 365

Ala Leu Arg Pro Asn Lys Pro Tyr Arg Gln Phe Asn Thr Trp Met Gly
    370                 375                 380

Asp Pro Ser Arg Ala Leu Ile Phe Arg Gly Ile Ile Glu Glu Ile Glu
385                 390                 395                 400

Arg Leu Phe Leu Val Glu Asn Thr Ala Ala Thr Gly Asp Tyr Leu Tyr
                405                 410                 415

Ser Gly Leu Glu Arg Leu Ala Lys Gln Tyr Pro Glu His Leu Gln Asn
            420                 425                 430

Leu Arg Gly Lys Gly Gln Gly Thr Phe Ile Ala Trp Asp Thr Pro Lys
        435                 440                 445

Arg Asp Glu Phe Leu Val Lys Gly Lys Gly Val Gly Ile Asn Ile Gly
    450                 455                 460

Gly Ser Gly Gln Asn Ala Val Arg Leu Arg Pro Met Leu Ile Phe Gln
465                 470                 475                 480

Lys His His Ala Asp Ile Leu Leu Glu Ser Ile Glu Lys Ile Ile Lys
                485                 490                 495

Gln Leu

<210> 67
<211> 509
<212> PRT
<213> Ustilago maydis

<400> 67

Met Ile Ser Ser Lys Ala Thr Ser Ala Ala Arg Ala Cys Ala Arg Thr
1               5                   10                  15

Ser Arg Val Met Gln Gln Gln Gln Arg Arg Leu Leu Ala Thr Val Val
            20                  25                  30

Ser Ser Ser Ser Leu Phe Pro Gly Glu Pro Ser Ser Pro His Val Val
        35                  40                  45

Thr Ser Gln Ile Pro Gly Pro Lys Ser Lys Glu Leu Ser Asp Arg Ile
    50                  55                  60

Gly Thr Phe Gln Glu Asn Arg Thr His Gly Phe Val Val Asp Tyr Ala
65                  70                  75                  80

Lys Ser Gln Gly Asn Trp Ile Ala Asp Ala Asp Gly Asn Val Leu Leu
                85                  90                  95

Asp Met Phe Ala Gln Ile Ala Ser Ile Ala Ile Gly Tyr Asn Asn Pro
            100                 105                 110

Asp Leu Leu Ala Leu Ala Lys Thr Asp Glu Phe Ile Thr Ala Thr Met
        115                 120                 125

Asn Arg Ala Ala Leu Gly Ser Phe Pro Pro Thr Asn Trp Gln Glu Leu
    130                 135                 140

Val Glu Thr Ser Phe Gly Thr Val Lys Pro Asp Gly Leu Asn Asn Ile
145                 150                 155                 160

Phe Thr Ala Met Cys Gly Ser Cys Ala Asn Glu Asn Ala Phe Lys Ala
                165                 170                 175

Ser Phe Met Ala Tyr Arg Ala Arg Glu Arg Gly Glu Lys Ala Glu Phe
            180                 185                 190

Thr Pro Glu Glu Met Ser Ser Cys Met Lys Asn Gln Ser Pro Gly Ser
        195                 200                 205

Pro Asp Leu Ser Ile Leu Ser Phe Thr Ser Ala Phe His Gly Arg Leu
    210                 215                 220

Phe Gly Ser Leu Ser Ala Thr Arg Ser Lys Ala Ile His Lys Leu Asp
225                 230                 235                 240

Ile Pro Ser Phe Asn Trp Pro Val Val Glu Trp Pro Asp Val Lys Tyr
                245                 250                 255

Pro Phe Ala Gln Asn Ser Arg Glu Asn Ala Glu Ala Glu Lys Val Ala
            260                 265                 270

Leu Ala Ala Val Glu Glu Ala Ile Val Ser Ser Lys Lys Thr Gly Ser
        275                 280                 285

Ser Tyr Gly Pro Val Ala Ala Leu Ile Val Glu Pro Ile Gln Ser Glu

290                 295                 300

Gly Gly Asp Asn His Ala Ser Pro Ala Phe Phe Gln Gly Leu Arg Asp
305                 310                 315                 320

Val Thr Lys Lys His Gly Val Phe Met Ile Val Asp Glu Val Gln Thr
                325                 330                 335

Gly Val Gly Ala Thr Gly Ala Phe Trp Ala His Ser Lys Trp Asn Leu
            340                 345                 350

Thr Ser Pro Pro Asp Phe Val Thr Phe Ser Lys Lys Met Gln Ala Ala
        355                 360                 365

Gly Phe Tyr His Asn Ile Glu Thr Arg Pro Ser Leu Pro Tyr Arg Asn
    370                 375                 380

Tyr Asn Thr Trp Met Gly Asp Pro Ala Arg Thr Leu Gln Ala Arg Gln
385                 390                 395                 400

Ile Ile Arg Thr Ile Gln Asp His Asn Leu Ile Gln Lys Thr Asp Lys
                405                 410                 415

Val Gly Asn Tyr Ile Tyr Glu Lys Leu Phe Asp Leu Ile Glu Asn Gly
            420                 425                 430

Ala Gly Arg Gly Lys Ile Glu Lys Leu Arg Gly Glu Asn Ala Gly Thr
        435                 440                 445

Phe Leu Ala Phe Asp Gly Arg Thr Ala Lys Val Arg Asp Gln Leu Ile
    450                 455                 460

Met Glu Met Arg Lys Leu Gly Val His Met Gly Gly Cys Gly Asp Lys
465                 470                 475                 480

Ala Leu Arg Leu Arg Pro Met Leu Val Phe Glu Gln Lys His Ala Asp
                485                 490                 495

Ile Phe Leu Asp Lys Leu Glu Thr Ala Leu Gly Gln Leu
            500                 505

<210>  68
<211>  472
<212>  PRT
<213>  Kluyveromyces lactis

<400>  68

Met Ser Val Ala Ala Lys Tyr Tyr Pro Asn Glu Pro Thr Glu Pro Lys

1               5                   10                  15

Val Val Thr Ser Glu Ile Pro Gly Pro Glu Ser Lys Ala Lys Val Ala
            20                  25                  30

Ser Leu Gly Glu Val Phe Asp Ser Arg Pro Ala Tyr Phe Val Ala Asp
        35                  40                  45

Tyr Ala Lys Ser Ser Gly Asn Tyr Ile Val Asp Val Asp Gly Asn Lys
    50                  55                  60

Phe Leu Asp Val Tyr Ala Gln Ile Ser Ser Ile Ala Leu Gly Tyr Asn
65                  70                  75                  80

Asn Pro Ala Leu Ile Glu Ala Ala Lys Ser Asp Lys Met Ile Arg Ala
                85                  90                  95

Leu Val Asp Arg Pro Ala Leu Gly Asn Phe Pro Gly Ala Asp Leu Glu
            100                 105                 110

Asp Ile Leu Lys Gln Leu Leu Lys Phe Ala Pro Lys Gly Gln Asn Lys
        115                 120                 125

Ile Trp Ser Gly Leu Ser Gly Ala Asp Ala Asn Glu Leu Ala Phe Lys
    130                 135                 140

Ala Ala Phe Met Tyr Tyr Arg Gln Leu Gln Arg Gly Gly His Gly Ile
145                 150                 155                 160

Asp Phe Ser Glu Glu Glu Asn Ser Ser Val Met Glu Asn Thr Ser Pro
                165                 170                 175

Gly Ser Pro Gln Leu Ala Val Leu Ser Phe Lys Lys Ala Phe His Gly
            180                 185                 190

Arg Leu Phe Ala Ser Gly Ser Ser Thr Cys Ser Lys Pro Ile His Lys
        195                 200                 205

Leu Asp Phe Pro Ala Phe Asn Trp Pro His Gly Glu Tyr Pro Val Tyr
    210                 215                 220

Lys Tyr Pro Leu Ser Glu Asn Glu Glu Glu Asn Lys Lys Glu Asp Asp
225                 230                 235                 240

Arg Cys Leu Ala Ile Val Glu Asp Leu Ile Lys Ser Trp Pro Thr Pro
                245                 250                 255

Val Ala Ala Leu Ile Ile Glu Pro Ile Gln Ser Glu Gly Gly Asp Asn
            260                 265                 270

His Ala Ser Lys Tyr Phe Leu Gln Ser Leu Arg Asp Leu Thr Ser Lys
        275                 280                 285

Tyr Asn Val Val Tyr Ile Ile Asp Glu Val Gln Thr Gly Val Gly Ala
    290                 295                 300

Thr Gly Lys Phe Trp Cys His Glu Trp Ala Asp Ile Gln Pro Pro Val
305                 310                 315                 320

Asp Leu Val Thr Phe Ser Lys Lys Phe Gln Ser Ala Gly Tyr Trp Phe
                325                 330                 335

His Asp Asp Arg Phe Ile Pro Asn Lys Ala Tyr Arg Gln Phe Asn Thr
            340                 345                 350

Trp Cys Gly Asp Pro Ala Arg Met Ile Ile Ala Gly Ala Ile Gly Gln
        355                 360                 365

Glu Ile Val Asp Asn Asn Leu Val Asp Gln Cys Ala Arg Val Gly Asp
    370                 375                 380

Tyr Leu Phe Glu Lys Leu Glu Lys Leu Gln Ala Lys Tyr Pro Thr Arg
385                 390                 395                 400

Leu Ile Asn Leu Arg Gly Lys Asn Arg Gly Thr Phe Ile Ala Phe Asp
                405                 410                 415

Leu Glu Thr Ser Ala Glu Arg Asp Gln Leu Leu Lys Leu Leu Lys Ser
            420                 425                 430

Asn Gly Cys Asn Val Gly Gly Cys Ala Glu Lys Ser Val Arg Leu Arg
        435                 440                 445

Pro Ser Leu Thr Phe Glu Glu Lys His Ala Asp Ile Phe Val Asp Ala
    450                 455                 460

Leu Glu Lys Ser Ile Gly Gln Leu
465                 470

<210> 69
<211> 436
<212> PRT
<213> Bacillus subtilis

<400> 69
   Met Ser Gln Thr Thr Ala Ser Ile Thr Thr Ala Gln Trp Gln Gln Lys
   1               5                   10                  15
   Arg Asp Gln Phe Val Ser Lys Gly Val Ser Asn Gly Asn Arg Ser Leu
               20                  25                  30
   Ala Val Lys Gly Glu Gly Ala Glu Leu Tyr Asp Leu Asp Gly Arg Arg
           35                  40                  45
   Phe Ile Asp Phe Ala Gly Ala Ile Gly Thr Leu Asn Val Gly His Ser
       50                  55                  60
   His Pro Lys Val Val Glu Ala Val Lys Arg Gln Ala Glu Glu Leu Ile
   65                  70                  75                  80
   His Pro Gly Phe Asn Val Met Met Tyr Pro Thr Tyr Ile Glu Leu Ala
                   85                  90                  95
   Glu Lys Leu Cys Gly Ile Ala Pro Gly Ser His Glu Lys Lys Ala Ile
               100                 105                 110
   Phe Leu Asn Ser Gly Ala Glu Ala Val Glu Asn Ala Val Lys Ile Ala
           115                 120                 125
   Arg Lys Tyr Thr Lys Arg Gln Gly Val Val Ser Phe Thr Arg Gly Phe
       130                 135                 140
   His Gly Arg Thr Asn Met Thr Met Ser Met Thr Ser Lys Val Lys Pro
   145                 150                 155                 160
   Tyr Lys Phe Gly Phe Gly Pro Phe Ala Pro Glu Val Tyr Gln Ala Pro
                   165                 170                 175
   Phe Pro Tyr Tyr Tyr Gln Lys Pro Ala Gly Met Ser Asp Glu Ser Tyr
               180                 185                 190
   Asp Asp Met Val Ile Gln Ala Phe Asn Asp Phe Phe Ile Ala Ser Val
           195                 200                 205
   Ala Pro Glu Thr Val Ala Cys Val Val Met Glu Pro Val Gln Gly Glu
       210                 215                 220
   Gly Gly Phe Ile Ile Pro Ser Lys Arg Phe Val Gln His Val Ala Ser
   225                 230                 235                 240
   Phe Cys Lys Glu His Gly Ile Val Phe Val Ala Asp Glu Ile Gln Thr
                   245                 250                 255
   Gly Phe Ala Arg Thr Gly Thr Tyr Phe Ala Ile Glu His Phe Asp Val
   260 265 270
   Val Pro Asp Leu Ile Thr Val Ser Lys Ser Leu Ala Ala Gly Leu Pro
   275 280 285
   Leu Ser Gly Val Ile Gly Arg Ala Glu Met Leu Asp Ala Ala Ala Pro
   290 295 300
   Gly Glu Leu Gly Gly Thr Tyr Ala Gly Ser Pro Leu Gly Cys Ala Ala
   305 310 315 320
   Ala Leu Ala Val Leu Asp Ile Ile Glu Glu Glu Gly Leu Asn Glu Arg
   325 330 335
   Ser Glu Glu Ile Gly Lys Ile Ile Glu Asp Lys Ala Tyr Glu Trp Lys
   340 345 350
   Gln Glu Phe Pro Phe Ile Gly Asp Ile Arg Arg Leu Gly Ala Met Ala
   355 360 365
   Ala Ile Glu Ile Val Lys Asp Pro Asp Thr Arg Glu Pro Asp Lys Thr
   370 375 380
   Lys Ala Ala Ala Ile Ala Ala Tyr Ala Asn Gln Asn Gly Leu Leu Leu
   385 390 395 400
   Leu Thr Ala Gly Ile Asn Gly Asn Ile Ile Arg Phe Leu Thr Pro Leu
   405 410 415
   Val Ile Ser Asp Ser Leu Leu Asn Glu Gly Leu Ser Ile Leu Glu Ala
   420 425 430
   Gly Leu Arg Ala
   435

<210>  70
<211>  495
<212>  PRT
<213>  Dictyostelium discoideum

<400>  70

Met Ser Ser Ser Arg Leu Ile Lys Cys Leu Ser Ser Asn Asn Tyr Ile
1               5                   10                  15

Val Arg Ser Phe Ser Lys Ser Ser Ile Pro Thr Thr Pro Thr Pro Asp
            20                  25                  30

Phe Pro Gly Glu Tyr Lys Glu Pro Ile Val Lys Thr Gln Ile Pro Gly
        35                  40                  45

Pro Gln Ser Lys Ala Leu Ile Glu Arg Leu Asn Lys Leu Gln Asp Pro
    50                  55                  60

Arg Ala Ala His Phe Phe Ala Asp Tyr Ala Asn Ser Arg Gly Asn Tyr
65                  70                  75                  80

Ile Ser Asp Val Asp Gly Asn Ile Leu Leu Asp Leu Tyr Cys Gln Ile
                85                  90                  95

Ala Ser Ile Pro Ile Gly Tyr Asn Asn Pro Glu Leu Ile Lys Ala Ala
            100                 105                 110

Lys Ser Asp Arg Trp Val Ser Ala Ile Ile Asn Arg Pro Ser Leu Gly
        115                 120                 125

Val Leu Pro Pro Lys Asp Trp Pro Ala Leu Ile Glu Asn Ser Phe Met
    130                 135                 140

Gln Val Ser Pro Lys Gly Leu Asn Gln Val Phe Thr Ala Met Cys Gly
145                 150                 155                 160

Ser Cys Ala Asn Glu Cys Ala Tyr Lys Ala Val Phe Met His Tyr Gln
                165                 170                 175

His Val Lys Arg Gly Gly Lys Pro Phe Thr Pro Glu Glu Leu Ser Ser
            180                 185                 190

Cys Met Lys Asn Gln Glu Pro Gly Ser Pro Ser Leu Ser Ile Leu Ser
        195                 200                 205

Phe Lys Lys Gly Phe His Gly Arg Thr Phe Gly Thr Leu Ser Thr Thr
    210                 215                 220

Arg Ser Lys Ala Ile His Lys Leu Asp Ile Pro Ala Phe Asp Trp Pro
225                 230                 235                 240

Ala Ala Thr Phe Pro Asp Leu Lys Tyr Pro Leu Ala Glu His Ala Lys
                245                 250                 255

Glu Asn Arg Glu Ile Glu Asp Arg Cys Leu Gln Glu Val Glu Gln Leu
            260                 265                 270

Ile Lys Thr Trp His Ile Pro Val Ala Gly Ile Ile Val Glu Pro Ile
        275                 280                 285

Gln Ala Glu Gly Gly Asp Asn Tyr Ala Thr Pro Tyr Phe Phe Gln Gly
    290                 295                 300

Leu Arg Asp Ile Thr Lys Lys His Gly Val Ser Met Ile Val Asp Glu
305                 310                 315                 320

Val Gln Thr Gly Met Gly Ala Thr Gly Lys Phe Trp Ala His Glu His
                325                 330                 335

Trp Asn Leu Thr Ser Pro Pro Asp Ile Val Thr Phe Ser Lys Lys Met
            340                 345                 350

Gln Ala Ala Gly Phe Tyr His Asn Leu Asp Tyr Arg Pro Ser Glu Ser
        355                 360                 365

Tyr Arg Asn Phe Asn Thr Trp Met Gly Asp Pro Val Arg Ala Leu Glu
    370                 375                 380

Leu Glu Val Val Ile Gly Glu Ile Lys Lys Asn His Leu Leu Asp Asn
385                 390                 395                 400

Val Val Ile Thr Gly Asn Tyr Leu Lys Asp Gly Leu Phe Asp Ile Ala
                405                 410                 415

Ala Arg Tyr Pro Gly Leu Ile Gln Asn Ile Arg Gly Glu Gly Thr Phe
            420                 425                 430

Leu Ala Ile Asp Phe Pro Thr Pro Ala Glu Arg Asp Arg Val Ile Ser
        435                 440                 445

His Ile Arg Leu Leu Gly Val Glu Met Gly Gly Cys Gly Glu Arg Ser
    450                 455                 460

Ile Arg Phe Arg Pro Met Leu Val Cys Gln Pro Ser His Ile Asn Gln
465                 470                 475                 480

Phe Leu Asn Arg Phe Asp Gln Thr Met Lys Glu Leu Tyr Lys Asn
                485                 490                 495

<210>  71
<211>  449
<212>  PRT
<213>  Mycobacterium tuberculosis

<400>  71

Met Ala Ser Leu Gln Gln Ser Arg Arg Leu Val Thr Glu Ile Pro Gly
1               5                   10                  15

Pro Ala Ser Gln Ala Leu Thr His Arg Arg Ala Ala Ala Val Ser Ser
            20                  25                  30

Gly Val Gly Val Thr Leu Pro Val Phe Val Ala Arg Ala Gly Gly Gly
        35                  40                  45

Ile Val Glu Asp Val Asp Gly Asn Arg Leu Ile Asp Leu Gly Ser Gly
    50                  55                  60

Ile Ala Val Thr Thr Ile Gly Asn Ser Ser Pro Arg Val Val Asp Ala
65                  70                  75                  80

Val Arg Thr Gln Val Ala Glu Phe Thr His Thr Cys Phe Met Val Thr
                85                  90                  95

Pro Tyr Glu Gly Tyr Val Ala Val Ala Glu Gln Leu Asn Arg Ile Thr
            100                 105                 110

Pro Gly Ser Gly Pro Lys Arg Ser Val Leu Phe Asn Ser Gly Ala Glu
        115                 120                 125

Ala Val Glu Asn Ala Val Lys Ile Ala Arg Ser Tyr Thr Gly Lys Pro
    130                 135                 140

Ala Val Val Ala Phe Asp His Ala Tyr His Gly Arg Thr Asn Leu Thr
145                 150                 155                 160

Met Ala Leu Thr Ala Lys Ser Met Pro Tyr Lys Ser Gly Phe Gly Pro
                165                 170                 175

Phe Ala Pro Glu Ile Tyr Arg Ala Pro Leu Ser Tyr Pro Tyr Arg Asp
            180                 185                 190

Gly Leu Leu Asp Lys Gln Leu Ala Thr Asn Gly Glu Leu Ala Ala Ala
        195                 200                 205

Arg Ala Ile Gly Val Ile Asp Lys Gln Val Gly Ala Asn Asn Leu Ala
    210                 215                 220

Ala Leu Val Ile Glu Pro Ile Gln Gly Glu Gly Gly Phe Ile Val Pro
225                 230                 235                 240

Ala Glu Gly Phe Leu Pro Ala Leu Leu Asp Trp Cys Arg Lys Asn His
                245                 250                 255

Val Val Phe Ile Ala Asp Glu Val Gln Thr Gly Phe Ala Arg Thr Gly

            260                 265                 270

Ala Met Phe Ala Cys Glu His Glu Gly Pro Asp Gly Leu Glu Pro Asp
        275                 280                 285

Leu Ile Cys Thr Ala Lys Gly Ile Ala Asp Gly Leu Pro Leu Ser Ala
    290                 295                 300

Val Thr Gly Arg Ala Glu Ile Met Asn Ala Pro His Val Gly Gly Leu
305                 310                 315                 320

Gly Gly Thr Phe Gly Gly Asn Pro Val Ala Cys Ala Ala Ala Leu Ala
                325                 330                 335

Thr Ile Ala Thr Ile Glu Ser Asp Gly Leu Ile Glu Arg Ala Arg Gln
            340                 345                 350

Ile Glu Arg Leu Val Thr Asp Arg Leu Thr Thr Leu Gln Ala Val Asp
        355                 360                 365

Asp Arg Ile Gly Asp Val Arg Gly Arg Gly Ala Met Ile Ala Val Glu
    370                 375                 380

Leu Val Lys Ser Gly Thr Thr Glu Pro Asp Ala Gly Leu Thr Glu Arg
385                 390                 395                 400

Leu Ala Thr Ala Ala His Ala Ala Gly Val Ile Ile Leu Thr Cys Gly
                405                 410                 415

Met Phe Gly Asn Ile Ile Arg Leu Leu Pro Pro Leu Thr Ile Gly Asp
            420                 425                 430

Glu Leu Leu Ser Glu Gly Leu Asp Ile Val Cys Ala Ile Leu Ala Asp
        435                 440                 445

Leu

<210> 72
<211> 446
<212> PRT
<213> Mycobacterium leprae

<400> 72

Met Thr Ser Val Glu Gln Ser Arg Gln Leu Val Thr Glu Ile Pro Gly
1               5                   10                  15

Pro Val Ser Leu Glu Leu Ala Lys Arg Leu Asn Ala Ala Val Pro Arg

20                  25                  30

Gly Val Gly Val Thr Leu Pro Val Phe Val Thr Arg Ala Ala Gly Gly
        35                  40                  45

Val Ile Glu Asp Val Asp Gly Asn Arg Leu Ile Asp Leu Gly Ser Gly
    50                  55                  60

Ile Ala Val Thr Thr Ile Gly Asn Ser Ser Pro Arg Val Val Asp Ala
65                  70                  75                  80

Val Arg Ala Gln Val Ala Asp Phe Thr His Thr Cys Phe Ile Ile Thr
                85                  90                  95

Pro Tyr Glu Glu Tyr Val Ala Val Thr Glu Gln Leu Asn Arg Ile Thr
            100                 105                 110

Pro Gly Ser Gly Glu Lys Arg Ser Val Leu Phe Asn Ser Gly Ala Glu
        115                 120                 125

Ala Val Glu Asn Ser Ile Lys Val Ala Arg Ser Tyr Thr Arg Lys Pro
    130                 135                 140

Ala Val Val Ala Phe Asp His Ala Tyr His Gly Arg Thr Asn Leu Thr
145                 150                 155                 160

Met Ala Leu Thr Ala Lys Ser Met Pro Tyr Lys Ser Gly Phe Gly Pro
                165                 170                 175

Phe Ala Pro Glu Ile Tyr Arg Ala Pro Leu Ser Tyr Pro Tyr Arg Asp
            180                 185                 190

Gly Leu Leu Asn Lys Asp Leu Ala Thr Asp Gly Lys Leu Ala Gly Ala
        195                 200                 205

Arg Ala Ile Asn Val Ile Glu Lys Gln Val Gly Ala Asp Asp Leu Ala
    210                 215                 220

Ala Val Ile Ile Glu Pro Ile Gln Gly Glu Gly Gly Phe Ile Val Pro
225                 230                 235                 240

Ala Glu Gly Phe Leu Ala Thr Leu Leu Asp Trp Cys Arg Lys Asn Asn
                245                 250                 255

Val Met Phe Ile Ala Asp Glu Val Gln Thr Gly Phe Ala Arg Thr Gly
            260                 265                 270

Ala Met Phe Ala Cys Glu His Asp Gly Ile Val Pro Asp Leu Ile Cys
        275                 280                 285

Thr Ala Lys Gly Ile Ala Asp Gly Leu Pro Leu Ala Ala Val Thr Gly
    290                 295                 300

Arg Ala Glu Ile Met Asn Ala Pro His Val Ser Gly Leu Gly Gly Thr
305                 310                 315                 320

Phe Gly Gly Asn Pro Val Ala Cys Ala Ala Ala Leu Ala Thr Ile Thr
                325                 330                 335

Thr Ile Glu Asn Asp Gly Leu Ile Gln Arg Ala Gln Gln Ile Glu Arg
            340                 345                 350

Leu Ile Thr Asp Arg Leu Leu Arg Leu Gln Asp Ala Asp Asp Arg Ile
        355                 360                 365

Gly Asp Val Arg Gly Arg Gly Ala Met Ile Ala Val Glu Leu Val Lys
    370                 375                 380

Ser Gly Thr Ala Glu Pro Asp Pro Glu Leu Thr Glu Lys Val Ala Thr
385                 390                 395                 400

Ala Ala His Ala Thr Gly Val Ile Ile Leu Thr Cys Gly Met Phe Gly
                405                 410                 415

Asn Ile Ile Arg Leu Leu Pro Pro Leu Thr Ile Ser Asp Glu Leu Leu
            420                 425                 430

Ala Glu Gly Leu Asp Ile Leu Ser Arg Ile Leu Gly Asp Phe
        435                 440                 445

<210>  73
<211>  449
<212>  PRT
<213>  Mycobacterium bovis

<400>  73

Met Ala Ser Leu Gln Gln Ser Arg Arg Leu Val Thr Glu Ile Pro Gly
1               5                   10                  15

Pro Ala Ser Gln Ala Leu Thr His Arg Arg Ala Ala Ala Val Ser Ser
            20                  25                  30

Gly Val Gly Val Thr Leu Pro Val Phe Val Ala Arg Ala Gly Gly Gly
        35                  40                  45

Ile Val Glu Asp Val Asp Gly Asn Arg Leu Ile Asp Leu Gly Ser Gly
    50                  55                  60

Ile Ala Val Thr Thr Ile Gly Asn Ser Ser Pro Arg Val Val Asp Ala
65                  70                  75                  80

Val Arg Thr Gln Val Ala Glu Phe Thr His Thr Cys Phe Met Val Thr
                85                  90                  95

Pro Tyr Glu Gly Tyr Val Ala Val Ala Glu Gln Leu Asn Arg Ile Thr
            100                 105                 110

Pro Gly Ser Gly Pro Lys Arg Ser Val Leu Phe Asn Ser Gly Ala Glu
        115                 120                 125

Ala Val Glu Asn Ala Val Lys Ile Ala Arg Ser Tyr Thr Gly Lys Pro
    130                 135                 140

Ala Val Val Ala Phe Asp His Ala Tyr His Gly Arg Thr Asn Leu Thr
145                 150                 155                 160

Met Ala Leu Thr Ala Lys Ser Met Pro Tyr Lys Ser Gly Phe Gly Pro
                165                 170                 175

Phe Ala Pro Glu Ile Tyr Arg Ala Pro Leu Ser Tyr Pro Tyr Arg Asp
            180                 185                 190

Gly Leu Leu Asp Lys Gln Leu Ala Thr Asn Gly Glu Leu Ala Ala Ala
        195                 200                 205

Arg Ala Ile Gly Val Ile Asp Lys Gln Val Gly Ala Asn Asn Leu Ala
    210                 215                 220

Ala Leu Val Ile Glu Pro Ile Gln Gly Glu Gly Gly Phe Ile Val Pro
225                 230                 235                 240

Ala Glu Gly Phe Leu Pro Ala Leu Leu Asp Trp Cys Arg Lys Asn His
                245                 250                 255

Val Val Phe Ile Ala Asp Glu Val Gln Thr Gly Phe Ala Arg Thr Gly
            260                 265                 270

Ala Met Phe Ala Cys Glu His Glu Gly Pro Asp Gly Leu Glu Pro Asp
        275                 280                 285

Leu Ile Cys Thr Ala Lys Gly Ile Ala Asp Gly Leu Pro Leu Ser Ala
    290                 295                 300

Val Thr Gly Arg Ala Glu Ile Met Asn Ala Pro His Val Gly Gly Leu
305                 310                 315                 320

Gly Gly Thr Phe Gly Gly Asn Pro Val Ala Cys Ala Ala Ala Leu Ala
                325                 330                 335

Thr Ile Ala Thr Ile Glu Ser Asp Gly Leu Ile Glu Arg Ala Arg Gln
            340                 345                 350

Ile Glu Arg Leu Val Thr Asp Arg Leu Thr Thr Leu Gln Ala Val Asp
        355                 360                 365

Asp Arg Ile Gly Asp Val Arg Gly Arg Gly Ala Met Ile Ala Val Glu
    370                 375                 380

Leu Val Lys Ser Gly Thr Thr Glu Pro Asp Ala Gly Leu Thr Glu Arg
385                 390                 395                 400

Leu Ala Thr Ala Ala His Ala Ala Gly Val Ile Ile Leu Thr Cys Gly
                405                 410                 415

Met Phe Gly Asn Ile Ile Arg Leu Leu Pro Pro Leu Thr Ile Gly Asp
            420                 425                 430

Glu Leu Leu Ser Glu Gly Leu Asp Ile Val Cys Ala Ile Leu Ala Asp
        435                 440                 445

Leu

<210>  74
<211>  449
<212>  PRT
<213>  Mycobacterium tuberculosis

<400>  74

Met Ala Ser Leu Gln Gln Ser Arg Arg Leu Val Thr Glu Ile Pro Gly
1               5                   10                  15

Pro Ala Ser Gln Ala Leu Thr His Arg Arg Ala Ala Ala Val Ser Ser
            20                  25                  30

Gly Val Gly Val Thr Leu Pro Val Phe Val Ala Arg Ala Gly Gly Gly
        35                  40                  45

Ile Val Glu Asp Val Asp Gly Asn Arg Leu Ile Asp Leu Gly Ser Gly
    50                  55                  60

Ile Ala Val Thr Thr Ile Gly Asn Ser Ser Pro Arg Val Val Asp Ala
65                  70                  75                  80

Val Arg Thr Gln Val Ala Glu Phe Thr His Thr Cys Phe Met Val Thr
                85                  90                  95

Pro Tyr Glu Gly Tyr Val Ala Val Ala Glu Gln Leu Asn Arg Ile Thr
            100                 105                 110

Pro Gly Ser Gly Pro Lys Arg Ser Val Leu Phe Asn Ser Gly Ala Glu
        115                 120                 125

Ala Val Glu Asn Ala Val Lys Ile Ala Arg Ser Tyr Thr Gly Lys Pro
    130                 135                 140

Ala Val Val Ala Phe Asp His Ala Tyr His Gly Arg Thr Asn Leu Thr
145                 150                 155                 160

Met Ala Leu Thr Ala Lys Ser Met Pro Tyr Lys Ser Gly Phe Gly Pro
                165                 170                 175

Phe Ala Pro Glu Ile Tyr Arg Ala Pro Leu Ser Tyr Pro Tyr Arg Asp
            180                 185                 190

Gly Leu Leu Asp Lys Gln Leu Ala Thr Asn Gly Glu Leu Ala Ala Ala
        195                 200                 205

Arg Ala Ile Gly Val Ile Asp Lys Gln Val Gly Ala Asn Asn Leu Ala
    210                 215                 220

Ala Leu Val Ile Glu Pro Ile Gln Gly Glu Gly Gly Phe Ile Val Pro
225                 230                 235                 240

Ala Glu Gly Phe Leu Pro Ala Leu Leu Asp Trp Cys Arg Lys Asn His
                245                 250                 255

Val Val Phe Ile Ala Asp Glu Val Gln Thr Gly Phe Ala Arg Thr Gly
            260                 265                 270

Ala Met Phe Ala Cys Glu His Glu Gly Pro Asp Gly Leu Glu Pro Asp
        275                 280                 285

Leu Ile Cys Thr Ala Lys Gly Ile Ala Asp Gly Leu Pro Leu Ser Ala
    290                 295                 300

Val Thr Gly Arg Ala Glu Ile Met Asn Ala Pro His Val Gly Gly Leu
305                 310                 315                 320

Gly Gly Thr Phe Gly Gly Asn Pro Val Ala Cys Ala Ala Ala Leu Ala
                325                 330                 335

Thr Ile Ala Thr Ile Glu Ser Asp Gly Leu Ile Glu Arg Ala Arg Gln
            340                 345                 350

Ile Glu Arg Leu Val Thr Asp Arg Leu Thr Thr Leu Gln Ala Val Asp
        355                 360                 365

Asp Arg Ile Gly Asp Val Arg Gly Arg Gly Ala Met Ile Ala Val Glu
    370                 375                 380

Leu Val Lys Ser Gly Thr Thr Glu Pro Asp Ala Gly Leu Thr Glu Arg
385                 390                 395                 400

Leu Ala Thr Ala Ala His Ala Ala Gly Val Ile Ile Leu Thr Cys Gly
                405                 410                 415

Met Phe Gly Asn Ile Ile Arg Leu Leu Pro Pro Leu Thr Ile Gly Asp
            420                 425                 430

Glu Leu Leu Ser Glu Gly Leu Asp Ile Val Cys Ala Ile Leu Ala Asp
        435                 440                 445

Leu

<210>  75
<211>  470
<212>  PRT
<213>  Streptomyces aizunensis

<400>  75

Met Arg Pro Met Thr Ala Lys Ile Phe Ala Val Asp Ser Val Arg Pro
1               5                   10                  15

Ile Asp Glu Phe Glu Gln Asp Ala Leu Arg Val Ala Asp Val Ile Arg
            20                  25                  30

Glu Arg Gly Val Cys Leu Gly Asp Arg Val Met Leu Lys Ala Gly Asn
        35                  40                  45

Ser Ala Ser Tyr Val Cys Val Leu Tyr Ala Leu Met His Ile Gly Ala
    50                  55                  60

Ser Ile Val Leu Val Asp Gln Gln Glu His Lys Glu Glu Thr Arg Arg
65                  70                  75                  80

Ile Ala Leu Arg Thr Gly Val Lys Val Thr Phe Val Asp Asp Glu Thr
                85                  90                  95

Pro Ile Asp Gln Asp Ala Asp Pro Ile His Leu Tyr Glu Leu Met Val
            100                 105                 110

Ala Thr Gln Asn Arg Pro Pro Met Asp Ser Ala Leu Ser Phe Asp Ala
        115                 120                 125

Trp Gly Glu Leu Ser Asp Gly Leu Ile Met Trp Thr Ser Gly Ser Thr
    130                 135                 140

Gly Ser Pro Lys Gly Val Val Lys Ser Gly Gly Lys Phe Leu Ala Asn
145                 150                 155                 160

Leu Arg Arg Asn Ala His Gln Val Gly His Arg Pro Asp Asp Val Leu
                165                 170                 175

Met Pro Leu Leu Pro Phe Ala His Gln Tyr Gly Leu Ser Met Val Leu
            180                 185                 190

Ile Ala Trp Leu Thr Arg Cys Ser Leu Val Ile Ala Pro Tyr Arg Arg
        195                 200                 205

Leu Asp Arg Ala Leu Arg Met Ala Arg Asp Ser Gly Thr Thr Val Ile
    210                 215                 220

Asp Ala Thr Pro Ser Ser Tyr Arg Ser Ile Leu Gly Leu Val Thr Arg
225                 230                 235                 240

Lys Pro Ala Leu Arg Ala His Leu Ala Gly Thr Arg Met Phe Cys Val
                245                 250                 255

Gly Ala Ala Pro Leu Asp Ala Pro Leu Val Glu Ser Tyr Val Gln Glu
            260                 265                 270

Phe Gly Leu Pro Leu Leu Asp Ser Tyr Gly Ser Thr Glu Leu Asn Asn
        275                 280                 285

Ile Ala Phe Ala Thr Leu Asp Asn Pro Val Ser Cys Gly Arg Ala Met
    290                 295                 300

Glu Gly Ile Gly Leu Arg Ile Val Asp Glu Asp Gly Arg Glu Val Ala
305                 310                 315                 320

Ala Gly Gln Pro Gly Glu Ile Glu Val Asp Thr Pro Asp Ala Leu Glu

325                 330                 335

Gly Gln Ile Ala Glu Asp Gly Ser Ile Ile Pro Ala Pro Thr Gly Trp
            340                 345                 350

Gln Arg Thr Gly Asp Leu Gly His Leu Asp Ala Asp Gly Asn Leu Tyr
        355                 360                 365

Val Leu Gly Arg Lys Phe Ala Val His Arg Met Gly Tyr Thr Leu Tyr
    370                 375                 380

Pro Glu Leu Ile Glu Arg Lys Val Ala Ala Glu Gly Cys Pro Thr Arg
385                 390                 395                 400

Ile Val Pro Leu Pro Asp Glu Leu Arg Gly Ser Gln Leu Val Phe Phe
                405                 410                 415

Val Glu Asp Asp Glu Gln Arg Asp Ala Gly Tyr Trp Arg Glu Arg Leu
            420                 425                 430

Cys Gly Leu Leu Pro Ala Phe Glu Gln Pro Asn Lys Val Val Val Leu
        435                 440                 445

Glu Gln Phe Pro Leu Asn Arg Asn Gly Lys Pro Asp Lys Lys Glu Leu
    450                 455                 460

Thr Arg Met Ala Ala Glu
465                 470

<210>  76
<211>  238
<212>  PRT
<213>  Artificial Sequence

<220>
<223>  GFP protein

<400>  76

Met Ser Lys Gly Glu Glu Leu Phe Thr Gly Val Val Pro Ile Leu Val
1               5                   10                  15

Glu Leu Asp Gly Asp Val Asn Gly His Lys Phe Ser Val Ser Gly Glu
            20                  25                  30

Gly Glu Gly Asp Ala Thr Tyr Gly Lys Leu Thr Leu Lys Phe Ile Cys
        35                  40                  45

Thr Thr Gly Lys Leu Pro Val Pro Trp Pro Thr Leu Val Thr Thr Phe
    50                  55                  60

Ser Tyr Gly Val Gln Cys Phe Ser Arg Tyr Pro Asp His Met Lys Arg
65                  70                  75                  80

His Asp Phe Phe Lys Ser Ala Met Pro Glu Gly Tyr Val Gln Glu Arg
                85                  90                  95

Thr Ile Ser Phe Lys Asp Asp Gly Asn Tyr Lys Thr Arg Ala Glu Val
            100                 105                 110

Lys Phe Glu Gly Asp Thr Leu Val Asn Arg Ile Glu Leu Lys Gly Ile
        115                 120                 125

Asp Phe Lys Glu Asp Gly Asn Ile Leu Gly His Lys Leu Glu Tyr Asn
    130                 135                 140

Tyr Asn Ser His Asn Val Tyr Ile Thr Ala Asp Lys Gln Lys Asn Gly
145                 150                 155                 160

Ile Lys Ala Asn Phe Lys Ile Arg His Asn Ile Glu Asp Gly Ser Val
                165                 170                 175

Gln Leu Ala Asp His Tyr Gln Gln Asn Thr Pro Ile Gly Asp Gly Pro
            180                 185                 190

Val Leu Leu Pro Asp Asn His Tyr Leu Ser Thr Gln Ser Ala Leu Ser
        195                 200                 205

Lys Asp Pro Asn Glu Lys Arg Asp His Met Val Leu Leu Glu Phe Val
    210                 215                 220

Thr Ala Ala Gly Ile Thr His Gly Met Asp Glu Leu Tyr Lys
225                 230                 235

<210>  77
<211>  236
<212>  PRT
<213>  Artificial Sequence

<220>
<223>  RFP protein

<400>  77

Met Val Ser Lys Gly Glu Glu Asp Asn Met Ala Ile Ile Lys Glu Phe
1               5                   10                  15

Met Arg Phe Lys Val His Met Glu Gly Ser Val Asn Gly His Glu Phe
            20                  25                  30

Glu Ile Glu Gly Glu Gly Glu Gly Arg Pro Tyr Glu Gly Thr Gln Thr
        35                  40                  45

Ala Lys Leu Lys Val Thr Lys Gly Gly Pro Leu Pro Phe Ala Trp Asp
    50                  55                  60

Ile Leu Ser Pro Gln Phe Met Tyr Gly Ser Lys Ala Tyr Val Lys His
65                  70                  75                  80

Pro Ala Asp Ile Pro Asp Tyr Leu Lys Leu Ser Phe Pro Glu Gly Phe
                85                  90                  95

Lys Trp Glu Arg Val Met Asn Phe Glu Asp Gly Gly Val Val Thr Val
            100                 105                 110

Thr Gln Asp Ser Ser Leu Gln Asp Gly Glu Phe Ile Tyr Lys Val Lys
        115                 120                 125

Leu Arg Gly Thr Asn Phe Pro Ser Asp Gly Pro Val Met Gln Lys Lys
    130                 135                 140

Thr Met Gly Trp Glu Ala Ser Ser Glu Arg Met Tyr Pro Glu Asp Gly
145                 150                 155                 160

Ala Leu Lys Gly Glu Ile Lys Gln Arg Leu Lys Leu Lys Asp Gly Gly
                165                 170                 175

His Tyr Asp Ala Glu Val Lys Thr Thr Tyr Lys Ala Lys Lys Pro Val
            180                 185                 190

Gln Leu Pro Gly Ala Tyr Asn Val Asn Ile Lys Leu Asp Ile Thr Ser
        195                 200                 205

His Asn Glu Asp Tyr Thr Ile Val Glu Gln Tyr Glu Arg Ala Glu Gly
    210                 215                 220

Arg His Ser Thr Gly Gly Met Asp Glu Leu Tyr Lys
225                 230                 235

<210>  78
<211>  717
<212>  DNA
<213>  Artificial Sequence

<220>
<223>  GFP gene

<400>  78
atgagtaaag gagaagaact tttcactgga gttgtcccaa ttcttgttga attagatggt        60

gatgttaatg ggcacaaatt ttctgtcagt ggagagggtg aaggtgatgc aacatacgga 120

aaacttaccc ttaaatttat ttgcactact ggaaaactac ctgttccgtg gccaacactt 180

gtcactactt tctcttatgg tgttcaatgc ttttcccgtt atccggatca catgaaacgg 240

catgactttt tcaagagtgc catgcccgaa ggttatgtac aggaacgcac tatatctttc 300

aaagatgacg ggaactacaa gacgcgtgct gaagtcaagt ttgaaggtga tacccttgtt 360

aatcgtatcg agttaaaagg tattgatttt aaagaagatg gaaacattct cggacacaaa 420

ctggagtaca actataactc acacaatgta tacatcacgg cagacaaaca aaagaatgga 480

atcaaagcta acttcaaaat tcgccacaac attgaagatg gctccgttca actagcagac 540

cattatcaac aaaatactcc aattggcgat ggccctgtcc ttttaccaga caaccattac 600

ctgtccacac aatctgccct ttcgaaagat cccaacgaaa agcgtgacca catggtcctt 660

cttgagtttg taactgctgc tgggattaca catggcatgg atgagctcta caaataa 717

<210> 79
<211> 711
<212> DNA
<213> Artificial Sequence

<220>
<223> mCherry gene

<400> 79
atggtgtcaa agggagagga agacaatatg gcgattatca aagagttcat gcgatttaag 60

gtacacatgg agggaagcgt gaatggccat gaatttgaaa tagaggggga aggggaggga 120

cgcccatacg agggcacaca aacggctaaa ttgaaagtta ctaaaggagg cccgcttcct 180

tttgcgtggg atatactttc accccaattc atgtacggtt ctaaagcata cgtcaagcac 240

ccagcagata tacctgatta cttaaagctg agctttcctg aaggctttaa atgggaacgc 300

gtaatgaact ttgaagacgg aggagtcgtc accgtaacgc aggattcttc cttacaggat 360

ggtgagttta tttataaggt aaaattacgt ggcacaaatt ttccttctga cgggcctgtc 420

atgcagaaaa aaaccatggg atgggaagcg tcttcagaaa gaatgtatcc tgaagacggg 480

gcactgaaag gagagataaa gcagcgtttg aaactgaaag atggtggcca ctacgatgcg 540

gaagtaaaaa cgacgtataa ggcaaaaaaa cccgtgcagc tacctggtgc ttataatgtg 600

aatattaaac ttgacataac gtcgcacaac gaggattaca caatcgtgga acagtacgaa 660

agggccgagg gccgccattc aacgggcggc atggatgaac tgtataagta a 711

<210> 80
<211> 1401
<212> DNA
<213> Escherichia coli

<400> 80
atggataaga agcaagtaac ggatttaagg tcggaactac tcgattcacg ttttggtgcg 60

aagtctattt ccactatcgc agaatcaaaa cgttttccgc tgcacgaaat gcgcgacgat 120

gtcgcattcc agattatcaa tgacgaatta tatcttgatg gcaacgctcg tcagaacctg 180

gccactttct gccagacctg ggacgacgaa aatgtccaca aattgatgga tttatccatt 240

aacaaaaact ggatcgacaa agaagaatat ccgcaatccg cagccatcga cctgcgttgc 300

gtaaatatgg ttgccgatct gtggcatgcg cctgcgccga aaaatggtca ggccgttggc 360

accaacacca ttggttcttc cgaggcctgt atgctcggcg ggatggcgat gaaatggcgt 420

tggcgcaagc gtatggaagc tgcaggcaaa ccaacggata aaccaaacct ggtgtgcggt 480

ccggtacaaa tctgctggca taaattcgcc cgctactggg atgtggagct gcgtgagatc 540

cctatgcgcc ccggtcagtt gtttatggac ccgaaacgca tgattgaagc ctgtgacgaa 600

aacaccatcg gcgtggtgcc gactttcggc gtgacctaca ctggtaacta tgagttccca 660

caaccgctgc acgatgcgct ggataaattc caggccgata ccggtatcga catcgacatg 720

cacatcgacg ctgccagcgg tggcttcctg gcaccgttcg tcgccccgga tatcgtctgg 780

gacttccgcc tgccgcgtgt gaaatcgatc agtgcttcag gccataaatt cggtctggct 840

ccgctgggct gcggctgggt tatctggcgt gacgaagaag cgctgccgca ggaactggtg 900

ttcaacgttg actacctggg tggtcaaatt ggtacttttg ccatcaactt ctcccgcccg 960

gcgggtcagg taattgcaca gtactatgaa ttcctgcgcc tcggtcgtga aggctatacc 1020

aaagtacaga acgcctctta ccaggttgcc gcttatctgg cggatgaaat cgccaaactg 1080

gggccgtatg agttcatctg tacgggtcgc ccggacgaag gcatcccggc ggtttgcttc 1140

aaactgaaag atggtgaaga tccgggatac accctgtatg acctctctga acgtctgcgt 1200

ctgcgcggct ggcaggttcc ggccttcact ctcggcggtg aagccaccga catcgtggtg 1260

atgcgcatta tgtgtcgtcg cggcttcgaa atggactttg ctgaactgtt gctggaagac 1320

tacaaagcct ccctgaaata tctcagcgat cacccgaaac tgcagggtat tgcccaacag 1380

aacagcttta aacatacctg a 1401


<210> 81
<211> 1377
<212> DNA
<213> Sulfobacillus thermosulfidooxidans

<400> 81
   atgaaaaaag catcggagga agtggcgatt aatcccttgt ttgcccgccg cggagaagaa 60
   tcggttcccc gatatcgtct ctcggaagaa ggattattgc ctgaaacagc ctatcaaatt 120
   gttcatgatg aattgacttt agacggcagt gctcggttaa acttggcgac ttttgtggga 180
   acgtggatgg agccagaagc tcagaaactg tatgtcgaag cggccaataa aaatatcatc 240
   gataaagatg aatatcctga gacggctgct attgaggaac ggtgtctgaa aatcctggca 300
   aacttatgga atgctccgga tcccgccaat agtattgggg tgtccacaac aggttcatcg 360
   gaagcttgca tgttaggagg cctggcttta aaaagacggt ggcaattatc ccggcgtcaa 420
   caaggaaaat ccaccgacca tcccaatatt gtctttagtt cagctgtcca agtcgtctgg 480
   gaaaagtttg cgaattattt tgaagtcgaa ccccgctatg tgaacatcac gcccgaacat 540
   ccttatttga cgccagaagg tgtgatcaag gcggtggacg aaaatactat tggagttgtc 600
   cccattttag gggtgaccta taccggcagt tacgaacccg ttgaagctat cgcacaggct 660
   ttagatgatt tagaaaaacg cacgggcctg tccattccca ttcatgtgga tgcggcatcg 720
   ggcggttttg tggcgccctt cttgcaacca gatttggtat gggattttag actgccgcgg 780
   gtccattcta ttaatacctc aggccataaa tacggactgg tctatccggg gctgggatgg 840
   gttttatggc gggataagac gttattgcca gaggaattaa tcttcaatgt ctcatattta 900
   gggggcagta tgccaacgtt tggactcaat ttttccagac ccgggcccca agttctcctt 960
   caatactata attttcttcg gttgggacgt cagggctatt acacggtcca aaagacgtgt 1020
   caaaatgttg ctcatttttt ggcacaggaa attggggcta tggagccgtt tgagctggta 1080
   actgacggat cggatttgcc ggtatttgcc tggcgaaaaa gaccggaaga gaatccttta 1140
   tggtctttag aggatttatc ttttgtattg cgggaacggg gatggcaagt accagcctat 1200
   ccgttaccac caactatgca agacgttacc ataatgcgca ttgttgttcg caacagtttt 1260
   tctatggata tggcacagct ctttttggat gacttaaacc gggcggtggc gtatttacag 1320
   gggttagatc atccctttcc ccaggcttta cgccaacata aaccgtttgc tcattga 1377

<210> 82
<211> 1407
<212> DNA
<213> Lactobacillus brevis

<400> 82
atggctatgt tgtatggaaa acacacgcat gaaacagatg agacgctcac gccaatcttc 60

ggggccacag ctgaacgcca cgacctcccc aaatataaat tggcaaagca cgcgctcgag 120

ccccgtgaag ccgatcgatt ggttcgcgat caactattgg atgaaggaaa ctcgcggctg 180

aatctcgcca cgttctgtca gacttacatg gaaccggaag cggttgaact catgaaagat 240

acactggaga aaaacgccat cgataaatcc gagtatcctc ggaccgctga aattgaaaat 300

cgttgcgtta atatcattgc caacctctgg catgctccag aagctgagtc gttcactggc 360

acctcgacga ttggttcctc cgaggcctgc atgctggccg gtttggcgat gaagtttgct 420

tggcgtaagc gcgccaaagc gaacggtctt gacttaactg cccatcaacc taatattgtc 480

atctcagccg gttatcaagt ttgttgggaa aaattctgtg tctattggga catcgacatg 540

catgtcgttc ccatggacga tgaccacatg tccttgaatg tcgatcacgt gttagattac 600

gtggatgact acaccattgg tatcgttggc attatgggca tcacttatac tggacaatac 660

gacgatttag cccgattaga tgccgttgta gagcggtaca atcggacgac taagttcccg 720

gtatatatcc atgtcgatgc cgcttccggc ggattttaca cgccgtttat tgaacccgag 780

ctcaagtggg acttccgttt aaacaacgtg atttccatca atgcctccgg ccacaaatat 840

ggcttggttt atcccggagt cggctgggta atctggcgtg accaacagta tctaccaaaa 900

gagctggtct ttaaggtcag ctacttgggt ggtgaactac ctacgatggc catcaacttc 960

tcccacagtg cctcccaatt aatcggtcag tattacaact ttattcgctt tggttttgat 1020

ggctatcgtg aaattcaaga aaaaactcac gacgttgccc gctatctcgc gaaatcgctc 1080

actaaattag ggggcttttc cctcattaat gacggccacg agttaccgct gatctgttat 1140

gaactcactg ccgattctga tcgcgaatgg accctctacg atttatccga tcggttatta 1200

atgaagggct ggcaggttcc cacctatccc ttaccaaaaa acatgacgga ccgcgttatt 1260

caacggatcg tggttcgggc tgactttggt atgagtatgg cccacgactt tattgatgat 1320

ctaacccaag ccattcacga tctcgaccaa gcacacatcg ttttccatag tgatccgcaa 1380

cctaaaaaat acgggttcac gcactaa 1407

<210> 83
<211> 1401
<212> DNA
<213> Artificial Sequence

<220>
<223> E. coli GAD mutant E89Q gene

<400> 83
atggataaga agcaagtaac ggatttaagg tcggaactac tcgattcacg ttttggtgcg 60

aagtctattt ccactatcgc agaatcaaaa cgttttccgc tgcacgaaat gcgcgacgat 120

gtcgcattcc agattatcaa tgacgaatta tatcttgatg gcaacgctcg tcagaacctg 180

gccactttct gccagacctg ggacgacgaa aatgtccaca aattgatgga tttatccatt 240

aacaaaaact ggatcgacaa agaacagtat ccgcaatccg cagccatcga cctgcgttgc 300

gtaaatatgg ttgccgatct gtggcatgcg cctgcgccga aaaatggtca ggccgttggc 360

accaacacca ttggttcttc cgaggcctgt atgctcggcg ggatggcgat gaaatggcgt 420

tggcgcaagc gtatggaagc tgcaggcaaa ccaacggata aaccaaacct ggtgtgcggt 480

ccggtacaaa tctgctggca taaattcgcc cgctactggg atgtggagct gcgtgagatc 540

cctatgcgcc ccggtcagtt gtttatggac ccgaaacgca tgattgaagc ctgtgacgaa 600

aacaccatcg gcgtggtgcc gactttcggc gtgacctaca ctggtaacta tgagttccca 660

caaccgctgc acgatgcgct ggataaattc caggccgata ccggtatcga catcgacatg 720

cacatcgacg ctgccagcgg tggcttcctg gcaccgttcg tcgccccgga tatcgtctgg 780

gacttccgcc tgccgcgtgt gaaatcgatc agtgcttcag gccataaatt cggtctggct 840

ccgctgggct gcggctgggt tatctggcgt gacgaagaag cgctgccgca ggaactggtg 900

ttcaacgttg actacctggg tggtcaaatt ggtacttttg ccatcaactt ctcccgcccg 960

gcgggtcagg taattgcaca gtactatgaa ttcctgcgcc tcggtcgtga aggctatacc 1020

aaagtacaga acgcctctta ccaggttgcc gcttatctgg cggatgaaat cgccaaactg 1080

gggccgtatg agttcatctg tacgggtcgc ccggacgaag gcatcccggc ggtttgcttc 1140

aaactgaaag atggtgaaga tccgggatac accctgtatg acctctctga acgtctgcgt 1200

ctgcgcggct ggcaggttcc ggccttcact ctcggcggtg aagccaccga catcgtggtg 1260

atgcgcatta tgtgtcgtcg cggcttcgaa atggactttg ctgaactgtt gctggaagac 1320

tacaaagcct ccctgaaata tctcagcgat cacccgaaac tgcagggtat tgcccaacag 1380

aacagcttta aacatacctg a 1401

<210> 84
<211> 1335
<212> DNA
<213> Artificial Sequence

<220>
<223> Sth GAD mutant E84Q delta C14 gene

<400> 84
atgaaaaaag catcggagga agtggcgatt aatcccttgt ttgcccgccg cggagaagaa 60

tcggttcccc gatatcgtct ctcggaagaa ggattattgc ctgaaacagc ctatcaaatt 120

gttcatgatg aattgacttt agacggcagt gctcggttaa acttggcgac ttttgtggga 180

acgtggatgg agccagaagc tcagaaactg tatgtcgaag cggccaataa aaatatcatc 240

gataaagatc aatatcctga gacggctgct attgaggaac ggtgtctgaa aatcctggca 300

aacttatgga atgctccgga tcccgccaat agtattgggg tgtccacaac aggttcatcg 360

gaagcttgca tgttaggagg cctggcttta aaaagacggt ggcaattatc ccggcgtcaa 420

caaggaaaat ccaccgacca tcccaatatt gtctttagtt cagctgtcca agtcgtctgg 480

gaaaagtttg cgaattattt tgaagtcgaa ccccgctatg tgaacatcac gcccgaacat 540

ccttatttga cgccagaagg tgtgatcaag gcggtggacg aaaatactat tggagttgtc 600

cccattttag ggtgacctta taccggcagt tacgaacccg ttgaagctat cgcacaggct 660

ttagatgatt tagaaaaacg cacgggcctg tccattccca ttcatgtgga tgcggcatcg 720

ggcggttttg tggcgccctt cttgcaacca gatttggtat gggattttag actgccgcgg 780

gtccattcta ttaatacctc aggccataaa tacggactgg tctatccggg gctgggatgg 840

gttttatggc gggataagac gttattgcca gaggaattaa tcttcaatgt ctcatattta 900

gggggcagta tgccaacgtt tggactcaat ttttccagac ccggggccca agttctcctt 960

caatactata attttcttcg gttgggacgt cagggctatt acacggtcca aaagacgtgt 1020

caaaatgttg ctcatttttt ggcacaggaa attggggcta tggagccgtt tgagctggta 1080

actgacggat cggatttgcc ggtatttgcc tggcgaaaaa gaccggaaga gaatccttta 1140

tggtctttag aggatttatc ttttgtattg cgggaacggg gatggcaagt accagcctat 1200

ccgttaccac caactatgca agacgttacc ataatgcgca ttgttgttcg caacagtttt 1260

tctatggata tggcacagct ctttttggat gacttaaacc gggcggtggc gtatttacag 1320

gggttagatc attga 1335

<210> 85
<211> 1407
<212> DNA
<213> Artificial Sequence

<220>
<223> Lbr GAD 4AA mutant gene

<400> 85
atggccatgc tgtatggtaa gcacacccat gaaactgatg agaccctcat acccatattc 60

ggtgccactg ccgaacgtca cgacctccca aagtataagc tggcgaagca cgcgctcgag 120

ccacgtgaag ccgatcgtct ggtgcgtgat cagttgctgg atgaaggtaa ctcccgcttg 180

aatctcgcca ccttctgcca aacttacatg gaaccagaag cggtggaact catgaaggat 240

actttggaga agaacgccat agataagtcc gagtatccac gcaccgccga aatcgaaaat 300

cgttgtgtga atataatcgc caacctctgg catgcccccg aagccgagtc cttcactggt 360

acctccacca tcggctcctc cgaggcctgt atgttggccg gcctggcgat gaagtttgcc 420

tggcgtaagc gtgccaaggc gaacggcctc gacctgactg cccatcagcc aaatatcgtc 480

ataagcgccg gctatcaggt gtgctgggaa aagttctgcg tctattggga catagacatg 540

catgtcgtgc caatggacga tgaccacatg tccctgaatg tcgatcacgt gctggattac 600

gtggatgact acaccatcgg catagtgggt atcatgggta taacttatac tggtcagtac 660

gacgatctgg cccgtctgga tgccgtggta gagcgctaca atcgcaccac taagttccca 720

gtatatatac atgtcgatgc cgcctccggt ggtttttaca ccccatttat cgaaccagag 780

ctcaagtggg acttccgtct gaacaacgtg atctccataa atgcctccgg tcacaagtat 840

ggtctggtgt atccaggtgt cggttgggta atatggcgtg gtcagcaata tttgcccaag 900

gagttggtct ttaaggtctc ctacctgggc ggcagcttgc caaccatggc cataaacttc 960

tcccacagcg cctcccagct gataggccaa tattacaact ttatccgttt tggctttgat 1020

ggttatcgtg aaatccatga aaagactcac gacgtggccc gttatctcgc gaagtccctc 1080

actaagctgg gtggtttttc cctcatcaat gacggtcacg agctgccatt gatatgctat 1140

gaactcactg ccgattccga tcgtgaatgg accctctacg atctgtccga tcgcctgctg 1200

atgaagggtt ggcaagtgcc aacctatcca ctgcccaaga acatgaccga ccgtgtgatc 1260

cagcgcatag tggtgcgcgc cgactttggc atgagcatgg cccacgactt tatcgatgat 1320

ttgacccagg ccatccacga tctcgaccag gcgcacatag tgttccatag cgatccacag 1380

ccaaagaagt acggtttcac ccactaa 1407

<210> 86
<211> 1845
<212> DNA
<213> Artificial Sequence

<220>
<223> Egr kdc (harmonized) gene

<400> 86
atgggcatcg ccctggatgg tcctgaccgc gaccgcatgt tcaacgccta cttgcattct 60

tcaaggccct cctttggacc ggccaccgat gtgttcgtgc agtgcttgga ggcagaaaag 120

atcgactgga tctttggaat ccccggggag gagaacttgg acctcctcca cagcctggca 180

aaggcgcacc gccgggaggg tggggagacg atgaagctcg tggtgacccg gcatgagcag 240

gcagctgggt ttatggcagc gactgtgggc cggctgacgg gacaccctgc ggcttgcctg 300

tcaaccctcg ggcctggagc caccaacttc accaccgctc tggcgtacag caagttgggc 360

ggtttccctt tgatctgtat cactgggcag aagcccatcc gtcacagcaa acagggtgcc 420

tttcagatcc tggacatctg cggtcacttc cgggatgtgg tgaagtccac aaagagcatc 480

gaggaccccg acctcatccc cagcacaatt cgcaatgccg tgctgcaggc gcgagaggag 540

aagcctggcc ccgtccacat tgaactggcc gaggacattg ccgccctgga ggtctccaag 600

gagggcggct gggtgttccc cgcccccacc cggacgccca tcgcccgcgg ccgtcgtccg 660

ctggtggagc ccaagtgcat tgagcacgcc gtgaccctta ttcatgccgc caagcgcccg 720

ctgatttgta tcgccgctgg agccaaccgc aagaacaccc accatgctat ggaggtgttg 780

atgcggaaga cggggctgat ggtggtgtgc acacagatgg gcaagggggt tgtgtacgag 840

gacaacctcg gatacatcgg ctgcacggcc ctgtcggaca aggacctggt gcacgtggcc 900

atcaacaccg cggacctcat cgtcaacgtg ggccacgaca tcagcgagaa gccgccgttc 960

atcatgaacc acaacaagcc ccccctggtc atccacgcca acttcacccg gcccctggtg 1020

gaccccgtgt acttcccaca cctcgtgctg gttggagaca tcaacgactg cctctggcag 1080

ctcgcgaccc gcatccggcc tcagccacac tgggacttca accccttcca gatggtccgc 1140

cgggagatcg agcgcaccgt gtggaacagc ccctacgccc agtccgatgc gttcccactg 1200

acggtccagc gcctcgtgtc ggacgtccgg aaggcgatgc cggtggatgg tatcgtcagc 1260

ctcgacaatg gcatgtacaa gatctggttt gcccggcagt acaagaccat gatgggcaac 1320

accctgctgt tggacaatgc gttggccacc atgggtgctg gactgccctc ttgcattgcg 1380

gccaagctag tgtaccctga gcgggtctgc atcgcagtgt gcggcgatgg cgggttcatg 1440

atgaacagcc aggagatcga gacagcattg aggctgaacc ttcacattgt ggtcatcgtg 1500

ctcaacaaca atagctatgg catgattgcc tggaaggcga ctgccatggg catggacgac 1560

ttcgggctga actacggcaa cccggacttc gcccagtacg cccgcgccta cggggccatc 1620

ggccacaacg tcaagtccac agccgagttc ctccccaccc tggagaaggc catcaaagag 1680

cacggcgtgc acatcatcga cctccccatc tcgtacgaga cgtcggacaa ggcccttttt 1740

gaggacctgc ccaaggaagt ggaggagctc aagaaggcgg tggccaaagc catctccgag 1800

gagaagaagt tcgactggga tgccgtgacg gcagctcaga gctag 1845

<210> 87
<211> 1638
<212> DNA
<213> Artificial Sequence

<220>
<223> Ssc kdc (harmonized) gene

<400> 87
atgaacactg ctgaattgtt gattcggtgc ctcgaaaacg aaggcgtgga gtatatcttc 60

ggcttgccag gcgaagaaaa cctgcatatt ctggaagccc tcaaagagtc cccgattcgc 120

ttcattaccg tccgccatga acaaggcgcc gctttcatgg ccgacgtgta tggccgtttg 180

accggcaagg ctggcgtctg cttgtccacc ttgggcccag gcgctaccaa cctcatgact 240

ggcgtcgccg acgcaaatct ggacggcgca ccgttgatcg caatcactgg ccaagtgggc 300

accgatcgca tgcatatcga aagccatcag tatctcgact tggtggcaat gttcgccccg 360

gtcaccaaat ggaacaagca gatcgtccgg ccaaatacga ccccagaggt ggtacgtcgt 420

gccttcaaga tcgcccaaca agaaaagcca ggcgctgtac atattgacct gccagaaaac 480

atcgcagcaa tgccggtaga aggccaaccg ctgcaacgcg acggccgtga aaagatttat 540

gctagtagcc gcagcttgaa tcgcgctgcc gaggcaattg cccatgccaa aagcccattg 600

atcttggtgg gcaacggcat catccgcgcc gacgccgccg aagccctgac cgacttcgcc 660

acccaattga acatcccggt agtcaatacc ttcatgggca agggcgctat cccgtacacc 720

catccgttga gcttgtggac ggtaggtctg cagcaacgcg acttcgtcac ctgcgccttc 780

gaacaaagcg acttggtgat cgctgtgggc tacgacttga ttgaatatag cccgaagcgc 840

tggaatccag agggtacgac cccaatcatt catatcggcg aagtggccgc cgaaatcgac 900

agcagctata tcccgctgac tgaagtcgtc ggcgatatcg gcgacgcctt gaacgaaatc 960

cgtaagcgca ctgatcgtga gggcaagacc gcaccaaagt tcctgaacgt ccgcgctgag 1020

atccgcgagg attatgaacg ccatggcacc gatgctagct tcccagtcaa gccgcagaag 1080

attatttacg acctgcgcca ggtgatggcc ccagaggata ttgtcatctc cgacgtgggc 1140

gcccataaga tgtggatggc ccgccattac cattgtgacc gcccgaacac ttgtttgatc 1200

agcaacggtt tcgcagcaat gggcatcgca atcccgggcg ctgtagctgc caagttggtc 1260

tacccagaaa agaacgtcgt ggctgtcact ggcgatggcg gtttcatgat gaattgtcaa 1320

gagctggaaa cggccttgcg catcggcgca aatttcgtca ccctcatctt taacgacggc 1380

ggctatggct tgattggctg gaagcaaatc aatcaatttg gcgctccagc cttcgtggag 1440

ttcggcaacc cggacttcgt gcaattcgcc gaaagcatgg gcctgaaagg ctatcgcatc 1500

accgccgccg ccgatctcgt gccaaccttg aaggaagccc tcgcccaaga cgtaccagca 1560

gtgattgact gtccggtgga ttacagcgag aacgtgaagt ttagccagaa gagtggcgac 1620

ttgatttgtc gtatgtga 1638

<210> 88
<211> 1281
<212> DNA
<213> Escherichia coli

<400> 88
atgaacagca ataaagagtt aatgcagcgc cgcagtcagg cgattccccg tggcgttggg 60

caaattcacc cgattttcgc tgaccgcgcg gaaaactgcc gggtgtggga cgttgaaggc 120

cgtgagtatc ttgatttcgc gggcgggatt gcggtgctca ataccgggca cctgcatccg 180

aaggtggtgg ccgcggtgga agcgcagttg aaaaaactgt cgcacacctg cttccaggtg 240

ctggcttacg agccgtatct ggagctgtgc gagattatga atcagaaggt gccgggcgat 300

ttcgccaaga aaacgctgct ggttacgacc ggttccgaag cggtggaaaa cgcggtaaaa 360

atcgcccgcg ccgccaccaa acgtagcggc accatcgctt ttagcggcgc gtatcacggg 420

cgcacgcatt acacgctggc gctgaccggc aaggtgaatc cgtactctgc gggcatgggg 480

ctgatgccgg gtcatgttta tcgcgcgctt tatccttgcc cgctgcacgg cataagcgag 540

gatgacgcta tcgccagcat ccaccggatc ttcaaaaatg atgccgcgcc ggaagatatc 600

gccgccatcg tgattgagcc ggttcagggc gaaggcggtt tctacgcctc gtcgccagcc 660

tttatgcagc gtttacgcgc tctgtgtgac gagcacggga tcatgctgat tgccgatgaa 720

gtgcagagcg gcgcggggcg taccggcacg ctgtttgcga tggagcagat gggcgttgcg 780

ccggatctta ccacctttgc gaaatcgatc gcgggcggct tcccgctggc gggcgtcacc 840

gggcgcgcgg aagtaatgga tgccgtcgct ccaggcggtc tgggcggcac ctatgcgggt 900

aacccgattg cctgcgtggc tgcgctggaa gtgttgaagg tgtttgagca ggaaaatctg 960

ctgcaaaaag ccaacgatct ggggcagaag ttgaaagacg gattgctggc gatagccgaa 1020

aaacacccgg agatcggcga cgtacgcggg ctgggggcga tgatcgccat tgagctgttt 1080

gaagacggcg atcacaacaa gccggacgcc aaactcaccg ccgagatcgt ggctcgcgcc 1140

cgcgataaag gcctgattct tctctcctgc ggcccgtatt acaacgtgct gcgcatcctt 1200

gtaccgctca ccattgaaga cgctcagatc cgtcagggtc tggagatcat cagccagtgt 1260

tttgatgagg cgaagcagta g 1281

<210> 89
<211> 1413
<212> DNA
<213> Streptomyces aizunensis

<400> 89
atgcgcccca tgaccgccaa aattttcgca gtcgattcgg tccgccctat cgatgagttt 60

gagcaagatg cattacgtgt agcagacgtc attcgtgaac gtggtgtatg ccttggtgat 120

cgtgtcatgt taaaagcagg taattcggcc tcgtatgtct gcgtcttata tgcacttatg 180

cacattggag ctagtattgt tttagttgat caacaagaac acaaagagga gactcgtcgt 240

attgcccttc gtacgggtgt taaagtcacg tttgtcgatg atgagacgcc tattgatcaa 300

gatgcagatc ccattcacct ttatgaatta atggtcgcga cgcaaaatcg cccaccaatg 360

gatagtgcgt tgtcttttga tgcatggggt gagctcagtg atggactcat tatgtggacg 420

agcggttcca cgggtagccc aaaaggtgtt gttaaatccg gtggcaaatt tctcgctaat 480

ttgcgtcgta atgcacacca agtaggacac cgtcctgatg atgttcttat gcccctttta 540

ccatttgcac accaatatgg gttgtcgatg gtcttgattg cgtggcttac acgttgttcc 600

cttgttattg cgccctatcg tcgtttagat cgtgcccttc gtatggcgcg tgattcgggt 660

acaaccgtta ttgatgccac tccttcgtca tatcgtagca ttttgggttt agtcacacgc 720

aaacccgcat tgcgtgccca cctcgctggt acacgtatgt tttgcgttgg agccgcgccc 780

ttggatgccc cacttgttga gtcgtatgtg caagagtttg gtttgccatt actcgatagc 840

tatggttcaa cagagctcaa taatattgcg tttgcaactc ttgataatcc cgtaagttgt 900

gggcgtgcga tggaaggtat tggcttacgt attgttgatg aggatgggcg tgaggttgcc 960

gctggccaac ccggtgagat tgaggtagat acgcccgatg cgttggaagg gcaaatcgca 1020

gaggatggct cgattattcc cgcccctacg ggttggcaac gtaccgggga tctcggtcac 1080

ttggatgccg atggaaattt atatgtactc ggacgtaaat ttgcggtaca ccgtatgggg 1140

tatacattat accctgagtt gattgagcgt aaagtcgcgg ctgaggggtg tcctacgcgt 1200

attgtacctt tacccgatga attgcgtggt tcgcaacttg ttttctttgt cgaggatgat 1260

gagcaacgtg atgcggggta ttggcgtgag cgtctttgtg gattattgcc agcatttgag     1320

caacctaata aagtagttgt cttggagcaa tttcccctta atcgtaatgg aaaacccgat     1380

aagaaagagt taactcgtat ggcggcagaa taa                                  1413

<210>  90
<211>  452
<212>  PRT
<213>  Artificial Sequence

<220>
<223>  E. coli GAD mutant E89Q delta C14

<400>  90

Met Asp Lys Lys Gln Val Thr Asp Leu Arg Ser Glu Leu Leu Asp Ser
1               5                   10                  15

Arg Phe Gly Ala Lys Ser Ile Ser Thr Ile Ala Glu Ser Lys Arg Phe
            20                  25                  30

Pro Leu His Glu Met Arg Asp Asp Val Ala Phe Gln Ile Ile Asn Asp
        35                  40                  45

Glu Leu Tyr Leu Asp Gly Asn Ala Arg Gln Asn Leu Ala Thr Phe Cys
    50                  55                  60

Gln Thr Trp Asp Asp Glu Asn Val His Lys Leu Met Asp Leu Ser Ile
65                  70                  75                  80

Asn Lys Asn Trp Ile Asp Lys Glu Gln Tyr Pro Gln Ser Ala Ala Ile
                85                  90                  95

Asp Leu Arg Cys Val Asn Met Val Ala Asp Leu Trp His Ala Pro Ala
            100                 105                 110

Pro Lys Asn Gly Gln Ala Val Gly Thr Asn Thr Ile Gly Ser Ser Glu
        115                 120                 125

Ala Cys Met Leu Gly Gly Met Ala Met Lys Trp Arg Trp Arg Lys Arg
    130                 135                 140

Met Glu Ala Ala Gly Lys Pro Thr Asp Lys Pro Asn Leu Val Cys Gly
145                 150                 155                 160

Pro Val Gln Ile Cys Trp His Lys Phe Ala Arg Tyr Trp Asp Val Glu
                165                 170                 175

Leu Arg Glu Ile Pro Met Arg Pro Gly Gln Leu Phe Met Asp Pro Lys
            180                 185                 190

Arg Met Ile Glu Ala Cys Asp Glu Asn Thr Ile Gly Val Val Pro Thr
        195                 200                 205

Phe Gly Val Thr Tyr Thr Gly Asn Tyr Glu Phe Pro Gln Pro Leu His
    210                 215                 220

Asp Ala Leu Asp Lys Phe Gln Ala Asp Thr Gly Ile Asp Ile Asp Met
225                 230                 235                 240

His Ile Asp Ala Ala Ser Gly Gly Phe Leu Ala Pro Phe Val Ala Pro
                245                 250                 255

Asp Ile Val Trp Asp Phe Arg Leu Pro Arg Val Lys Ser Ile Ser Ala
            260                 265                 270

Ser Gly His Lys Phe Gly Leu Ala Pro Leu Gly Cys Gly Trp Val Ile
        275                 280                 285

Trp Arg Asp Glu Glu Ala Leu Pro Gln Glu Leu Val Phe Asn Val Asp
    290                 295                 300

Tyr Leu Gly Gly Gln Ile Gly Thr Phe Ala Ile Asn Phe Ser Arg Pro
305                 310                 315                 320

Ala Gly Gln Val Ile Ala Gln Tyr Tyr Glu Phe Leu Arg Leu Gly Arg
                325                 330                 335

Glu Gly Tyr Thr Lys Val Gln Asn Ala Ser Tyr Gln Val Ala Ala Tyr
            340                 345                 350

Leu Ala Asp Glu Ile Ala Lys Leu Gly Pro Tyr Glu Phe Ile Cys Thr
        355                 360                 365

Gly Arg Pro Asp Glu Gly Ile Pro Ala Val Cys Phe Lys Leu Lys Asp
    370                 375                 380

Gly Glu Asp Pro Gly Tyr Thr Leu Tyr Asp Leu Ser Glu Arg Leu Arg
385                 390                 395                 400

Leu Arg Gly Trp Gln Val Pro Ala Phe Thr Leu Gly Gly Glu Ala Thr
                405                 410                 415

Asp Ile Val Val Met Arg Ile Met Cys Arg Arg Gly Phe Glu Met Asp
            420                 425                 430

Phe Ala Glu Leu Leu Leu Glu Asp Tyr Lys Ala Ser Leu Lys Tyr Leu

435                 440                 445

Ser Asp His Pro
    450

<210>  91
<211>  1359
<212>  DNA
<213>  Artificial Sequence

<220>
<223>  E. coli GAD mutant E89Q delta C14 gene

<400>  91
atggataaga agcaagtaac ggatttaagg tcggaactac tcgattcacg ttttggtgcg      60

aagtctattt ccactatcgc agaatcaaaa cgttttccgc tgcacgaaat gcgcgacgat     120

gtcgcattcc agattatcaa tgacgaatta tatcttgatg gcaacgctcg tcagaacctg     180

gccactttct gccagacctg ggacgacgaa aatgtccaca aattgatgga tttatccatt     240

aacaaaaact ggatcgacaa agaacagtat ccgcaatccg cagccatcga cctgcgttgc     300

gtaaatatgg ttgccgatct gtggcatgcg cctgcgccga aaaatggtca ggccgttggc     360

accaacacca ttggttcttc cgaggcctgt atgctcggcg ggatggcgat gaaatggcgt     420

tggcgcaagc gtatggaagc tgcaggcaaa ccaacggata aaccaaacct ggtgtgcggt     480

ccggtacaaa tctgctggca taaattcgcc cgctactggg atgtggagct gcgtgagatc     540

cctatgcgcc ccggtcagtt gtttatggac ccgaaacgca tgattgaagc ctgtgacgaa     600

aacaccatcg gcgtggtgcc gactttcggc gtgacctaca ctggtaacta tgagttccca     660

caaccgctgc acgatgcgct ggataaattc caggccgata ccggtatcga catcgacatg     720

cacatcgacg ctgccagcgg tggcttcctg gcaccgttcg tcgccccgga tatcgtctgg     780

gacttccgcc tgccgcgtgt gaaatcgatc agtgcttcag gccataaatt cggtctggct     840

ccgctgggct gcggctgggt tatctggcgt gacgaagaag cgctgccgca ggaactggtg     900

ttcaacgttg actacctggg tggtcaaatt ggtacttttg ccatcaactt ctcccgcccg     960

gcgggtcagg taattgcaca gtactatgaa ttcctgcgcc tcggtcgtga aggctatacc    1020

aaagtacaga acgcctctta ccaggttgcc gcttatctgg cggatgaaat cgccaaactg    1080

gggccgtatg agttcatctg tacgggtcgc ccggacgaag gcatcccggc ggtttgcttc    1140

aaactgaaag atggtgaaga tccgggatac accctgtatg acctctctga acgtctgcgt    1200

ctgcgcggct ggcaggttcc ggccttcact ctcggcggtg aagccaccga catcgtggtg    1260

atgcgcatta tgtgtcgtcg cggcttcgaa atggactttg ctgaactgtt gctggaagac    1320

tacaaagcct ccctgaaata tctcagcgat cacccgtga                           1359

<210>  92
<211>  443
<212>  PRT
<213>  Artificial Sequence

<220>
<223>  S. thermosulfidooxidans GAD mutant E84Q delta C14

<400>  92

Met Lys Lys Ala Ser Glu Glu Val Ala Ile Asn Pro Leu Phe Ala Arg
1               5                   10                  15

Arg Gly Glu Glu Ser Val Pro Arg Tyr Arg Leu Ser Glu Glu Gly Leu
            20                  25                  30

Leu Pro Glu Thr Ala Tyr Gln Ile Val His Asp Glu Leu Thr Leu Asp
        35                  40                  45

Gly Ser Ala Arg Leu Asn Leu Ala Thr Phe Val Gly Thr Trp Met Glu
    50                  55                  60

Pro Glu Ala Gln Lys Leu Tyr Val Glu Ala Ala Asn Lys Asn Ile Ile
65                  70                  75                  80

Asp Lys Asp Gln Tyr Pro Glu Thr Ala Ala Ile Glu Glu Arg Cys Leu
                85                  90                  95

Lys Ile Leu Ala Asn Leu Trp Asn Ala Pro Asp Pro Ala Asn Ser Ile
            100                 105                 110

Gly Val Ser Thr Thr Gly Ser Ser Glu Ala Cys Met Leu Gly Gly Leu
        115                 120                 125

Ala Leu Lys Arg Arg Trp Gln Leu Ser Arg Arg Gln Gln Gly Lys Ser
    130                 135                 140

Thr Asp His Pro Asn Ile Val Phe Ser Ser Ala Val Gln Val Val Trp
145                 150                 155                 160

Glu Lys Phe Ala Asn Tyr Phe Glu Val Glu Pro Arg Tyr Val Asn Ile
                165                 170                 175

Thr Pro Glu His Pro Tyr Leu Thr Pro Glu Gly Val Ile Lys Ala Val
            180                 185                 190

Asp Glu Asn Thr Ile Gly Val Val Pro Ile Leu Gly Val Thr Tyr Thr
        195                 200                 205

Gly Ser Tyr Glu Pro Val Glu Ala Ile Ala Gln Ala Leu Asp Asp Leu

210                 215                 220

Glu Lys Arg Thr Gly Leu Ser Ile Pro Ile Val Asp Ala Ala Ser Gly
225                 230                 235                 240

Gly Phe Val Ala Pro Phe Leu Gln Pro Asp Leu Val Trp Asp Phe Arg
                245                 250                 255

Leu Pro Arg Val His Ser Ile Asn Thr Ser Gly His Lys Tyr Gly Leu
            260                 265                 270

Val Tyr Pro Gly Leu Gly Trp Val Leu Trp Arg Asp Lys Thr Leu Leu
        275                 280                 285

Pro Glu Glu Leu Ile Phe Asn Val Ser Tyr Leu Gly Gly Ser Met Pro
    290                 295                 300

Thr Phe Gly Leu Asn Phe Ser Arg Pro Gly Ala Gln Val Leu Leu Gln
305                 310                 315                 320

Tyr Tyr Asn Phe Leu Arg Leu Gly Arg Gln Gly Tyr Tyr Thr Val Gln
                325                 330                 335

Lys Thr Cys Gln Asn Val Ala His Phe Leu Ala Gln Glu Ile Gly Ala
            340                 345                 350

Met Glu Pro Phe Glu Leu Val Thr Asp Gly Ser Asp Leu Pro Val Phe
        355                 360                 365

Ala Trp Arg Lys Arg Pro Glu Glu Asn Pro Leu Trp Ser Leu Glu Asp
    370                 375                 380

Leu Ser Phe Val Leu Arg Glu Arg Gly Trp Gln Val Pro Ala Tyr Pro
385                 390                 395                 400

Leu Pro Pro Thr Met Gln Asp Val Thr Ile Met Arg Ile Val Val Arg
                405                 410                 415

Asn Ser Phe Ser Met Asp Met Ala Gln Leu Phe Leu Asp Asp Leu Asn
            420                 425                 430

Arg Ala Val Ala Tyr Leu Gln Gly Leu Asp His
        435                 440

## Description

### Technical field of the invention

The present invention generally relates to the biotechnology engineering, and specifically to mesophilic, methylotrophic bacteria, which allow for the production of biochemicals in a pH-independent manner. More specifically, the present invention provides a mesophilic, methylotrophic bacterium which expresses a polypeptide which has glutamate decarboxylase (GAD) activity and is catalytically active at a pH above 5.5. The present invention further provides a mesophilic, methylotrophic bacterium which expresses a) a polypeptide having α-ketoglutarate decarboxylase (KDC) activity and b) a polypeptide having GABA transaminase (GABA-TA) activity. The present invention also provides methods for the pH-independent production of biochemicals, such as γ-amino butyric acid (GABA) or related compounds, using a bacterium of the invention.

### Background of the invention

γ-amino butyric acid (GABA) is a ubiquitous non-proteinogenic amino acid, that plays various metabolic and regulatory roles in microbes, plants, animals and humans. Commercially, GABA is sold as a food supplement that promotes relaxation. GABA is also a bulk chemical that can be converted chemically or biologically to 2-pyrrolidone, which can be further converted to other bulk chemicals, such as N-methyl-2-pyrrolidone and Nylon-4. GABA and 2-pyrrolidone are currently produced from oil, which ultimately results in the release of greenhouse gasses, particularly CO2, into the atmosphere and contributes to global warming. To transition to a CO2-neutral society, alternative production processes for GABA and 2-pyrrolidone are required.

Sustainable GABA and 2-pyrrolidone production can be achieved by microbial conversion of carbohydrates or methanol. Carbohydrates have been the dominant substrate used for microbial chemical production due to their widespread availability. While carbohydrates may be a sustainable substrate in terms of atmospheric CO2 release, they compete with food and feed chains. Because of the growing global population, carbohydrate-derived substrates will become less and less sustainable in the long term. On the other hand, biobased methanol production from wood biomass or CO2-sequestration does not interfere with food and feed production.

Bacteria that can utilize methanol for growth and product formation (methylotrophs) have been identified. Examples include Bacillus methanolicus, Methylobacterium extorquens, Methylobacillus glycogenes and Methylobacillus flagellatus. Methylotrophic bacteria can be divided into two broad groups based on the underlying metabolism that they use to assimilate methanol. In both groups, methanol is first converted to formaldehyde using a methanol dehydrogenase. The first group of methylotrophic bacteria use the Ribulose-monophosphate (RuMP) cycle to react formaldehyde with RuMP to form C6-compounds that are then metabolized further. B. methanolicus and Methylobacilli belong to the RuMP cycle group of methylotrophs. The second group, which includes M. extorquens, assimilate formaldehyde by reacting it with glycine, forming serine in the pathway known as the serine cycle. Both groups of methylotrophic bacteria have been used for the production of various biochemicals from methanol. However, chemical production with methylotrophs is not as well-developed than carbohydrate-based fermentations, including GABA or 2-pyrrolidone.

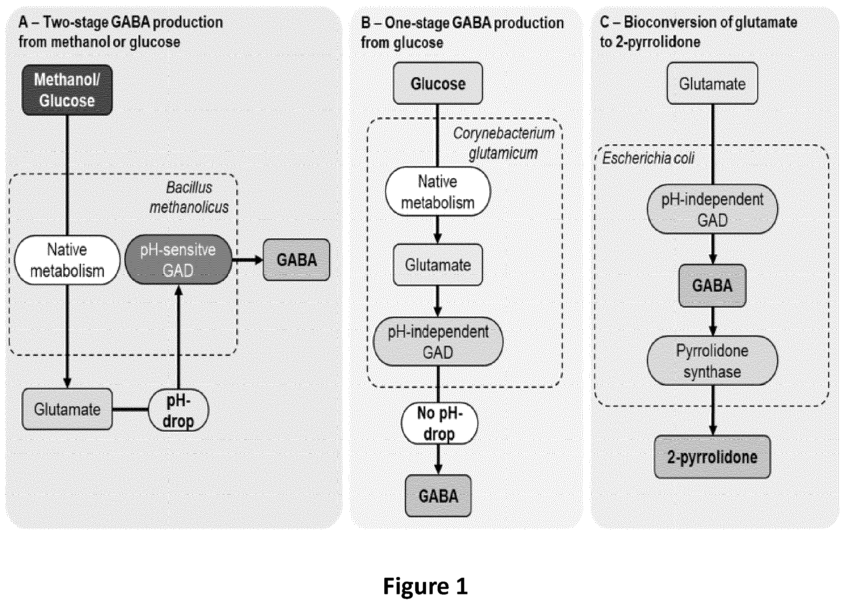

To produce GABA, most GABA-producing bacteria use the glutamate decarboxylase (GAD) enzyme, which converts glutamate to GABA through the removal of CO2. In bacteria, the GAD enzyme participates in the acid stress response. When some bacteria are exposed to low pH, they increase expression of the gad gene. This leads to rapid decarboxylation of glutamate, which consumes intracellular protons and increases the pH. GABA is produced in the process. Since GAD activity is required in acidic environments, it shows optimal activity at pH 4.0-5.0, but is inactive at neutral pH 7.0. The lack of GAD activity under normal physiological conditions of the cytosol, in particular neutral pH, prevents continuous GABA production at neutral pH. To stimulate GABA production, several studies have utilized a pH drop (Figure 1A), where external pH is reduced to stimulate GAD activity and extracellular glutamate import.

Alternatively, mutant GAD enzymes have been developed with improved activity at neutral pH. Two approaches have been used to improve the pH range of GAD enzymes. One method removed part of the C-terminus of GAD, which physically blocks the catalytic site of GAD when the environmental pH is neutral. By truncating the C-terminus of the Lactobacillus brevis and Lactobacillus plantarum GAD enzyme, the pH range of the enzyme was expanded to 4.0-8.0 (Yu et al., 2012). In the second approach, specific sites on the GAD enzymes were mutated via random mutagenesis to obtain mutant enzyme active at neutral pH. By mutating the penultimate His residue of the *E. coli* GAD (H465), the C-terminus was again unable to block GAD activity at neutral pH (Pennacchieti, et al., 2009). An E89Q mutation in *E. coli* GAD also prevents the loss of activity at neutral pH (Thu Ho et al., 2013). A similar effect was observed in *Lactobacillus brevis* GAD where four amino acid changes were introduced (T17I, D294G, E312S, Q346H).

Some lactic acid bacteria (LAB) are natural producers of GABA. Examples include Lactobacillus brevis and Lactobacillus buchneri, which formed 345 mM (35.5 g/L) and 251 mM (28.5 g/L) GABA at pH 5.0. However, the high titres of GABA were only observed when L-glutamate was added externally, which is economically unfeasible. Some studies have engineered organisms that produce GABA directly from glucose by expressing pH-insensitive GAD enzymes (Figure 1B). This enabled production of 38.6 g/L GABA from glucose in a fed-batch process by Corynebacterium glutamicum, a natural glutamate producer.

Other genes related to GABA metabolism are involved in the GABA shunt, which is mostly used for controlling intracellular GABA levels and GABA degradation. In the first step, GABA is deaminated to succinate semialdehyde by GABA transaminase. In the second step, succinate semialdehyde is oxidized to succinate by succinate semialdehyde dehydrogenase, which enters the tricarboxylic acid (TCA) cycle.

GABA production directly from methanol was previously described in the thermophilic methylotroph Bacillus methanolicus MGA3. This bacterium is a natural glutamate overproducer and can form as much as 59 g/L glutamate from methanol. By expressing a heterologous thermostable GAD enzyme from Sulfobacillus thermosulfidooxidans that converts glutamate to GABA, B. methanolicus MGA3 could convert methanol to GABA (Irla et al., 2017). However, high GABA titers could only be produced in a two-phase process, where methanol was first converted to glutamate at pH 6.5. pH then had to be lowered to 4.6 to stimulate GAD activity and GABA formation (Figure 1B). The pH-drop resulted in the production of 9 g/L GABA from methanol, which was 90-fold higher compared to a process without a pH drop (Irla et al., 2017). However, such a two-stage bioprocess is not commercially viable on large scale. The thermophilic nature of B. methanolicus MGA3 additionally complicates direct GABA formation from methanol, since most known GAD enzymes are not active at high temperatures. To enable GABA production in bioreactors, thermophilic GAD enzymes had to be used (Irla et al., 2017). However, thermophilic GAD enzymes are sensitive to pH and thus require a pH-drop and two-phase bioprocess to produce GABA from methanol with B. methanolicus MGA3. As a consequence, GABA has not been produced continuously from methanol, which is economically unfeasible.

GABA can be further converted to 2-pyrrolidone via a single enzymatic reaction. To date, only conversion of glutamate to GABA and further to 2-pyrrolidone (Figure 1C) has been demonstrated (Zhang et al., 2016). Direct conversion of methanol to 2-pyrrolidone has not been shown. This pathway can only function when GABA is continuously produced in the absence of a pH drop. 2-pyrrolidone production from methanol therefore faces the same problems as pH-independent GABA production.

### Summary of the invention

The object of the present invention is to provide means allowing a more efficient production of biochemicals, such as GABA or related compounds. More particularly, it is an object of the present invention to provide means allowing the production of biochemicals, such as GABA or related compounds, at higher nominal yield directly from methanol.

This is achieved by the present inventors who have engineered different mesophilic, methylotrophic bacteria that are able to produce biochemicals such as GABA and 2-pyrrolidone, from methanol independently of a pH drop. This has not been reported before and will make GABA production from methanol economically feasible.

The present invention thus provides in a first aspect a mesophilic, methylotrophic bacterium which expresses a polypeptide which has glutamate decarboxylase (GAD) activity and is pH-insensitive. In other words, a bacterium of this aspect of the present invention expresses a GAD enzyme, such as a GAD mutant, which shows catalytic activity at a pH above 5.5, preferably in the range of pH 6 to pH 8, more preferably at pH 7.0.

The present invention further provides in a second aspect a mesophilic, methylotrophic bacterium which expresses a) a polypeptide having α-ketoglutarate decarboxylase (KDC) activity and b) a polypeptide having GABA transaminase (GABA-TA) activity.

The present invention further provides a further aspect a method for producing a biochemical comprising cultivating a bacterium of the present invention under suitable culture conditions in a culture medium comprising a suitable substrate, such as methanol.

The present invention may be summarized by the following items:
1. A mesophilic, methylotrophic bacterium which expresses a polypeptide which has glutamate decarboxylase (GAD) activity and is catalytically active at a pH above 5.5.
2. The bacterium according to item 1, wherein the polypeptide is catalytically active at a pH in the range of pH 6 to pH 8.
3. The bacterium according to item 1 or 2, wherein the polypeptide is catalytically active at a pH in the range of pH 6.5 to pH 7.5.
4. The bacterium according to any one of items 1 to 3, wherein the polypeptide is catalytically active at pH 7.0.
5. The bacterium according to any one of items 1 to 4, where the polypeptide is a GAD mutant comprising in its amino acid sequence at least one amino acid substitution compared to the wild type GAD enzyme from which it is derived rending the mutant active at a pH above 5.5, preferably active in the range of pH 6 to pH 8.
6. The bacterium according to item 5, wherein the amino acid substitution is in a position corresponding to position 89 and/or position 465 of SEQ ID NO: 1.
7. The bacterium according to any one of items 1 to 6, wherein the polypeptide is a GAD mutant having 2 to 20 amino acids, such as 2 to 14 amino acids, deleted from the C-terminus compared to the wild type GAD enzyme from which it is derived.
8. The bacterium according to any one of items 1 to 4, wherein the polypeptide is selected from any one of the following:
   a) a polypeptide having glutamate decarboxylase (GAD) activity which comprises an amino acid sequence having at least 70%, such as at least 80%, sequence identity with SEQ ID NO: 1 and comprising an amino acid substitution at a position corresponding to position 89 and/or position 465 in SEQ ID NO: 1;
   b) a polypeptide having glutamate decarboxylase (GAD) activity which comprises an amino acid sequence having at least 70%, such as at least 80%, sequence identity with SEQ ID NO: 2 and comprising an amino acid substitution at a position corresponding to position 84 and/or position 457 in SEQ ID NO: 2;
   c) a polypeptide having glutamate decarboxylase (GAD) activity which comprises an amino acid sequence having at least 70%, such as at least 80%, sequence identity with SEQ ID NO: 3 and comprising an amino acid substitution at a position corresponding to position 93 and/or position 467 in SEQ ID NO: 3;
   d) a polypeptide having glutamate decarboxylase (GAD) activity which comprises an amino acid sequence having at least 70%, such as at least 80%, sequence identity with SEQ ID NO: 4 and comprising an amino acid substitution at a position corresponding to position 90 and/or position 467 in SEQ ID NO: 4;
   e) a polypeptide having glutamate decarboxylase (GAD) activity which comprises an amino acid sequence having at least 70%, such as at least 80%, sequence identity with SEQ ID NO: 5 and comprising an amino acid substitution at a position corresponding to position 85 and/or position 462 in SEQ ID NO: 5;
   f) a polypeptide having glutamate decarboxylase (GAD) activity which comprises an amino acid sequence having at least 70%, such as at least 80%, sequence identity with SEQ ID NO: 6 and comprising an amino acid substitution at a position corresponding to position 89 and/or position 465 in SEQ ID NO: 6;
   g) a polypeptide having glutamate decarboxylase (GAD) activity which comprises an amino acid sequence having at least 70%, such as at least 80%, sequence identity with SEQ ID NO: 7 and comprising an amino acid substitution at a position corresponding to position 89 and/or position 466 in SEQ ID NO: 7;
   h) a polypeptide having glutamate decarboxylase (GAD) activity which comprises an amino acid sequence having at least 70%, such as at least 80%, sequence identity with SEQ ID NO: 8 and comprising an amino acid substitution at a position corresponding to position 89 and/or position 466 in SEQ ID NO: 8;
   i) a polypeptide having glutamate decarboxylase (GAD) activity which comprises an amino acid sequence having at least 70%, such as at least 80%, sequence identity with SEQ ID NO: 9 and comprising an amino acid substitution at a position corresponding to position 89 and/or position 463 in SEQ ID NO: 9;
   j) a polypeptide having glutamate decarboxylase (GAD) activity which comprises an amino acid sequence having at least 70%, such as at least 80%, sequence identity with SEQ ID NO: 10 and comprising an amino acid substitution at a position corresponding to position 17, position 91, position 94, position 213, position 346 and/or position 468 in SEQ ID NO: 10; and
   k) a polypeptide having glutamate decarboxylase (GAD) activity which comprises an amino acid sequence having at least 70%, such as at least 80%, sequence identity with SEQ ID NO: 1, 2, 3, 4, 5, 6, 7, 8, 9 or 10 wherein 2 to 20 amino acids, such as 2 to 14 amino acids, are deleted from the C-terminus.
9. The bacterium according to any one of items 1 to 4, wherein the polypeptide comprises an amino acid sequence having at least 70%, such as at least 80%, sequence identity with SEQ ID NO: 1 and comprising an amino acid substitution at a position corresponding to position 89 and/or position 465 in SEQ ID NO: 1.
10. The bacterium according to any one of items 1 to 4, wherein the polypeptide is selected from any one of the following:
   aa) a polypeptide having glutamate decarboxylase (GAD) activity which comprises the amino acid sequence of SEQ ID NO: 11, 12 or 13 or an amino acid sequence corresponding to SEQ ID NO: 11, 12 or 13, wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A;
   bb) a polypeptide having glutamate decarboxylase (GAD) activity which comprises the amino acid sequence of SEQ ID NO: 14, 15 or 16 or an amino acid sequence corresponding to SEQ ID NO: 14, 15 or 16 wherein 1 to 40, such as 1 to 20, amino acids are substituted with the proviso that the amino acid at position 84 is not E and/or the amino acid at position 457 is not H, preferably with the proviso that the amino acid at position 84 is Q and/or the amino acid at position 457 is A;
   cc) a polypeptide having glutamate decarboxylase (GAD) activity which comprises the amino acid sequence of SEQ ID NO: 17, 18 or 19 or an amino acid sequence corresponding to SEQ ID NO: 17, 18 or 19 wherein 1 to 40, such as 1 to 20, amino acids are substituted with the proviso that the amino acid at position 93 is not E and/or the amino acid at position 467 is not H, preferably with the proviso that the amino acid at position 93 is Q and/or the amino acid at position 467 is A;
   dd) a polypeptide having glutamate decarboxylase (GAD) activity which comprises the amino acid sequence of SEQ ID NO: 20, 21 or 22 or an amino acid sequence corresponding to SEQ ID NO: 20, 21 or 22 wherein 1 to 40, such as 1 to 20, amino acids are substituted with the proviso that the amino acid at position 90 is not E and/or the amino acid at position 467 is not H, preferably with the proviso that the amino acid at position 90 is Q and/or the amino acid at position 467 is A;
   ee) a polypeptide having glutamate decarboxylase (GAD) activity which comprises the amino acid sequence of SEQ ID NO: 23, 24 or 25 or an amino acid sequence corresponding to SEQ ID NO: 23, 24 or 25 wherein 1 to 40, such as 1 to 20, amino acids are substituted with the proviso that the amino acid at position 85 is not E and/or the amino acid at position 462 is not H, preferably with the proviso that the amino acid at position 85 is Q and/or the amino acid at position 462 is A;
   ff) a polypeptide having glutamate decarboxylase (GAD) activity which comprises the amino acid sequence of SEQ ID NO: 26, 27 or 28 or an amino acid sequence corresponding to SEQ ID NO: 26, 27 or 28 wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A;
   gg) a polypeptide having glutamate decarboxylase (GAD) activity which comprises the amino acid sequence of SEQ ID NO: 29, 30 or 31 or an amino acid sequence corresponding to SEQ ID NO: 29, 30 or 31 wherein 1 to 40, such as 1 to 20, amino acids are substituted with the proviso that the amino acid at position 89 is not E and/or the amino acid at position 466 is not H, preferably with the proviso that the amino acid at position 89 is Q and/or the amino acid at position 466 is A;
   hh) a polypeptide having glutamate decarboxylase (GAD) activity which comprises the amino acid sequence of SEQ ID NO: 32, 33 or 34 or an amino acid sequence corresponding to SEQ ID NO: 32, 33 or 34 wherein 1 to 40, such as 1 to 20, amino acids are substituted with the proviso that the amino acid at position 89 is not E and/or the amino acid at position 466 is not H, preferably with the proviso that the amino acid at position 89 is Q and/or the amino acid at position 466 is A;
   ii) a polypeptide having glutamate decarboxylase (GAD) activity which comprises the amino acid sequence of SEQ ID NO: 35, 36 or 37 or an amino acid sequence corresponding to SEQ ID NO: 35, 36 or 37 wherein 1 to 40, such as 1 to 20, amino acids are substituted with the proviso that the amino acid at position 89 is not E and/or the amino acid at position 463 is not H, preferably with the proviso that the amino acid at position 89 is Q and/or the amino acid at position 463 is A;
   jj) a polypeptide having glutamate decarboxylase (GAD) activity which comprises the amino acid sequence of SEQ ID NO: 38 or an amino acid sequence corresponding to SEQ ID NO: 38 wherein 1 to 40, such as 1 to 20, amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H; and
   kk) a polypeptide having glutamate decarboxylase (GAD) activity which comprises an amino acid sequence having at least 70%, such as at least 80%, sequence identity with any one of SEQ ID NOs: 11 to 38, wherein 2 to 20 amino acids, such as 2 to 14 amino acids, are deleted from the C-terminus.
11. The bacterium according to any one of items 1 to 4, wherein the polypeptide comprises the amino acid sequence of SEQ ID NO: 11, 12 or 13 or an amino acid sequence corresponding to SEQ ID NO: 11, 12, or 13, wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A.
12. The bacterium according to any one of items 1 to 4, wherein the polypeptide comprises the amino acid sequence of SEQ ID NO: 90 or an amino acid sequence corresponding to SEQ ID NO: 90, wherein 1 to 40, such as 1 to 20, amino acids are substituted with the proviso that the amino acid at position 89 is not E, preferably with the proviso that the amino acid at position 89 is Q.
13. A mesophilic, methylotrophic bacterium which expresses a) a polypeptide having α-ketoglutarate decarboxylase (KDC) activity and b) a polypeptide having GABA transaminase (GABA-TA) activity.
14. The bacterium according to item 13, wherein the polypeptide having KDC activity comprises the amino acid sequence of SEQ ID NO: 39, 40, 41 or 42 or an amino acid sequence having at least 70%, such as at least 80%, sequence identity with SEQ ID NO: 39, 40, 41 or 42.
15. The bacterium according to item 13 or 14, wherein the polypeptide having KDC activity comprises the amino acid sequence of SEQ ID NO: 40 or an amino acid sequence having at least 70%, such as at least 80%, sequence identity with SEQ ID NO: 40.
16. The bacterium according to any one of items 13 to 15, wherein the polypeptide having GABA-TA activity comprises the amino acid sequence of any one of SEQ ID NOs: 43 to 74 or an amino acid sequence having at least 70%, such as at least 80%, sequence identity with any one of SEQ ID NO: 43 to 74.
17. The bacterium according to any one of items 13 to 15, wherein the polypeptide having GABA-TA activity comprises the amino acid sequence of SEQ ID NO: 43 or an amino acid sequence having at least 70%, such as at least 80%, sequence identity with SEQ ID NO: 43.
18. The bacterium according to any one of items 1 to 17, wherein said bacterium belongs to the genus *Methylobacillus* or *Methylobacterium.*
19. The bacterium according to any one of items 1 to 18, wherein said bacterium is selected from *Methylobacillus flagellatus, Methylobacillus glycogenes, Methylobacillus pratensis, Methylobacillus rhizosphaerae, Methylobacterium extorquens* and *Methylobacterium organophilum.*
20. The bacterium according to any one of items 1 to 19, wherein said bacterium is *Methylobacillusflagellatus.*
21. The bacterium according to any one of items 1 to 20, wherein the bacterium further expresses a polypeptide having pyrrolidone synthase (PYS) activity.
22. The bacterium according to item 21, wherein the polypeptide having pyrrolidone synthase (PYS) activity comprises the amino acid sequence of SEQ ID NO: 75 or an amino acid sequence having at least 70%, such as at least 80%, sequence identity with SEQ ID NO: 75.
23. Method for the production of a biochemical comprising cultivating a bacterium according to any one of items 1 to 22 under suitable culture conditions in a culture medium comprising a reduced one-carbon compound, such as methanol, or a multi-carbon compound that contains no carbon-carbon bonds, such as dimethyl ether and dimethylamine.
24. Method according to item 23, wherein the culture medium comprises methanol.
25. The method according to item 23 or 24, wherein the method is for producing GABA or a derivative thereof.
26. The method according to item 25, wherein the GABA derivative is selected from the group consisting of 2-pyrrolidone, N-methyl-2-pyrrolidone and polybutyrolactam.
27. The method according to item 23 or 24, wherein the method is for producing an 2-pyrrolidone or a derivative thereof, such as N-methyl-2-pyrrolidone.

### Brief description of the figures

**Figure 1****:** Existing processes for the production of GABA and 2-pyrrolidone from methanol or glucose. A - Two stage production of GABA from glucose or methanol. The substrate is first converted to glutamate and exported. After the external pH is lowered, the glutamate is reimported and converted to GABA as a part of the acid stress response. B - One stage GABA production from glucose, where a pH-independent GAD enzyme directly converts glutamate into GABA without an external pH drop. C - 2-pyrrolidone formation by a pyrrolidone synthase enzyme, which converts GABA to 2-pyrrolidone.
**Figure 2****:** Novel and inventive approach for direct, pH-independent GABA and 2-pyrrolidone synthesis from methanol. A - comparison of the two approaches that can both be used to form GABA in a pH-independent manner. In both cases, GABA can be converted to 2-pyrrolidone. B - The novel GAD-bypass pathway which uses a α-ketoglutarate decarboxylase (KDC) and the reverse reaction of GABA-transaminase (GABA-TA) to produce GABA from methanol in a pH-independent manner.
**Figure 3****:** Sequence alignment of GAD enzymes.
**Figure 4****:** Fluorescene of GFP and mCherry expressed by methylotrophic cultures.
**Figure 5**: In vitro test of three GAD enzymes in strains ABME 38, ABME 39 and ABME 40.
**Figure 6****:** In vivo GABA production by ABME 35 expressing Sth GAD variant in shake flasks. 30 g/L glutamate was supplemented externally at the beginning of the cultivation. GABA was measured after 24 h of growth.
**Figure 7****:** In vivo GABA production by ABME 5-derived strains (A) and ABME 6-derived strains (B) expressing GAD variants in shake flasks.
**Figure 8****:** GABA production by ABME 40 in 5L (A) and 20 L (B) pH-controlled bioreactors. The process was oxygen limited with automatic methanol addition.
**Figure 9****:** GABA production from methanol in ABME 126, which was derived from ABME 6 by integrating the GAD into the epsD-G cluster. The native epsD-G promoter was used to drive gene expression.
**Figure 10****:** GABA production by ABME 126 in 5L pH-controlled bioreactors. The process was carbon limited with automatic methanol addition.
**Figure 11****:** Comparison of the conventional GAD variant and the novel GAD-bypass pathways to convert α-ketoglutarate to GABA.
**Figure 12****:** GABA production from methanol in shake flasks using the GAD-bypass expressed from plasmid in strains derived from ABME 5 (A) and ABME 6 (B).

The present invention is now described in more detail below.

### Detailed description of the invention

Unless specifically defined herein, all technical and scientific terms used have the same meaning as commonly understood by a skilled artisan in the fields of biochemistry, genetics, and microbiology.

All methods and materials similar or equivalent to those described herein can be used in the practice or testing of the present invention, with suitable methods and materials being described herein. All publications, patent applications, patents, and other references mentioned herein are incorporated by reference in their entirety. In case of conflict, the present specification, including definitions, will prevail. Further, the materials, methods, and examples are illustrative only and are not intended to be limiting, unless otherwise specified.

The practice of the present invention will employ, unless otherwise indicated, conventional techniques of cell biology, cell culture, molecular biology, transgenic biology, microbiology, and recombinant DNA, which are within the skill of the art. Such techniques are explained fully in the literature. See, for example, Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gait ed., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols.154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory).

### Bacterium of the invention

As indicated above, the present inventors have engineered different mesophilic, methylotrophic bacteria that are able to produce biochemicals such as GABA and 2-pyrrolidone from methanol independently of a pH drop. This has not been reported before and will make the production of biochemicals from methanol economically feasible.

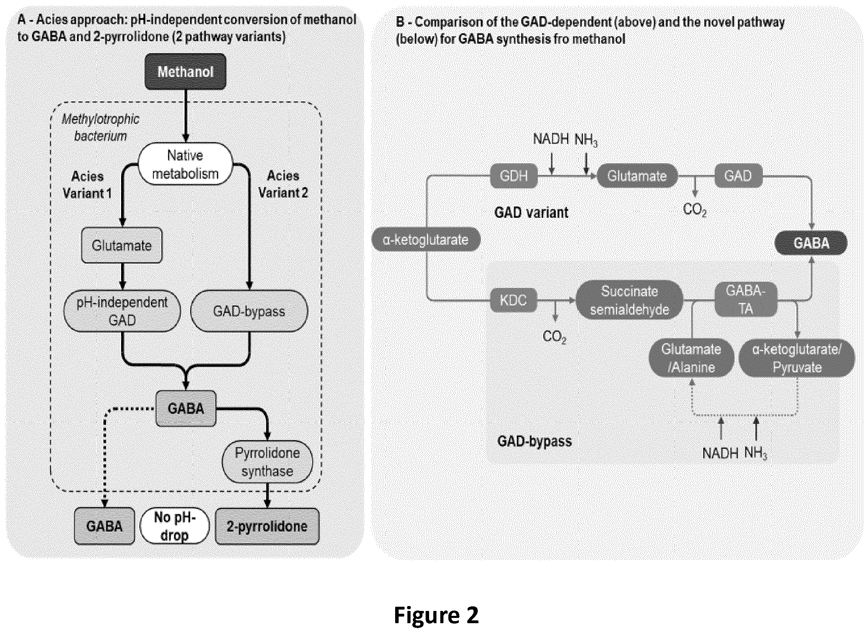

Particularly, the present inventors have developed two alternative approaches for direct, pH-independent production of biochemicals from methanol (Figure 2). The first approach is based on mesophilic, methylotrophic bacteria which express GAD enzymes which are active under neutral to alkaline conditions, the other is based on mesophilic, methylotrophic bacteria which express two enzymes, GABA transaminase (GABA-TA) and α-ketoglutarate decarboxylase (KDC).

The present invention thus provides in a first aspect, a mesophilic, methylotrophic bacterium which expresses a polypeptide which has glutamate decarboxylase (GAD) activity and is catalytically active at a pH above 5.5.

According to some embodiments, the polypeptide having glutamate decarboxylase (GAD) activity is catalytically active at a pH in the range of pH 6 to pH 8.

According to some embodiments, the polypeptide having glutamate decarboxylase (GAD) activity is catalytically active at a pH in the range of pH 6.5 to pH 7.5.

According to some embodiments, the polypeptide having glutamate decarboxylase (GAD) activity is catalytically active at pH 7.0.

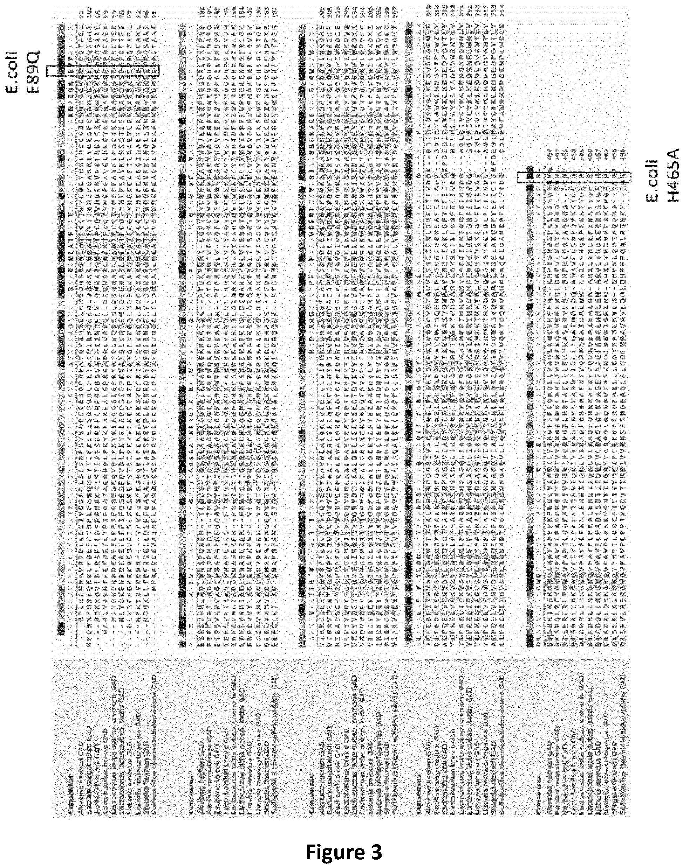

A polypeptide which has glutamate decarboxylase (GAD) activity and is catalytically active at a pH above 5.5 may be naturally occurring or may be genetically modified to improve the pH range. In the latter case, two approaches have so far been used to improve the pH range of GAD enzymes. One approach removes part of the C-terminus of GAD, which physically blocks the catalytic site of GAD when the environmental pH is neutral. In the second approach, specific sites in the GAD enzymes are mutated via random mutagenesis to obtain mutant enzyme active at neutral pH. In this respect, GAD enzymes, and particularly bacterial GAD enzymes, share several conserved regions, which containing a number of residues which have been shown to cause a shift in the pH range. By way of non-limiting example, mutating the penultimate His residue of the *E. coli* GAD (H465), the C-terminus was again unable to block GAD activity at neutral pH (Pennacchieti, et al., 2009). A E89Q mutation in *E. coli* GAD also prevents the loss of activity at neutral pH (Thu Ho et al., 2013). A similar effect was observed in *Lactobacillus brevis* GAD where four amino acid changes were introduced (T17I, D294G, E312S, Q346H). Of particular note, the two sites that improve the pH range in *E. coli* are also conserved in other GAD genes (as shown in Figure 3).

Thus, according to some embodiments, the polypeptide having glutamate decarboxylase (GAD) activity and being active at a pH above 5.5 is a GAD mutant, such as mutant derived from a bacterial GAD enzyme.

According to some embodiments, the polypeptide having glutamate decarboxylase (GAD) activity is a GAD mutant comprising in its amino acid sequence at least one amino acid substitution compared to the wild type GAD enzyme from which it is derived rending the mutant active at a pH above 5.5.

According to some embodiments, the polypeptide having glutamate decarboxylase (GAD) activity is a GAD mutant comprising in its amino acid sequence at least one amino acid substitution compared to the wild type GAD enzyme from which it is derived rending the mutant active at a pH in the range of pH 6 to pH 8.

According to some embodiments, the polypeptide having glutamate decarboxylase (GAD) activity is a GAD mutant comprising in its amino acid sequence at least one amino acid substitution compared to the wild type GAD enzyme from which it is derived rending the mutant active at a pH in the range of pH 6.5 to pH 7.5.

According to some embodiments, the polypeptide having glutamate decarboxylase (GAD) activity is a GAD mutant comprising in its amino acid sequence at least one amino acid substitution compared to the wild type GAD enzyme from which it is derived rending the mutant active at pH 7.0.

According to some embodiments, the at least one amino acid substitution is in a position corresponding to position 89 and/or position 465 of SEQ ID NO: 1. Thus, according to some embodiments, the GAD mutant comprises in its amino acid sequence an amino acid substitution in a position corresponding to position 89 of SEQ ID NO: 1. According to some embodiments, the GAD mutant comprises in its amino acid sequence an amino acid substitution in a position corresponding to position 465 of SEQ ID NO: 1. According to some embodiments, the GAD mutant comprises in its amino acid sequence two amino acid substitutions in positions corresponding to position 89 and position 465 of SEQ ID NO: 1.

According to some embodiments, the polypeptide is a GAD mutant having 2 to 20 amino acids, such as 2 to 14 amino acids, deleted from the C-terminus compared to the wild type GAD enzyme from which it is derived.

According to some embodiments, the polypeptide having glutamate decarboxylase (GAD) activity is selected from any one of the following:
a) a polypeptide having glutamate decarboxylase (GAD) activity which comprises an amino acid sequence having at least 70%, such as at least 80%, sequence identity with SEQ ID NO: 1 and comprising an amino acid substitution at a position corresponding to position 89 and/or position 465 in SEQ ID NO: 1;
b) a polypeptide having glutamate decarboxylase (GAD) activity which comprises an amino acid sequence having at least 70%, such as at least 80%, sequence identity with SEQ ID NO: 2 and comprising an amino acid substitution at a position corresponding to position 84 and/or position 457 in SEQ ID NO: 2;
c) a polypeptide having glutamate decarboxylase (GAD) activity which comprises an amino acid sequence having at least 70%, such as at least 80%, sequence identity with SEQ ID NO: 3 and comprising an amino acid substitution at a position corresponding to position 93 and/or position 467 in SEQ ID NO: 3;
d) a polypeptide having glutamate decarboxylase (GAD) activity which comprises an amino acid sequence having at least 70%, such as at least 80%, sequence identity with SEQ ID NO: 4 and comprising an amino acid substitution at a position corresponding to position 90 and/or position 467 in SEQ ID NO: 4;
e) a polypeptide having glutamate decarboxylase (GAD) activity which comprises an amino acid sequence having at least 70%, such as at least 80%, sequence identity with SEQ ID NO: 5 and comprising an amino acid substitution at a position corresponding to position 85 and/or position 462 in SEQ ID NO: 5;
f) a polypeptide having glutamate decarboxylase (GAD) activity which comprises an amino acid sequence having at least 70%, such as at least 80%, sequence identity with SEQ ID NO: 6 and comprising an amino acid substitution at a position corresponding to position 89 and/or position 465 in SEQ ID NO: 6;
g) a polypeptide having glutamate decarboxylase (GAD) activity which comprises an amino acid sequence having at least 70%, such as at least 80%, sequence identity with SEQ ID NO: 7 and comprising an amino acid substitution at a position corresponding to position 89 and/or position 466 in SEQ ID NO: 7;
h) a polypeptide having glutamate decarboxylase (GAD) activity which comprises an amino acid sequence having at least 70%, such as at least 80%, sequence identity with SEQ ID NO: 8 and comprising an amino acid substitution at a position corresponding to position 89 and/or position 466 in SEQ ID NO: 8;
i) a polypeptide having glutamate decarboxylase (GAD) activity which comprises an amino acid sequence having at least 70%, such as at least 80%, sequence identity with SEQ ID NO: 9 and comprising an amino acid substitution at a position corresponding to position 89 and/or position 463 in SEQ ID NO: 9;
j) a polypeptide having glutamate decarboxylase (GAD) activity which comprises an amino acid sequence having at least 70%, such as at least 80%, sequence identity with SEQ ID NO: 10 and comprising an amino acid substitution at a position corresponding to position 17, position 91, position 94, position 213, position 346 and/or position 468 in SEQ ID NO: 10; and
k) a polypeptide having glutamate decarboxylase (GAD) activity which comprises an amino acid sequence having at least 70%, such as at least 80%, sequence identity with SEQ ID NO: 1, 2, 3, 4, 5, 6, 7, 8, 9 or 10 wherein 2 to 20 amino acids, such as 2 to 14 amino acids, are deleted from the C-terminus.

According to some embodiments, the polypeptide having glutamate decarboxylase (GAD) activity comprises an amino acid sequence which has at least 70%, such as at least 75%, sequence identity with SEQ ID NO: 1 and comprises an amino acid substitution at a position corresponding to position 89 and/or position 465 in SEQ ID NO: 1. According to some embodiments, the polypeptide having glutamate decarboxylase (GAD) activity comprises an amino acid sequence which has at least 80%, such as at least 85%, sequence identity with SEQ ID NO: 1 and comprises an amino acid substitution at a position corresponding to position 89 and/or position 465 in SEQ ID NO: 1. According to some embodiments, the polypeptide having glutamate decarboxylase (GAD) activity comprises an amino acid sequence which has at least 90%, such as at least 95%, sequence identity with SEQ ID NO: 1 and comprises an amino acid substitution at a position corresponding to position 89 and/or position 465 in SEQ ID NO: 1.

According to some embodiments, the polypeptide having glutamate decarboxylase (GAD) activity comprises an amino acid sequence which has at least 70%, such as at least 75%, sequence identity with SEQ ID NO: 1 and comprises an amino acid substitution at a position corresponding to position 89 in SEQ ID NO: 1. According to some embodiments, the polypeptide having glutamate decarboxylase (GAD) activity comprises an amino acid sequence which has at least 80%, such as at least 85%, sequence identity with SEQ ID NO: 1 and comprises an amino acid substitution at a position corresponding to position 89 in SEQ ID NO: 1. According to some embodiments, the polypeptide having glutamate decarboxylase (GAD) activity comprises an amino acid sequence which has at least 90%, such as at least 95%, sequence identity with SEQ ID NO: 1 and comprises an amino acid substitution at a position corresponding to position 89 in SEQ ID NO: 1.

According to some embodiments, the polypeptide having glutamate decarboxylase (GAD) activity comprises an amino acid sequence which has at least 70%, such as at least 75%, sequence identity with SEQ ID NO: 1 and comprises an amino acid substitution at a position corresponding to position 465 in SEQ ID NO: 1. According to some embodiments, the polypeptide having glutamate decarboxylase (GAD) activity comprises an amino acid sequence which has at least 80%, such as at least 85%, sequence identity with SEQ ID NO: 1 and comprises an amino acid substitution at a position corresponding to position 465 in SEQ ID NO: 1. According to some embodiments, the polypeptide having glutamate decarboxylase (GAD) activity comprises an amino acid sequence which has at least 90%, such as at least 90%, sequence identity with SEQ ID NO: 1 and comprises an amino acid substitution at a position corresponding to position 465 in SEQ ID NO: 1.

According to some embodiments, the polypeptide having glutamate decarboxylase (GAD) activity comprises an amino acid sequence which has at least 70%, such as at least 75%, sequence identity with SEQ ID NO: 1 and comprises an amino acid substitution at a position corresponding to position 89 and position 465 in SEQ ID NO: 1. According to some embodiments, the polypeptide having glutamate decarboxylase (GAD) activity comprises an amino acid sequence which has at least 80%, such as at least 85%, sequence identity with SEQ ID NO: 1 and comprises an amino acid substitution at a position corresponding to position 89 and position 465 in SEQ ID NO: 1. According to some embodiments, the polypeptide having glutamate decarboxylase (GAD) activity comprises an amino acid sequence which has at least 90%, such as at least 95%, sequence identity with SEQ ID NO: 1 and comprises an amino acid substitution at a position corresponding to position 89 and position 465 in SEQ ID NO: 1.

According to some embodiments, the polypeptide having glutamate decarboxylase (GAD) activity comprises an amino acid sequence which has at least 70%, such as at least 75%, sequence identity with SEQ ID NO: 2 and comprises an amino acid substitution at a position corresponding to position 84 and/or position 457 in SEQ ID NO: 2. According to some embodiments, the polypeptide having glutamate decarboxylase (GAD) activity comprises an amino acid sequence which has at least 80%, such as at least 85%, sequence identity with SEQ ID NO: 2 and comprises an amino acid substitution at a position corresponding to position 84 and/or position 457 in SEQ ID NO: 2. According to some embodiments, the polypeptide having glutamate decarboxylase (GAD) activity comprises an amino acid sequence which has at least 90%, such as at least 95%, sequence identity with SEQ ID NO: 2 and comprises an amino acid substitution at a position corresponding to position 84 and/or position 457 in SEQ ID NO: 2.

According to some embodiments, the polypeptide having glutamate decarboxylase (GAD) activity comprises an amino acid sequence which has at least 70%, such as at least 75%, sequence identity with SEQ ID NO: 2 and comprises an amino acid substitution at a position corresponding to position 84 in SEQ ID NO: 2. According to some embodiments, the polypeptide having glutamate decarboxylase (GAD) activity comprises an amino acid sequence which has at least 80%, such as at least 85%, sequence identity with SEQ ID NO: 2 and comprises an amino acid substitution at a position corresponding to position 84 in SEQ ID NO: 2. According to some embodiments, the polypeptide having glutamate decarboxylase (GAD) activity comprises an amino acid sequence which has at least 90%, such as at least 95%, sequence identity with SEQ ID NO: 2 and comprises an amino acid substitution at a position corresponding to position 84 in SEQ ID NO: 2.

According to some embodiments, the polypeptide having glutamate decarboxylase (GAD) activity comprises an amino acid sequence which has at least 70%, such as at least 75%, sequence identity with SEQ ID NO: 2 and comprises an amino acid substitution at a position corresponding to position 457 in SEQ ID NO: 2. According to some embodiments, the polypeptide having glutamate decarboxylase (GAD) activity comprises an amino acid sequence which has at least 80%, such as at least 85%, sequence identity with SEQ ID NO: 2 and comprises an amino acid substitution at a position corresponding to position 457 in SEQ ID NO: 2. According to some embodiments, the polypeptide having glutamate decarboxylase (GAD) activity comprises an amino acid sequence which has at least 90%, such as at least 95%, sequence identity with SEQ ID NO: 2 and comprises an amino acid substitution at a position corresponding to position 457 in SEQ ID NO: 2.

According to some embodiments, the polypeptide having glutamate decarboxylase (GAD) activity comprises an amino acid sequence which has at least 70%, such as at least 75%, sequence identity with SEQ ID NO: 2 and comprises an amino acid substitution at a position corresponding to position 84 and position 457 in SEQ ID NO: 2. According to some embodiments, the polypeptide having glutamate decarboxylase (GAD) activity comprises an amino acid sequence which has at least 80%, such as at least 85%, sequence identity with SEQ ID NO: 2 and comprises an amino acid substitution at a position corresponding to position 84 and position 457 in SEQ ID NO: 2. According to some embodiments, the polypeptide having glutamate decarboxylase (GAD) activity comprises an amino acid sequence which has at least 90%, such as at least 95%, sequence identity with SEQ ID NO: 2 and comprises an amino acid substitution at a position corresponding to position 84 and position 457 in SEQ ID NO: 2.

According to some embodiments, the polypeptide having glutamate decarboxylase (GAD) activity comprises an amino acid sequence which has at least 70%, such as at least 75%, sequence identity with SEQ ID NO: 3 and comprises an amino acid substitution at a position corresponding to position 93 and/or position 467 in SEQ ID NO: 3. According to some embodiments, the polypeptide having glutamate decarboxylase (GAD) activity comprises an amino acid sequence which has at least 80%, such as at least 85%, sequence identity with SEQ ID NO: 3 and comprises an amino acid substitution at a position corresponding to position 93 and/or position 467 in SEQ ID NO: 3. According to some embodiments, the polypeptide having glutamate decarboxylase (GAD) activity comprises an amino acid sequence which has at least 90%, such as at least 95%, sequence identity with SEQ ID NO: 3 and comprises an amino acid substitution at a position corresponding to position 93 and/or position 467 in SEQ ID NO: 3.

According to some embodiments, the polypeptide having glutamate decarboxylase (GAD) activity comprises an amino acid sequence which has at least 70%, such as at least 75%, sequence identity with SEQ ID NO: 4 and comprises an amino acid substitution at a position corresponding to position 90 and/or position 467 in SEQ ID NO: 4. According to some embodiments, the polypeptide having glutamate decarboxylase (GAD) activity comprises an amino acid sequence which has at least 80%, such as at least 85%, sequence identity with SEQ ID NO: 4 and comprises an amino acid substitution at a position corresponding to position 90 and/or position 467 in SEQ ID NO: 4. According to some embodiments, the polypeptide having glutamate decarboxylase (GAD) activity comprises an amino acid sequence which has at least 90%, such as at least 95%, sequence identity with SEQ ID NO: 4 and comprises an amino acid substitution at a position corresponding to position 90 and/or position 467 in SEQ ID NO: 4.

According to some embodiments, the polypeptide having glutamate decarboxylase (GAD) activity comprises an amino acid sequence which has at least 70%, such as at least 75%, sequence identity with SEQ ID NO: 5 and comprises an amino acid substitution at a position corresponding to position 85 and/or position 462 in SEQ ID NO: 5. According to some embodiments, the polypeptide having glutamate decarboxylase (GAD) activity comprises an amino acid sequence which has at least 80%, such as at least 85%, sequence identity with SEQ ID NO: 5 and comprises an amino acid substitution at a position corresponding to position 85 and/or position 462 in SEQ ID NO: 5. According to some embodiments, the polypeptide having glutamate decarboxylase (GAD) activity comprises an amino acid sequence which has at least 90%, such as at least 95%, sequence identity with SEQ ID NO: 5 and comprises an amino acid substitution at a position corresponding to position 85 and/or position 462 in SEQ ID NO: 5.

According to some embodiments, the polypeptide having glutamate decarboxylase (GAD) activity comprises an amino acid sequence which has at least 70%, such as at least 75%, sequence identity with SEQ ID NO: 6 and comprises an amino acid substitution at a position corresponding to position 89 and/or position 465 in SEQ ID NO: 6. According to some embodiments, the polypeptide having glutamate decarboxylase (GAD) activity comprises an amino acid sequence which has at least 80%, such as at least 85%, sequence identity with SEQ ID NO: 6 and comprises an amino acid substitution at a position corresponding to position 89 and/or position 465 in SEQ ID NO: 6. According to some embodiments, the polypeptide having glutamate decarboxylase (GAD) activity comprises an amino acid sequence which has at least 90%, such as at least 95%, sequence identity with SEQ ID NO: 6 and comprises an amino acid substitution at a position corresponding to position 89 and/or position 465 in SEQ ID NO: 6.

According to some embodiments, the polypeptide having glutamate decarboxylase (GAD) activity comprises an amino acid sequence which has at least 70%, such as at least 75%, sequence identity with SEQ ID NO: 7 and comprises an amino acid substitution at a position corresponding to position 89 and/or position 466 in SEQ ID NO: 7. According to some embodiments, the polypeptide having glutamate decarboxylase (GAD) activity comprises an amino acid sequence which has at least 80%, such as at least 85%, sequence identity with SEQ ID NO: 7 and comprises an amino acid substitution at a position corresponding to position 89 and/or position 466 in SEQ ID NO: 7. According to some embodiments, the polypeptide having glutamate decarboxylase (GAD) activity comprises an amino acid sequence which has at least 90%, such as at least 95%, sequence identity with SEQ ID NO: 7 and comprises an amino acid substitution at a position corresponding to position 89 and/or position 466 in SEQ ID NO: 7.

According to some embodiments, the polypeptide having glutamate decarboxylase (GAD) activity comprises an amino acid sequence which has at least 70%, such as at least 75%, sequence identity with SEQ ID NO: 8 and comprises an amino acid substitution at a position corresponding to position 89 and/or position 466 in SEQ ID NO: 8. According to some embodiments, the polypeptide having glutamate decarboxylase (GAD) activity comprises an amino acid sequence which has at least 80%, such as at least 85%, sequence identity with SEQ ID NO: 8 and comprises an amino acid substitution at a position corresponding to position 89 and/or position 466 in SEQ ID NO: 8. According to some embodiments, the polypeptide having glutamate decarboxylase (GAD) activity comprises an amino acid sequence which has at least 90%, such as at least 95%, sequence identity with SEQ ID NO: 8 and comprises an amino acid substitution at a position corresponding to position 89 and/or position 466 in SEQ ID NO: 8.

According to some embodiments, the polypeptide having glutamate decarboxylase (GAD) activity comprises an amino acid sequence which has at least 70%, such as at least 75%, sequence identity with SEQ ID NO: 9 and comprises an amino acid substitution at a position corresponding to position 89 and/or position 463 in SEQ ID NO: 9. According to some embodiments, the polypeptide having glutamate decarboxylase (GAD) activity comprises an amino acid sequence which has at least 80%, such as at least 85%, sequence identity with SEQ ID NO: 9 and comprises an amino acid substitution at a position corresponding to position 89 and/or position 463 in SEQ ID NO: 9. According to some embodiments, the polypeptide having glutamate decarboxylase (GAD) activity comprises an amino acid sequence which has at least 90%, such as at least 95%, sequence identity with SEQ ID NO: 9 and comprises an amino acid substitution at a position corresponding to position 89 and/or position 463 in SEQ ID NO: 9.

According to some embodiments, the polypeptide having glutamate decarboxylase (GAD) activity comprises an amino acid sequence which has at least 70%, such as at least 75%, sequence identity with SEQ ID NO: 10 and comprises an amino acid substitution at a position corresponding to position 17, position 91, position 94, position 213, position 346 and/or position 468 in SEQ ID NO: 10. According to some embodiments, the polypeptide having glutamate decarboxylase (GAD) activity comprises an amino acid sequence which has at least 80%, such as at least 85%, sequence identity with SEQ ID NO: 10 and comprises an amino acid substitution at a position corresponding to position 17, position 91, position 94, position 213, position 346 and/or position 468 in SEQ ID NO: 10. According to some embodiments, the polypeptide having glutamate decarboxylase (GAD) activity comprises an amino acid sequence which has at least 90%, such as at least 95%, sequence identity with SEQ ID NO: 10 and comprises an amino acid substitution at a position corresponding to position 17, position 91, position 94, position 213, position 346 and/or position 468 in SEQ ID NO: 10.

According to some embodiments, the polypeptide having glutamate decarboxylase (GAD) activity comprises an amino acid sequence which has at least 70%, such as at least 75%, sequence identity with SEQ ID NO: 10 and comprises an amino acid substitution at a position corresponding to position 17, position 94, position 213 and/or position 346 in SEQ ID NO: 10. According to some embodiments, the polypeptide having glutamate decarboxylase (GAD) activity comprises an amino acid sequence which has at least 80%, such as at least 85%, sequence identity with SEQ ID NO: 10 and comprises an amino acid substitution at a position corresponding to position 17, position 94, position 213 and/or position 346 in SEQ ID NO: 10. According to some embodiments, the polypeptide having glutamate decarboxylase (GAD) activity comprises an amino acid sequence which has at least 90%, such as at least 95%, sequence identity with SEQ ID NO: 10 and comprises an amino acid substitution at a position corresponding to position 17, position 91, position 94, position 213, position 346 and/or position 468 in SEQ ID NO: 10.

According to some embodiments, the polypeptide having glutamate decarboxylase (GAD) activity comprises an amino acid sequence having at least 70%, such as at least 75%, sequence identity with SEQ ID NO: 1, 2, 3, 4, 5, 6, 7, 8, 9 or 10 wherein 2 to 20 amino acids are deleted from the C-terminus. According to some embodiments, the polypeptide having glutamate decarboxylase (GAD) activity comprises an amino acid sequence having at least 80%, such as at least 85%, sequence identity with SEQ ID NO: 1, 2, 3, 4, 5, 6, 7, 8, 9 or 10 wherein 2 to 20 amino acids are deleted from the C-terminus. According to some embodiments, the polypeptide having glutamate decarboxylase (GAD) activity comprises an amino acid sequence having at least 90%, such as at least 95%, sequence identity with SEQ ID NO: 1, 2, 3, 4, 5, 6, 7, 8, 9 or 10 wherein 2 to 20 amino acids, are deleted from the C-terminus.

According to some embodiments, the polypeptide having glutamate decarboxylase (GAD) activity comprises an amino acid sequence having at least 70%, such as at least 75%, sequence identity with SEQ ID NO: 1, 2, 3, 4, 5, 6, 7, 8, 9 or 10 wherein 2 to 14 amino acids are deleted from the C-terminus. According to some embodiments, the polypeptide having glutamate decarboxylase (GAD) activity comprises an amino acid sequence having at least 80%, such as at least 85%, sequence identity with SEQ ID NO: 1, 2, 3, 4, 5, 6, 7, 8, 9 or 10 wherein 2 to 14 amino acids are deleted from the C-terminus. According to some embodiments, the polypeptide having glutamate decarboxylase (GAD) activity comprises an amino acid sequence having at least 90%, such as at least 95%, sequence identity with SEQ ID NO: 1, 2, 3, 4, 5, 6, 7, 8, 9 or 10 wherein 2 to 14 amino acids are deleted from the C-terminus.

According to some embodiments, the polypeptide having glutamate decarboxylase (GAD) activity comprises an amino acid sequence having at least 70%, such as at least 75%, sequence identity with SEQ ID NO: 1, 2, 3, 4, 5, 6, 7, 8, 9 or 10 wherein 4 to 10 amino acids are deleted from the C-terminus. According to some embodiments, the polypeptide having glutamate decarboxylase (GAD) activity comprises an amino acid sequence having at least 80%, such as at least 85%, sequence identity with SEQ ID NO: 1, 2, 3, 4, 5, 6, 7, 8, 9 or 10 wherein 4 to 10 amino acids are deleted from the C-terminus. According to some embodiments, the polypeptide having glutamate decarboxylase (GAD) activity comprises an amino acid sequence having at least 90%, such as at least 95%, sequence identity with SEQ ID NO: 1, 2, 3, 4, 5, 6, 7, 8, 9 or 10 wherein 4 to 10 amino acids are deleted from the C-terminus.

According to some embodiments, the polypeptide having glutamate decarboxylase (GAD) activity is selected from any one of the following:
aa) a polypeptide having glutamate decarboxylase (GAD) activity which comprises the amino acid sequence of SEQ ID NO: 11, 12 or 13 or an amino acid sequence corresponding to SEQ ID NO: 11, 12 or 13, wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A;
bb) a polypeptide having glutamate decarboxylase (GAD) activity which comprises the amino acid sequence of SEQ ID NO: 14, 15 or 16 or an amino acid sequence corresponding to SEQ ID NO: 14, 15 or 16 wherein 1 to 40, such as 1 to 20, amino acids are substituted with the proviso that the amino acid at position 84 is not E and/or the amino acid at position 457 is not H, preferably with the proviso that the amino acid at position 84 is Q and/or the amino acid at position 457 is A;
cc) a polypeptide having glutamate decarboxylase (GAD) activity which comprises the amino acid sequence of SEQ ID NO: 17, 18 or 19 or an amino acid sequence corresponding to SEQ ID NO: 17, 18 or 19 wherein 1 to 40, such as 1 to 20, amino acids are substituted with the proviso that the amino acid at position 93 is not E and/or the amino acid at position 467 is not H, preferably with the proviso that the amino acid at position 93 is Q and/or the amino acid at position 467 is A;
dd) a polypeptide having glutamate decarboxylase (GAD) activity which comprises the amino acid sequence of SEQ ID NO: 20, 21 or 22 or an amino acid sequence corresponding to SEQ ID NO: 20, 21 or 22 wherein 1 to 40, such as 1 to 20, amino acids are substituted with the proviso that the amino acid at position 90 is not E and/or the amino acid at position 467 is not H, preferably with the proviso that the amino acid at position 90 is Q and/or the amino acid at position 467 is A;
ee) a polypeptide having glutamate decarboxylase (GAD) activity which comprises the amino acid sequence of SEQ ID NO: 23, 24 or 25 or an amino acid sequence corresponding to SEQ ID NO: 23, 24 or 25 wherein 1 to 40, such as 1 to 20, amino acids are substituted with the proviso that the amino acid at position 85 is not E and/or the amino acid at position 462 is not H, preferably with the proviso that the amino acid at position 85 is Q and/or the amino acid at position 462 is A;
ff) a polypeptide having glutamate decarboxylase (GAD) activity which comprises the amino acid sequence of SEQ ID NO: 26, 27 or 28 or an amino acid sequence corresponding to SEQ ID NO: 26, 27 or 28 wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A;
gg) a polypeptide having glutamate decarboxylase (GAD) activity which comprises the amino acid sequence of SEQ ID NO: 29, 30 or 31 or an amino acid sequence corresponding to SEQ ID NO: 29, 30 or 31 wherein 1 to 40, such as 1 to 20, amino acids are substituted with the proviso that the amino acid at position 89 is not E and/or the amino acid at position 466 is not H, preferably with the proviso that the amino acid at position 89 is Q and/or the amino acid at position 466 is A;
hh) a polypeptide having glutamate decarboxylase (GAD) activity which comprises the amino acid sequence of SEQ ID NO: 32, 33 or 34 or an amino acid sequence corresponding to SEQ ID NO: 32, 33 or 34 wherein 1 to 40, such as 1 to 20, amino acids are substituted with the proviso that the amino acid at position 89 is not E and/or the amino acid at position 466 is not H, preferably with the proviso that the amino acid at position 89 is Q and/or the amino acid at position 466 is A;
ii) a polypeptide having glutamate decarboxylase (GAD) activity which comprises the amino acid sequence of SEQ ID NO: 35, 36 or 37 or an amino acid sequence corresponding to SEQ ID NO: 35, 36 or 37 wherein 1 to 40, such as 1 to 20, amino acids are substituted with the proviso that the amino acid at position 89 is not E and/or the amino acid at position 463 is not H, preferably with the proviso that the amino acid at position 89 is Q and/or the amino acid at position 463 is A;
jj) a polypeptide having glutamate decarboxylase (GAD) activity which comprises the amino acid sequence of SEQ ID NO: 38 or an amino acid sequence corresponding to SEQ ID NO: 38 wherein 1 to 40, such as 1 to 20, amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H; and
kk) a polypeptide having glutamate decarboxylase (GAD) activity which comprises an amino acid sequence having at least 70%, such as at least 80%, sequence identity with any one of SEQ ID NOs: 11 to 38, wherein 2 to 20 amino acids, such as 2 to 14 amino acids, are deleted from the C-terminus.

According to some embodiments, the polypeptide having glutamate decarboxylase (GAD) activity comprises the amino acid sequence of SEQ ID NO: 11, 12 or 13 or an amino acid sequence corresponding to SEQ ID NO: 11, 12 or 13, wherein 1 to 40 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 11, 12 or 13 or an amino acid sequence corresponding to SEQ ID NO: 11, 12 or 13, wherein 1 to 20 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 11, 12 or 13 or an amino acid sequence corresponding to SEQ ID NO: 11, 12 or 13, wherein 1 to 10 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 11, 12 or 13 or an amino acid sequence corresponding to SEQ ID NO: 11, 12 or 13, wherein 1 to 5 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 11, 12 or 13.

According to some embodiments, the polypeptide having glutamate decarboxylase (GAD) activity comprises the amino acid sequence of SEQ ID NO: 14, 15 or 16 or an amino acid sequence corresponding to SEQ ID NO: 14, 15 or 16 wherein 1 to 40 amino acids are substituted with the proviso that the amino acid at position 84 is not E and/or the amino acid at position 457 is not H, preferably with the proviso that the amino acid at position 84 is Q and/or the amino acid at position 457 is A. According to some embodiments, the polypeptide having glutamate decarboxylase (GAD) activity comprises the amino acid sequence of SEQ ID NO: 14, 15 or 16 or an amino acid sequence corresponding to SEQ ID NO: 14, 15 or 16 wherein 1 to 20 amino acids are substituted with the proviso that the amino acid at position 84 is not E and/or the amino acid at position 457 is not H, preferably with the proviso that the amino acid at position 84 is Q and/or the amino acid at position 457 is A. According to some embodiments, the polypeptide having glutamate decarboxylase (GAD) activity comprises the amino acid sequence of SEQ ID NO: 14, 15 or 16 or an amino acid sequence corresponding to SEQ ID NO: 14, 15 or 16 wherein 1 to 20 amino acids are substituted with the proviso that the amino acid at position 84 is not E and/or the amino acid at position 457 is not H, preferably with the proviso that the amino acid at position 84 is Q and/or the amino acid at position 457 is A. According to some embodiments, the polypeptide having glutamate decarboxylase (GAD) activity comprises the amino acid sequence of SEQ ID NO: 14, 15 or 16 or an amino acid sequence corresponding to SEQ ID NO: 14, 15 or 16 wherein 1 to 10 amino acids are substituted with the proviso that the amino acid at position 84 is not E and/or the amino acid at position 457 is not H, preferably with the proviso that the amino acid at position 84 is Q and/or the amino acid at position 457 is A. According to some embodiments, the polypeptide having glutamate decarboxylase (GAD) activity comprises the amino acid sequence of SEQ ID NO: 14, 15 or 16 or an amino acid sequence corresponding to SEQ ID NO: 14, 15 or 16 wherein 1 to 5 amino acids are substituted with the proviso that the amino acid at position 84 is not E and/or the amino acid at position 457 is not H, preferably with the proviso that the amino acid at position 84 is Q and/or the amino acid at position 457 is A. According to some embodiments, the polypeptide having glutamate decarboxylase (GAD) activity comprises the amino acid sequence of SEQ ID NO: 14, 15 or 16.

According to some embodiments, the polypeptide having glutamate decarboxylase (GAD) activity comprises the amino acid sequence of SEQ ID NO: 17, 18 or 19 or an amino acid sequence corresponding to SEQ ID NO: 17, 18 or 19 wherein 1 to 40 amino acids are substituted with the proviso that the amino acid at position 93 is not E and/or the amino acid at position 467 is not H, preferably with the proviso that the amino acid at position 93 is Q and/or the amino acid at position 467 is A. According to some embodiments, the polypeptide having glutamate decarboxylase (GAD) activity comprises the amino acid sequence of SEQ ID NO: 17, 18 or 19 or an amino acid sequence corresponding to SEQ ID NO: 17, 18 or 19 wherein 1 to 20 amino acids are substituted with the proviso that the amino acid at position 93 is not E and/or the amino acid at position 467 is not H, preferably with the proviso that the amino acid at position 93 is Q and/or the amino acid at position 467 is A. According to some embodiments, the polypeptide having glutamate decarboxylase (GAD) activity comprises the amino acid sequence of SEQ ID NO: 17, 18 or 19 or an amino acid sequence corresponding to SEQ ID NO: 17, 18 or 19 wherein 1 to 10 amino acids are substituted with the proviso that the amino acid at position 93 is not E and/or the amino acid at position 467 is not H, preferably with the proviso that the amino acid at position 93 is Q and/or the amino acid at position 467 is A. According to some embodiments, the polypeptide having glutamate decarboxylase (GAD) activity comprises the amino acid sequence of SEQ ID NO: 17, 18 or 19 or an amino acid sequence corresponding to SEQ ID NO: 17, 18 or 19 wherein 1 to 5 amino acids are substituted with the proviso that the amino acid at position 93 is not E and/or the amino acid at position 467 is not H, preferably with the proviso that the amino acid at position 93 is Q and/or the amino acid at position 467 is A. According to some embodiments, the polypeptide having glutamate decarboxylase (GAD) activity comprises the amino acid sequence of SEQ ID NO: 17, 18 or 19.

According to some embodiments, the polypeptide having glutamate decarboxylase (GAD) activity comprises the amino acid sequence of SEQ ID NO: 20, 21 or 22 or an amino acid sequence corresponding to SEQ ID NO: 20, 21 or 22 wherein 1 to 40 amino acids are substituted with the proviso that the amino acid at position 90 is not E and/or the amino acid at position 467 is not H, preferably with the proviso that the amino acid at position 90 is Q and/or the amino acid at position 467 is A. According to some embodiments, the polypeptide having glutamate decarboxylase (GAD) activity comprises the amino acid sequence of SEQ ID NO: 20, 21 or 22 or an amino acid sequence corresponding to SEQ ID NO: 20, 21 or 22 wherein 1 to 20 amino acids are substituted with the proviso that the amino acid at position 90 is not E and/or the amino acid at position 467 is not H, preferably with the proviso that the amino acid at position 90 is Q and/or the amino acid at position 467 is A. According to some embodiments, the polypeptide having glutamate decarboxylase (GAD) activity comprises the amino acid sequence of SEQ ID NO: 20, 21 or 22 or an amino acid sequence corresponding to SEQ ID NO: 20, 21 or 22 wherein 1 to 10 amino acids are substituted with the proviso that the amino acid at position 90 is not E and/or the amino acid at position 467 is not H, preferably with the proviso that the amino acid at position 90 is Q and/or the amino acid at position 467 is A. According to some embodiments, the polypeptide having glutamate decarboxylase (GAD) activity comprises the amino acid sequence of SEQ ID NO: 20, 21 or 22 or an amino acid sequence corresponding to SEQ ID NO: 20, 21 or 22 wherein 1 to 5 amino acids are substituted with the proviso that the amino acid at position 90 is not E and/or the amino acid at position 467 is not H, preferably with the proviso that the amino acid at position 90 is Q and/or the amino acid at position 467 is A. According to some embodiments, the polypeptide having glutamate decarboxylase (GAD) activity comprises the amino acid sequence of SEQ ID NO: 20, 21 or 22

According to some embodiments, the polypeptide having glutamate decarboxylase (GAD) activity comprises the amino acid sequence of SEQ ID NO: 23, 24 or 25 or an amino acid sequence corresponding to SEQ ID NO: 23, 24 or 25 wherein 1 to 40 amino acids are substituted with the proviso that the amino acid at position 85 is not E and/or the amino acid at position 462 is not H, preferably with the proviso that the amino acid at position 85 is Q and/or the amino acid at position 462 is A. According to some embodiments, the polypeptide having glutamate decarboxylase (GAD) activity comprises the amino acid sequence of SEQ ID NO: 23, 24 or 25 or an amino acid sequence corresponding to SEQ ID NO: 23, 24 or 25 wherein 1 to 20 amino acids are substituted with the proviso that the amino acid at position 85 is not E and/or the amino acid at position 462 is not H, preferably with the proviso that the amino acid at position 85 is Q and/or the amino acid at position 462 is A. According to some embodiments, the polypeptide having glutamate decarboxylase (GAD) activity comprises the amino acid sequence of SEQ ID NO: 23, 24 or 25 or an amino acid sequence corresponding to SEQ ID NO: 23, 24 or 25 wherein 1 to 10 amino acids are substituted with the proviso that the amino acid at position 85 is not E and/or the amino acid at position 462 is not H, preferably with the proviso that the amino acid at position 85 is Q and/or the amino acid at position 462 is A. According to some embodiments, the polypeptide having glutamate decarboxylase (GAD) activity comprises the amino acid sequence of SEQ ID NO: 23, 24 or 25 or an amino acid sequence corresponding to SEQ ID NO: 23, 24 or 25 wherein 1 to 5 amino acids are substituted with the proviso that the amino acid at position 85 is not E and/or the amino acid at position 462 is not H, preferably with the proviso that the amino acid at position 85 is Q and/or the amino acid at position 462 is A. According to some embodiments, the polypeptide having glutamate decarboxylase (GAD) activity comprises the amino acid sequence of SEQ ID NO: 23, 24 or 25.

According to some embodiments, the polypeptide having glutamate decarboxylase (GAD) activity comprises the amino acid sequence of SEQ ID NO: 26, 27 or 28 or an amino acid sequence corresponding to SEQ ID NO: 26, 27 or 28 wherein 1 to 40 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 26, 27 or 28 or an amino acid sequence corresponding to SEQ ID NO: 26, 27 or 28 wherein 1 to 20 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 26, 27 or 28 or an amino acid sequence corresponding to SEQ ID NO: 26, 27 or 28 wherein 1 to 10 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 26, 27 or 28 or an amino acid sequence corresponding to SEQ ID NO: 26, 27 or 28 wherein 1 to 5 amino acids are substituted with the proviso that the amino acid at position 89 is not E and/or the amino acid at position 465 is not H, preferably with the proviso that the amino acid at position 89 is Q and/or the amino acid at position 465 is A. According to some embodiments, the polypeptide having glutamate decarboxylase (GAD) activity comprises the amino acid sequence of SEQ ID NO: 26, 27 or 28.

According to some embodiments, the polypeptide having glutamate decarboxylase (GAD) activity comprises the amino acid sequence of SEQ ID NO: 29, 30 or 31 or an amino acid sequence corresponding to SEQ ID NO: 29, 30 or 31 wherein 1 to 40 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 29, 30 or 31 or an amino acid sequence corresponding to SEQ ID NO: 29, 30 or 31 wherein 1 to 20 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 29, 30 or 31 or an amino acid sequence corresponding to SEQ ID NO: 29, 30 or 31 wherein 1 to 10 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 29, 30 or 31 or an amino acid sequence corresponding to SEQ ID NO: 29, 30 or 31 wherein 1 to 5 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 29, 30 or 31.

According to some embodiments, the polypeptide having glutamate decarboxylase (GAD) activity comprises the amino acid sequence of SEQ ID NO: 32, 33 or 34 or an amino acid sequence corresponding to SEQ ID NO: 32, 33 or 34 wherein 1 to 40 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 32, 33 or 34 or an amino acid sequence corresponding to SEQ ID NO: 32, 33 or 34 wherein 1 to 20 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 32, 33 or 34 or an amino acid sequence corresponding to SEQ ID NO: 32, 33 or 34 wherein 1 to 10 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 32, 33 or 34 or an amino acid sequence corresponding to SEQ ID NO: 32, 33 or 34 wherein 1 to 5 amino acids are substituted with the proviso that the amino acid at position 89 is not E and/or the amino acid at position 466 is not H, preferably with the proviso that the amino acid at position 89 is Q and/or the amino acid at position 466 is A. According to some embodiments, the polypeptide having glutamate decarboxylase (GAD) activity comprises the amino acid sequence of SEQ ID NO: 32, 33 or 34.

According to some embodiments, the polypeptide having glutamate decarboxylase (GAD) activity comprises the amino acid sequence of SEQ ID NO: 35, 36 or 37 or an amino acid sequence corresponding to SEQ ID NO: 35, 36 or 37 wherein 1 to 40 amino acids are substituted with the proviso that the amino acid at position 89 is not E and/or the amino acid at position 463 is not H, preferably with the proviso that the amino acid at position 89 is Q and/or the amino acid at position 463 is A. According to some embodiments, the polypeptide having glutamate decarboxylase (GAD) activity comprises the amino acid sequence of SEQ ID NO: 35, 36 or 37 or an amino acid sequence corresponding to SEQ ID NO: 35, 36 or 37 wherein 1 to 20 amino acids are substituted with the proviso that the amino acid at position 89 is not E and/or the amino acid at position 463 is not H, preferably with the proviso that the amino acid at position 89 is Q and/or the amino acid at position 463 is A. According to some embodiments, the polypeptide having glutamate decarboxylase (GAD) activity comprises the amino acid sequence of SEQ ID NO: 35, 36 or 37 or an amino acid sequence corresponding to SEQ ID NO: 35, 36 or 37 wherein 1 to 10 amino acids are substituted with the proviso that the amino acid at position 89 is not E and/or the amino acid at position 463 is not H, preferably with the proviso that the amino acid at position 89 is Q and/or the amino acid at position 463 is A. According to some embodiments, the polypeptide having glutamate decarboxylase (GAD) activity comprises the amino acid sequence of SEQ ID NO: 35, 36 or 37 or an amino acid sequence corresponding to SEQ ID NO: 35, 36 or 37 wherein 1 to 5 amino acids are substituted with the proviso that the amino acid at position 89 is not E and/or the amino acid at position 463 is not H, preferably with the proviso that the amino acid at position 89 is Q and/or the amino acid at position 463 is A. According to some embodiments, the polypeptide having glutamate decarboxylase (GAD) activity comprises the amino acid sequence of SEQ ID NO: 35, 36 or 37.

According to some embodiments, the polypeptide having glutamate decarboxylase (GAD) activity comprises the amino acid sequence of SEQ ID NO: 38 or an amino acid sequence corresponding to SEQ ID NO: 38 wherein 1 to 40 amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H. According to some embodiments, the polypeptide having glutamate decarboxylase (GAD) activity comprises the amino acid sequence of SEQ ID NO: 38 or an amino acid sequence corresponding to SEQ ID NO: 38 wherein 1 to 20 amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H. According to some embodiments, the polypeptide having glutamate decarboxylase (GAD) activity comprises the amino acid sequence of SEQ ID NO: 38 or an amino acid sequence corresponding to SEQ ID NO: 38 wherein 1 to 10 amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H. According to some embodiments, the polypeptide having glutamate decarboxylase (GAD) activity comprises the amino acid sequence of SEQ ID NO: 38 or an amino acid sequence corresponding to SEQ ID NO: 38 wherein 1 to 5 amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H. According to some embodiments, the polypeptide having glutamate decarboxylase (GAD) activity comprises the amino acid sequence of SEQ ID NO: 38 or an amino acid sequence corresponding to SEQ ID NO: 38.

According to some embodiments, the polypeptide having glutamate decarboxylase (GAD) activity comprises an amino acid sequence having at least 70%, such as at least 75%, sequence identity with any one of SEQ ID NOs: 11 to 38, wherein 2 to 20 amino acids are deleted from the C-terminus. According to some embodiments, the polypeptide having glutamate decarboxylase (GAD) activity comprises an amino acid sequence having at least 80%, such as at least 85%, sequence identity with any one of SEQ ID NOs: 11 to 38, wherein 2 to 20 amino acids are deleted from the C-terminus. According to some embodiments, the polypeptide having glutamate decarboxylase (GAD) activity comprises an amino acid sequence having at least 90%, such as at least 95%, sequence identity with any one of SEQ ID NOs: 11 to 38, wherein 2 to 20 amino acids are deleted from the C-terminus.

According to some embodiments, the polypeptide having glutamate decarboxylase (GAD) activity comprises an amino acid sequence having at least 70%, such as at least 75%, sequence identity with any one of SEQ ID NOs: 11 to 38, wherein 2 to 14 amino acids are deleted from the C-terminus. According to some embodiments, the polypeptide having glutamate decarboxylase (GAD) activity comprises an amino acid sequence having at least 80%, such as at least 85%, sequence identity with any one of SEQ ID NOs: 11 to 38, wherein 2 to 14 amino acids are deleted from the C-terminus. According to some embodiments, the polypeptide having glutamate decarboxylase (GAD) activity comprises an amino acid sequence having at least 90%, such as at least 95%, sequence identity with any one of SEQ ID NOs: 11 to 38, wherein 2 to 14 amino acids are deleted from the C-terminus.

According to some embodiments, the polypeptide having glutamate decarboxylase (GAD) activity comprises an amino acid sequence having at least 70%, such as at least 75%, sequence identity with any one of SEQ ID NOs: 11 to 38, wherein 4 to 10 amino acids are deleted from the C-terminus. According to some embodiments, the polypeptide having glutamate decarboxylase (GAD) activity comprises an amino acid sequence having at least 80%, such as at least 85%, sequence identity with any one of SEQ ID NOs: 11 to 38, wherein 4 to 10 amino acids are deleted from the C-terminus. According to some embodiments, the polypeptide having glutamate decarboxylase (GAD) activity comprises an amino acid sequence having at least 90%, such as at least 95%, sequence identity with any one of SEQ ID NOs: 11 to 38, wherein 4 to 10 amino acids are deleted from the C-terminus.

According to some embodiments, the polypeptide having glutamate decarboxylase (GAD) activity comprises the amino acid sequence of SEQ ID NO: 90 or an amino acid sequence corresponding to SEQ ID NO: 90, wherein 1 to 40, such as 1 to 20, amino acids are substituted with the proviso that the amino acid at position 89 is not E, preferably with the proviso that the amino acid at position 89 is Q.

According to some embodiments, the polypeptide having glutamate decarboxylase (GAD) activity comprises the amino acid sequence of SEQ ID NO: 92 or an amino acid sequence corresponding to SEQ ID NO: 92, wherein 1 to 40, such as 1 to 20, amino acids are substituted with the proviso that the amino acid at position 84 is not E, preferably with the proviso that the amino acid at position 84 is Q.

The present invention further provides in a second aspect a mesophilic, methylotrophic bacterium which expresses a) a polypeptide having α-ketoglutarate decarboxylase (KDC) activity and b) a polypeptide having GABA transaminase (GABA-TA) activity.

According to some embodiments, the polypeptide having KDC activity comprises the amino acid sequence of SEQ ID NO: 39, 40, 41 or 42 or an amino acid sequence having at least 70%, such as at least 80%, sequence identity with SEQ ID NO: 39, 40, 41 or 42. According to some embodiments, the polypeptide having KDC activity comprises the amino acid sequence of SEQ ID NO: 39, 40, 41 or 42. According to some embodiments, the polypeptide having KDC activity comprises an amino acid sequence having at least 70%, such as at least 80%, sequence identity with SEQ ID NO: 39, 40, 41 or 42.

According to some embodiments, the polypeptide having KDC activity comprises the amino acid sequence of SEQ ID NO: 39 or an amino acid sequence having at least 70%, such as at least 75%, sequence identity with SEQ ID NO: 39. According to some embodiments, the polypeptide having KDC activity comprises the amino acid sequence of SEQ ID NO: 39 or an amino acid sequence having at least 80%, such as at least 85%, sequence identity with SEQ ID NO: 39. According to some embodiments, the polypeptide having KDC activity comprises the amino acid sequence of SEQ ID NO: 39 or an amino acid sequence having at least 90%, such as at least 95%, sequence identity with SEQ ID NO: 39. According to some embodiments, the polypeptide having KDC activity comprises the amino acid sequence of SEQ ID NO: 39.

According to some embodiments, the polypeptide having KDC activity comprises the amino acid sequence of SEQ ID NO: 40 or an amino acid sequence having at least 70%, such as at least 75%, sequence identity with SEQ ID NO: 40. According to some embodiments, the polypeptide having KDC activity comprises the amino acid sequence of SEQ ID NO: 40 or an amino acid sequence having at least 80%, such as at least 85%, sequence identity with SEQ ID NO: 40. According to some embodiments, the polypeptide having KDC activity comprises the amino acid sequence of SEQ ID NO: 40 or an amino acid sequence having at least 90%, such as at least 95%, sequence identity with SEQ ID NO: 40. According to some embodiments, the polypeptide having KDC activity comprises the amino acid sequence of SEQ ID NO: 40.

According to some embodiments, the polypeptide having KDC activity comprises the amino acid sequence of SEQ ID NO: 41 or an amino acid sequence having at least 70%, such as at least 75%, sequence identity with SEQ ID NO: 41. According to some embodiments, the polypeptide having KDC activity comprises the amino acid sequence of SEQ ID NO: 41 or an amino acid sequence having at least 80%, such as at least 85%, sequence identity with SEQ ID NO: 41. According to some embodiments, the polypeptide having KDC activity comprises the amino acid sequence of SEQ ID NO: 41 or an amino acid sequence having at least 90%, such as at least 95%, sequence identity with SEQ ID NO: 41. According to some embodiments, the polypeptide having KDC activity comprises the amino acid sequence of SEQ ID NO: 41.

According to some embodiments, the polypeptide having KDC activity comprises the amino acid sequence of SEQ ID NO: 42 or an amino acid sequence having at least 70%, such as at least 75%, sequence identity with SEQ ID NO: 42. According to some embodiments, the polypeptide having KDC activity comprises the amino acid sequence of SEQ ID NO: 42 or an amino acid sequence having at least 80%, such as at least 85%, sequence identity with SEQ ID NO: 42. According to some embodiments, the polypeptide having KDC activity comprises the amino acid sequence of SEQ ID NO: 42 or an amino acid sequence having at least 90%, such as at least 95%, sequence identity with SEQ ID NO: 42. According to some embodiments, the polypeptide having KDC activity comprises the amino acid sequence of SEQ ID NO: 42.

According to some embodiments, the polypeptide having GABA-TA activity comprises the amino acid sequence of any one of SEQ ID NOs: 43 to 74 or an amino acid sequence having at least 70%, such as at least 75%, sequence identity with any one of SEQ ID NO: 43 to 74. According to some embodiments, the polypeptide having GABA-TA activity comprises the amino acid sequence of any one of SEQ ID NOs: 43 to 74 or an amino acid sequence having at least 80%, such as at least 85%, sequence identity with any one of SEQ ID NO: 43 to 74. According to some embodiments, the polypeptide having GABA-TA activity comprises the amino acid sequence of any one of SEQ ID NOs: 43 to 74 or an amino acid sequence having at least 90%, such as at least 95%, sequence identity with any one of SEQ ID NO: 43 to 74. According to some embodiments, the polypeptide having GABA-TA activity comprises the amino acid sequence of any one of SEQ ID NOs: 43 to 74.

According to some embodiments, the polypeptide having GABA-TA activity comprises the amino acid sequence of SEQ ID NO: 43 or an amino acid sequence having at least 70%, such as at least 75%, sequence identity with SEQ ID NO: 43. According to some embodiments, the polypeptide having GABA-TA activity comprises the amino acid sequence of SEQ ID NO: 43 or an amino acid sequence having at least 80%, such as at least 85%, sequence identity with SEQ ID NO: 43. According to some embodiments, the polypeptide having GABA-TA activity comprises the amino acid sequence of SEQ ID NO: 43 or an amino acid sequence having at least 90%, such as at least 95%, sequence identity with SEQ ID NO: 43. According to some embodiments, the polypeptide having GABA-TA activity comprises the amino acid sequence of SEQ ID NO: 43.

The present invention also contemplates the conversion of GABA into 2-pyrrolidone via a single enzymatic reaction. Thus, according to some embodiments, a bacterium of the present invention further expresses a polypeptide having pyrrolidone synthase (PYS) activity.

According to some embodiments, the polypeptide having pyrrolidone synthase (PYS) activity comprises the amino acid sequence of SEQ ID NO: 75 or an amino acid sequence having at least 70%, such as at least 75%, sequence identity with SEQ ID NO: 75. According to some embodiments, the polypeptide having pyrrolidone synthase (PYS) activity comprises the amino acid sequence of SEQ ID NO: 75 or an amino acid sequence having at least 80%, such as at least 85%, sequence identity with SEQ ID NO: 75. According to some embodiments, the polypeptide having pyrrolidone synthase (PYS) activity comprises the amino acid sequence of SEQ ID NO: 75 or an amino acid sequence having at least 90%, such as at least 95%, sequence identity with SEQ ID NO: 75.

Generally, a polypeptide as detailed herein and employed in accordance with the invention will be heterologous to the bacterium of the invention, which means that said polypeptide is normally not found in or made (i.e. expressed) by the bacterium of the invention, but derived from a different species. However, the present invention also contemplates embodiments, where only one of the polypeptide having α-ketoglutarate decarboxylase (KDC) activity and the polypeptide having GABA transaminase (GABA-TA) activity is heterologous to the bacterium, e.g. where the polypeptide having α-ketoglutarate decarboxylase (KDC) activity is heterologous, while the polypeptide having GABA transaminase (GABA-TA) activity is endogenous to the bacterium.

A bacterium of the invention may thus have been modified to express at least one polypeptide as detailed herein, which means that an exogenous nucleic acid molecule, such as a DNA molecule, which comprises a nucleotide sequence encoding said polypeptide has been introduced in the bacterium. Thus, a bacterium of the present invention may comprise an exogenous nucleic acid molecule, such as a DNA molecule, which comprises a nucleotide sequence encoding the polypeptide in question. Techniques for introducing an exogenous nucleic acid molecule, such as a DNA molecule, into a bacterial cell are well-known to those of skill in the art, and include transformation (e.g., heat shock or natural transformation) among others.

In order to facilitate (over)expression of a polypeptide in the bacterium, the exogenous nucleic acid molecule may comprise suitable regulatory elements such as a promoter that is functional in the bacterial cell to cause the production of an mRNA molecule and that is operably linked to the nucleotide sequence encoding said polypeptide.

Promoters useful in accordance with the invention are any known promoters that are functional in a given host cell to cause the production of an mRNA molecule. Many such promoters are known to the skilled person. Such promoters include promoters normally associated with other genes, and/or promoters isolated from any bacteria. The use of promoters for protein expression is generally known to those of skilled in the art of molecular biology, for example, see Sambrook et al., Molecular cloning: A Laboratory Manual, Cold Spring Harbor Laboratory, Cold Spring Harbor, N.Y., 1989. The promoter employed may be inducible, such as a temperature inducible promoter (e.g., a pL or pR phage lambda promoters, each of which can be controlled by the temperature-sensitive lambda repressor c1857). The term "inducible" used in the context of a promoter means that the promoter only directs transcription of an operably linked nucleotide sequence if a stimulus is present, such as a change in temperature or the presence of a chemical substance ("chemical inducer"). As used herein, "chemical induction" according to the present invention refers to the physical application of an exogenous or endogenous substance (incl. macromolecules, e.g., proteins or nucleic acids) to a host cell. This has the effect of causing the target promoter present in the host cell to increase the rate of transcription. Alternatively, the promoter employed may be constitutive. The term "constitutive" used in the context of a promoter means that the promoter is capable of directing transcription of an operably linked nucleotide sequence in the absence of stimulus (such as heat shock, chemicals etc.).

Temperature induction systems work, for example, by employing promoters that are repressed by thermolabile repressors. These repressors are active at lower temperatures for example at 30°C, while unable to fold correctly at 37 °C and are therefore inactive. Such circuits therefore can be used to directly regulate the genes of interest (St-Pierre et al. 2013) also by genome integration of the genes along with the repressors. Examples of such as a temperature inducible expression system are based on the pL and/or pR λ phage promoters which are regulated by the thermolabile cl857 repressor. Similar to the genome integrated DE3 system, the expression of the T7 RNA polymerase gene may also be controlled using a temperature controlled promoter system (Mertens et al. 1995), while the expression of the genes of interest can be controlled using a T7 promoter.

Non-limiting examples of promoters functional in bacteria include both constitutive and inducible promoters such as T7 promoter, the beta-lactamase and lactose promoter systems; alkaline phosphatase (phoA) promoter, a tryptophan (trp) promoter system, tetracycline promoter, lambda-phage promoter, ribosomal protein promoters; and hybrid promoters such as the tac promoter. Other bacterial and synthetic promoters are also suitable.

Besides a promoter, the exogenous nucleic acid molecule may further comprise at least one regulatory element selected from a 5' untranslated region (5'UTR) and 3' untranslated region (3' UTR). Many such 5' UTRs and 3' UTRs derived from prokaryotes and eukaryotes are well known to the skilled person. Such regulatory elements include 5' UTRs and 3' UTRs normally associated with other genes, and/or 5' UTRs and 3' UTRs isolated from any bacteria.

Usually, the 5' UTR contains a ribosome binding site (RBS), also known as the Shine Dalgarno sequence which is usually 3-10 base pairs upstream from the initiation codon.

The exogenous nucleic acid molecule may be a vector or part of a vector, such as an expression vector. Normally, such a vector remains extrachromosomal within the bacterial cell which means that it is found outside of the nucleus or nucleoid region of the bacterium.

It is also contemplated by the present invention that the exogenous nucleic acid molecule is stably integrated into the genome of the bacterium. Means for stable integration into the genome of a host cell, e.g., by homologous recombination, are well known to the skilled person.

A bacterium in accordance with the present invention can be produced from any suitable mesophilic and methylotrophic bacterium. The bacterium may be Gram-positive or Gram-negative.

According to some embodiments, the bacterium of the present invention is of the genus *Methylobacillus* or *Methylobacterium.*

According to some embodiments, the bacterium of the present invention is selected from *Methylobacillus flagellatus, Methylobacillus glycogenes, Methylobacillus pratensis, Methylobacillus rhizosphaerae, Methylobacterium extorquens* and *Methylobacterium organophilum.*

According to some embodiments, the bacterium of the present invention is *Methylobacillus flagellutes.*

According to some embodiments, the bacterium of the present invention is *Methylobacillus glycogenes.*

According to some embodiments, the bacterium of the present invention is *Methylobacillus pratensis.*

According to some embodiments, the bacterium of the present invention is *Methylobacillus rhizosphaerae.*

According to some embodiments, the bacterium of the present invention is *Methylobacterium extorquens.*

According to some embodiments, the bacterium of the present invention is *Methylobacterium organophilum.*

### Method of the invention

The present invention also provides methods for producing a biochemical comprising cultivating a bacterium according to the invention under suitable culture conditions in a culture medium comprising a reduced one-carbon compound, such as methanol, or a multi-carbon compound that contain no carbon-carbon bonds, such as dimethyl ether and dimethylamine.

The method may further comprise collecting the biochemical from the culture medium.

According to certain embodiments, present invention provides a method for producing GABA or a derivative thereof. Particularly, the present invention provides a method for producing GABA or a derivative thereof; said method comprises cultivating a bacterium as detailed herein in a culture medium. The method may further comprise collecting GABA or the derivative thereof from the culture medium.

According to some embodiments, the GABA derivative is selected from the group consisting of 2-pyrrolidone, N-methyl-2-pyrrolidone and polybutyrolactam.

According to certain embodiments, present invention provides a method for producing 2-pyrrolidone or a derivative thereof, such as N-methyl-2-pyrrolidone. Particularly, the present invention provides a method for producing 2-pyrrolidone or a derivative thereof, such as N-methylpyrrolidone, said method comprises cultivating a bacterium as detailed herein in a culture medium. The method may further comprise collecting 2-pyrrolidone or the derivative thereof, such as N-methyl-2-pyrrolidone, from the culture medium.

The culture medium employed may be any conventional medium suitable for culturing a bacterium cell in question, and may be composed according to the principles of the prior art. The medium will usually contain all nutrients necessary for the growth and survival of the respective bacterium, such as carbon and nitrogen sources and other inorganic salts. Suitable media, e.g. minimal or complex media, are available from commercial suppliers, or may be prepared according to published receipts, e.g. the American Type Culture Collection (ATCC) Catalogue of strains. Non-limiting standard medium well known to the skilled person include Luria Bertani (LB) broth, Sabouraud Dextrose (SD) broth, MS broth, Yeast Peptone Dextrose, BMMY, GMMY, or Yeast Malt Extract (YM) broth, which are all commercially available. A non-limiting example of suitable media for culturing bacterial cells, such as E. coli cells, including minimal media and rich media such as Luria Broth (LB), M9 media, M17 media, SA media, MOPS media, Terrific Broth, YT and others.

The carbon source may be any suitable carbon substrate known in the art, and in particularly any carbon substrate commonly used in the cultivation of methylotrophic bacteria and/or fermentation. A carbon source of particular interest is a reduced one-carbon compound, such as methanol, methane, formate, or methylamine, or a multi-carbon compound that contains no carbon-carbon bonds, such as dimethyl ether and dimethylamine. Thus, according to some embodiments, the culture medium comprises methanol as a carbon source. The concentration of methanol in the culture medium may generally be between in the range from about 0.5% w/v to about 4 % w/v, such as from about 2% w/v to about 4 % w/v. According to some embodiments, the concentration of methanol in the culture medium is in the range from about 2.5% w/v to about 3.5% w/v.

As the nitrogen source, various ammonium salts such as ammonia and ammonium sulfate, other nitrogen compounds such as amines, a natural nitrogen source such as peptone, soybean-hydrolysate, and digested fermentative microorganism can be used. As minerals, potassium monophosphate, magnesium sulfate, sodium chloride, ferrous sulfate, manganese sulfate, calcium chloride, and the like can be used.

The cultivation can be preferably performed under aerobic conditions, such as by a shaking culture, and by a stirring culture with aeration, at a temperature of about 20 to about 45 °C, such as about 30 to 38 °C, such as at about 37°C. The pH of the culture is usually above 5, such as in a range from about 6 to about 8, preferably from about 6.5 to about 7.5, more preferably from about 6.8 to about 7.2. The pH of the culture can be adjusted with ammonia, calcium carbonate, various acids, various bases, and buffers. The cultivation may be carried out for a period in the range from 10 to 70 h, preferably in a range from 24 to 60 h, more preferably in a range from 36 to 50 h.

After cultivation, solids such as cells can be removed from the culture medium by centrifugation or membrane filtration. The biochemical can be collected by conventional method for isolation and purification chemical compounds from a medium. Well-known purification procedures include, but are not limited to, centrifugation or filtration, precipitation, ion exchange, chromatographic methods such as e.g. ion exchange chromatography or gel filtration chromatography, and crystallization methods.

The present invention thus provides a biochemical obtainable by a method as detailed herein.

### Certain other definitions

The term "mesophilic" as used herein in the context of a bacterium means that the bacterium grows best in moderate temperature with an optimum growth range from 20 to 45 °C.

The term "methylotrophic" as used herein in the context of a bacterium means that the bacterium can use reduced one-carbon compounds, such as methanol, methane, formate, or methylamine, as the carbon source for their growth, and multi-carbon compounds that contain no carbon-carbon bonds, such as dimethyl ether and dimethylamine.

As used herein, a "polypeptide having glutamate decarboxylase (GAD) activity" or a "polypeptide which has glutamate decarboxylase (GAD) activity" means a polypeptide that catalyzes the reaction: L-glutamate <=> 4-aminobutanoate + CO(2) (EC 4.1.1.15). Non-limiting examples of such polypeptides are provided in SEQ ID NOs: 1 to 10.

As used herein, a "polypeptide having α-ketoglutarate decarboxylase (KDC) activity" or a " polypeptide which has α-ketoglutarate decarboxylase (KDC) activity" means a polypeptide that catalyzes the reaction: 2-oxoglutarate <=> succinate semialdehyde + CO(2) (EC 4.1.1.71).Non-limiting examples of such polypeptides are provided in SEQ ID NOs: 39, 40, 41 and 42.

As used herein, a "polypeptide having GABA transaminase (GABA-TA) activity" or a "polypeptide which has GABA transaminase (GABA-TA) activity" means a polypeptide that catalyzes the reaction: 4-aminobutanoate + 2-oxoglutarate <=> succinate semialdehyde + L-glutamate (EC 2.6.1.19). Non-limiting examples of such polypeptides are provided in SEQ ID NOs: 43 to 74.

As used herein, a "polypeptide having pyrrolidone synthase (PYS) activity" or a "polypeptide which has pyrrolidone synthase (PYS) activity" means a polypeptide that catalyzes the ring closing dehydration of γ-aminobutyrate. A non-limiting example of such polypeptide is provided in SEQ ID NO: 75, and is further described in, e.g, US 2016/0304922.

"Polypeptide" and "protein" are used interchangeably herein to denote a polymer of at least two amino acids covalently linked by an amide bond, regardless of length or post- translational modification (e.g., glycosylation, phosphorylation, lipidation, myristilation, ubiquitination, etc.). Included within this definition are D- and L-amino acids, and mixtures of D- and L-amino acids.

"Nucleic acid" or "polynucleotide" are used interchangeably herein to denote a polymer of at least two nucleic acid monomer units or bases (e.g., adenine, cytosine, guanine, thymine) covalently linked by a phosphodiester bond, regardless of length or base modification.

"Recombinant" or "non-naturally occurring" when used with reference to, e.g., a host cell, nucleic acid, or polypeptide, refers to a material, or a material corresponding to the natural or native form of the material, that has been modified in a manner that would not otherwise exist in nature, or is identical thereto but produced or derived from synthetic materials and/or by manipulation using recombinant techniques. Non-limiting examples include, among others, recombinant bacterial cells expressing genes that are not found within the native (non-recombinant) form of the cell or express native genes that are otherwise expressed at a different level.

"Heterologous" or "exogenous" as used herein in the context of a gene or nucleic acid molecule refer to a gene or nucleic acid molecule (i.e. DNA or RNA molecule) that does not occur naturally as part of the genome of the bacterium in which it is present or which is found in a location or locations in the genome that differ from that in which it occurs in nature. Thus, a "heterologous" or "exogenous" gene or nucleic acid molecule is not endogenous to the bacterium and has been exogenously introduced into the microorganism. A "heterologous" gene or nucleic acid molecule DNA molecule may be from a different organism, a different species, a different genus or a different kingdom, as the host DNA.

"Heterologous" as used herein in the context of a polypeptide means that a polypeptide is normally not found in or made (i.e. expressed) by the host microorganism, but derived from a different organism, a different species, a different genus or a different kingdom.

As used herein, the term "ortholog" or "orthologs" refers to genes, nucleic acid molecules encoded thereby, i.e., mRNA, or proteins encoded thereby that are derived from a common ancestor gene but are present in different species.

"Substitution" or "substituted" refers to modification of the polypeptide by replacing one amino acid residue with another, for instance the replacement of a Serine residue with a Glycine or Alanine residue in a polypeptide sequence is an amino acid substitution. When used with reference to a polynucleotide, "substitution" or "substituted" refers to modification of the polynucleotide by replacing one nucleotide with another, for instance the replacement of a cytosine with a thymine in a polynucleotide sequence is a nucleotide substitution.

"Conservative substitution", when used with reference to a polypeptide, refers to a substitution of an amino acid residue with a different residue having a similar side chain, and thus typically involves substitution of the amino acid in the polypeptide with amino acids within the same or similar class of amino acids. By way of example and not limitation, an amino acid with an aliphatic side chain may be substituted with another aliphatic amino acid, e.g., alanine, valine, leucine, and isoleucine; an amino acid with hydroxyl side chain is substituted with another amino acid with a hydroxyl side chain, e.g., serine and threonine; an amino acid having an aromatic side chain is substituted with another amino acid having an aromatic side chain, e.g., phenylalanine, tyrosine, tryptophan, and histidine; an amino acid with a basic side chain is substituted with another amino acid with a basic side chain, e.g., lysine and arginine; an amino acid with an acidic side chain is substituted with another amino acid with an acidic side chain, e.g., aspartic acid or glutamic acid; and a hydrophobic or hydrophilic amino acid is replaced with another hydrophobic or hydrophilic amino acid, respectively.

"Non-conservative substitution", when used with reference to a polypeptide, refers to a substitution of an amino acid in a polypeptide with an amino acid with significantly differing side chain properties. Non-conservative substitutions may use amino acids between, rather than within, the defined groups and affects (a) the structure of the peptide backbone in the area of the substitution (e.g., serine for glycine), (b) the charge or hydrophobicity, or (c) the bulk of the side chain. By way of example and not limitation, an exemplary non-conservative substitution can be an acidic amino acid substituted with a basic or aliphatic amino acid; an aromatic amino acid substituted with a small amino acid; and a hydrophilic amino acid substituted with a hydrophobic amino acid.

"Expression" includes any step involved in the production of a polypeptide (e.g., encoded enzyme) including, but not limited to, transcription, post-transcriptional modification, translation, post-translational modification, and secretion.

As used herein, "vector" refers to a nucleic acid molecule capable of transporting another nucleic acid molecule to which it has been linked. One type of vector is a "plasmid", which refers to a circular double stranded nucleic acid loop into which additional nucleic acid segments can be ligated. Certain vectors are capable of directing the expression of genes to which they are operatively linked. Such vectors are referred to herein as "expression vectors". Certain other vectors are capable of facilitating the insertion of an exogenous nucleic acid molecule into a genome of a bacterium. Such vectors are referred to herein as "transformation vectors". In general, vectors of utility in recombinant nucleic acid techniques are often in the form of plasmids. In the present specification, "plasmid" and "vector" can be used interchangeably as the plasmid is the most commonly used form of a vector. Large numbers of suitable vectors are known to those of skill in the art and commercially available.

As used herein, "promoter" refers to a sequence of DNA, usually upstream (5') of the coding region of a structural gene, which controls the expression of the coding region by providing recognition and binding sites for RNA polymerase and other factors which may be required for initiation of transcription. The selection of the promoter will depend upon the nucleic acid sequence of interest. A suitable "promoter" is generally one which is capable of supporting the initiation of transcription in a bacterium of the invention, causing the production of an mRNA molecule.

As used herein, "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequence. A promoter sequence is "operably-linked" to a gene when it is in sufficient proximity to the transcription start site of a gene to regulate transcription of the gene.

"Percentage of sequence identity," "% sequence identity" and "percent identity" are used herein to refer to comparisons between an amino acid sequence and a reference amino acid sequence. The "% sequence identity", as used herein, is calculated from the two amino acid sequences as follows: The sequences are aligned using Version 9 of the Genetic Computing Group's GAP (global alignment program), using the default BLOSUM62 matrix with a gap open penalty of -12 (for the first null of a gap) and a gap extension penalty of -4 (for each additional null in the gap). After alignment, percentage identity is calculated by expressing the number of matches as a percentage of the number of amino acids in the reference amino acid sequence.

"Reference sequence" or "reference amino acid sequence" refers to a defined sequence to which another sequence is compared. In the context of the present invention a reference amino acid sequence may, for example, be an amino acid sequence set forth in SEQ ID NO: 1.

As used herein, "derivative" of GABA refers to a compound derived from GABA by modification thereof. Non-limiting examples of a GABA "derivative" include 2-pyrrolidone, N-methyl-2-pyrrolidone and polybutyrolactam (Nylon 4).

As used herein, the term "about" means plus or minus 10% of the numerical value of the number with which it is being used.

Where a numerical limit or range is stated herein, the endpoints are included. Also, all values and sub ranges within a numerical limit or range are specifically included as if explicitly written out.

As used herein, the indefinite articles "a" and "an" mean "at least one" or "one or more" unless the context clearly dictates otherwise.

As used herein, the terms "comprising", "including", "having" and grammatical variants thereof are to be taken as specifying the stated features, steps or components but do not preclude the addition of one or more additional features, steps, components or groups thereof.

Having generally described this invention, a further understanding can be obtained by reference to certain specific examples, which are provided herein for purposes of illustration only, and are not intended to be limiting unless otherwise specified.

### Examples

### I. Genetic methods

### Example 1: Plasmid preparation and transformation

Plasmids contained a pBBR1 origin or replication, a kanamycin resistance or a rifampicin resistance cassette, and a lacO/trc promoter for controlling gene expression. Custom genes were inserted downstream of the promotor using HiFi assembly. Plasmids were constructed to express single genes, i.e. pABM36 was used to express the gene SEQ ID NO: 91. In some cases, plasmids were used to express two genes under the control of a single promoter. An additional RBS sequence was placed immediately downstream of the first gene and immediately upstream of the second genes. Examples include pABM45, which expresses the genes SEQ ID NO: 86 and SEQ ID NO: 87.

Plasmid DNAwas prepared using the NEB HiFi 2x Master Mix according to manufacturer instruction. HiFi assemblies were transformed to *Escherichia coli* DH5α strains. Transformants were selected with 50 µg/mL kanamycin or 50 µg/mL rifampicin in solid and liquid 2TY medium. Plasmid DNA was extracted from overgrown liquid cultures using the GeneJet MiniPrep kit according to manufacturer instructions.

Plasmid DNA (e.g. pABM36) was transformed to ABME 3, ABME 5, ABME 6, and their derivative strains via electroporation. Transformations plated onto selective PM7 plates containing 20 g/L agar, 50 µg/mL kanamycin, or 5 µg/mL rifampicin and incubated at 37 °C for 24-48 h.

All the plasmids that were prepared are listed in Table 1. The strains prepared by plasmid transformation are listed in Table 2.

**Table 1: List of plasmids**

| **Plasmid** | **Purpose** |
|---|---|
| pABM4 | Expression of GFP with gene sequence SEQ ID NO: 78 and protein sequence SEQ ID NO: 76 |
| pABM28 | Co-expression of mCherry with gene sequence SEQ ID NO: 79 and protein sequence SEQ ID NO: 77, and GPF with gene sequence SEQ ID NO: 78and protein sequence SEQ ID NO: 76 |
| pABM34 | Expression of Lbr GAD 4AAmut with gene sequence SEQ ID NO: 85 and protein sequence SEQ ID NO: 38 |
| pABM35 | Expression of Lbr GAD with gene sequence SEQ ID NO: 82 and protein sequence SEQ ID NO: 10 |
| pABM36 | Expression of Eco GAD E89QΔ14C with gene sequence SEQ ID NO: 91 and protein sequence SEQ ID NO: 90 |
| pABM37 | Expression of Sth GAD E84QΔ14C with gene sequence SEQ ID NO: 84 and protein sequence SEQ ID NO: 92 |
| pABM44 | Co-expression of Egr KDC with gene sequence SEQ ID NO: 86 and protein sequence SEQ ID NO: 39, and Eco GABA-TA with gene sequence SEQ ID NO: 88 and protein sequence SEQ ID NO: 43 |
| pABM45 | Co-expression of Scc KDC with gene sequence SEQ ID NO: 87 and protein sequence SEQ ID NO: 40, and Eco GABA-TA with gene sequence SEQ ID NO: 88 and protein sequence SEQ ID NO: 43 |
| pABM TH1 | Expression of Sai PYS with gene sequence SEQ ID NO: 89 and protein sequence SEQ ID NO: 75 |
| pABM TH2 | Co-expression of Sai PYS with gene sequence SEQ ID NO: 89 and protein sequence SEQ ID NO: 75 and Eco GAD E89QΔ14C with gene sequence SEQ ID NO: 91 and protein sequence SEQ ID NO: 90 |

**Table 2: List of strains prepared by plasmid transformation**

| **Strain** | **Genotype/parent strain** | **Plasmid** |
|---|---|---|
| ABME 5 | Wild-type *Methylobacillus glycogenes* DSM 5685 | / |
| ABME 23 | ABME 5 | pABM4 |
| ABME 27 | ABME 5 | pABM28 |
| ABME 32 | ABME 5 | pABM 34 |
| ABME 33 | ABME 5 | pABM 35 |
| ABME 34 | ABME 5 | pABM 36 |
| ABME 35 | ABME 5 | pABM 37 |
| ABME 99 | ABME 5 | pABM44 |
| ABME 101 | ABME 5 | pABM45 |
| ABME 6 | Wild-type *Methylobacillus flagellatus* DSM 6875 | / |
| ABME 25 | ABME 6 | pABM4 |
| ABME 65 | ABME 6 | pABM28 |
| ABME 38 | ABME 6 | pABM34 |
| ABME 39 | ABME 6 | pABM35 |
| ABME 40 | ABME 6 | pABM36 |
| ABME 41 | ABME 6 | pABM37 |
| ABME 90 | ABME 6 | pABM44 |
| ABME 104 | ABME 6 | pABM45 |
| ABME 6TH2 | ABME 126 | pABMTH1 |
| ABME 6TH3 | ABME 6 | pABM TH2 |

### Example 2: Gene integration

GABA producing genes were integrated into the genome of ABME 6, or ABME 6-derived strains. The genome edits were achieved by introducing a linear DNA fragment consisting of the gene of interest and a kanamycin or chloramphenicol resistance cassette flanked by 2 kb long sequences upstream and downstream of the integration sites. DNA fragments were transformed into ABME 6 by electroporation. Transformations were plated onto selective PM7 plates containing 20 g/L agar, 50 µg/mL kanamycin, or 500 µg/mL thiamphenicol and incubated at 37 °C for 24-48. Correct clones were confirmed by colony PCR.

Strain ABME 126 was generated by integrating the gene with SEQ ID NO: 91 by replacing the AMBE 6 *epsD-G* genes.

Strain ABME 6TH1 was generated by integrating the gene with SEQ ID NO: 87 by replacing the genes Mfla_1268-1280, and by integrating the gene with SEQ ID NO: 88 by replacing the AMBE 6 *epsD-G* genes.

The strains prepared by genome integration are listed in Table 3.

**Table 3: List of strains prepared with genomic integration.**

| **Strain** | **Genotype/parent strain** | **Plasmid** |
|---|---|---|
| ABME 6 | Wild-type *Methylobacillus flagellatus* DSM 6875 | / |
| ABME 126 | epsD-G::Eco_gad_E89Q-kanR / ABME 6 | / |

### Example 3: Expression of fluorescent proteins in the genera Metylobacillus and Methylobacterium

To test the capacity of methylotrophs from the genera *Methylobacillus* and *Methylobacterium,* GFP and mCherry were expressed from plasmid. GFP was expressed alone (ABME 23, ABME 25) or co-expressed in combination with mCherry (ABME 27 and ABME 65). The strains and plasmids were prepared as described in Example 1.

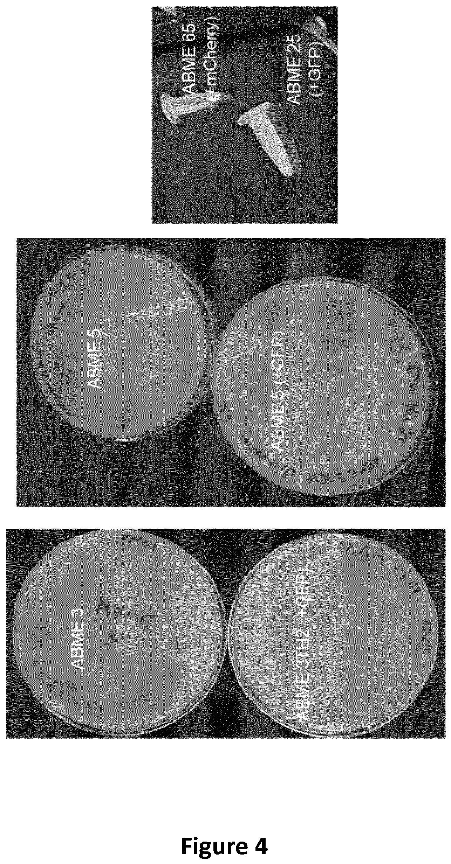

To assess observe GFP and RFP fluorescence, fresh single colonies were grown overnight on PM7 + 50 µg/mL kanamycin agar plates. Single colonies were resuspended in 25 mL PM7 medium + 25 µg/mL kanamycin in baffled 250 mL shake flasks, and incubated overnight at 200 rpm. Next day, 50 mL PM7 medium + 25 µg/mL kanamycin in baffled 250 mL shake flasks was inoculated with 5 mL overnight culture. After 4 h of growth, 0.5 g/L IPTG was added to induce gene expression. The medium was supplemented with 30 g/L glutamate. All ABME 3-derived strains and ABME 5-derived strains were cultivated at 30°C, and all ABME 6-derived strains were cultivated at 37°C. Cultures were analyzed after 24 h of growth. The GFP and RFP expression was sufficiently high to detect visually when plates or cell pellets were exposed to UV light (Figure 4).

### II. pH-independent GABA production via a GAD enzyme

### Example 4: Functional expression of three GAD enzymes in ABME 6 - in vitro test

Three GAD enzymes were tested *in vitro* to assess their capacity for functional GABA production when expressed in ABME 6-derived strains (ABME 38, ABME 39, and ABME40).

GAD enzymes were taken from *E. coli* and *Lactobacillus brevis* (Lbr). Eco GAD had an amino acid substitution, where glutamate at position 89 was replaced by glutamine (SEQ ID NO: 90) and 14 AA removed from the C-terminus. Lbr GAD was tested both as a wild-type enzyme (SEQ ID NO: 10), as well as a mutated version, where threonine at position 17 was substituted with isoleucine, aspartate at position 94 was substituted with glycine, glutamate at position 213 was substituted with serine, and glutamine at position 346 was replaced with histidine (SEQ ID NO: 38). The GAD variants were individually expressed in strains ABME 39, ABME 38, and ABME 40 which were obtained by transforming ABME 6 with the plasmids the plasmids pABM34, pABM35, and pABM36 as described in Example 1.

To prepare the cell lysates, fresh single colonies were grown overnight on PM7+50 µg/mL kanamycin agar plates at 37 °C. Single colonies were resuspended in 25 mL PM7 medium + 25 µg/mL kanamycin in baffled 250 mL shake flasks, and incubated overnight at 37 °C and 200 rpm. Next day, 25 mL PM7 medium + 25 µg/mL kanamycin in baffled 250 mL shake flasks was inoculated with 2.5 mL overnight culture and grown to an OD600=1.0 at 37 °C and 200 rpm. The cultures were induced with 0.5 g/L IPTG for 3 h and then centrifuged to concentrate the cells. The cell pellet was lysed by three freeze-thaw cycles at -80°C. The cell lysates were clarified by centrifugation at 4500 rpm and 4°C.

The clarified cell lysates were used to determine the *in vitro* activity of the three GAD variants in individual GAD enzyme assays reaction mixtures (Table 6). The reactions were incubated for 4 h at 37°C. The resulting GABA was measured by HPLC.

The HPLC method was set-up on Thermo Surveyor + HPLC system, equipped with a PDA detector and coupled to Corona + charged aerosol detector. Chromatographic separation was based on an AkzoNobel Kromasil 100-5 C18 HPLC column, 250x4.6 mm, with 5 um particle size, thermostated at 60 °C in the column oven. Mobile phase A was 0.09 % aqueous solution of heptafluorobutyric acid (HFBA) and mobile phase B was methanol (MeOH), with flow 1.0 ml / min on column with the gradient program as described in Table 4. Samples were injected with 10 % MeOH vs 90 % aqueous solution of % 0.05 TFA, and analytes are detected with CAD set at 36 psi nitrogen, within 100 pA range.

**Table 4: Gradient used in the HPLC method for GABA detection.**

| Time / min: | % A | % B |
|---|---|---|
| 0.0 | 85 | 15 |
| 4.5 | 85 | 15 |
| 4.6 | 1 | 99 |
| 6.5 | 1 | 99 |
| 6.6 | 85 | 15 |
| 15.0 | 85 | 15 |

**Table 5: Composition of PM7 medium**

| **Compound** | **Per 1L** |
|---|---|
| MeOH | 10 g |
| KH2PO4 | 0.65 g |
| NA2HPO4 | 1.05 g |
| MgSO4 ^{∗} 7H2O | 0.1 g |
| NH4Cl | 0.2 g |
| (NH4)2SO4 | 2g |
| TE1 1000X | 1 mL |
| d-Biotin | 0.1 mg |
| Thiamine^{∗}HCl | 0.1 mg |
| Riboflavin | 0.1 mg |
| Pyridoxine^{∗}HCl | 0.1 mg |
| Pantothenate | 0.1 mg |
| Nicotinamide | 0.1 mg |
| p-Aminobenzoic acid | 0.02 mg |
| Folic acid | 0.01 mg |
| Vitamin B12 | 0.01 mg |
| Lipoic acid | 0.01 mg |
| CaCl2 ^{∗} 2H2O | 3.3 mg |
| FeSO4 ^{∗} 7H2O | 1.3 mg |
| MnSO4 ^{∗} H2O | 0.1 mg |
| ZnSO4 ^{∗} 7H2O | 0.13 mg |
| CuSO4 ^{∗} 5H2O | 0.04 mg |
| Na2MoO4 ^{∗} 2H2O | 0.04 mg |
| CoCl2 ^{∗} 6H2O | 0.04 mg |
| H3BO3 | 0.03 mg |
| pH | 7.0 |
| Agar (for solid media) | 20 g |

**Table 6: GAD enzyme assay reaction**

| **Component** | **Concentration** |
|---|---|
| Ammonium-Acetate buffer, pH 5.5 | 0.5 M |
| Glutamate | 5 g/L |
| Pyridoxal-phosphate | 750 µM |
| Cell lysate | 100 µg |
| MQ water | To 1 mL |

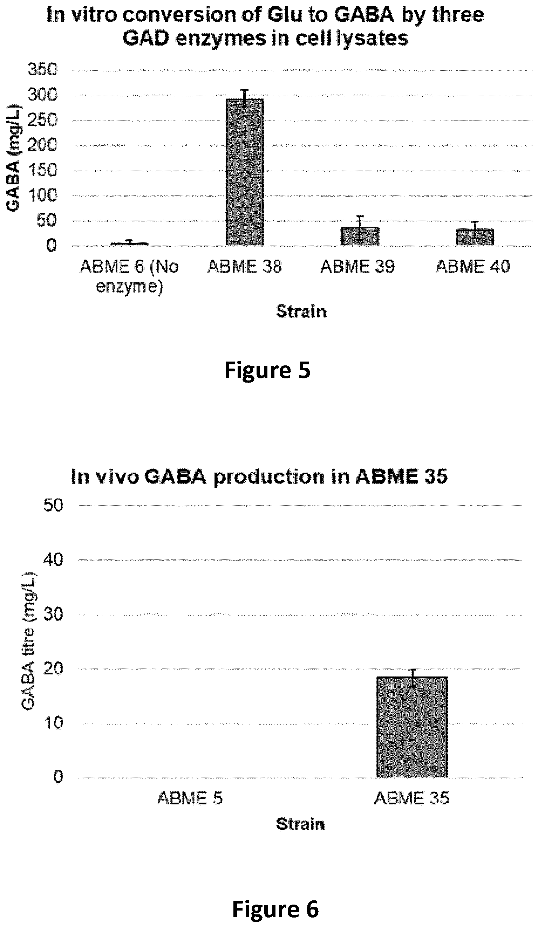

GABA formation was detected in all lysates that contained the GAD enzyme. ABME 38 cell lysate formed the most GABA, 239 ± 18 mg/L, while the lysates of ABME 39 and ABME 40 formed approximately 30 mg/L GABA (Figure 5). The *in vitro* GAD activity observed in strains ABME 38, ABME 39, and ABME 40 demonstrated for the first time that expression of GAD in *Methylobacillus flagellatus* is possible.

### Example 5: Sth GAD enzyme in ABME 5

The Sulfobacillus thermosulfidooxidans (Sth) GAD enzyme was tested *in vivo* to assess its capacity for GABA production when expressed from plasmid in the ABME 5-derived strain ABME 34 with external glutamate supplementation.

The GAD enzyme was taken from *Sulfobacillus thermosulfidooxidans* (Sth) with an amino acid substitution, where glutamate at position 84 was replaced by glutamine, and the C-terminal 14 amino acids deleted (SEQ ID NO: 84). The enzyme was expressed from plasmid pABM37 as described in Example 1.

Strain ABME 35 was obtained by transforming ABME 5 with the plasmid pABM37 as described in Example 1.

To assess *in vivo* GABA production by Sth GAD in shake flasks, fresh single colonies were grown overnight on PM7 + 50 µg/mL kanamycin agar plates. Single colonies were resuspended in 25 mL PM7 medium + 25 µg/mL kanamycin in baffled 250 mL shake flasks, and incubated overnight at 200 rpm. Next day, 50 mL PM7 medium + 25 µg/mL kanamycin in baffled 250 mL shake flasks was inoculated with 5 mL overnight culture. After 4 h of growth, 0.5 g/L IPTG was added to induce gene expression. The medium was supplemented with 30 g/L glutamate. All ABM E 5-derived strains were cultivated at 30°C. Cultures were sampled after 24 h of growth. GABA was measured by HPLC.

ABME 35 produced 18 ± 2 mg/L GABA (Figure 6). No GABA formation was detected in the negative controls (ABME 5).

### Example 6: Direct GABA production from methanol with Methylobacillus in shake flasks by GAD expression from a plasmid

Two GAD enzymes were further tested *in vivo* to assess their capacity for GABA production from methanol when expressed from plasmid in ABME 5-derived strain (ABME 32 and ABME 34) and ABME 6-derived strains (ABME 38 and ABME 40). In this example, no external glutamate was supplemented.

GAD enzymes were taken from *E. coli* and *Lactobacillus brevis* (Lbr). Eco GAD had an amino acid substitution, where glutamate at position 89 was replaced by glutamine, and the C-terminal 14 amino acids deleted (SEQ ID NO: 90). Lbr GAD was tested both as a wild-type enzyme (SEQ ID NO: 10), as well as a mutated version, where threonine at position 17 was substituted with isoleucine, aspartate at position 94 was substituted with glycine, glutamate at position 213 was substituted with serine, and glutamine at position 346 was replaced with histidine (SEQ ID NO: 38).

Strains ABME 32, ABME 34, ABME 38, and ABME 40 were obtained as described in Example 1. *In vivo* GABA production by the strains was tested in shake flasks, as described in Example with the omission of external glutamate. The cultures were sampled after 24 h and 48 h of growth. GABA production was measured by HPLC as described in Example .

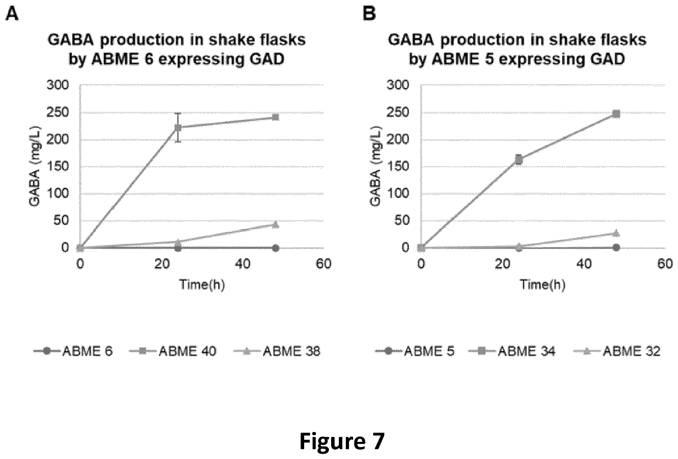

All four strains (ABME 32, ABME 34, ABME 38, and ABME 40) expressing GAD enzymes formed GABA directly from methanol without external glutamate addition (Figure 7AB). ABME 5 and ABME 6 did not express GAD enzymes and were used as negative controls. The strains ABME 34 and ABME 40 formed the most GABA (approximately 250 mg/L). This was surprising, as the *in vitro* test of four GAD enzymes (Example 4) showed that the Eco GAD E89Q Δ14C enzyme is less efficient compared to the Lbr GAD 4AAmut. In comparison, the strains expressing Lbr GAD 4AAmut (ABME 32 and ABME 38) formed less than 50 mg/L GABA.

This experiment is the first known example of direct GABA synthesis from methanol in any methylotroph without a pH-drop, which was reported so far in literature. To achieve this, it was necessary to use a mesophilic methylotroph to express the GAD enzymes, which was not expected.

### Example 7: Direct GABA production from methanol with Methylobacillus in reactors by GAD expression from a plasmid

The best performing strain, ABME 40 was tested in 5L and 20 L pH-controlled bioreactors to prove that GABA production was independent from pH and extend the GABA production phase. The process was oxygen limited with gradual methanol feeding.

The strain ABME 40 was prepared as described in Example 1. The bioreactors contained PM3 medium + 25 µg/mL kanamycin and were inoculated with an 8% (5L reactors) or 1% (20 L reactors) inoculum of ABME 40 with an OD600 = 1.0. pH was maintained at 7.0 by automatic addition of NH₄OH. Methanol concentrations were measured online and were kept at approximately 5 g/L throughout the fermentation by automatic addition of pure methanol. Reactor temperature was maintained at 37 °C throughout the process. The reactor was kept oxygen limited by flushing with 2 VVM (5L reactors) or 1VVM (20 L reactors) air while maintaining the pO₂ value at 0.0%. 0.5 g/L IPTG was added to the reactors after 19 h to induce GAD expression. Reactors were sampled regularly throughout the process. GABA and methanol were analyzed by HPLC as described in Example . Growth was monitored by measuring OD600.

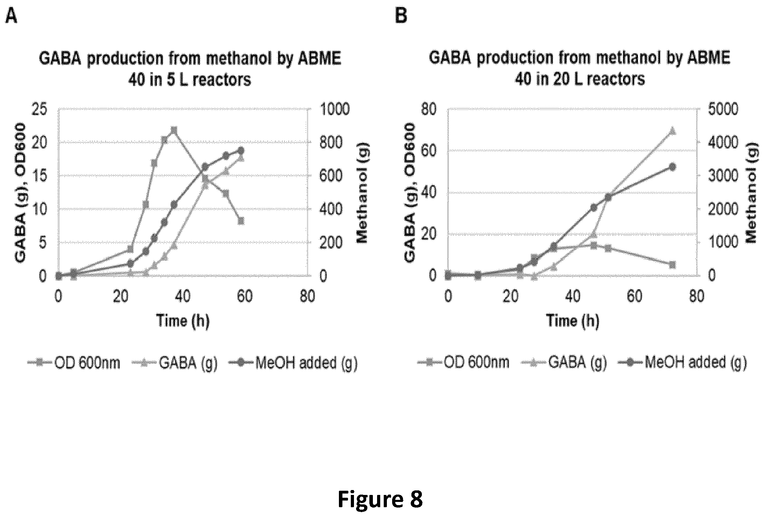

The strain ABME 40 produced GABA directly from methanol at pH 7.0 in absence of a pH-drop in both 5 L and 20 L bioreactors (Figure 8A and B, respectively). The strain produced 17.8 g of GABA in 5 L bioreactors, and 69.8 g GABA in 20 L reactors. These amounts were significantly higher compared to the amounts reached in shake flasks (Example 6). They were also surprising since the parent strain (ABME 6) is not known as a natural producer of glutamate, which is a precursor for GABA formation. This experiment further demonstrated the feasibility of pH-independent direct GABA synthesis from methanol without a pH drop using the stains disclosed in this invention.

**Table 7: Composition of the PM3 medium**

| **Compound** | **Per 1L** |
|---|---|
| KH2PO4 | 1.95 g |
| NA2HPO4 | 3.15 g |
| MgSO4 * 7H2O | 0.1 g |
| (NH4)2SO4 | 2g |
| d-Biotin | 0.1 mg |
| Thiamine*HCl | 0.1 mg |
| Riboflavin | 0.1 mg |
| Pyridoxine*HCl | 0.1 mg |
| Pantothenate | 0.1 mg |
| Nicotinamide | 0.1 mg |
| p-Aminobenzoic acid | 0.02 mg |
| Folic acid | 0.01 mg |
| Vitamin B12 | 0.01 mg |
| Lipoic acid | 0.01 mg |
| CaCl2 * 2H2O | 33 mg |
| FeSO4 * 7H2O | 13 mg |
| MnSO4 * H2O | 1 mg |
| ZnSO4 * 7H2O | 1.3 mg |
| CuSO4 * 5H2O | 0.4 mg |
| Na2MoO4 * 2H2O | 0.4 mg |
| CoCl2 * 6H2O | 0.4 mg |
| H3BO3 | 0.3 mg |
| pH | 7.0 |

### Example 8: Direct GABA production from methanol with Methylobacillus in shake flasks by genomic GAD expression

The best performing GAD gene, Eco GAD E89Q Δ14C (SEQ ID NO: 91) was integrated in the strain ABME 126, as described in Example. The genomic integration of GAD is beneficial since plasmid-based gene expression is not commercially viable in the long term. In vivo GABA production by ABME 126 was assessed in shake flasks, as described in Example . The cultures were sampled after 24 h of growth. GABA production was measured by HPLC, as described in Example 3.

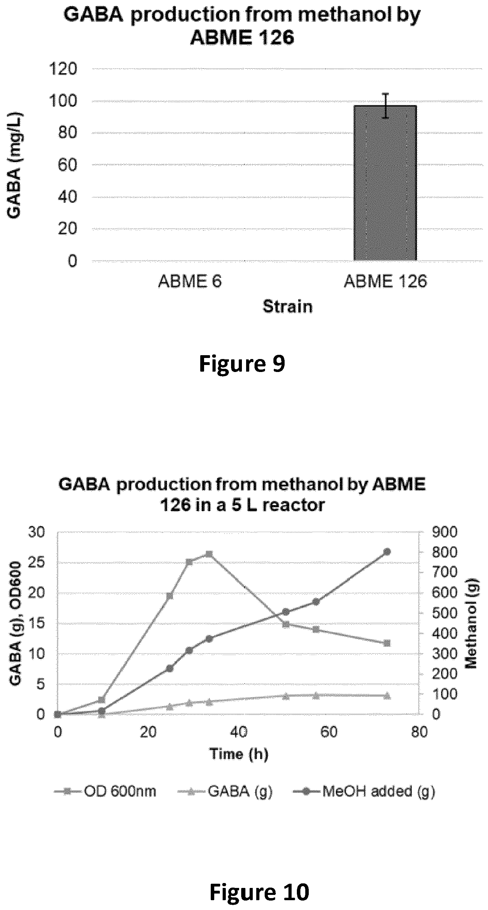

The strain ABME 126 produced almost 97 ± 7 mg/L GABA, while the negative control strain ABME 6 did not produce any GABA (Figure 9). This experiment demonstrated that GABA formation from methanol is also possible when the Eco gad E89Q Δ14C gene (SEQ ID NO: 91) was expressed as a single copy from the genome. It was unexpected and surprising that the native epsD-G promoter would be sufficiently strong to result in GABA production without optimization.

### Example 9: Direct GABA production from methanol with Methylobacillus in reactors by genomic GAD expression

The strain ABME 126, which was prepared as described in Example 1 was tested in 5 L bioreactors to prove that GABA production was independent from pH and extend the GABA production phase. The process was carbon limited with gradual methanol feeding to extend the GABA production phase.

The bioreactors contained PM3 medium and were inoculated with an 8% (5L reactors) inoculum of ABME 126 with an OD600 = 1.0. pH was maintained at 7.0 by automatic addition of NH4OH. The reactor temperature was maintained at 37 °C throughout the process. The reactor was kept carbon-limited by controlling the automatic addition of 50% methanol at a rate that maintained pO2-value at approximately 30%. Methanol concentration in the liquid were measured online and were zero throughout the process. The reactor was sampled regularly. GABA and methanol were analyzed by HPLC. Growth was monitored by measuring OD600.

The strain ABME 126 formed 3.2 g GABA in pH-controlled bioreactors (Figure 10), which was significantly higher compared to the amount produced in shake flasks (Example 8). This experiment provides further evidence that genomic expression of the Eco_gad E89Q Δ14C (SEQ ID NO: 91) gene enables pH-independent GABA production from methanol.

### III. pH-independent GABA production via an alternative pathway

### Example 10: Identification of a potential novel pathway for GABA production from methanol

In this example, the identification of a theoretical novel GABA-producing pathway (GAD-bypass) is disclosed. The benefit of this alternative pathway is that does not use the GAD enzyme, which is pH-dependent in its native from.

The pathway was identified by reversing the order of reactions that convert α-ketoglutarate to GABA. In the conventional GABA-forming pathway described in the Examples above, α-ketoglutarate is first converted to glutamate via a reductive amination reaction, catalyzed by glutamate dehydrogenase (GDH). Next, glutamate is decarboxylated to GABA by removing the α-carbon-adjacent carbonyl group. The general reaction sequence in the conventional GABA pathway is thereby amination, followed by decarboxylation (Example 10A).

We investigated whether the reaction sequence could be reversed, i.e. decarboxylation that is followed by amination. For this pathway to function, α-ketoglutarate would first have to be decarboxylated by removing the keto-adjacent carbonyl group. This would yield succinate semialdehyde, which would then have to be aminated at the aldehyde group to form GABA. After establishing the necessary chemical conversion steps needed to convert α-ketoglutarate to GABA by decarboxylation followed by amination, we examined whether enzymes that catalyze these reactions exist. Surprisingly, we were able to identify enzymes that perform both required reactions, and are theoretically able to form GABA form α-ketoglutarate independently from the GAD enzyme.

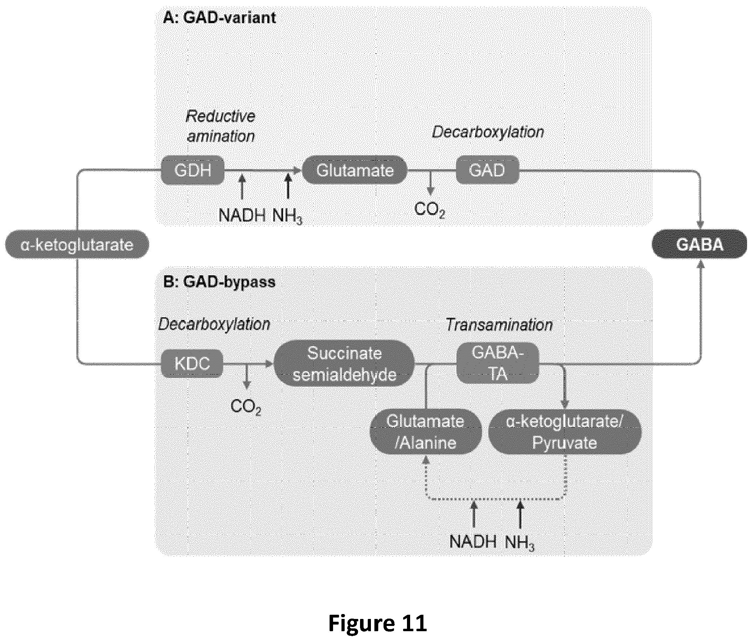

The decarboxylation of α-ketoglutarate to succinate semialdehyde can be performed α-ketoglutarate decarboxylase (KDC), an enzyme used in some rare variants of the TCA cycle by *Euglena gracilis* (SEQ ID NO: 39) and *Synechococcus sp.* (SEQ ID NO: 40) The next step of succinate semialdehyde amination could then theoretically be catalyzed by GABA transaminase (GABA-TA,). Unexpectedly, this enzyme is usually involved in GABA degradation. However, the ΔGr0 of the reaction is near 0, indicating that the reaction may be reversible, and could catalyze the formation of GABA as well. The GABA-TA enzyme is present in many organisms, including *E. coli* (SEQ ID NO: 43). The transamination reaction transfers the amino group from glutamate or alanine to succinate semialdehyde and forms GABA and α-ketoglutarate or pyruvate in the process (Figure 11B).

The identification of the novel GAD-bypass provides a previously unknown and unexpected pathway to form GABA from methanol in a pH-independent manner.

### Example 11: Direct GABA production from methanol with Methylobacillus in shake flasks by expression of the GAD-bypass from a plasmid

To test whether the GAD-bypass is able to form GABA from methanol in vivo, two KDC enzymes from *Euglena gracilis* (Egr, SEQ ID NO: 86) and *Synechococcus sp.* (Scc, SEQ ID NO: 87) PCC7002 were expressed in combination with the *E. coli* (Eco) GABA-TA (gabT gene, SEQ ID NO: 88). SEQ ID NO: 86 and SEQ ID NO: 87 were codon harmonized for expression in ABME 6. The genes were expressed in strains ABME 99, ABME 101, ABME 90, and ABME 104, respectively, as described in Example 1.

In vivo GABA production by the strains was assessed as described in Example 6. The cultures were sampled after 24 h of growth. GABA production was measured by HPLC, as described in Example 4.

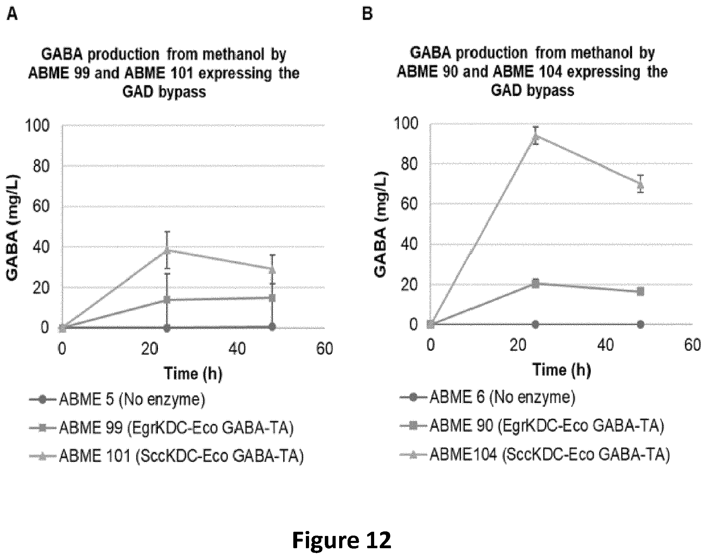

The strains ABME 99, ABME 101, ABME 90, and ABME 104 were all able to produce GABA from methanol in shake flasks (Figure 12). This confirmed that the novel GAD-bypass pathway is indeed able to form GABA from methanol independently of the GAD enzyme. The pathway variant utilizing the SccKDC (SEQ ID NO: 40) and Eco GABA-TA (SEQ ID NO: 43) enzymes was more efficient compared to the EgrKDC (SEQ ID NO: 39) variant. The ABME 6-derived strain ABME 104 formed the most GABA via the GAD bypass in this example (94 ± 4 mg/L, Figure 12B).

GABA production by the GAD-bypass was theoretically possible, but still surprising since neither the KDC nor GABA-TA enzymes are native to *Methylobacillus glycogenes* or *Methylobacillus flagellatus.* It was surprising that they would be able to produce GABA immediately without any optimization of enzyme activity. It was particularly surprising that the GABA-TA enzyme, which normally catalyzes GABA degradation, was able to form GABA as part of the GAD bypass.

This experiment demonstrates for the first time a novel pathway for GABA formation from methanol in a GAD- and pH-independent manner.

### Example 12: Direct GABA production from methanol with Methylobacillus in reactors by expression of the GAD-bypass from a plasmid

The strain ABME 104, which was prepared as described in Example 1 was tested in 5 L bioreactors to prove that GABA production was independent from pH and extend the GABA production phase. The process was oxygen limited with gradual methanol feeding. The process was performed as described in Example 7 using the strain ABME 104.

In pH-controlled bioreactors, the strain ABME 104 expressing the GAD bypass was able to form 0.8 g GABA directly from methanol. The GABA formation occurred at pH 7.0, confirming that the GAD-bypass is able to produce GABA in a pH independent manner, and represents a novel pathway for GABA production from methanol.

### List of references cited in the description

Irla, M., Nærdal, I., Brautaset, T., Wendisch, V.F., 2017. Methanol-based γ-aminobutyric acid (GABA) production by genetically engineered Bacillus methanolicus strains. Ind. Crops Prod. 106, 12-20. https://doi.org/https://doi.org/10.1016/j.indcrop.2016.11.050
Pennacchietti E, Lammens TM, Capitani G, Franssen MC, John RA, Bossa F, De Biase D., 2009. Mutation of His465 alters the pH-dependent spectroscopic properties of Escherichia coli glutamate decarboxylase and broadens the range of its activity toward more alkaline pH. J Biol Chem. 284(46):31587-96. doi: 10.1074/jbc.M109.049577.
Thu Ho, N.A., Hou, C.Y., Kim, W.H., Kang, T.J., 2013. Expanding the active pH range of Escherichia coli glutamate decarboxylase by breaking the cooperativeness. J. Biosci. Bioeng. https://doi.org/10.1016/j.jbiosc.2012.09.002
Yu, K., Lin, L., Hu, S., Huang, J., Mei, L., 2012. C-terminal truncation of glutamate decarboxylase from Lactobacillus brevis CGMCC 1306 extends its activity toward near-neutral pH. Enzyme Microb. Technol. 50, 263-269. https://doi.org/10.1016/j.enzmictec.2012.01.010
Zhang, J., Kao, E., Wang, G., Baidoo, E.E.K., Chen, M., Keasling, J.D., 2016. Metabolic engineering of Escherichia coli for the biosynthesis of 2-pyrrolidone. Metab. Eng. Commun. https://doi.org/10.1016/j.meteno.2015.11.001

## Claims

1. A mesophilic, methylotrophic bacterium which expresses a polypeptide which has glutamate decarboxylase (GAD) activity and is catalytically active at a pH above 5.5.

2. The bacterium according to claim 1, wherein the polypeptide is catalytically active at a pH in the range of pH 6 to pH 8.

3. The bacterium according to claim 1 or 2, where the polypeptide is a GAD mutant comprising in its amino acid sequence at least one amino acid substitution compared to the wild type GAD enzyme from which it is derived rending the mutant active at a pH above 5.5, preferably active in the range of pH 6 to pH 8.

4. The bacterium according to claim 3, wherein the amino acid substitution is in a position corresponding to position 89 and/or position 465 of SEQ ID NO: 1.

5. The bacterium according to any one of claims 1 to 4, wherein the polypeptide is a GAD mutant having 2 to 20 amino acids, such as 2 to 14 amino acids, deleted from the C-terminus compared to the wild type GAD enzyme from which it is derived.

6. The bacterium according to claim 1 or 2, wherein the polypeptide is selected from any one of the following:
a) a polypeptide having glutamate decarboxylase (GAD) activity which comprises an amino acid sequence having at least 70%, such as at least 80%, sequence identity with SEQ ID NO: 1 and comprising an amino acid substitution at a position corresponding to position 89 and/or position 465 in SEQ ID NO: 1;
b) a polypeptide having glutamate decarboxylase (GAD) activity which comprises an amino acid sequence having at least 70%, such as at least 80%, sequence identity with SEQ ID NO: 2 and comprising an amino acid substitution at a position corresponding to position 84 and/or position 457 in SEQ ID NO: 2;
c) a polypeptide having glutamate decarboxylase (GAD) activity which comprises an amino acid sequence having at least 70%, such as at least 80%, sequence identity with SEQ ID NO: 3 and comprising an amino acid substitution at a position corresponding to position 93 and/or position 467 in SEQ ID NO: 3;
d) a polypeptide having glutamate decarboxylase (GAD) activity which comprises an amino acid sequence having at least 70%, such as at least 80%, sequence identity with SEQ ID NO: 4 and comprising an amino acid substitution at a position corresponding to position 90 and/or position 467 in SEQ ID NO: 4;
e) a polypeptide having glutamate decarboxylase (GAD) activity which comprises an amino acid sequence having at least 70%, such as at least 80%, sequence identity with SEQ ID NO: 5 and comprising an amino acid substitution at a position corresponding to position 85 and/or position 462 in SEQ ID NO: 5;
f) a polypeptide having glutamate decarboxylase (GAD) activity which comprises an amino acid sequence having at least 70%, such as at least 80%, sequence identity with SEQ ID NO: 6 and comprising an amino acid substitution at a position corresponding to position 89 and/or position 465 in SEQ ID NO: 6;
g) a polypeptide having glutamate decarboxylase (GAD) activity which comprises an amino acid sequence having at least 70%, such as at least 80%, sequence identity with SEQ ID NO: 7 and comprising an amino acid substitution at a position corresponding to position 89 and/or position 466 in SEQ ID NO: 7;
h) a polypeptide having glutamate decarboxylase (GAD) activity which comprises an amino acid sequence having at least 70%, such as at least 80%, sequence identity with SEQ ID NO: 8 and comprising an amino acid substitution at a position corresponding to position 89 and/or position 466 in SEQ ID NO: 8;
i) a polypeptide having glutamate decarboxylase (GAD) activity which comprises an amino acid sequence having at least 70%, such as at least 80%, sequence identity with SEQ ID NO: 9 and comprising an amino acid substitution at a position corresponding to position 89 and/or position 463 in SEQ ID NO: 9;
j) a polypeptide having glutamate decarboxylase (GAD) activity which comprises an amino acid sequence having at least 70%, such as at least 80%, sequence identity with SEQ ID NO: 10 and comprising an amino acid substitution at a position corresponding to position 17, position 91, position 94, position 213, position 346 and/or position 468 in SEQ ID NO: 10; and
k) a polypeptide having glutamate decarboxylase (GAD) activity which comprises an amino acid sequence having at least 70%, such as at least 80%, sequence identity with SEQ ID NO: 1, 2, 3, 4, 5, 6, 7, 8, 9 or 10 wherein 2 to 20 amino acids, such as 2 to 14 amino acids, are deleted from the C-terminus.

7. The bacterium according to claim 1 or 2, wherein the polypeptide is selected from any one of the following:
aa) a polypeptide having glutamate decarboxylase (GAD) activity which comprises the amino acid sequence of SEQ ID NO: 11, 12 or 13 or an amino acid sequence corresponding to SEQ ID NO: 11, 12 or 13, wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A;
bb) a polypeptide having glutamate decarboxylase (GAD) activity which comprises the amino acid sequence of SEQ ID NO: 14, 15 or 16 or an amino acid sequence corresponding to SEQ ID NO: 14, 15 or 16 wherein 1 to 40, such as 1 to 20, amino acids are substituted with the proviso that the amino acid at position 84 is not E and/or the amino acid at position 457 is not H, preferably with the proviso that the amino acid at position 84 is Q and/or the amino acid at position 457 is A;
cc) a polypeptide having glutamate decarboxylase (GAD) activity which comprises the amino acid sequence of SEQ ID NO: 17, 18 or 19 or an amino acid sequence corresponding to SEQ ID NO: 17, 18 or 19 wherein 1 to 40, such as 1 to 20, amino acids are substituted with the proviso that the amino acid at position 93 is not E and/or the amino acid at position 467 is not H, preferably with the proviso that the amino acid at position 93 is Q and/or the amino acid at position 467 is A;
dd) a polypeptide having glutamate decarboxylase (GAD) activity which comprises the amino acid sequence of SEQ ID NO: 20, 21 or 22 or an amino acid sequence corresponding to SEQ ID NO: 20, 21 or 22 wherein 1 to 40, such as 1 to 20, amino acids are substituted with the proviso that the amino acid at position 90 is not E and/or the amino acid at position 467 is not H, preferably with the proviso that the amino acid at position 90 is Q and/or the amino acid at position 467 is A;
ee) a polypeptide having glutamate decarboxylase (GAD) activity which comprises the amino acid sequence of SEQ ID NO: 23, 24 or 25 or an amino acid sequence corresponding to SEQ ID NO: 23, 24 or 25 wherein 1 to 40, such as 1 to 20, amino acids are substituted with the proviso that the amino acid at position 85 is not E and/or the amino acid at position 462 is not H, preferably with the proviso that the amino acid at position 85 is Q and/or the amino acid at position 462 is A;
ff) a polypeptide having glutamate decarboxylase (GAD) activity which comprises the amino acid sequence of SEQ ID NO: 26, 27 or 28 or an amino acid sequence corresponding to SEQ ID NO: 26, 27 or 28 wherein 1 to 40, such as 1 to 20, amino acids are substituted with the proviso that the amino acid at position 89 is not E and/or the amino acid at position 465 is not H, preferably with the proviso that the amino acid at position 89 is Q and/or the amino acid at position 465 is A;
gg) a polypeptide having glutamate decarboxylase (GAD) activity which comprises the amino acid sequence of SEQ ID NO: 29, 30 or 31 or an amino acid sequence corresponding to SEQ ID NO: 29, 30 or 31 wherein 1 to 40, such as 1 to 20, amino acids are substituted with the proviso that the amino acid at position 89 is not E and/or the amino acid at position 466 is not H, preferably with the proviso that the amino acid at position 89 is Q and/or the amino acid at position 466 is A;
hh) a polypeptide having glutamate decarboxylase (GAD) activity which comprises the amino acid sequence of SEQ ID NO: 32, 33 or 34 or an amino acid sequence corresponding to SEQ ID NO: 32, 33 or 34 wherein 1 to 40, such as 1 to 20, amino acids are substituted with the proviso that the amino acid at position 89 is not E and/or the amino acid at position 466 is not H, preferably with the proviso that the amino acid at position 89 is Q and/or the amino acid at position 466 is A;
ii) a polypeptide having glutamate decarboxylase (GAD) activity which comprises the amino acid sequence of SEQ ID NO: 35, 36 or 37 or an amino acid sequence corresponding to SEQ ID NO: 35, 36 or 37 wherein 1 to 40, such as 1 to 20, amino acids are substituted with the proviso that the amino acid at position 89 is not E and/or the amino acid at position 463 is not H, preferably with the proviso that the amino acid at position 89 is Q and/or the amino acid at position 463 is A;
jj) a polypeptide having glutamate decarboxylase (GAD) activity which comprises the amino acid sequence of SEQ ID NO: 38 or an amino acid sequence corresponding to SEQ ID NO: 38 wherein 1 to 40, such as 1 to 20, amino acids are substituted with the proviso that the amino acid at position 17 is not T, the amino acid at position 94 is not D, the amino acid at position 213 is not E and /or the amino acid at position 346 is not Q, preferably with the proviso that the amino acid at position 17 is I, the amino acid at position 94 is G, the amino acid at position 213 is S and /or the amino acid at position 346 is H; and
kk) a polypeptide having glutamate decarboxylase (GAD) activity which comprises an amino acid sequence having at least 70%, such as at least 80%, sequence identity with any one of SEQ ID NOs: 11 to 38, wherein 2 to 20 amino acids, such as 2 to 14 amino acids, are deleted from the C-terminus.

8. A mesophilic, methylotrophic bacterium which expresses a) a polypeptide having α-ketoglutarate decarboxylase (KDC) activity and b) a polypeptide having GABA transaminase (GABA-TA) activity.

9. The bacterium according to claim 8, wherein the polypeptide having KDC activity comprises the amino acid sequence of SEQ ID NO: 39, 40, 41 or 42 or an amino acid sequence having at least 70%, such as at least 80%, sequence identity with SEQ ID NO: 39, 40, 41 or 42, and/or wherein the polypeptide having GABA-TA activity comprises the amino acid sequence of any one of SEQ ID NOs: 43 to 74 or an amino acid sequence having at least 70%, such as at least 80%, sequence identity with any one of SEQ ID NO: 43 to 74.

10. The bacterium according to any one of claims 1 to 9, wherein said bacterium belongs to the genus *Methylobacillus* or *Methylobacterium,* preferably being selected from *Methylobacillus flagellatus, Methylobacillus glycogenes, Methylobacillus pratensis, Methylobacillus rhizosphaerae, Methylobacterium extorquens* and *Methylobacterium organophilum.*

11. The bacterium according to any one of claims 1 to 10, wherein said bacterium is *Methylobacillus flagellatus.*

12. The bacterium according to any one of claims 1 to 11, wherein the bacterium further expresses a polypeptide having pyrrolidone synthase (PYS) activity.

13. The bacterium according to claim 12, wherein the polypeptide having pyrrolidone synthase (PYS) activity comprises the amino acid sequence of SEQ ID NO: 75 or an amino acid sequence having at least 70%, such as at least 80%, sequence identity with SEQ ID NO: 75.

14. Method for the production of a biochemical comprising cultivating a bacterium according to any one of claims 1 to 13 under suitable culture conditions in a culture medium comprising a reduced one-carbon compound, such as methanol, or a multi-carbon compound that contains no carbon-carbon bonds, such as dimethyl ether and dimethylamine.

15. The method according to claim 14, wherein the method is for producing GABA or a derivative thereof.
